# EUROPEAN PATENT APPLICATION

(11) **EP 4 736 920 A2**
(43) Date of publication of application: **06.05.2026**
(21) Application number: 25224565.9
(22) Date of filing: 09.03.2022
(51) Int. Cl.: A61M 16/08

(54) **FULL-FACE PATIENT INTERFACE**

(30) Priority: 09.03.2021 SG 10202102386Q; 14.05.2021 SG 10202105065S
(62) Divisional of application: 22767607.9
(71) Applicant: ResMed Pty Ltd, Bella Vista, New South Wales 2153 (AU)
(72) Inventor: HARTONO, Marvin Sugi, Singapore 639443 (SG); MAUNG, Kyi Thu, Singapore 639443 (SG)
(74) Representative: Vossius & Partner Patentanwälte Rechtsanwälte mbB

(57) **Abstract**

The present invention relates to a patient interface comprising: a plenum chamber being formed entirely from a flexible material, the plenum chamber including: a cavity, an inlet port, and a groove formed on a surface of the plenum chamber outside the cavity and exposed to ambient pressure, the groove formed radially outside of the inlet port, and the groove forming an enclosed perimeter; a seal-forming structure; a positioning and stabilizing structure comprising: a frame coupled to the plenum chamber, the frame including, a central portion including an inner perimeter and an outer perimeter removably positionable within the groove, the inner perimeter configured to be spaced apart from the inlet port while positioned within the groove, a pair of upper arms, and a pair of lower arms; and headgear straps coupled to the pair of upper arms and the pair of lower arms of the frame.

## Description

### 1 CROSS-REFERENCE TO RELATED APPLICATION

This application claims the benefit of S.G. Provisional Patent Application No. 10202105065S, filed May 14, 2021, and S.G. Provisional Patent Application No. 10202102386Q, filed March 9, 2021, each of which is incorporated herein by reference in its entirety.

International Application No. PCT/AU2020/050953, filed September 9, 2020, which claims priority to U.S. Provisional Patent Application No. 63/058,001, filed July 29, 2020, are each incorporated herein by reference in their entirety.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

A range of respiratory disorders exist. Certain disorders may be characterised by particular events, e.g. apneas, hypopneas, and hyperpneas.

Examples of respiratory disorders include Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD) and Chest wall disorders.

Obstructive Sleep Apnea (OSA), a form of Sleep Disordered Breathing (SDB), is characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. See US Patent No. 6,532,959 (Berthon-Jones).

Respiratory failure is an umbrella term for respiratory disorders in which the lungs are unable to inspire sufficient oxygen or exhale sufficient CO₂ to meet the patient's needs. Respiratory failure may encompass some or all of the following disorders.

A patient with respiratory insufficiency (a form of respiratory failure) may experience abnormal shortness of breath on exercise.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 2.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that is held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO₂ from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched gas at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 2.2.2.3 Supplementary oxygen

For certain patients, oxygen therapy may be combined with a respiratory pressure therapy or HFT by adding supplementary oxygen to the pressurised flow of air. When oxygen is added to respiratory pressure therapy, this is referred to as RPT with supplementary oxygen. When oxygen is added to HFT, the resulting therapy is referred to as HFT with supplementary oxygen.

### 2.2.3 Respiratory therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFT^{™} nasal pillows mask, SWIFT^{™} II nasal pillows mask, SWIFT^{™} LT nasal pillows mask, SWIFT^{™} FX nasal pillows mask and MIRAGE LIBERTY^{™} full-face mask. The following patent applications, assigned to ResMed Limited, describe examples of nasal pillows masks: International Patent Application WO2004/073,778 (describing amongst other things aspects of the ResMed Limited SWIFT^{™} nasal pillows), US Patent Application 2009/0044808 (describing amongst other things aspects of the ResMed Limited SWIFT^{™} LT nasal pillows); International Patent Applications WO 2005/063,328 and WO 2006/130,903 (describing amongst other things aspects of the ResMed Limited MIRAGE LIBERTY^{™} full-face mask); International Patent Application WO 2009/052,560 (describing amongst other things aspects of the ResMed Limited SWIFT^{™} FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. Humidifiers therefore often have the capacity to heat the flow of air was well as humidifying it.

### 2.2.3.5 Oxygen source

Experts in this field have recognized that exercise for respiratory failure patients provides long term benefits that slow the progression of the disease, improve quality of life and extend patient longevity. Most stationary forms of exercise like tread mills and stationary bicycles, however, are too strenuous for these patients. As a result, the need for mobility has long been recognized. Until recently, this mobility has been facilitated by the use of small compressed oxygen tanks or cylinders mounted on a cart with dolly wheels. The disadvantage of these tanks is that they contain a finite amount of oxygen and are heavy, weighing about 50 pounds when mounted.

Oxygen concentrators have been in use for about 50 years to supply oxygen for respiratory therapy. Traditional oxygen concentrators have been bulky and heavy making ordinary ambulatory activities with them difficult and impractical. Recently, companies that manufacture large stationary oxygen concentrators began developing portable oxygen concentrators (POCs). The advantage of POCs is that they can produce a theoretically endless supply of oxygen. In order to make these devices small for mobility, the various systems necessary for the production of oxygen enriched gas are condensed. POCs seek to utilize their produced oxygen as efficiently as possible, in order to minimise weight, size, and power consumption. This may be achieved by delivering the oxygen as series of pulses or "boli", each bolus timed to coincide with the start of inspiration. This therapy mode is known as pulsed or demand (oxygen) delivery (POD), in contrast with traditional continuous flow delivery more suited to stationary oxygen concentrators.

### 2.2.3.6 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 2.2.3.7 Mandibular repositioning

A mandibular repositioning device (MRD) or mandibular advancement device (MAD) is one of the treatment options for sleep apnea and snoring. It is an adjustable oral appliance available from a dentist or other supplier that holds the lower jaw (mandible) in a forward position during sleep. The MRD is a removable device that a patient inserts into their mouth prior to going to sleep and removes following sleep. Thus, the MRD is not designed to be worn all of the time. The MRD may be custom made or produced in a standard form and includes a bite impression portion designed to allow fitting to a patient's teeth. This mechanical protrusion of the lower jaw expands the space behind the tongue, puts tension on the pharyngeal walls to reduce collapse of the airway and diminishes palate vibration.

In certain examples a mandibular advancement device may comprise an upper splint that is intended to engage with or fit over teeth on the upper jaw or maxilla and a lower splint that is intended to engage with or fit over teeth on the upper jaw or mandible. The upper and lower splints are connected together laterally via a pair of connecting rods. The pair of connecting rods are fixed symmetrically on the upper splint and on the lower splint.

In such a design the length of the connecting rods is selected such that when the MRD is placed in a patient's mouth the mandible is held in an advanced position. The length of the connecting rods may be adjusted to change the level of protrusion of the mandible. A dentist may determine a level of protrusion for the mandible that will determine the length of the connecting rods.

Some MRDs are structured to push the mandible forward relative to the maxilla while other MADs, such as the ResMed Narval CC^{™} MRD are designed to retain the mandible in a forward position. This device also reduces or minimises dental and temporo-mandibular joint (TMJ) side effects. Thus, it is configured to minimises or prevent any movement of one or more of the teeth.

### 2.2.3.8 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

**Table of noise of prior masks (ISO 17510-2:2007, 10 cmH₂O pressure at 1m)**

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage^{™} (*) | nasal | 29.5 | 21.5 | 1998 |
| ResMed UltraMirage^{TM} | nasal | 36 (3) | 28 (3) | 2000 |
| ResMed Mirage Activa^{™} | nasal | 32 (3) | 24 (3) | 2002 |
| ResMed Mirage Micro^{™} | nasal | 30 (3) | 22 (3) | 2008 |
| ResMed Mirage^{™} SoftGel | nasal | 29 (3) | 22 (3) | 2008 |
| ResMed Mirage^{™} FX | nasal | 26 (3) | 18 (3) | 2010 |
| ResMed Mirage Swift^{™} (*) | nasal pillows | 37 | 29 | 2004 |
| ResMed Mirage Swift^{™} II | nasal pillows | 28 (3) | 20 (3) | 2005 |
| ResMed Mirage Swift^{™} LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| [0068] (* one specimen only, measured using test method specified in ISO 3744 in CPAP mode at 10 cmH₂O) | | | | |

Sound pressure values of a variety of objects are listed below

| Object | A-weighted sound pressure dB(A) | Notes |
|---|---|---|
| Vacuum cleaner: Nilfisk | 68 | ISO 3744 at 1m distance |
| Walter Broadly Litter Hog: B+ Grade | | |
| Conversational speech | 60 | 1m distance |
| Average home | 50 | |
| Quiet library | 40 | |
| Quiet bedroom at night | 30 | |
| Background in TV studio | 20 | |

### 2.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

### 3 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One form of the present technology comprises a patient interface comprising:
a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient;
a first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use; and
a vent structure to allow a continuous flow of gases exhaled by the patient from an interior of the plenum chamber to vent to ambient, said vent structure being sized and shaped to maintain the therapeutic pressure in the plenum chamber in use;
the patient interface further comprising:
   a pair of support portions provided on opposite sides of the interface between the second seal forming structure and an anterior wall of the plenum chamber, wherein the support portions are configured to oppose compression in the anterior-posterior direction.

In embodiments:
a) the support portions are connected to portions of the second seal forming structure which seal, in use, against the patient's lip superior;
b) the support portions are connected to portions of the second seal forming structure which, in use, seal to the patient's lip superior, directly inferior to the lower corners of the patient's nose;
c) the support portions are curved when viewed in cross-section parallel to a sagittal plane;
d) the support portions are curved when viewed in cross-section parallel to a frontal plane;
e) the plenum chamber comprises an oral portion and a nasal portion;
f) each support portion is connected to the oral portion of the plenum chamber adjacent a boundary of a lateral side wall portion of the oral portion and a lateral side wall portion of the nasal portion;
g) each support portion is connected to the oral portion of the shell adjacent a boundary of an anterior wall portion of the oral portion and an anterior wall portion of the nasal portion;
h) the lateral side wall portions of the plenum chamber curve inwardly adjacent the boundary with the nasal portion, wherein each support portion is substantially contiguous with an adjacent lateral side wall portion;
i) the second seal-forming structure comprises at least one nasal aperture configured to deliver a flow of air at said therapeutic pressure to an entrance to the patient's nares, wherein, in use no part of either support portion is directly inferior to the or each nasal aperture;
j) the interface further comprises a positioning and stabilising structure configured to generate a force to hold the seal-forming structure in a therapeutically effective position on the patient's head;
k) the plenum chamber is at least partially formed by a shell and the vent structure is provided to the shell; and/or
l) the support portions are connected to the second seal forming structure and are connected to the anterior wall of the plenum chamber.

Another form of the technology comprises a patient interface comprising:
a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient;
a first seal-forming structure connected to an oral portion of the plenum chamber, the first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure connected to a nasal portion of the plenum chamber, the second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use; and
a vent structure to allow a continuous flow of gases exhaled by the patient from an interior of the plenum chamber to vent to ambient, said vent structure being sized and shaped to maintain the therapeutic pressure in the plenum chamber in use;
wherein
a first anterior wall portion of the nasal portion of the plenum chamber, adjacent a boundary with the oral portion of the plenum chamber, is more flexible than an immediately adjacent region of the oral portion of the plenum chamber, and a second anterior wall portion of the nasal portion of the plenum chamber, which is immediately adjacent the first anterior wall portion and is on an opposite side of the first anterior wall portion to the boundary with the oral portion of the plenum chamber, is less flexible than the immediately adjacent portions of the anterior wall.

In examples:
a) the first anterior wall portion is thinner than the immediately adjacent portions of the plenum chamber wall;
b) the second anterior wall portion is thicker than the immediately adjacent portions of the plenum chamber wall;
c) the first and second anterior wall portions are made from the same material;
d) the first anterior wall portion extends across substantially an entire width of the nasal portion of the plenum chamber;
e) the second anterior wall portion extends across at least a majority of a width of the nasal portion of the plenum chamber;
f) the first anterior wall portion extends in a superior direction around at least one lateral edge of the second anterior wall portion;
g) the second anterior wall portion extends across substantially an entire width of the nasal portion of the plenum chamber;
h) a central portion of the first anterior wall portion extends further in the superior direction than lateral portions of the first anterior wall portion;
i) an upper boundary of the first anterior wall portion is curved;
j) a lower boundary of the first anterior wall portion is curved;
k) the plenum chamber is at least partially formed by a shell and the vent structure is provided to the shell;
l) the second anterior wall portion includes a band that extends past the first anterior wall portion, and is configured to extend toward the patient, through the plenum chamber;
m) a transition within the plenum chamber between the first anterior wall portion and the second anterior wall portion is a substantially stepped surface;
n) a transition outside of the plenum chamber between the first anterior wall portion and the second anterior wall portion is a substantially smooth surface; and/or
o) the first anterior wall portion extends further superior than at least a portion of the band, in use.

Another form of the technology comprises a patient interface comprising:
a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient;
a first seal-forming structure connected to an oral portion of the plenum chamber, the first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure connected to a nasal portion of the plenum chamber, the second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use; and
a vent structure to allow a continuous flow of gases exhaled by the patient from an interior of the plenum chamber to vent to ambient, said vent structure being sized and shaped to maintain the therapeutic pressure in the plenum chamber in use;
wherein
posterior surfaces of the lateral portions of the second seal forming structure slope in a superior-anterior direction from a boundary of the first and second seal forming structures.

In examples:
a) the slope of each lateral portion forms an angle of between 20 degrees and 90 degrees with a mid-contact plane of the mask;
b) no part of the patent interface contacts the patient's alar crest point, in use;
c) the interface is configured to prevent occlusion of the patient's nares, or to at least reduce occlusion relative to the interfaces of the prior art; and/or
d) the plenum chamber is at least partially formed by a shell and the vent structure is provided to the shell.

Another form of the technology comprises a patient interface comprising:
a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient;
a first seal-forming structure connected to an oral portion of the plenum chamber, the first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure connected to a nasal portion of the plenum chamber, the second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use; and
a vent structure to allow a continuous flow of gases exhaled by the patient from an interior of the plenum chamber to vent to ambient, said vent structure being sized and shaped to maintain the therapeutic pressure in the plenum chamber in use;
wherein
a boundary between the first seal-forming structure and the second seal-forming structure comprises a ridge.

In examples:
a) the ridge has a radius of curvature of less than 2mm;
b) the ridge extends across substantially an entire boundary between the first seal forming structure and the second seal forming structure;
c) in use, the ridge engages a patient's face proximate the entrances to the nares where the ala meets the face above the lip superior;
d) the ridge resists creases forming in the first and/or second seal forming structure adjacent the ridge; and/or
e) in use the plenum chamber is at least partially formed by a shell and the vent structure is provided to the shell.

Another form of the technology comprises a patient interface comprising:
a plenum chamber pressurisable to a therapeutic pressure of at least 6 cmH₂O above ambient air pressure, said plenum chamber including a plenum chamber inlet port sized and structured to receive a flow of air at the therapeutic pressure for breathing by a patient;
a first seal-forming structure connected to an oral portion of the plenum chamber, the first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure connected to a nasal portion of the plenum chamber, the second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use; and
a vent structure to allow a continuous flow of gases exhaled by the patient from an interior of the plenum chamber to vent to ambient, said vent structure being sized and shaped to maintain the therapeutic pressure in the plenum chamber in use;
wherein,
at least a portion of the oral portion of plenum chamber comprises a flexible shell, wherein the flexible shell is formed from a material having a Young's modulus of less than 0.4GPa.

In examples:
a) the flexible shell is formed from a material having a Young's modulus less than 0.1GPa, preferably between 0.3-0.7MPa.
b) at least one component is connected to the flexible shell, wherein the at least one component is stiffer than a portion of the flexible shell adjacent the component;
c) the at least one component comprises one or more of: a vent module; a headgear connector; a headgear connector connected to a rigidizing arm; a rigidizing member; a less flexible shell portion;
d) the at least one component is releasably connectable to the flexible shell;
e) the at least one component is permanently connected to the flexible shell;
f) the at least one component is overmoulded to the flexible shell;
g) the flexible shell comprises stiffening portions having greater thickness than immediately adjacent portions of the flexible shell;
h) the at least one component is configured as stiffening ribs or bands;
i) a central portion of the oral portion of the plenum chamber has a greater stiffness than the remainder of the plenum chamber; and/or
j) the plenum chamber is at least partially formed by a shell and the vent structure is provided to the shell.

Another form of the technology comprises a frame configured to couple to the plenum chamber, the frame comprising a central portion coupled to the plenum chamber outside of the cavity and a plurality of connectors that comprises a pair of upper connector and a pair of lower connectors, the pair of lower connectors being flush with the central portion and the pair of upper connectors extend away from the central portion in a posterior direction configured to be past the plenum chamber.

In examples:
a) each upper connector of the pair of upper connectors includes a slot configured to receive a headgear strap;
b) each slot includes a raised portion proximate to the respective arm;
c) each raised portion having a curved shape;
d) each upper connector including a rib proximate to the central portion;
e) a combined width of the rib and the arm proximate to the central portion is substantially equal to a width of the arm proximate to the slot;
f) each lower connector of the pair of lower connectors includes a magnet;
g) each lower connector of the pair of lower connectors includes a groove configured to act as a hinge and allow each lower connector to bend relative to the central portion.

Another form of the technology comprises a frame configured to couple to the plenum chamber, the frame comprising a central portion coupled to the plenum chamber outside of the cavity and a pair of arms that extend away from the central portion in a posterior direction configured to be past the plenum chamber, the pair of arms being more flexible than the central portion.

In examples:
a) each arm of the pair of arms are more flexible than the frame;
b) the central portion is thicker than the each arm of the pair of arms;
c) the central portion and each arm of the pair of arms are constructed from the same material;
d) each arm of the pair of arms includes a first connection point, the headgear straps are coupled to the first connection point of each arm;
e) the first connection points are loops, and the headgear straps are coupled to the loops so that the straps are perpendicular to an edge of each loop and apply a force vector perpendicular to the edge;
f) the first connection points are loops, and include a region of reduced thickness, a headgear strap may contact the region of reduced thickness;
g) a first magnet is overmolded onto the central portion of the frame, the headgear straps include a second magnet removably coupled to the first magnet;
h) the first magnet includes an outer casing at least partially enclosing a magnetic material, the outer casing includes a planar surface and a lip extending from the planar surface;
i) an overhang is spaced apart from the second magnet and configured to engage the lip when the second magnet is coupled to the magnetic material;
j) the central portion configured to be positioned within a groove of the plenum chamber;
k) the central portion is removably positionable within the groove;
l) the groove includes a projection, the central portion includes a complimentary slot configured to receive the projection;
m) the slot is tapered and includes a wider opening and a narrower opening, the projection configured to be received through the wider opening before the narrower opening;
n) a sleeve covering at least a portion of one arm of the pair of arms;
o) the slot having a raised portion with a curvature configured to conform to a curvature of the sleeve; and/or
p) each arm of the pair of arms including a rib proximate to the central portion, the rub configured to hold the sleeve.

Another form of the technology comprises a patient interface comprising:
a plenum chamber including a cavity that is pressurisable to a therapeutic pressure of at least 6 cmH2O above ambient air pressure, said plenum chamber including a plenum chamber inlet port sized and structured to receive a flow of air at the therapeutic pressure for breathing by a patient;
a first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use;
a vent structure to allow a continuous flow of gases exhaled by the patient from an interior of the plenum chamber to vent to ambient, said vent structure being sized and shaped to maintain the therapeutic pressure in the plenum chamber in use; and
a positioning and stabilizing structure configured to maintain the first seal-forming structure and the second seal-forming structure in a therapeutically effective position, the positioning and stabilizing structure comprising:
   a frame coupled to the plenum chamber, the frame including,
      a central portion coupled to the plenum chamber outside of the cavity, and
      a pair of arms that extend away from the central portion in a posterior direction past the second seal-forming structure, the pair of arms being more flexible than the central portion; and
   headgear straps coupled to the frame, and configured to provide a tensile force to the first seal-forming structure and to the second seal-forming structure into the patient's face via the frame.

In examples:
a) each arm of the pair of arms are more flexible than the frame;
b) the central portion is thicker than the each arm of the pair of arms;
c) the central portion and each arm of the pair of arms are constructed from the same material;
d) each arm of the pair of arms includes a first connection point, the headgear straps are coupled to the first connection point of each arm;
e) the first connection points are loops, and the headgear straps are coupled to the loops so that the straps are perpendicular to an edge of each loop and apply a force vector perpendicular to the edge;
f) the first connection points are loops, and include a region of reduced thickness, a headgear strap may contact the region of reduced thickness;
g) a first magnet is overmolded onto the central portion of the frame, the headgear straps include a second magnet removably coupled to the first magnet;
h) the first magnet includes an outer casing at least partially enclosing a magnetic material, the outer casing includes a planar surface and a lip extending from the planar surface;
i) an overhang is spaced apart from the second magnet and configured to engage the lip when the second magnet is coupled to the magnetic material;
j) the plenum chamber includes a groove, the central portion positioned within the groove;
k) the central portion is removably positionable within the groove;
l) the groove includes a projection, the central portion includes a complimentary slot configured to receive the projection;
m) the slot is tapered and includes a wider opening and a narrower opening, the projection configured to be received through the wider opening before the narrower opening;
n) the projection includes an overhang configured to extend over the slot and retain the frame relative to the plenum chamber;
o) the plenum chamber includes a protrusion disposed adjacent to the groove, the protrusion configured to retain the central portion within the groove;
p) an outer surface of the central portion is flush with an outer surface of the plenum chamber, the outer surface of the central portion and the outer surface of the plenum chamber configured to face away from the patient in use;
q) the central portion includes an annular shape, the plenum chamber inlet port disposed radially within the central portion while the frame is coupled to the plenum chamber;
r) the plenum chamber inlet port is configured to receive an elbow, the elbow configured to be spaced apart from the central portion while received within the plenum chamber inlet port;
s) each arm is formed as a cantilevered structure relative to the central portion;
t) the thickness of each arm decreases from a fixed end toward a free end;
u) the arm includes a scalloped region on an inner surface configured to face the patient's skin;
v) each arm is pivotable relative to the central portion about a pivot point; and/or
w) the pivot point is a living hinge.

Another form of the technology comprises a patient interface comprising:
a plenum chamber including a cavity that is pressurisable to a therapeutic pressure of at least 6 cmH2O above ambient air pressure, said plenum chamber including a plenum chamber inlet port sized and structured to receive a flow of air at the therapeutic pressure for breathing by a patient;
a seal-forming structure constructed and arranged to form a seal with a region of the patient's face such that the flow of air at said therapeutic pressure is delivered to the airways, the seal-forming structure constructed and arranged to maintain said therapeutic pressure in the plenum chamber throughout the patient's respiratory cycle in use; and
a positioning and stabilizing structure configured to maintain the seal-forming structure in a therapeutically effective position.

Another aspect of one form of the present technology is an oro-nasal patient interface that is more compact and less obtrusive to the patient.

Another aspect of one form of the present technology is an oro-nasal patient interface that has a nasal cushion portion that provides an improved fit to the lower corners of the nose.

Another aspect of one form of the present technology is an oro-nasal patient interface that reduces occlusive contact on the nose.

Another aspect of one form of the present technology is an oro-nasal patient interface that can self adjust to accommodate patients with a wide variety of nasiolabal angles.

Another aspect of one form of the present technology is an oro-nasal patient interface that has a relatively flexible shell.

Another aspect of one form of the present technology is a patient interface that is moulded or otherwise constructed with a perimeter shape which is complementary to that of an intended wearer.

Another form of the present technology comprises a patient interface comprising:
a plenum chamber including a cavity that is pressurisable to a therapeutic pressure of at least 6 cmH₂O above ambient air pressure, said plenum chamber including a plenum chamber inlet port sized and structured to receive a flow of air at the therapeutic pressure for breathing by a patient;
a first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use;
a vent structure to allow a continuous flow of gases exhaled by the patient from an interior of the plenum chamber to vent to ambient, said vent structure being sized and shaped to maintain the therapeutic pressure in the plenum chamber in use; and
a positioning and stabilizing structure configured to maintain the first seal-forming structure and the second seal-forming structure in a therapeutically effective position, the positioning and stabilizing structure comprising:
   headgear straps configured to provide a tensile force to the first seal-forming structure and to the second seal-forming structure into the patient's face via the frame, wherein the headgear straps include a first width and a second width greater than the first width; and
wherein the headgear straps are selectively threaded through an opening in order to adjust a tensile force, the first width configured to fit through the opening and the second width configured to be blocked from passing through the opening.

Another form of the present technology comprises a positioning and stabilising structure configured to maintain a seal-forming structure in a therapeutically effective position, the positioning and stabilising structure comprising:
headgear straps configured to provide a tensile force to the seal-forming structure into the patient's face via the frame, wherein the headgear straps include a first width and a second width greater than the first width; and
wherein the headgear straps are selectively threaded through an opening in order to adjust a tensile force, the first width configured to fit through the opening and the second width configured to be blocked from passing through the opening.

Another form of the present technology comprises a plenum chamber comprising:
a plenum chamber including a cavity that is pressurisable to a therapeutic pressure of at least 6 cmH₂O above ambient air pressure, said plenum chamber including a plenum chamber inlet port sized and structured to receive a flow of air at the therapeutic pressure for breathing by a patient;
a first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use; and
a positioning and stabilizing structure configured to maintain the first seal-forming structure and the second seal-forming structure in a therapeutically effective position, the positioning and stabilizing structure comprising:
   a frame coupled to the plenum chamber, the frame including,
      a pair of upper arms, and
      a pair of lower arms, and
   headgear straps coupled to the pair of upper arms and to the pair of lower arms, and the headgear straps configured to provide a tensile force to the first seal-forming structure and to the second seal-forming structure into the patient's face via the frame.

Another form of the present technology comprises a frame comprising:
a central portion;
a pair of upper arms that extend away from the central portion in a posterior direction, the pair of arms being more flexible than the central portion;
a pair of first connectors coupled to the pair of upper arms, the pair of first connectors configured to selectively connect to a pair of upper straps;
a pair of lower arms that extend away from the central portion;
a pair of second connectors coupled to the pair of lower arms, the pair of second connectors configured to selectively connect to a pair of lower straps.

In examples:
a) the pair of first connectors are different than the pair of second connectors;
b) the pair of first connectors are selected from a group consisting of a slots, ladder locks, and a stopper;
c) the pair of first connectors are slots;
d) the pair of first connectors each include a first loop section and a second loop section;
e) the pair of second connectors are magnets; and/or
f) the pair of first connectors are longer than the pair of second connectors.

Another form of the present technology comprises a patient interface comprising:
a plenum chamber including a cavity that is pressurisable to a therapeutic pressure of at least 6 cmH₂O above ambient air pressure, said plenum chamber including a plenum chamber inlet port sized and structured to receive a flow of air at the therapeutic pressure for breathing by a patient;
a first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use; and
a positioning and stabilizing structure configured to maintain the first seal-forming structure and the second seal-forming structure in a therapeutically effective position, the positioning and stabilizing structure comprising:
   a frame including a central portion coupled to the plenum chamber;
wherein the plenum chamber includes a groove configured to removably receive the central portion of the frame.

In examples:
a) at least part of the central portion of the frame is spaced apart from the groove in an engaged position;
b) a superior bar of the central portion is spaced apart from the groove when the central portion is coupled to the frame; and/or
c) the second seal-forming structure is spaced apart from the superior bar prior to use, and the second seal-forming structure is configured to contact the superior bar in use.

Another form of the present technology comprises a patient interface comprises:
a plenum chamber pressurisable to a therapeutic pressure of at least 6 cmH2O above ambient air pressure, said plenum chamber including a plenum chamber inlet port sized and structured to receive a flow of air at the therapeutic pressure for breathing by a patient;
a first seal-forming structure constructed and arranged to form a seal with a region of the patient's face surrounding an entrance to the patient's mouth such that the flow of air at said therapeutic pressure is delivered to the mouth, the first seal-forming structure constructed and arranged to maintain said therapeutic pressure in the plenum chamber throughout the patient's respiratory cycle in use;
a second seal-forming structure constructed and arranged to form a seal with a region of the patient's face surrounding an entrance to the patient's nose such that the flow of air at said therapeutic pressure is delivered to the nose, the second seal-forming structure constructed and arranged to maintain said therapeutic pressure in the plenum chamber throughout the patient's respiratory cycle in use; and
a vent structure to allow a continuous flow of gases exhaled by the patient from an interior of the plenum chamber to vent to ambient, said vent structure being sized and shaped to maintain the therapeutic pressure in the plenum chamber in use;
the patent interface further comprising:
   at least one region disposed in the plenum chamber spaced apart from the first seal-forming structure, the at least one region configured to oppose compression of the first seal-forming structure in the anterior direction

In examples:
a) the at least one region is at least one stopper rib;
b) the at least one stopper rib includes a plurality of stopper ribs spaced apart from one another;
c) the plurality of stopper ribs are arranged in a c-shape;
d) the plurality of stopper ribs extend around substantially the entire perimeter of the first seal-forming structure;
e) the at least one stopper rib is an elongated member;
f) the at least one stopper rib includes an inclined edge;
g) the inclined edge is a posterior edge;
h) the inclined edge configured to contact the first seal-forming structure in use in order to limit anterior movement of the first seal-forming structure;
i) the at least one stopper rib includes a length measured substantially perpendicularly to the first seal-forming structure and a width measured substantially perpendicularly to the length;
j) the length is greater than the width;
k) the first seal-forming structure is configured to contact the at least one stopper rib along the width, in use;
l) the at least one region is at least one stiffened region;
m) the at least one stiffened region includes a plurality of stiffened regions;
n) the at least one stiffened region includes a first thickness proximate to the inlet port and a second thickness distal to the inlet port, the second thickness is less than the first thickness;
o) the at least one stiffened region is disposed on an anterior surface of the plenum chamber; and/or
p) the at least one region is integrally formed with the plenum chamber.

Another form of the present technology comprises a patient interface comprising:
a plenum chamber including a cavity that is pressurisable to a therapeutic pressure of at least 6 cmH2O above ambient air pressure, said plenum chamber including a plenum chamber inlet port sized and structured to receive a flow of air at the therapeutic pressure for breathing by a patient;
a seal-forming structure constructed and arranged to form a seal with a region of the patient's face surrounding an entrance to the patient's airways such that the flow of air at said therapeutic pressure is delivered to the airways, the seal-forming structure constructed and arranged to maintain said therapeutic pressure in the plenum chamber throughout the patient's respiratory cycle in use;
a positioning and stabilizing structure configured to maintain the seal-forming structure in a therapeutically effective position, the positioning and stabilizing structure comprising:
   a frame coupled to the plenum chamber, the frame including,
      a central portion removably positionable within a groove on the plenum chamber outside of the cavity, and
      a pair of upper arms that extend away from the central portion; and
      a pair of lower arms that extend away from the central portion;
   headgear straps coupled to the frame, and configured to provide a tensile force to the first seal-forming structure and to the second seal-forming structure into the patient's face via the frame.

In examples:
a) the pair of upper arms are longer than the pair of lower arms;
b) the pair of upper arms each include a first connector and the pair of lower arms each include a second connector that is different from the first connector;
c) the first connector is selected from a group consisting of a loop, a ladder lock, and a cord stopper;
d) the first connector is the loop, the loop including a first loop portion and a second loop portion adjacent to the first loop portion;
e) the loop includes a dividing wall at least partially dividing the first loop portion from the second loop portion;
f) a strap of the headgear straps is configured to be selectively inserted into the first loop portion and contact the dividing wall, in use while under an optimal tension, and wherein the strap is configured to move into the second loop portion while under tension greater than the optimal tension;
g) the second connector is a magnetic member;
h) the magnetic member includes a substantially elliptical shape;
i) the second connector is the magnetic member;
j) a housing is removably and magnetically connected to the magnetic member;
k) the housing includes a strap connector for connecting to a strap of the headgear straps, the strap connector is selected from a group consisting of a loop, a ladder lock, and a cord stopper;
l) the housing is configured to engage a lip of an outer casing;
m) at least one strap of the headgear straps include a first width and a second width that is greater than the first width;
n) the first width is selectively receivable through the first connector, and wherein the second width is substantially blocked from passing through the first connector; and/or
o) the pair of first arms and the pair of second arms are integrally formed with the central portion.

Another form of the present technology comprises a plenum chamber comprising:
a plenum chamber including a cavity that is pressurisable to a therapeutic pressure of at least 6 cmH2O above ambient air pressure, said plenum chamber including a plenum chamber inlet port sized and structured to receive a flow of air at the therapeutic pressure for breathing by a patient;
a seal-forming structure constructed and arranged to form a seal with a region of the patient's face surrounding an entrance to the patient's airways such that the flow of air at said therapeutic pressure is delivered to the airways, the seal-forming structure constructed and arranged to maintain said therapeutic pressure in the plenum chamber throughout the patient's respiratory cycle in use;
a positioning and stabilizing structure configured to maintain the seal-forming structure in a therapeutically effective position;
wherein the plenum chamber includes a lip extending around at least a portion of the inlet port and extending into the cavity.

Another form of the present technology comprises a plenum chamber comprising:

Another form of the present technology comprises a system for providing pressurized air to a patient, the system comprising:
a plenum chamber including a cavity that is pressurisable to a therapeutic pressure of at least 6 cmH2O above ambient air pressure, said plenum chamber including a plenum chamber inlet port sized and structured to receive a flow of air at the therapeutic pressure for breathing by a patient, wherein the plenum chamber includes a lip extending at least partially around the plenum chamber inlet port;
a seal-forming structure constructed and arranged to form a seal with a region of the patient's face surrounding an entrance to the patient's airways such that the flow of air at said therapeutic pressure is delivered to the airways, the seal-forming structure constructed and arranged to maintain said therapeutic pressure in the plenum chamber throughout the patient's respiratory cycle in use;
a positioning and stabilizing structure configured to maintain the seal-forming structure in a therapeutically effective position; and
an air circuit removably connected to the plenum chamber and configured to provide the flow of air at the therapeutic pressure from a flow generator to the cavity, the air circuit including a vent ring configured to be inserted at least partially through the plenum chamber inlet port, the vent ring comprising:
   a posterior surface having a first diameter,
   an anterior surface having a second diameter less than the first diameter, and
   a ring body extending between the anterior surface and the posterior surface;
wherein the posterior surface is configured to be positioned within the cavity and the anterior surface is configured to remain outside the cavity when connected to the plenum chamber;
wherein the lip is configured to contact the vent ring along the ring body between the posterior surface and the anterior surface; and
wherein the first diameter is greater than a dimeter of the lip.

In examples:
a) the vent body is inclined between the posterior surface and the anterior surface;
b) the posterior surface is configured to contact the lip and an anterior wall of the plenum chamber within the cavity, in use;
c) the ring body includes an inner wall, an outer wall, and a groove disposed between the inner wall and the outer wall;
d) a ring seal is positioned within the groove;
e) an outer diameter of the ring seal is less than an outer diameter of the groove;
f) the ring body includes a central opening configured to receive an elbow connector;
g) the system includes a quick-release connector;
h) the quick-release connector is a rotatable connector or a latch connector;
i) the quick-release connector is configured to connect to a complementary connector; and/or
j) the seal-forming structure is spaced apart from the vent ring, and wherein a patient's face is not configured to contact the vent ring.

Another form of the present technology comprises a patient interface comprising:
a plenum chamber having a cavity pressurisable to a therapeutic pressure of at least 6 cmH2O above ambient air pressure;
a first seal-forming structure constructed and arranged to form a seal with a region of the patient's face surrounding an entrance to the patient's mouth, the first seal-forming structure constructed and arranged to maintain said therapeutic pressure in the cavity of the plenum chamber throughout the patient's respiratory cycle in use;
a second seal-forming structure constructed and arranged to form a seal with a region of the patient's face surrounding an entrance to the patient's nares, the second seal-forming structure constructed and arranged to maintain said therapeutic pressure in the cavity of the plenum chamber throughout the patient's respiratory cycle in use; and
a vent structure to allow a continuous flow of gases exhaled by the patient from an interior of the plenum chamber to vent to ambient, said vent structure being sized and shaped to maintain the therapeutic pressure in the plenum chamber in use;
the patient interface further comprising:
   at least one stopper rib disposed in the cavity plenum chamber spaced apart from the first seal-forming structure in a rest position, the first seal-forming structure configured to contact the at least one stopper rib in an operational position, the at least one stopper rib configured to oppose compression of the first seal-forming structure in an anterior direction; and
   wherein the second seal-forming structure is not configured to contact the at least one stopper rib.

In examples:
a) a lip superior portion is disposed inferior to the second seal-forming structure and configured to contact the lip superior;
b) the lip superior portion not configured to contact the at least one stopper rib;
c) the plurality of stopper ribs are arranged in a c-shape;
d) the plurality of stopper ribs are unequally spaced apart from one another within the cavity;
e) the plurality of stopper ribs extend around a majority of the perimeter of the seal-forming structure;
f) the at least one stopper rib is an elongated member;
g) the at least one stopper rib includes an inclined edge;
h) the inclined edge is a posterior edge;
i) the inclined edge configured to contact the seal-forming structure in use in order to limit anterior movement of the seal-forming structure in the operational position;
j) the inclined edge is disposed further from the seal-forming structure toward a center of the plenum chamber in the rest position;
k) the at least one stopper rib includes a length measured substantially perpendicularly to the seal-forming structure and a width measured substantially perpendicularly to the length, and wherein the length is greater than the width;
l) first seal-forming structure is configured to contact the at least one stopper rib along the width, in the operational position;
m) the width is substantially uniform along the length of the at least one stopper rib;
n) the width varies along the length of the at least one stopper rib; and/or
o) the at least one stopper rib is integrally formed with the plenum chamber.

Another form of the present technology comprises a patient interface comprising:
a plenum chamber including a cavity that is pressurisable to a therapeutic pressure of at least 6 cmH2O above ambient air pressure and a groove formed on a surface of the plenum chamber outside the cavity and exposed to ambient pressure, the plenum chamber being formed entirely from a flexible material;
a seal-forming structure constructed and arranged to form a seal with a region of the patient's face surrounding an entrance to the patient's airways, the seal-forming structure constructed and arranged to maintain said therapeutic pressure in the cavity of the plenum chamber throughout the patient's respiratory cycle in use;
a positioning and stabilizing structure configured to maintain the seal-forming structure in a therapeutically effective position, the positioning and stabilizing structure comprising:
   a frame coupled to the plenum chamber, the frame including,
      a central portion including removably positionable within the groove,
      a pair of upper arms that extend away from the central portion, and
      a pair of lower arms that extend away from the central portion; and
   headgear straps coupled to the pair of upper arms and the pair of lower arms of the frame, and configured to provide a tensile force to the seal-forming structure into the patient's face via the frame.

In examples:
a) the pair of upper arms are longer than the pair of lower arms;
b) the pair of upper arms each include a first type of connector and the pair of lower arms each include a second type of connector that is different from the first type of connector;
c) the first type of connector is selected from a group consisting of a loop, a ladder lock, and a cord stopper;
d) the first type of connector is the loop, the loop including a first loop portion and a second loop portion adjacent to the first loop portion;
e) the loop includes a dividing wall at least partially dividing the first loop portion from the second loop portion;
f) a strap of the headgear straps is configured to be selectively inserted into the first loop portion and contact the dividing wall, in use while under an optimal tension;
g) the strap is configured to move into the second loop portion while under tension greater than the optimal tension;
h) the second type of connector is a magnetic member;
i) the magnetic member includes a substantially elliptical shape;
j) a housing is removably and magnetically connected to the magnetic member;
k) the housing includes a strap connector for connecting to a strap of the headgear straps, the strap connector is selected from a group consisting of a loop, a ladder lock, and a cord stopper;
l) the housing is configured to engage a lip of an outer casing;
m) at least one strap of the headgear straps include a first width and a second width that is greater than the first width;
n) the first width is selectively receivable through the first connector, and wherein the second width is substantially blocked from passing through the first connector;
o) the pair of upper arms and the pair of lower arms are integrally formed with the central portion;
p) the plenum chamber includes at least one projection that extends from the groove;
q) the central portion of the frame includes at least one slot configured to selectably receive the at least one projection;
r) the pair of lower arms are substantially flush with the central portion;
s) the seal-forming structure further includes a first seal-forming structure constructed and is arranged to form a seal with a region of the patient's face surrounding an entrance to the patient's mouth;
t) the seal-forming structure further includes a second seal-forming structure constructed and arranged to form a seal with a region of the patient's face surrounding an entrance to the patient's nares;
u) the groove is disposed on the plenum chamber inferior to the second seal-forming structure and opposite to the first seal-forming structure;
v) system for providing pressurized air to a patient, the system comprising: the patient interface of any one of the previous examples; a respiratory therapy system (RPT) device configured to provide a flow of air at the therapeutic pressure; and a conduit connecting the RPT device to the patient interface, the conduit configured to convey the flow of air to the patient interface;
w) the conduit is connected to an inlet port of the plenum chamber, which is spaced apart from an inner perimeter of the central portion of the frame;
x) a central portion includes an inner perimeter and an outer perimeter removably positionable within the groove;
y) the groove is formed radially outside of the inlet port, and the groove forms an enclosed perimeter; and/or
z) the inner perimeter is configured to be spaced apart from the inlet port while positioned within the groove.

Another form of the present technology comprises a patient interface comprising:
a plenum chamber including a cavity that is pressurisable to a therapeutic pressure of at least 6 cmH2O above ambient air pressure, said plenum chamber including a plenum chamber inlet port sized and structured to receive a flow of air at the therapeutic pressure for breathing by a patient, wherein the plenum chamber includes a lip extending at least partially around the plenum chamber inlet port and into the cavity;
a seal-forming structure constructed and arranged to form a seal with a region of the patient's face surrounding an entrance to the patient's airways such that the flow of air at said therapeutic pressure is delivered to the airways, the seal-forming structure constructed and arranged to maintain said therapeutic pressure in the plenum chamber throughout the patient's respiratory cycle in use;
a positioning and stabilizing structure configured to maintain the seal-forming structure in a therapeutically effective position; and
an air circuit removably connected to the plenum chamber and configured to provide the flow of air at the therapeutic pressure from a flow generator to the cavity, the air circuit including a vent ring configured to be inserted at least partially through the plenum chamber inlet port, the vent ring comprising:
   a posterior surface having a first diameter,
   an anterior surface having a second diameter less than the first diameter, and
   a ring body extending between the anterior surface and the posterior surface, ring body includes an inner wall, an outer wall, and a groove disposed between the inner wall and the outer wall;
wherein:
   the posterior surface is configured to be positioned within the cavity and the anterior surface is configured to remain outside the cavity when connected to the plenum chamber;
   the posterior surface includes at least one vent opening configured to provide communication between the cavity and an ambient environment through the groove;
   the lip is configured to contact the vent ring along the ring body between the posterior surface and the anterior surface; and
   the first diameter is greater than a diameter of the lip.

In examples:
a) the ring body is inclined between the posterior surface and the anterior surface;
b) the posterior surface is configured to contact the lip and an anterior wall of the plenum chamber within the cavity, in use;
c) a ring seal is positioned within the groove;
d) an outer diameter of the ring seal is less than an outer diameter of the groove;
e) the ring body includes a central opening and the system further comprises an elbow connector, the central opening is configured to receive the elbow connector;
f) the elbow connector is rotatably connected to the ring body and wherein the ring body is rotatably connected to the plenum chamber;
g) the at least one vent opening is a plurality of vent openings spaced around the perimeter of the groove;
h) the system includes a quick-release connector;
i) the quick-release connector is a rotatable connector or a latch connector;
j) wherein the quick-release connector is configured to connect to a complementary connector;
k) the quick-release connector includes at least one flat surface;
l) the at least one flat surface is a pair of flat surface spaced approximately 180° apart;
m) the at least one flat surface is configured to redistribute leak of the flow of air from the system;
n) the seal-forming structure is spaced apart from the vent ring, and wherein a patient's face is not configured to contact the vent ring;
o) the quick-release connector includes vent openings configured to exhaust a portion of the flow of air to ambient;
p) the ring body includes an extension;
q) the ring body includes disk configured to be positioned within the cavity and limit exhaled air from venting to ambient; and/or
r) the disk extends radially beyond a portion of the posterior surface including the at least one vent opening.

In examples:
a) the elbow connector includes at least one button configured to be selectively actuated in order to disengage the connection between the elbow connector and the central opening;
b) the at least one button is formed as a cantilever structure'
c) the at least one button includes a button lip configured to selectively engage the ring body and move relative to the remainder of the elbow when the at least one button is actuated;
d) the button lip is configured to selectively engage an inner perimeter of the inner wall of the ring body;
e) the at least one button includes a finger configured to selectively engage the ring body and move relative to the remainder of the elbow when the at least one button is actuated;
f) the finger is configured to selectively engage the ring body radially outside the inner wall; and/or
g) the plenum chamber inlet port includes an extension and the ring body is connected to the extension and spaced apart from a surface of the plenum chamber.

Another form of the present technology comprises an elbow configured to removably connect to a plenum chamber, the elbow comprising a button selectively engagable by the patient.

Another form of the present technology comprises an elbow configured to removably connect to a plenum chamber, the elbow comprising:
a body comprising a first end configured to be at least partially inserted into a cavity of the plenum chamber, and a second end configured to connect to an air circuit, the body further comprising:
   a button having a fixed end proximate to the first end and a free end extending away from the free end;
   an outer lip extending substantially around at least a portion perimeter of the body; and
wherein the button is selectively engagable by a patient in order to selectively disengage the body from the plenum chamber.

In examples:
a) the button forms a portion of a perimeter of the body;
b) an inner lip spaced apart from the outer lip and disposed closer to the first end than the outer lip,
c) a width of the inner lip is approximately the same as a width of the button;
d) the inner lip extends along a portion of the perimeter of the body;
e) a channel extends around a portion of the button creating the free end;
f) the button is a first button and the body further includes a second button;
g) the second button is spaced approximately 180° apart from the first button;
h) the first button and the second button are configured to move toward one another as a result of selective engagement by the patient;
i) the outer lip is configured to abut a vent ring and limit translational movement of the elbow;
j) the button is selectively engagable by a patient in order to selectively move the inner lip relative to the outer lip;
k) the inner lip is configured to be in a pressurized volume when connected to the plenum chamber and pressurized air is supplied;
l) the inner lip is configured to selectively engage a groove, and is configured to disengage the groove upon application of the button(s); and/or
m) the outer lip extends substantially around the perimeter of the body.

In examples:
a) the button includes a finger extending from the fixed end;
b) the finger extends toward the first end of the body;
c) the finger is formed as a cantilever structure with respect to the button;
d) the button is a first button and the body further includes a second button;
e) the second button is spaced approximately 180° apart from the first button;
f) the first button and the second button are configured to move toward one another as a result of selective engagement by the patient;
g) the outer lip is disposed between the first button and the second button;
h) a distance between an end of the finger and the first end of the body is less than a distance between the outer lip and the first end of the body;
i) the outer lip is configured to abut a vent ring and limit translational movement of the elbow;
j) the finger is configured to selectively connect to a vent ring outside of a pressurized volume; and/or
k) selective engagement of the button is configured to space the finger further from a surface of the body.

Another form of the present technology comprises an elbow configured to removably connect to a plenum chamber, the elbow comprising:
a body comprising a first end configured to be at least partially inserted into a cavity of the plenum chamber, and a second end configured to connect to an air circuit, the body further comprising:
   a button spaced apart from a surface of the body; and
   a finger coupled to the button;
wherein the button is selectively engagable by a patient in order to move the button relative to the surface of the body; and
wherein the finger is movable with the button.

In examples:
a) the button is configured to move toward the surface of the body and the finger is configured to move away from the surface of the body when the button is engaged;
b) the button is a first button and the body further includes a second button;
c) the second button is spaced approximately 180° apart from the first button;
d) the first button and the second button are configured to move toward one another as a result of selective engagement by the patient; and/or
e) the finger is configured to selectively connect to a vent ring outside of a pressurized volume.

Another form of the present technology comprises an elbow configured to removably connect to a plenum chamber, the elbow comprising:
a body comprising a first end configured to be at least partially inserted into a cavity of the plenum chamber, and a second end configured to connect to an air circuit, the body further comprising:
a button forming a portion of a surface of the body, the button configured to move into an interior of the body as a result of an external force; and
a finger coupled to the button and spaced apart from the surface of the body, the finger configured to move as a result of the external force applied to the button.

In examples:
a) the finger is configured to engage an outer surface of a vent ring;
b) the button is biased toward a relaxed position where the button is substantially even with the surrounding surface of the body;
c) the finger is spaced a first distance from the surface of the body in the relaxed position, and is spaced a second distance from the surface of the body when the external force is applied, the second distance being greater than the first distance;
d) the button is a first button and the body further includes a second button;
e) the second button is spaced approximately 180° apart from the first button;
f) the finger is substantially hook shaped; and/or
g) a fulcrum point is between the button and the finger, the button formed as a cantilever structure relative to the fulcrum point, and the finger formed as a cantilever structure relative to the fulcrum point.

Another form of the present technology comprises vent ring configured to connect to a plenum chamber and to increase the humidity within the plenum chamber, the vent ring comprising:
a posterior surface configured to be positioned within a pressurized environment, the posterior surface including at least one vent opening configured to exhaust air from the plenum chamber; and
an anterior surface spaced apart from the posterior surface and configured to be positioned outside the pressurized environment.

In example:
a) a disk is coupled to the posterior surface and is configured to be positioned within the pressurized environment;
b) a width of the disk is substantially the same as a width of the posterior surface;
c) the disk extends radially outside of a position of the at least one vent opening on the posterior surface;
d) the disk is configured to deflect exhaled air back into the plenum chamber;
e) an extension connectable to the vent ring proximate to the posterior surface, the extension configured to space the at least one vent opening apart from a surface of the plenum chamber;
f) the at least one vent opening is a plurality of vent openings spaced around a perimeter of the posterior surface;
g) the plurality of vent openings are equally spaced around the perimeter of the posterior surface; and/or
h) the plurality of vent openings extend around less than half of the perimeter of the posterior surface.

Another form of the present technology is a connector member having a first side and a second side opposite to the first side. The first side and the second side each including a connector material. The connector material is configured to removably connect to a positioning and stabilizing structure of a patient interface.

In examples:
a) the first side includes the same connector material as the second side;
b) wherein the connector material is either hook material or loop material; and/or
c) the connector is connectable to an infinite number of locations.

Another form of the present technology comprises a positioning and stabilizing structure comprising at least one side strap having an inner surface configured to overlay a patient's temporal bone and an outer surface opposite to the inner surface; and a connecting member removably connectable to the outer surface, the connecting member having a first side and a second side each including a connecting material; wherein the connecting member is connectable to a plurality of locations along the length of the at least one side strap.

In examples:
a) the first side and the second side of the connecting member include hook material;
b) the outer surface includes loop material;
c) the connecting member is connectable to an infinite number of locations along the length of the at least one side strap;
d) a patient interface includes a frame and the positioning and stabilizing structure; wherein the frame includes a loop configured to removably receive the at least one side strap; and wherein the at least one strap is folded after passing through the loop so that the other side of the connecting member is removably connected to the at least one strap;
e) in use, the first side and the second side are removably connected to the at least one strap;
f) the positioning and stabilizing structure further comprises a rear strap configured to overlay the patient's occipital bone, and wherein the at least one side strap includes a pair of lower side straps configured to removably connect to a frame, and a pair of upper straps configured to removably connect to the frame. The pair of upper side straps and the pair of lower side straps each include a connection member; and/or
g) the connection member on each of the upper straps and the lower straps is removably connected to an infinite number of positions along the length of the respective strap.

Another form of the present technology comprises a positioning and stabilizing structure comprising at least one side strap, at least one strap includes an elastic portion stretchable between a first length and a second length, the at least one strap includes a portion that is substantially hidden in the first position and is visible when the strap is extended in the second position.

In examples:
a) the hidden portion is a different color than the remainder of the at least one strap;
b) the hidden portion is luminescent;
c) the hidden portion include an electric light emitting element (e.g., an LED);
d) the at least one strap includes a tactile response element on either side of the hidden portion, the tactile response element configured to be coupled together in the first position and separate in the second position;
e) the tactile response element is a magnet; and/or
f) the tactile response element is a mechanical connector.

Another form of the present technology comprises a positioning and stabilizing structure having a rear strap configured to overlay the patient's occipital bone, a pair of lower side straps configured to removably connect to a frame, and a pair of upper straps configured to removably connect to the frame. The pair of upper side straps and the pair of lower side straps each include a connection member.

In examples:
a) the connection members are removably connected to the respective straps;
b) the connection members each include hook material on each side to facilitate removable connection;
c) at least one strap includes a first portion with a first width, a second portion with a second width, and a transition between the first portion and the second portion;
d) the second width is larger than the first width and is configured to be larger than a loop on a frame that receives the strap;
e) at least one strap includes a plurality of raised portions;
f) the plurality of raised portions are evenly spaced along at least a portion of the at least one strap;
g) the at least one strap includes a first density of raised portions and a second density of raised portions;
h) at least one strap includes a cut edges;
i) the cut edge is a square edge, a triangular edge, or a circular edge;
j) the cut edges are evenly spaced along at least a portion of the at least one strap;
k) the at least one strap includes a first density of cut edges and a second density of cut edges;
l) at least one strap includes an elastic portion stretchable between a first length and a second length;
m) the at least one strap includes a portion that is substantially hidden in the first position and is visible when the strap is extended in the second position;
n) the hidden portion is a different color and/or texture than the remainder of the strap;
o) the at least one strap includes a tactile response element on either side of the hidden portion, the tactile response element configured to be coupled together in the first position and separate in the second position;
p) a top strap configured to overlay the patient's temporal and/or frontal bones, the top strap having a first section and a second section and being selectively connected by a buckle; and/or
q) a patient interface removably connected to the positioning and stabilizing structure.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

Fig. 1A shows a system including a patient 1000 wearing a patient interface 3000, in the form of nasal pillows, receiving a supply of air at positive pressure from an RPT device 4000. Air from the RPT device 4000 is conditioned in a humidifier 5000, and passes along an air circuit 4170 to the patient 1000. A bed partner 1100 is also shown. The patient is sleeping in a supine sleeping position.
Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.
Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

Fig. 2A shows an overview of a human respiratory system including the nasal and oral cavities, the larynx, vocal folds, oesophagus, trachea, bronchus, lung, alveolar sacs, heart and diaphragm.
Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.
Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.
Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.
Fig. 2E is a further side view of a head. The approximate locations of the Frankfort horizontal and nasolabial angle are indicated. The coronal plane is also indicated.
Fig. 2F shows a base view of a nose with several features identified including naso-labial sulcus, lip inferior, upper Vermilion, naris, subnasale, columella, pronasale, the major axis of a naris and the midsagittal plane.
Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 21 shows a medial dissection of a nose, approximately several millimeters from the midsagittal plane, amongst other things showing the septum cartilage and medial crus of greater alar cartilage.

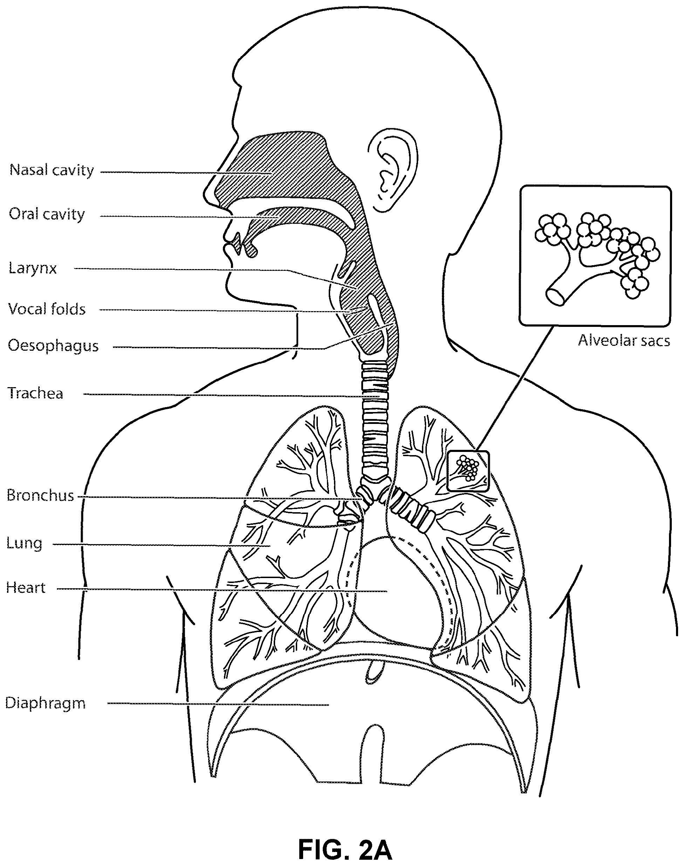

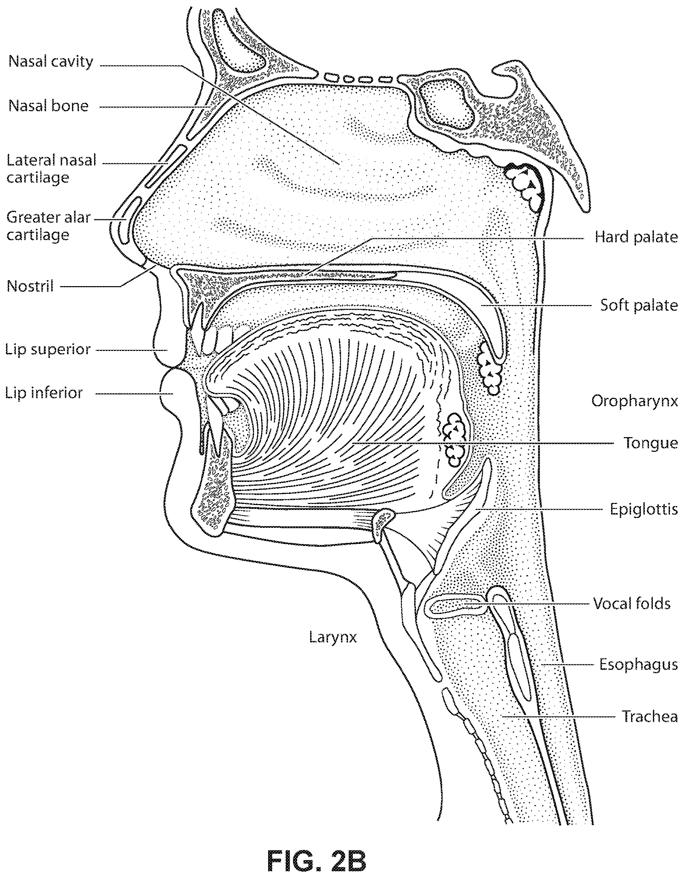

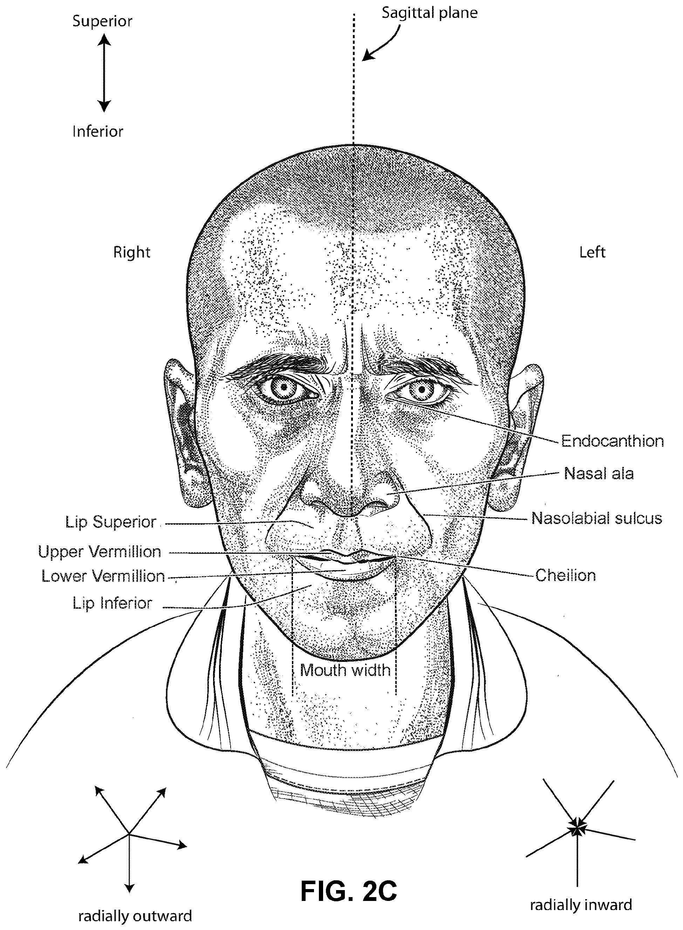

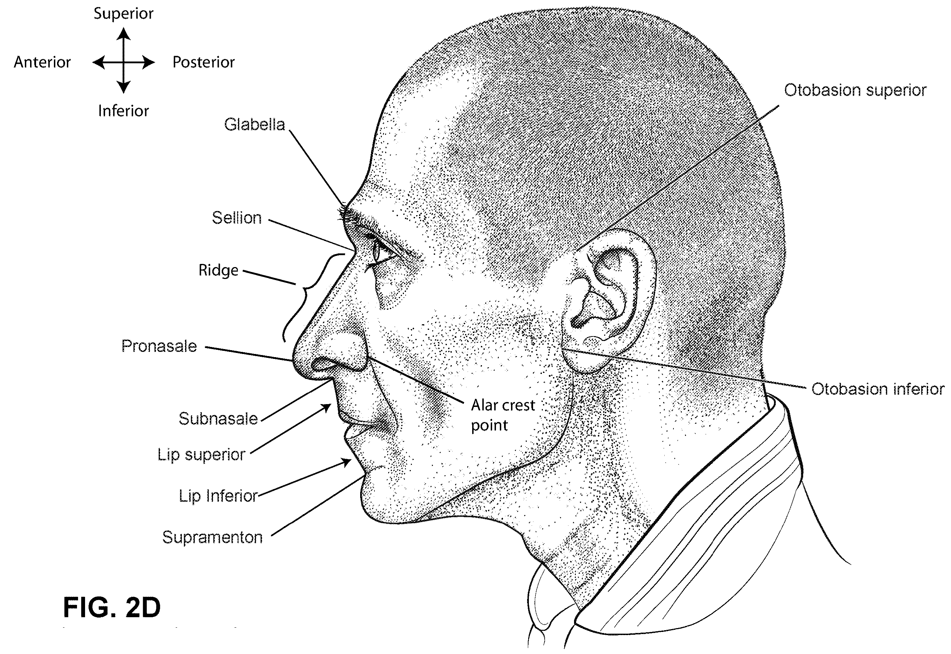

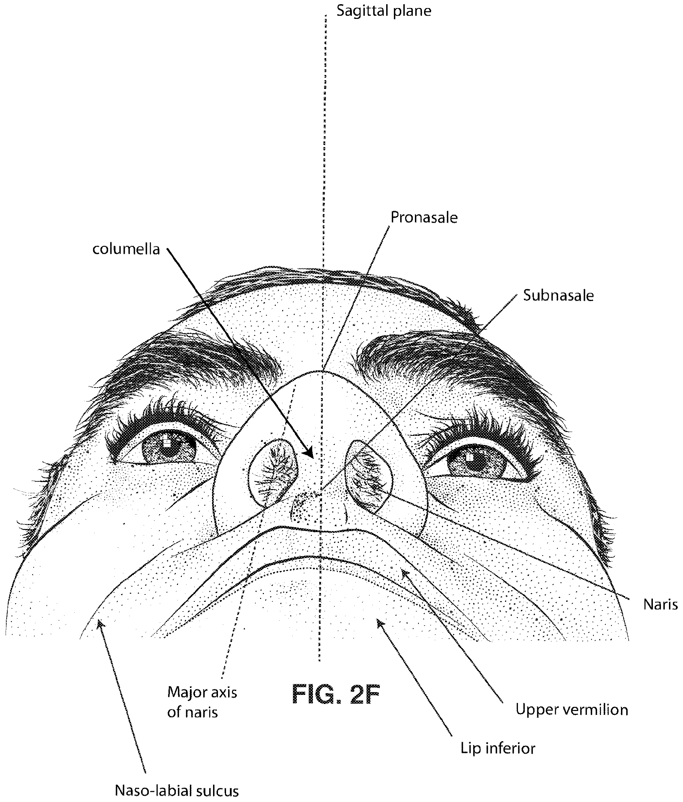

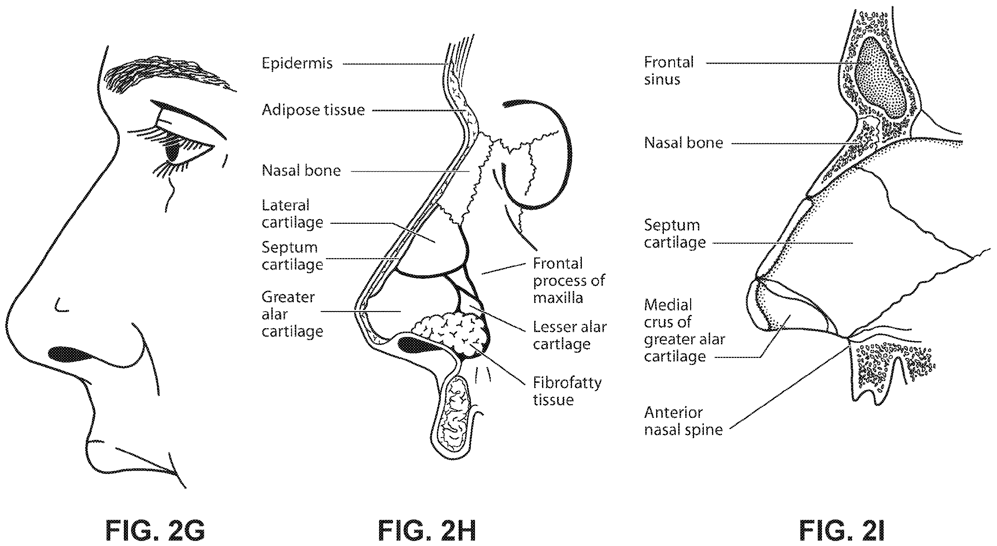

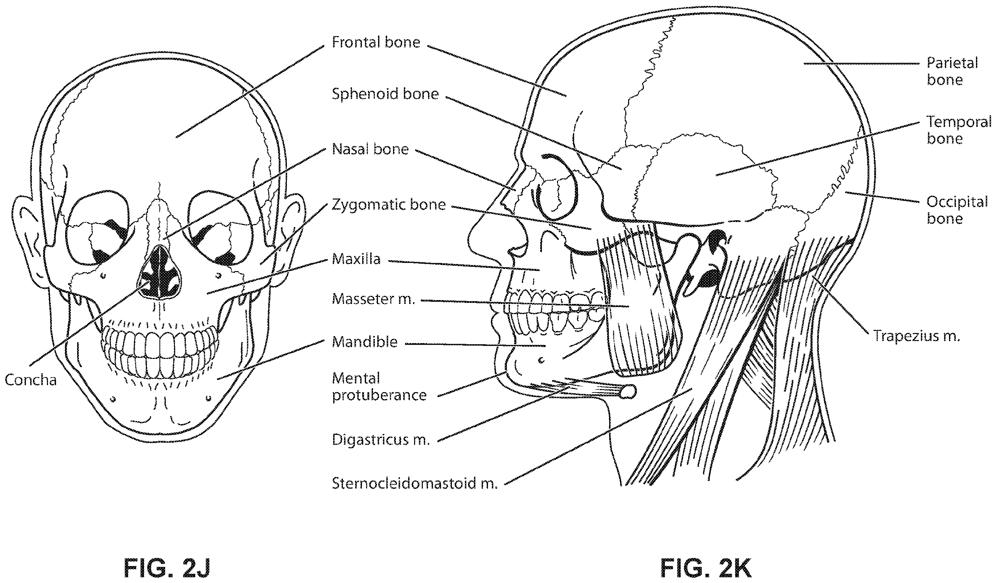

Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

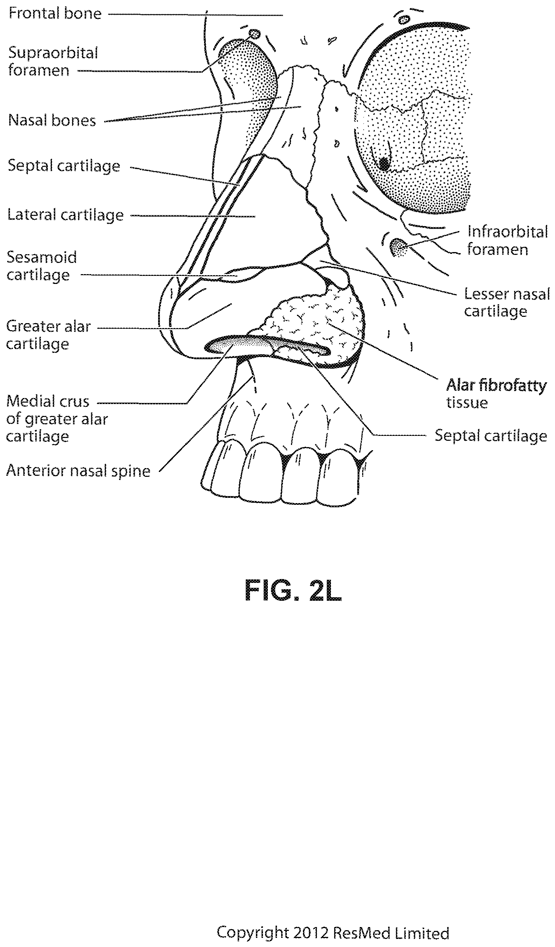

Fig. 2L shows an anterolateral view of a nose.

### 4.3 PATIENT INTERFACE

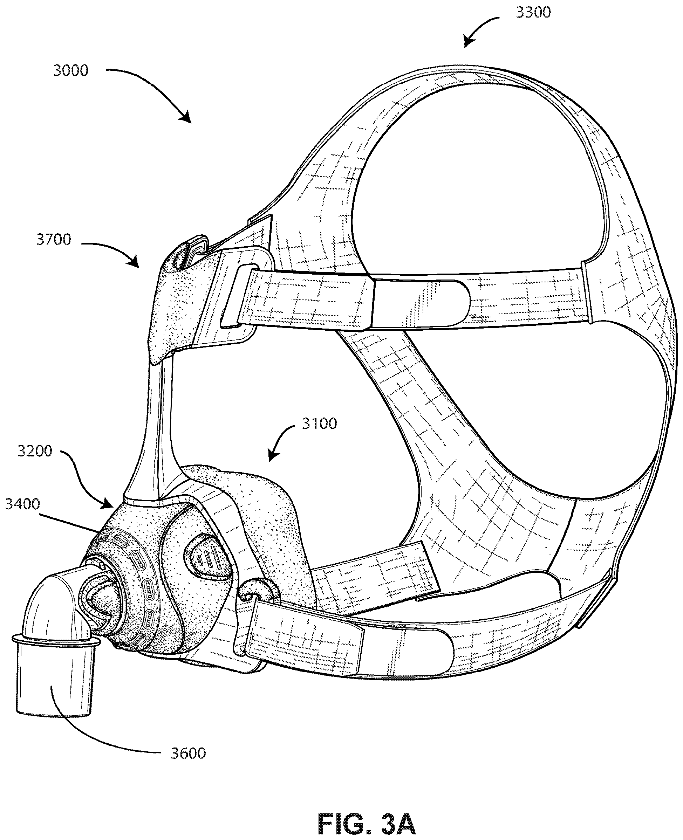

Fig. 3A shows a patient interface in the form of a nasal mask in accordance with one form of the present technology.

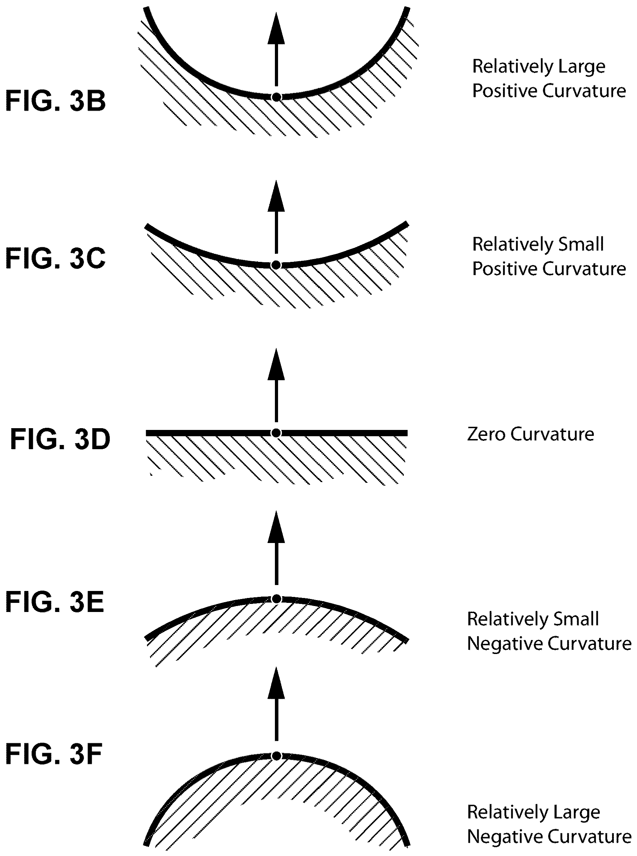

Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.

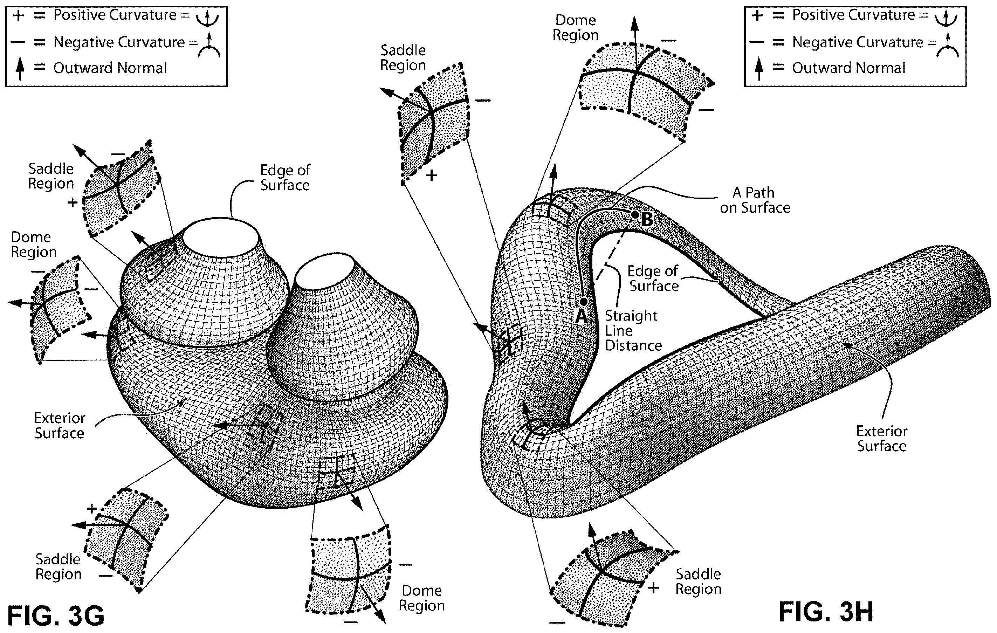

Fig. 3G shows a cushion for a mask that includes two pillows. An exterior surface of the cushion is indicated. An edge of the surface is indicated. Dome and saddle regions are indicated.
Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.

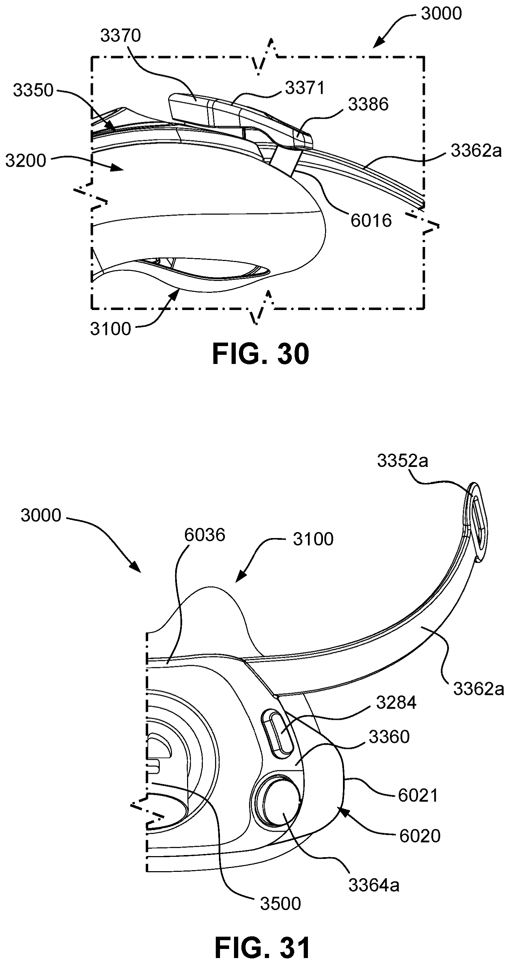

Fig. 31 shows the surface of a structure, with a one dimensional hole in the surface. The illustrated plane curve forms the boundary of a one dimensional hole.

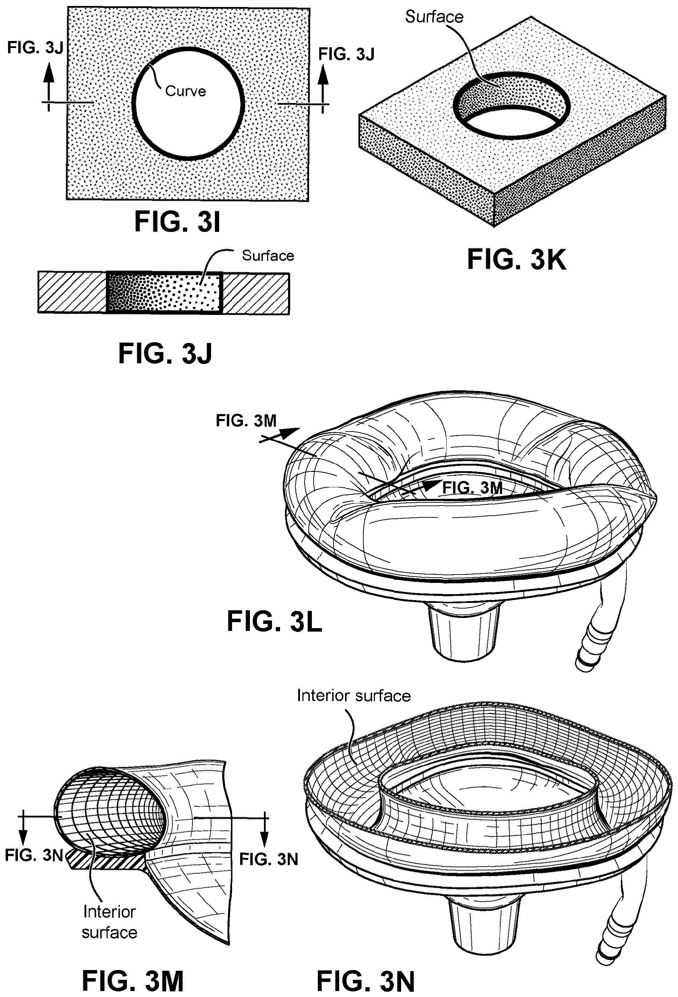

Fig. 3J shows a cross-section through the structure of Fig.3I. The illustrated surface bounds a two dimensional hole in the structure of Fig. 31.
Fig. 3K shows a perspective view of the structure of Fig. 31, including the two dimensional hole and the one dimensional hole. Also shown is the surface that bounds a two dimensional hole in the structure of Fig. 31.
Fig. 3L shows a mask having an inflatable bladder as a cushion.
Fig. 3M shows a cross-section through the mask of Fig. 3L, and shows the interior surface of the bladder. The interior surface bounds the two dimensional hole in the mask.
Fig. 3N shows a further cross-section through the mask of Fig. 3L. The interior surface is also indicated.

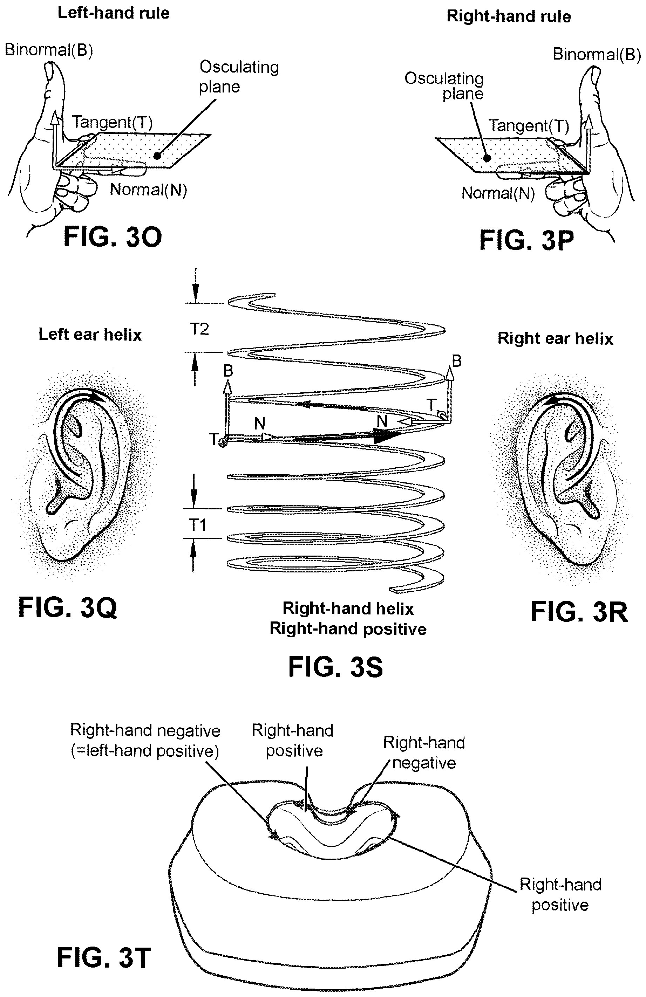

Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a mask, including the sign of the torsion of the space curve defined by the edge of the sealing membrane in different regions of the mask.

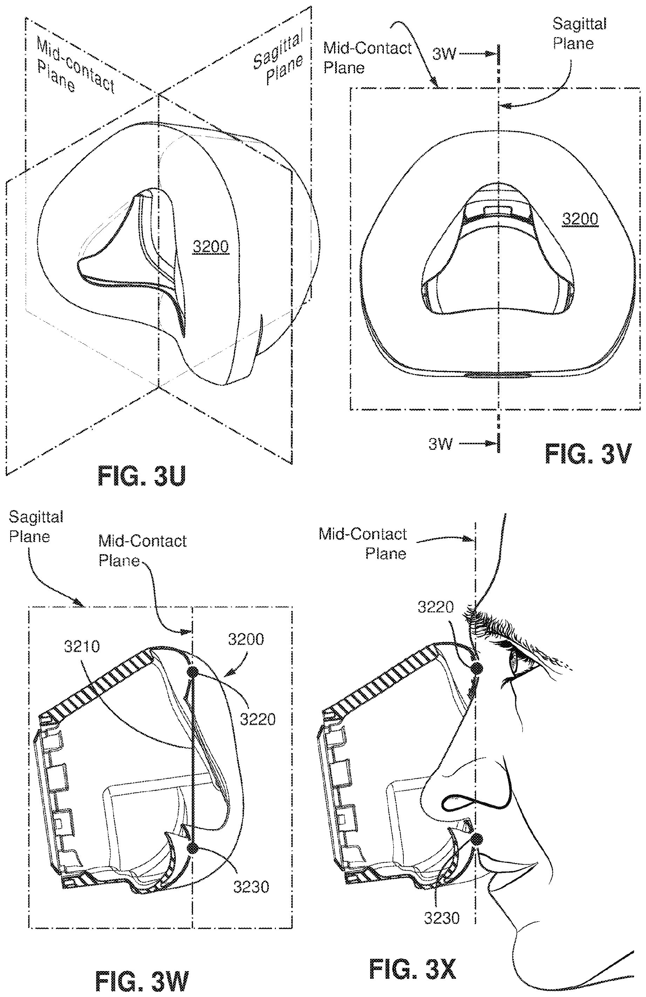

Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.
Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points. For oro-nasal interfaces configured with plenum chambers having separate oral and nasal portions, for example Ultra Compact Full Face (UCFF) masks, the superior point and inferior points are on the seal forming structure of the oral portion of the mask.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

### 4.4 RPT DEVICE

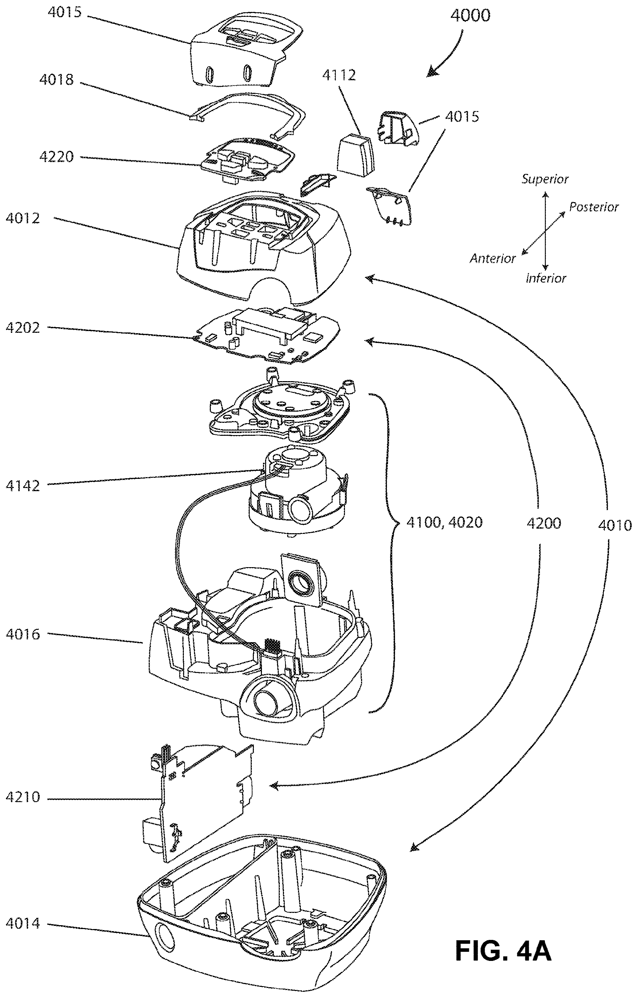

Fig. 4A shows an RPT device in accordance with one form of the present technology.

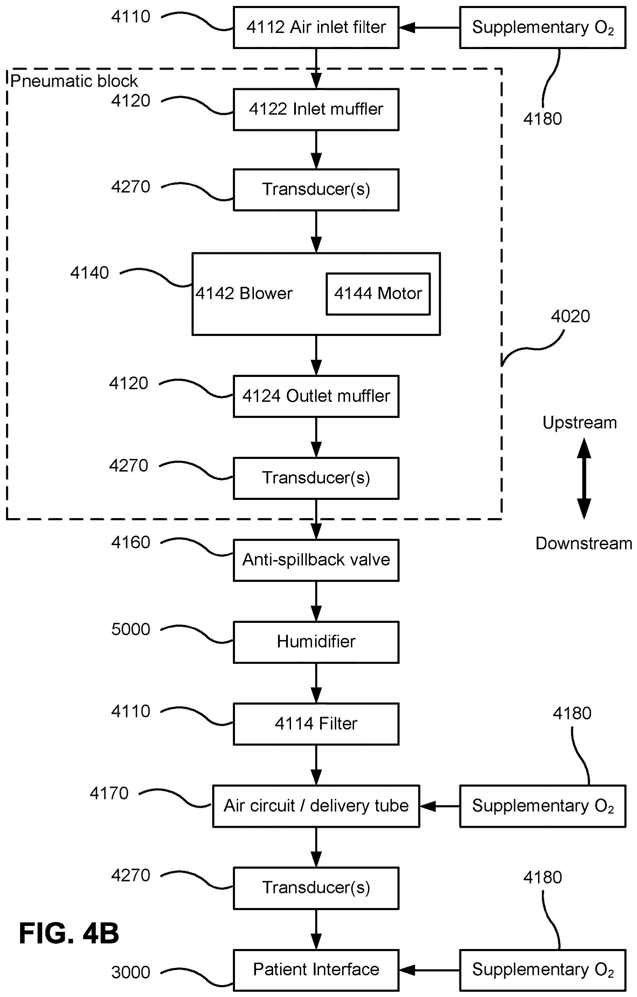

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 4.5 HUMIDIFIER

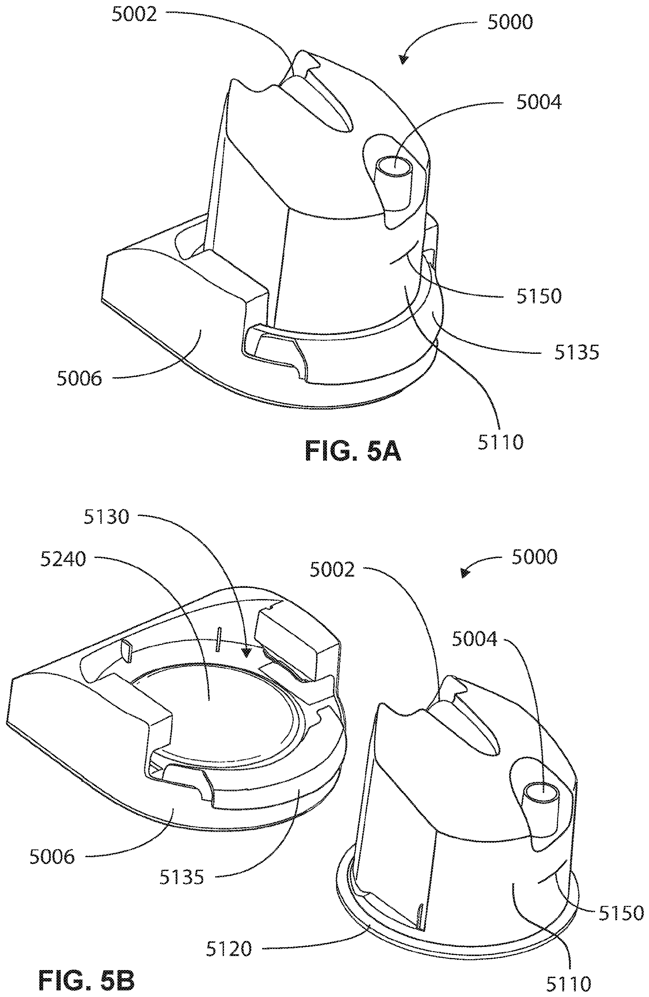

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 4.6 BREATHING WAVEFORMS

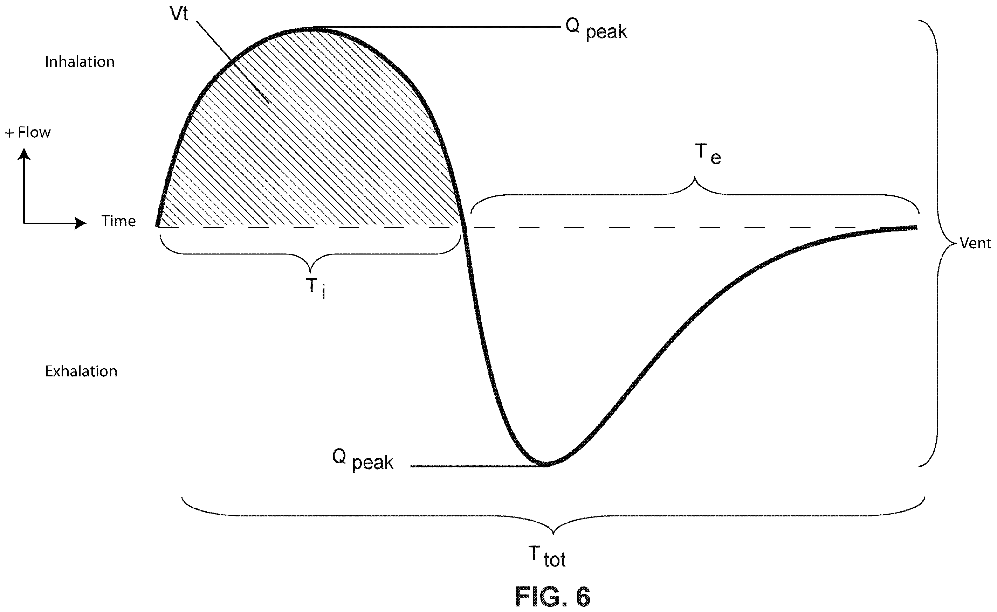

Fig. 6 shows a model typical breath waveform of a person while sleeping.

### 4.7 PATIENT INTERFACE EXAMPLES OF THE PRESENT TECHNOLOGY

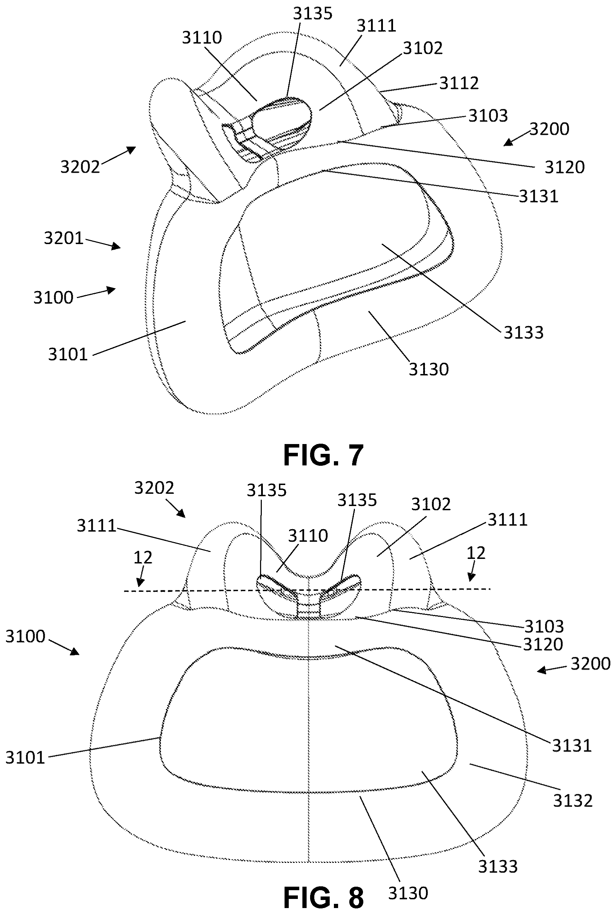

Fig. 7 is a rear perspective view of a plenum chamber in accordance with one form of the present technology, with inlet ports not shown.
Fig. 8 is a rear view of the plenum chamber of Fig 7.

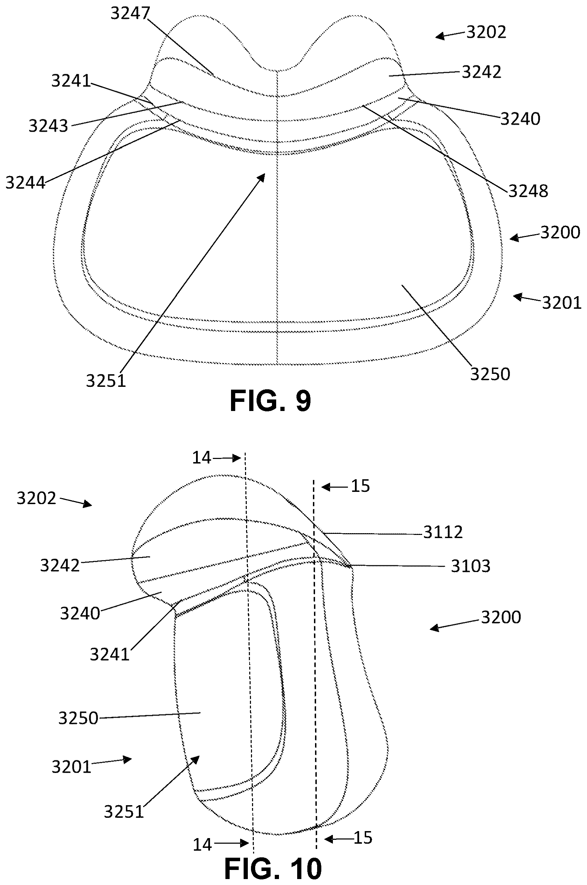

Fig. 9 is a front view of the plenum chamber of Fig. 7.
Fig. 10 is a side view of the plenum chamber of Fig. 7.

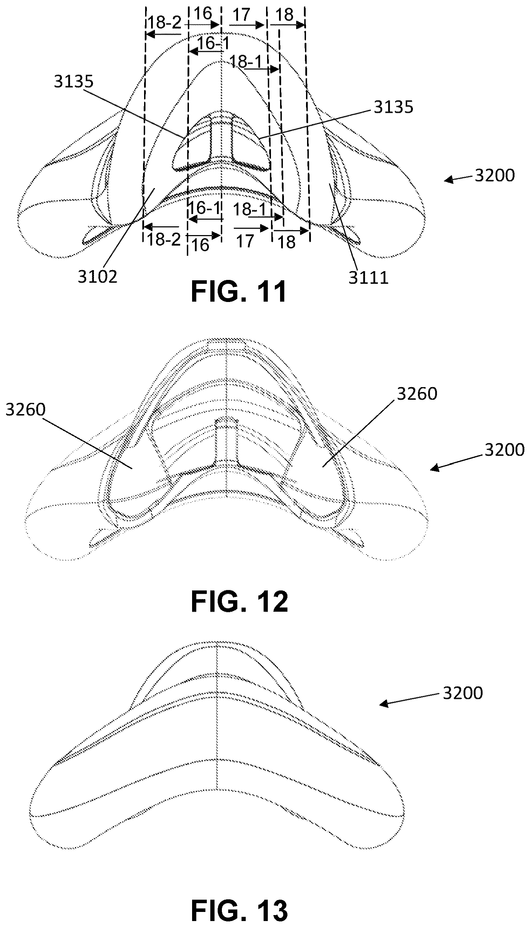

Fig. 11 is a top view of the plenum chamber of Fig. 7.
Fig. 12 is a cross-section of the plenum chamber through plane 12-12.
Fig. 13 is a bottom view of the plenum chamber of Fig. 7.

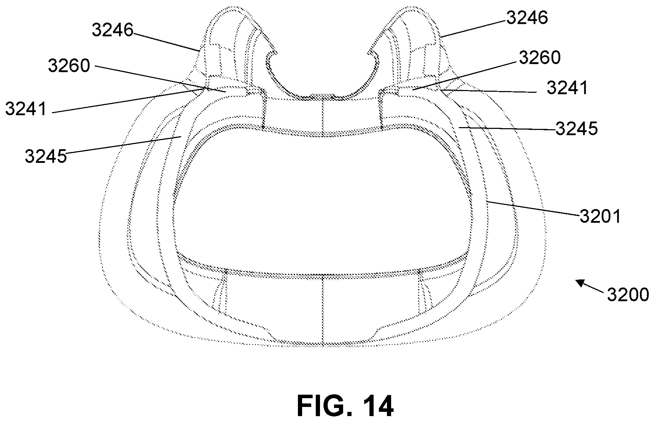

Fig. 14 is a cross-section of the plenum chamber through plane 14-14.

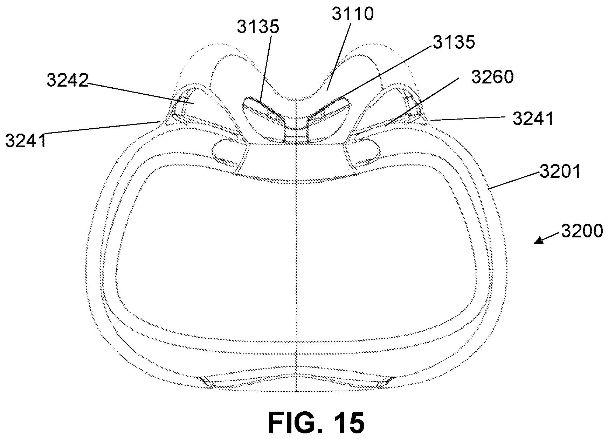

Fig. 15 is a cross-section of the plenum chamber through plane 15-15.

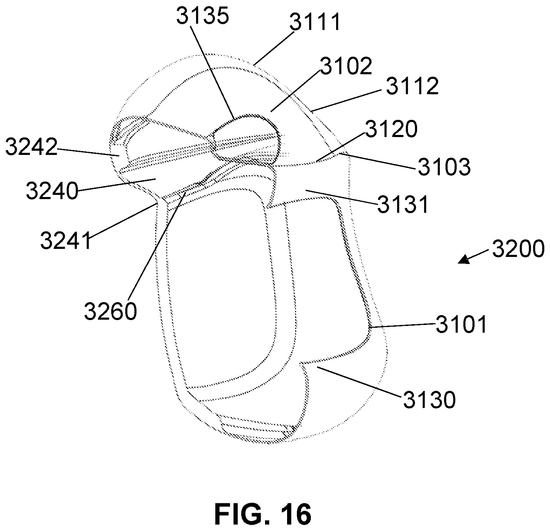

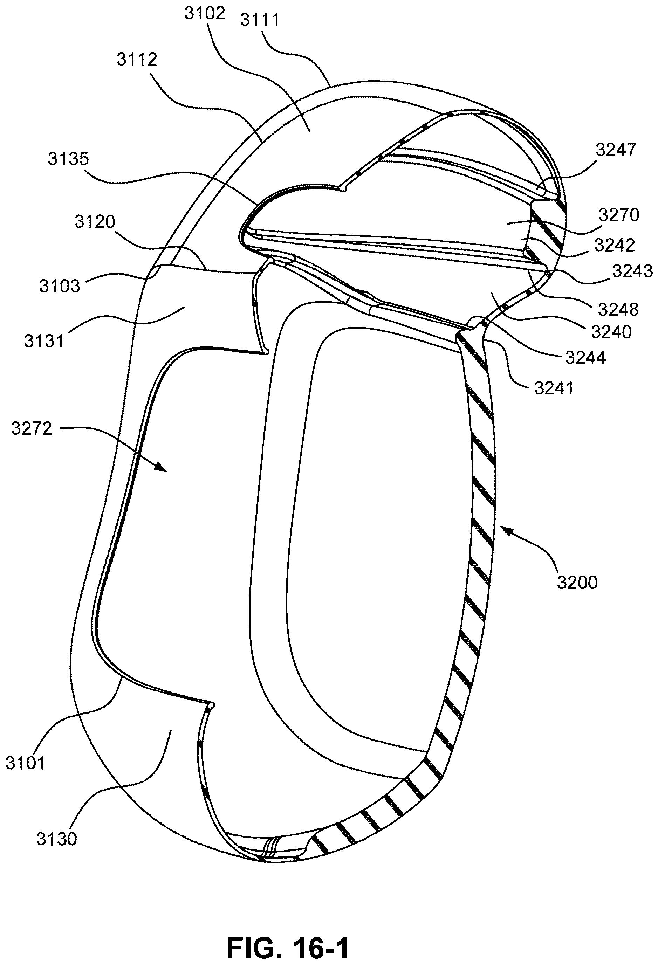

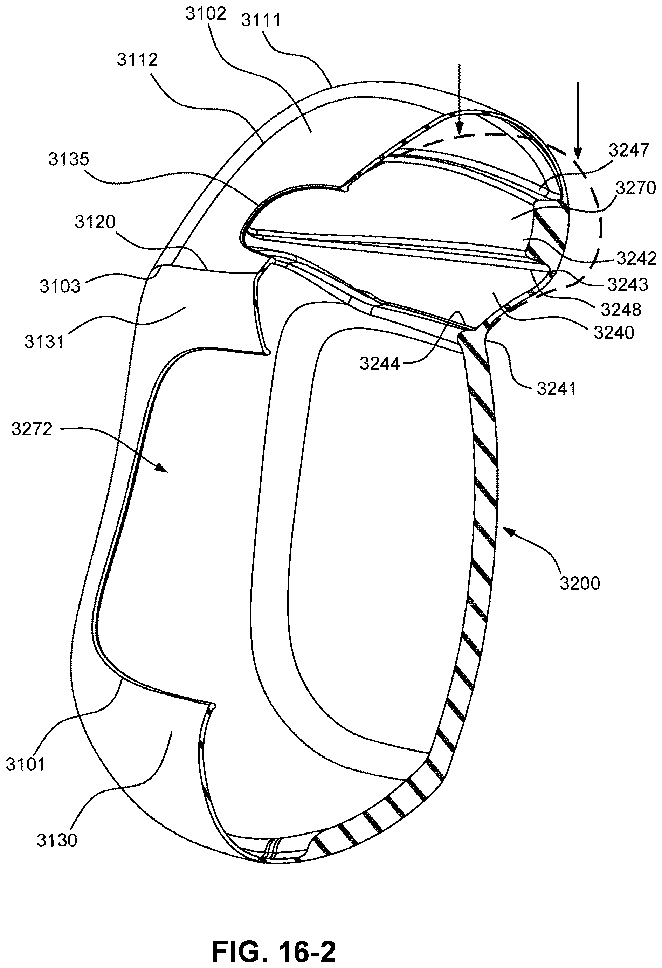

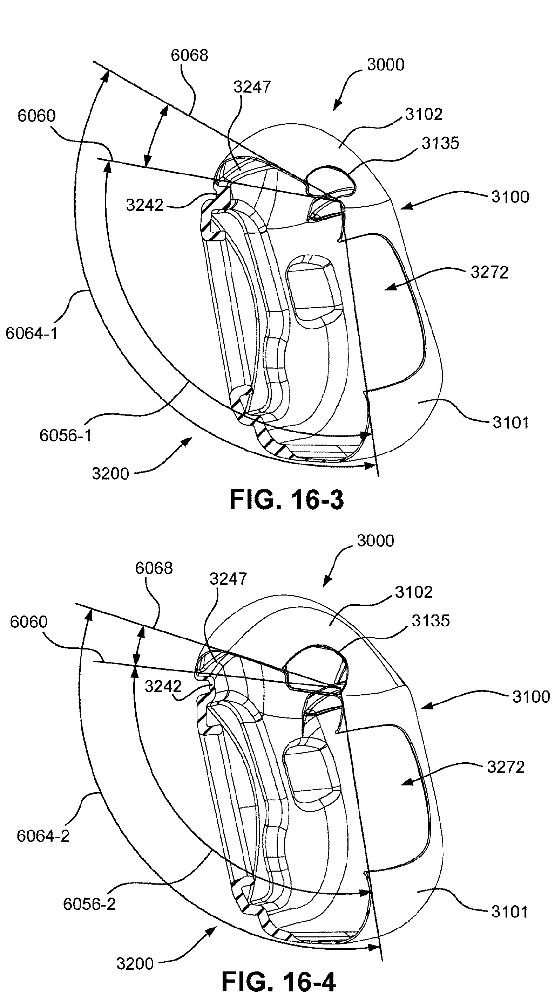

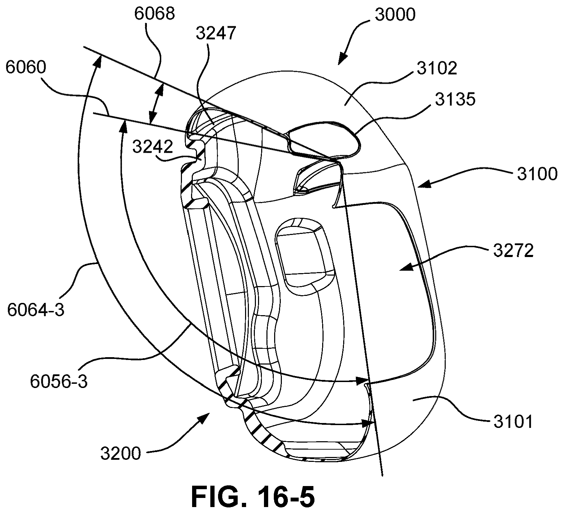

Fig. 16 is a cross-section of the plenum chamber through plane 16-16.
Fig. 16-1 is a cross-section of the plenum chamber through plane 16-1-16-1.
Fig. 16-2 is a cross-section of the plenum chamber of Fig. 16-1, illustrating the plenum chamber in a deformed position.
Fig. 16-3 is a cross-section of an alternate view of the plenum chamber illustrated in Fig. 16, illustrated a first sized cushion.
Fig. 16-4 is a cross-section of an alternate view of the plenum chamber illustrated in Fig. 16, illustrated a second sized cushion.
Fig. 16-5 is a cross-section of an alternate view of the plenum chamber illustrated in Fig. 16, illustrated a third sized cushion.

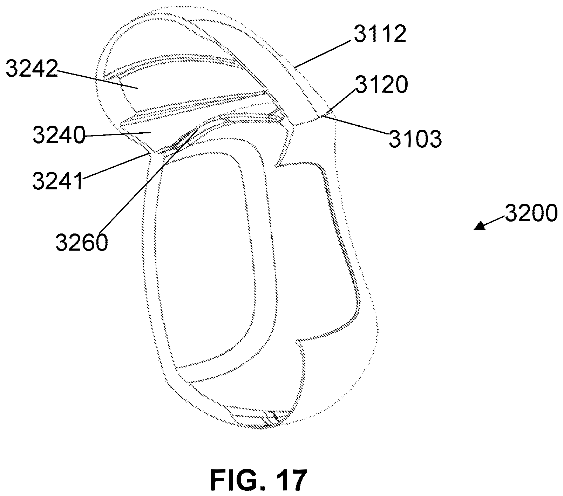

Fig. 17 is a cross-section of the plenum chamber through plane 17-17.

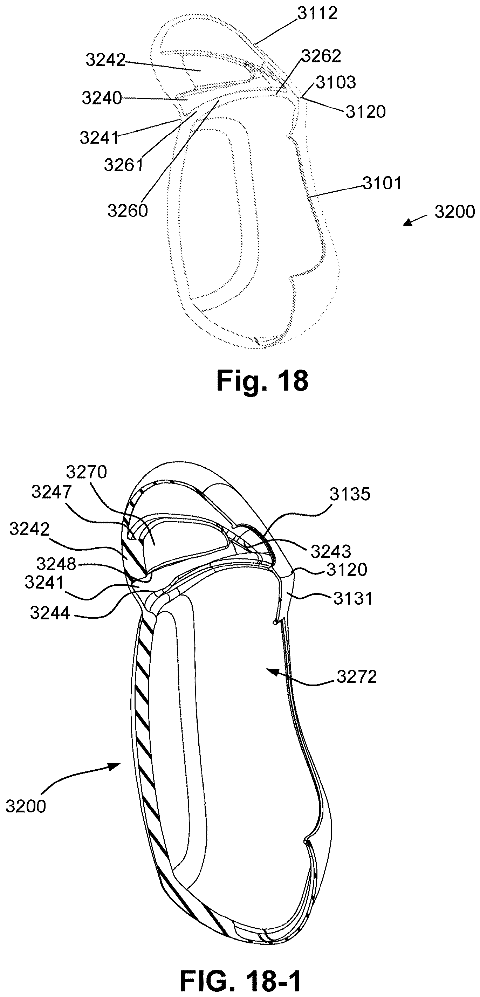

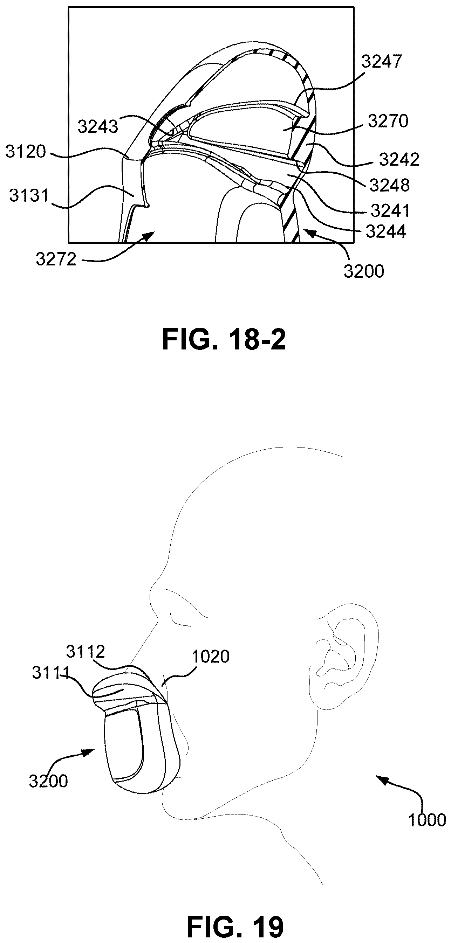

Fig. 18 is a cross-section of the plenum chamber through plane 18-18.
Fig. 18-1 is a cross-section of the plenum chamber through plane 18-1-18-1.
Fig. 18-2 is a cross-section of the plenum chamber through plane 18-2-18-2.
Fig. 19 shows a side view of the plenum chamber in an in-use position on a patient's face, with the plenum chamber shown in outline for clarity.

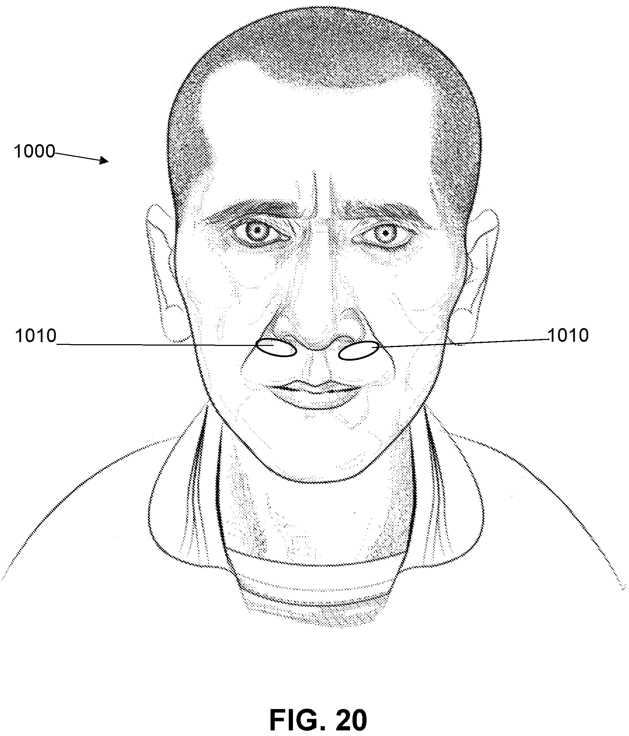

Fig. 20 shows a patient's face with particular areas of engagement by a seal forming structure indicated.

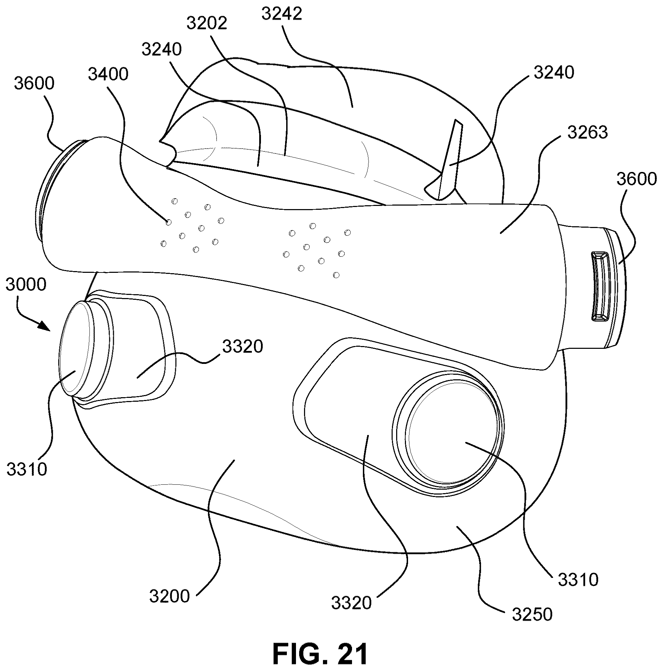

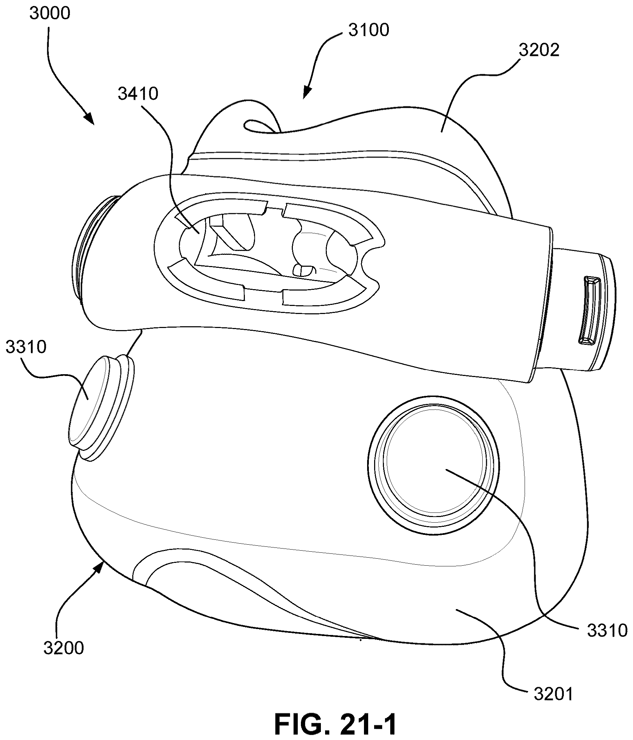

Fig. 21 is a front perspective view of a patient interface in accordance with another form of the technology.
Fig. 21-1 is a front perspective view of a patient interface in accordance with another form of the technology.

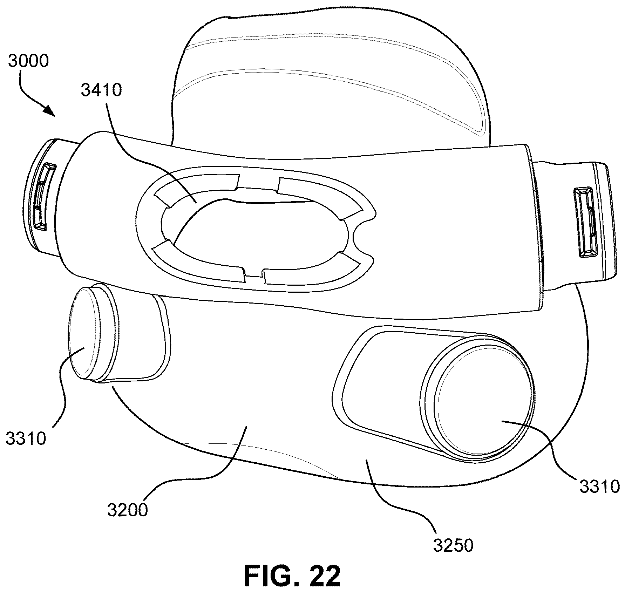

Fig. 22 is a front perspective view of a patient interface in accordance with yet another form of the technology, with a vent removed.

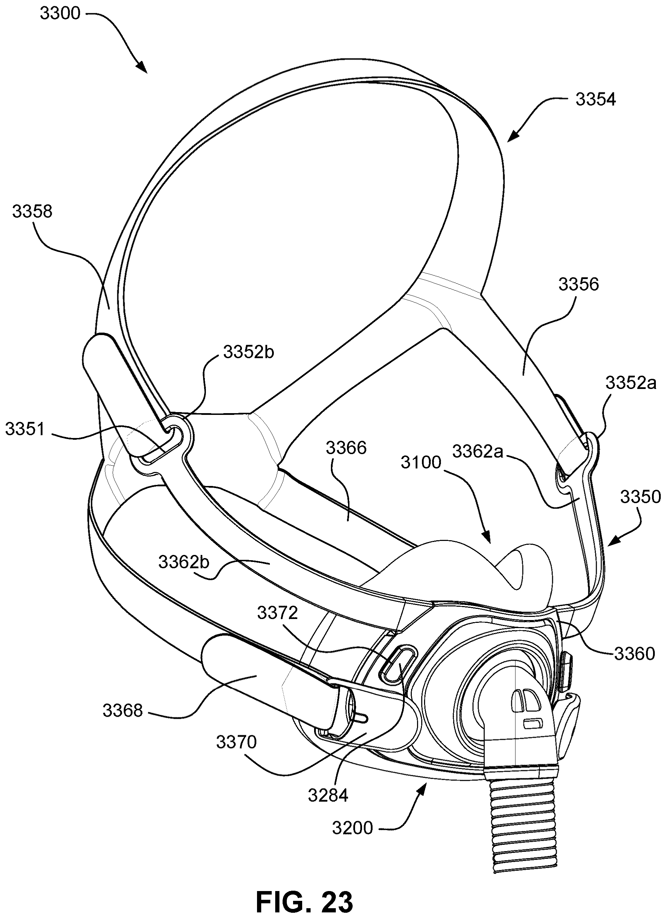

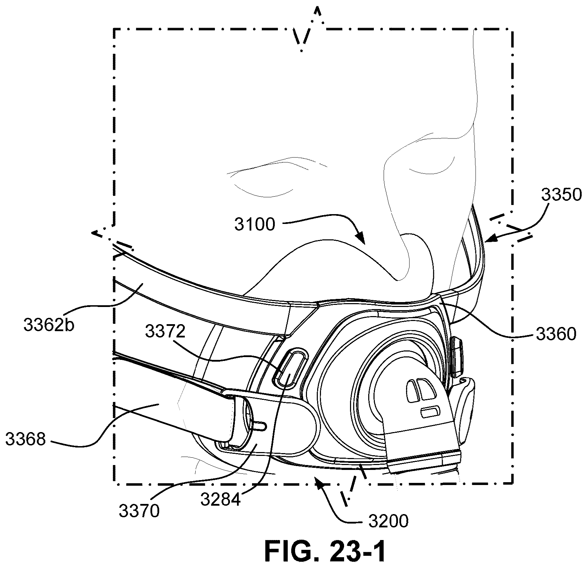

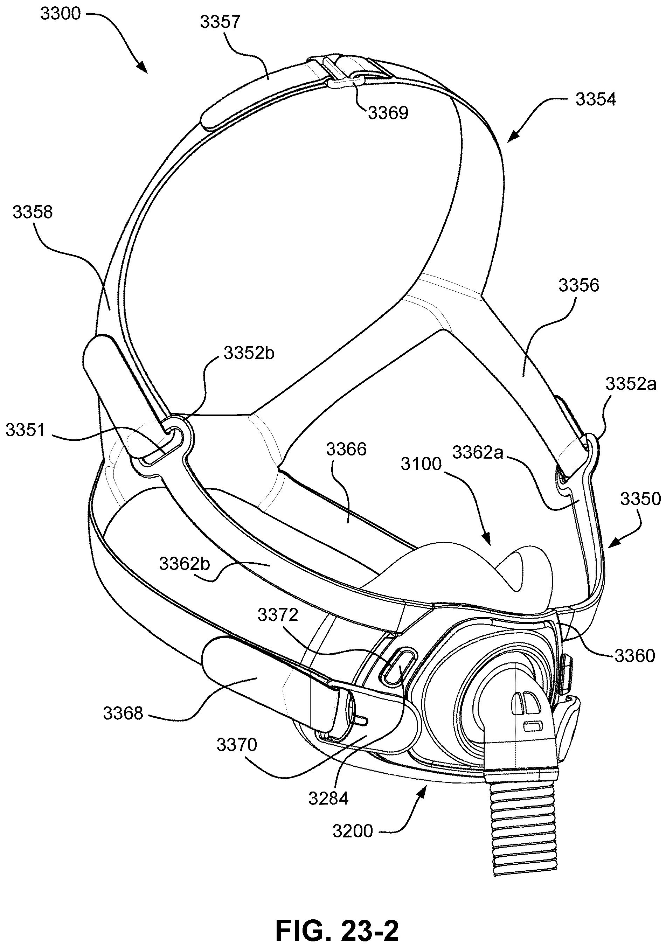

Fig. 23 is a front perspective view of a patient interface having a frame for connecting headgear straps to the plenum chamber.
Fig. 23-1 is a front perspective view of a patient wearing the patient interface of Fig. 23.
Fig. 23-2 is a front perspective view of an alternate version of a patient interface having a frame for connecting headgear straps to the plenum chamber.

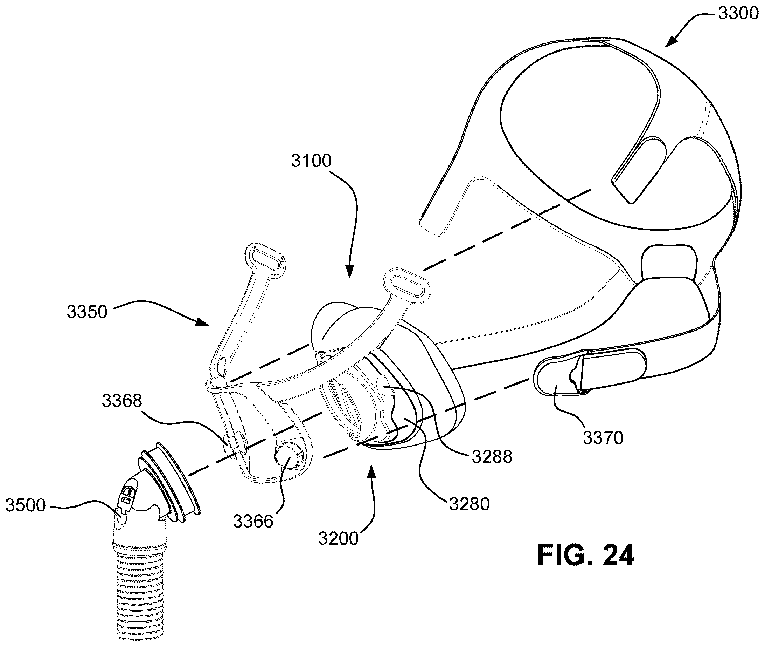

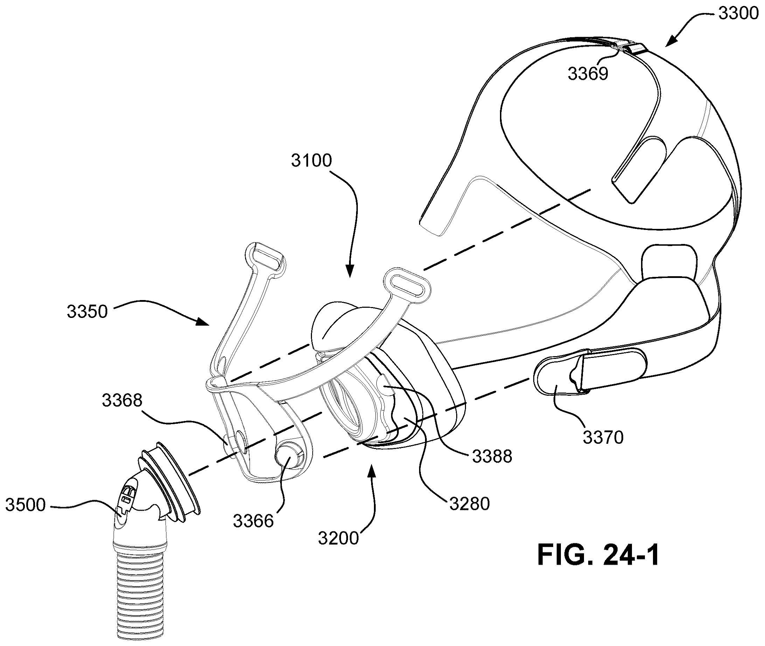

Fig. 24 is an exploded view of the patient interface of Fig. 23.
Fig. 24-1 is an exploded view of the patient interface of Fig. 23-2.

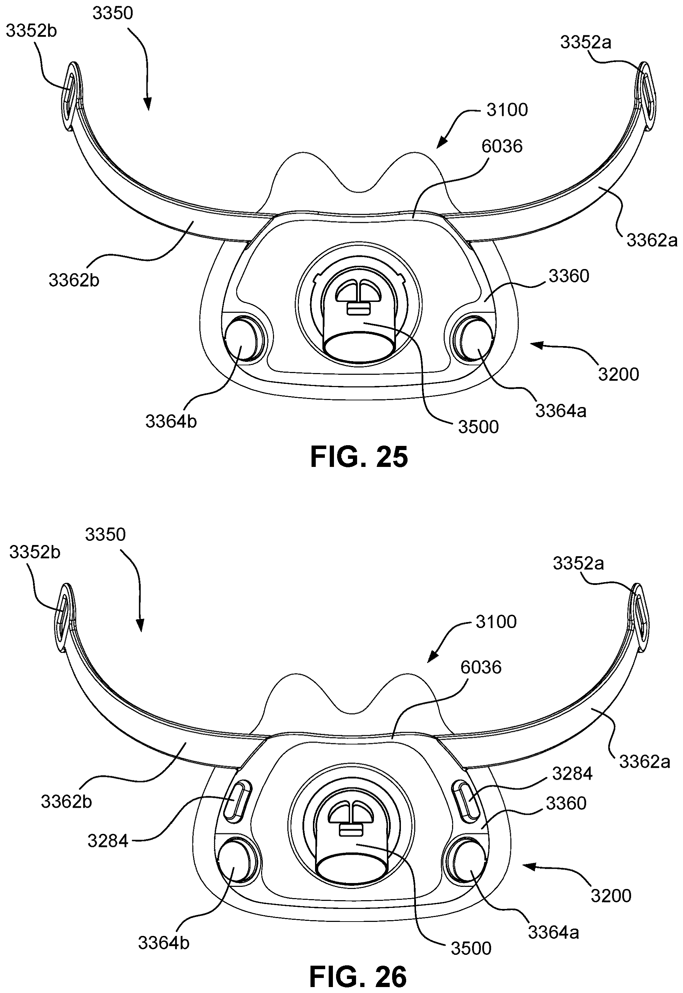

Fig. 25 is a front view of a frame and plenum chamber in accordance with one form of the technology.

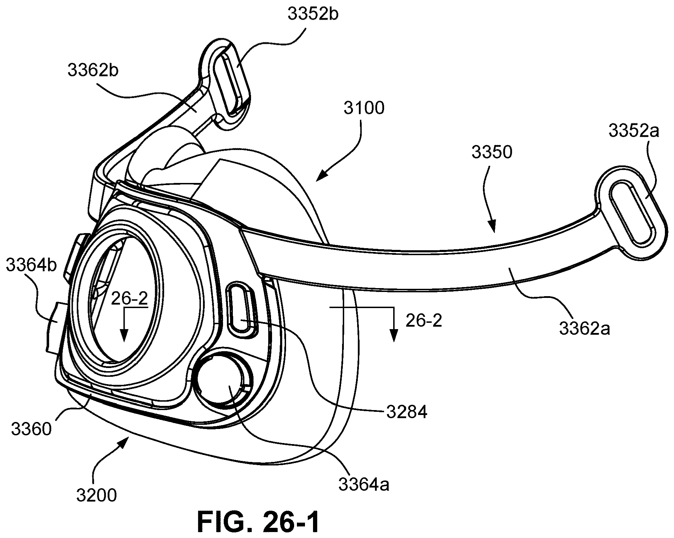

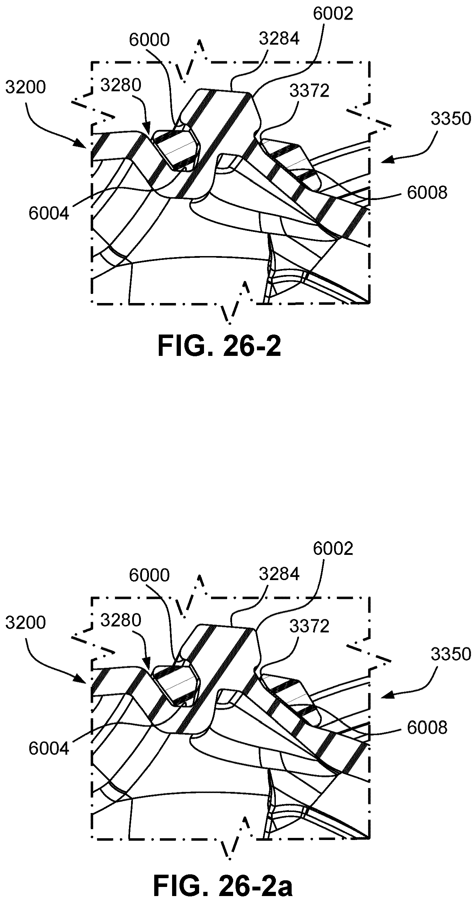

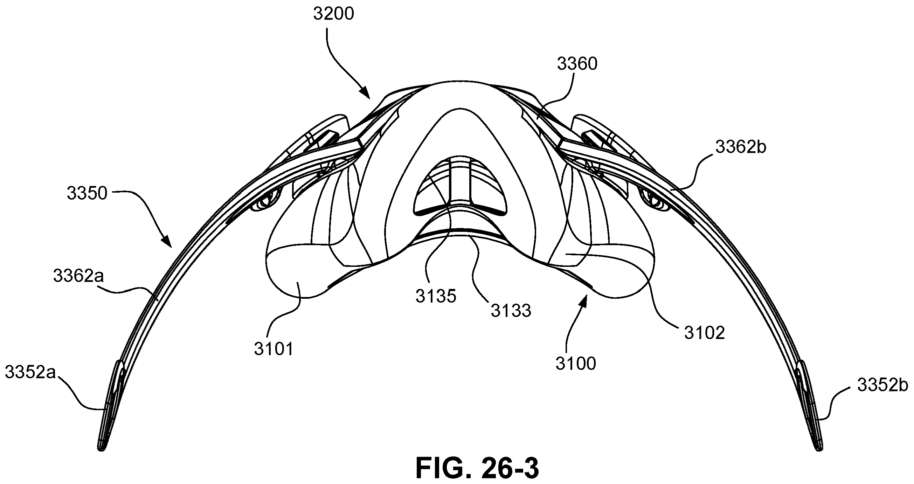

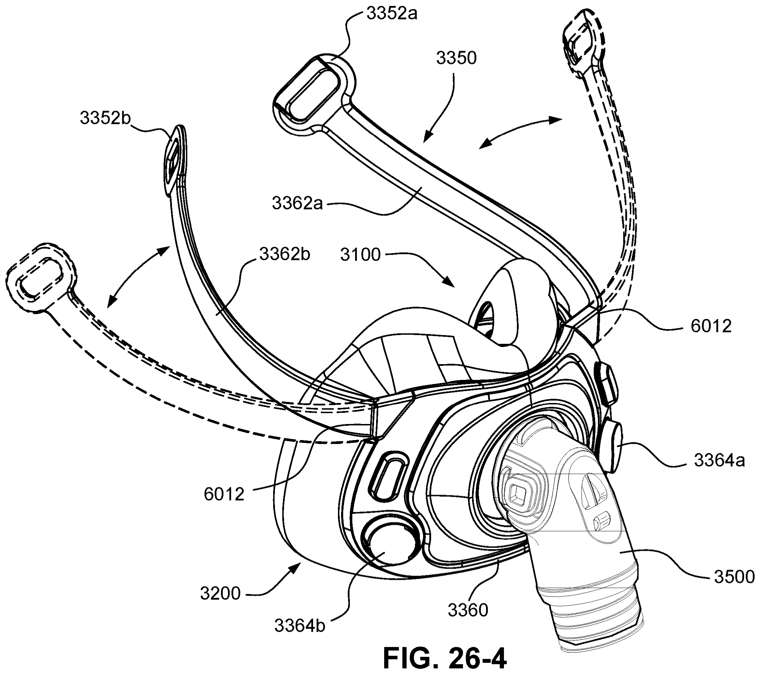

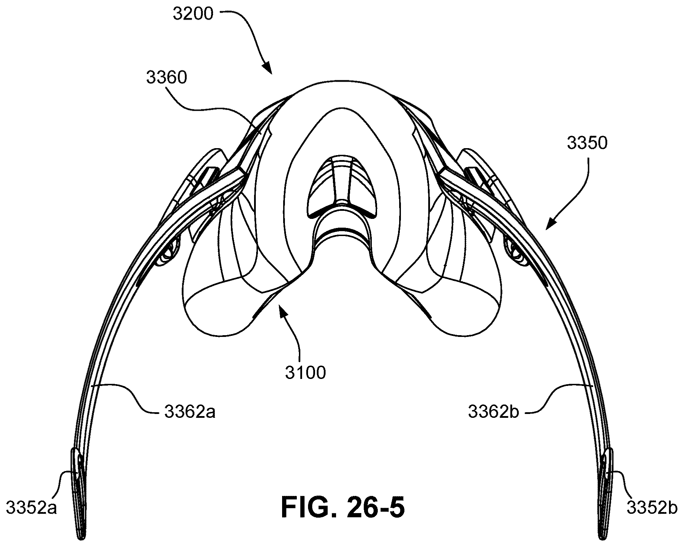

Fig. 26 is a front view of a frame and plenum chamber in accordance with another form of the technology.
Fig. 26-1 is a side perspective view of the frame and plenum chamber of Fig. 26.
Fig. 26-2 is a cross-sectional view of the frame and plenum chamber of Fig. 26-1, viewed along section 26--26.
Fig. 26-2a is a cross-sectional view of an alternate example of the frame and plenum chamber of Fig. 26-1, viewed along section 26--26.
Fig. 26-3 is a top view of the frame and plenum chamber of Fig. 26.
Fig. 26-4 is a perspective view of the frame and plenum chamber of Fig. 26, illustrating arms of the frame moveable between a first position and a second position.
Fig. 26-5 is a rear perspective view of the frame and plenum chamber of Fig. 26-4, illustrating the flexion of the plenum chamber in the second position.

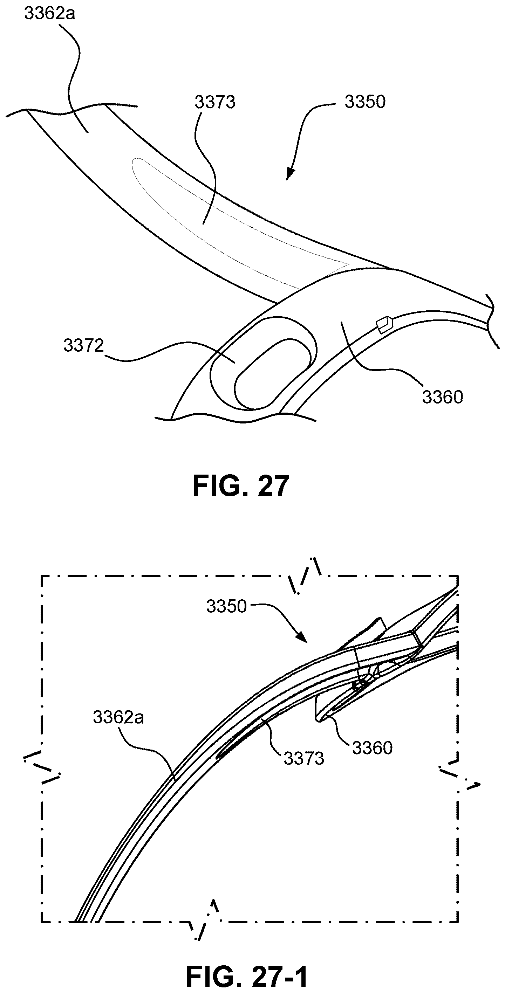

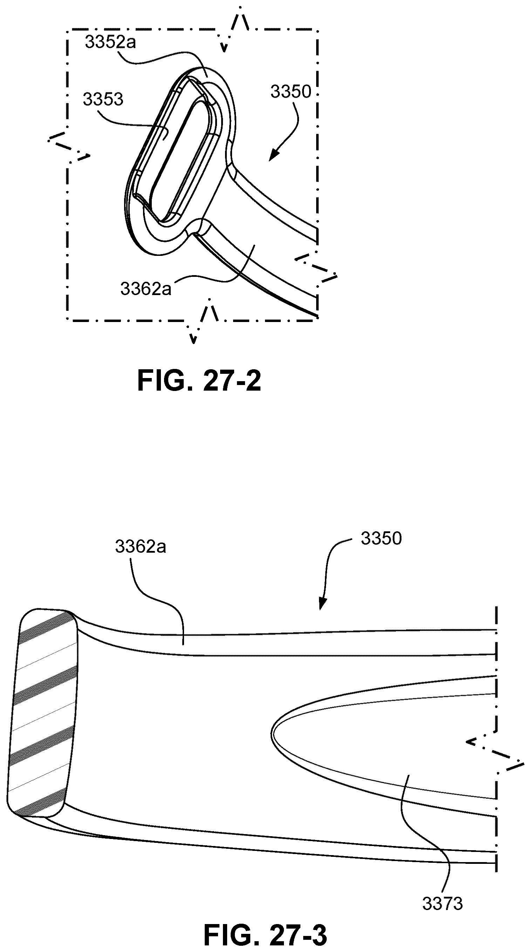

Fig. 27 is a rear view of the frame of Fig. 26, illustrating a tapered opening.
Fig. 27-1 is a top perspective view of the frame of Fig. 26, illustrating a decreasing thickness of an arm of the frame.
Fig. 27-2 is a perspective view of the frame of Fig. 26, illustrating a loop on a free end of the frame.
Fig. 27-3 is a perspective view of the frame of Fig. 26, illustrating a cross-section of the arm of the frame.

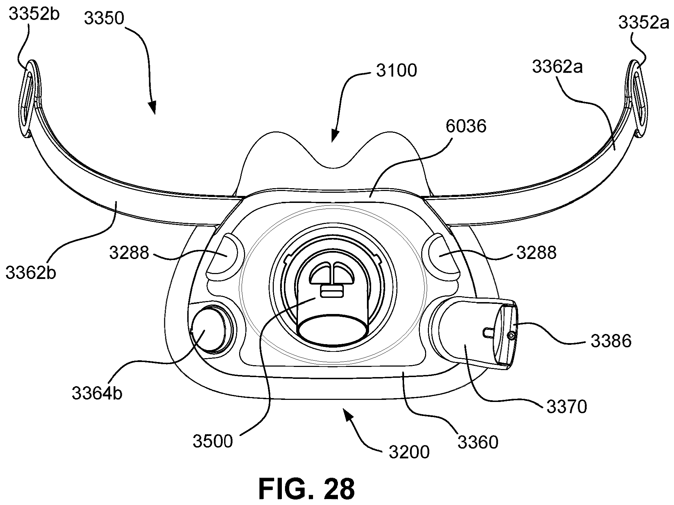

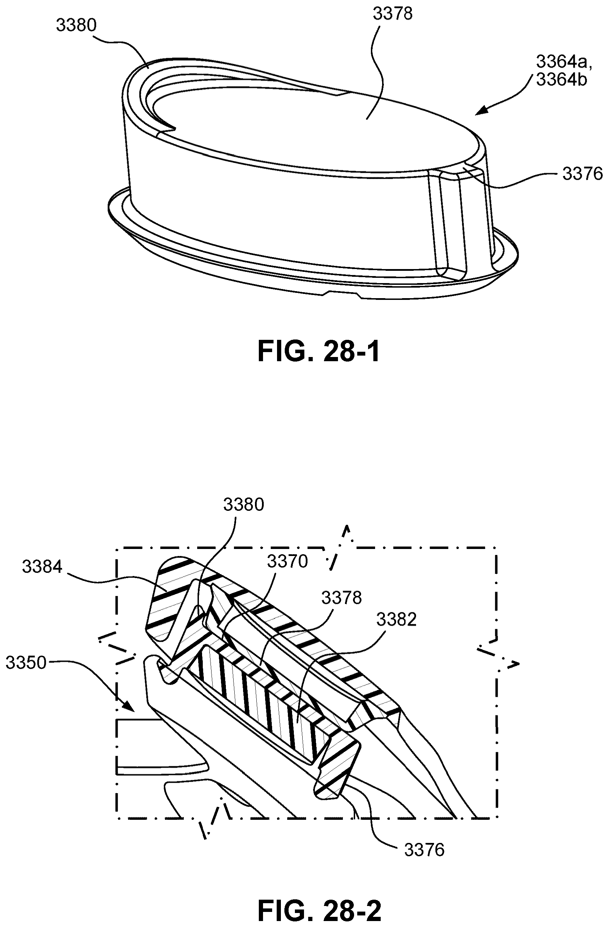

Fig. 28 is a front view of a frame and plenum chamber in accordance with yet another form of the technology.
Fig. 28-1 is a perspective view of a secondary connection point usable with the frame in one form of the technology.
Fig. 28-2 is a cross-sectional view of the frame and plenum chamber of Fig. 28, illustrating the connection between a magnet and a secondary connection point of Fig. 28-1.

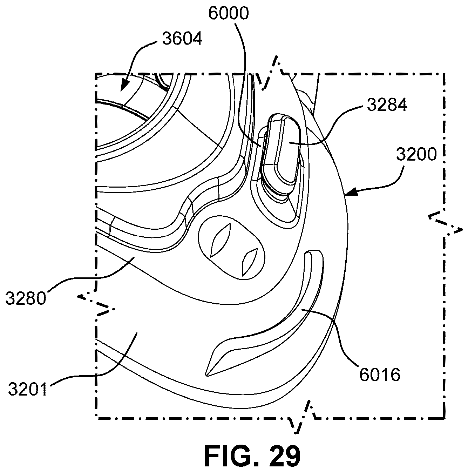

Fig. 29 is a detail view of a plenum chamber in accordance with yet another form of the technology, illustrating a clip support configured to selectively contact the frame.
Fig. 30 is a bottom view of the plenum chamber of Fig. 29 connected to a frame, which is in contact with the clip support.
Fig. 31 is a front view of a plenum chamber in accordance with yet another form of the technology connected to a frame, which includes a clip support.

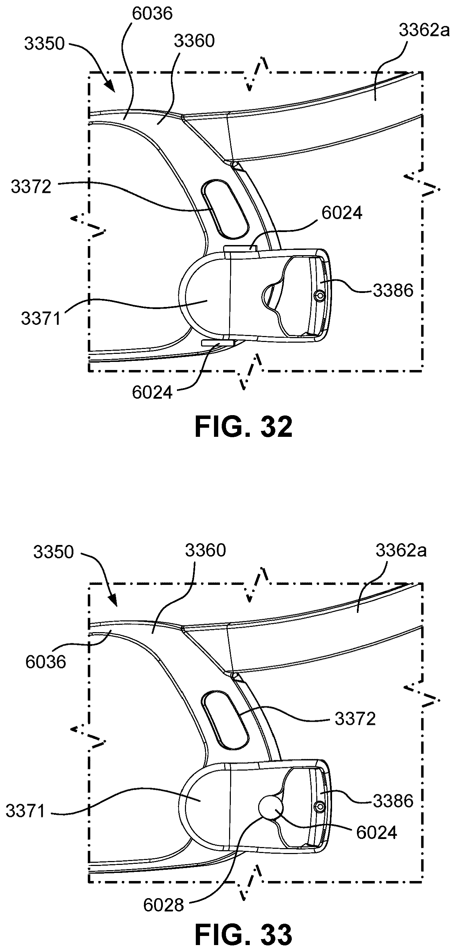

Fig. 32 is a front view of a frame in accordance with yet another form of the technology, illustrating a rib for limiting movement of the connection member.
Fig. 33 is a front view of a frame in accordance with yet another form of the technology, illustrating a pair of ribs for limiting movement of the connection member.

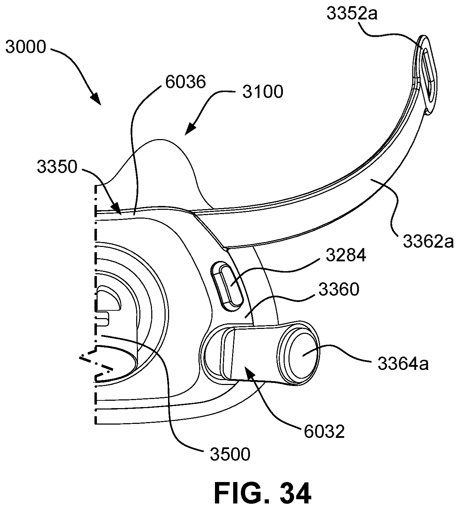

Fig. 34 is a front view of a frame in accordance with yet another form of the technology, illustrating an arm having a connection point.

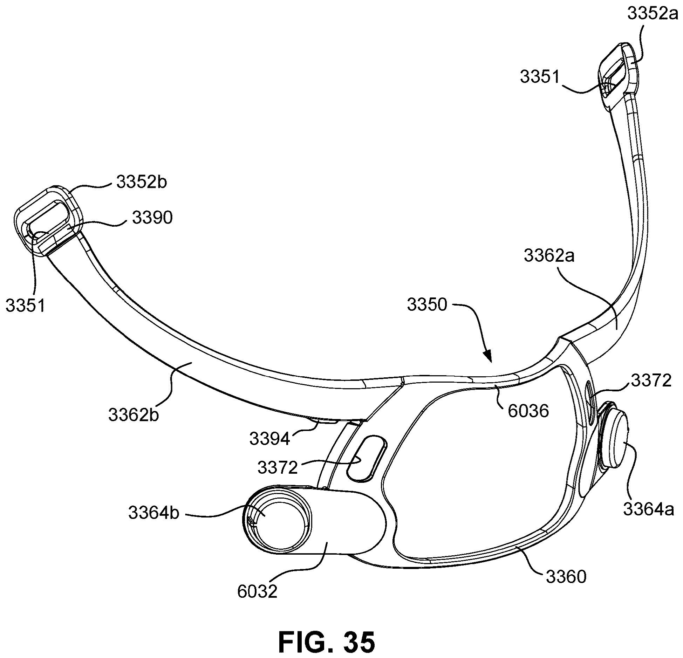

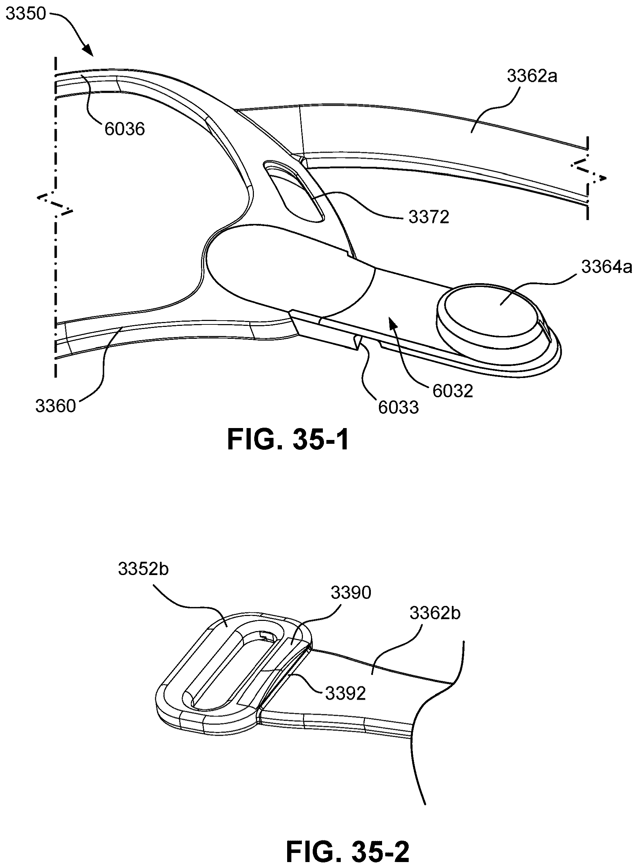

Fig. 35 is a perspective view of a frame in accordance with yet another form of the technology, illustrating a raised portion proximate to the eyelet.
Fig. 35-1 is a side perspective view of the frame in accordance to another example.
Fig. 35-2 is a detail view of the eyelet of Fig. 35.

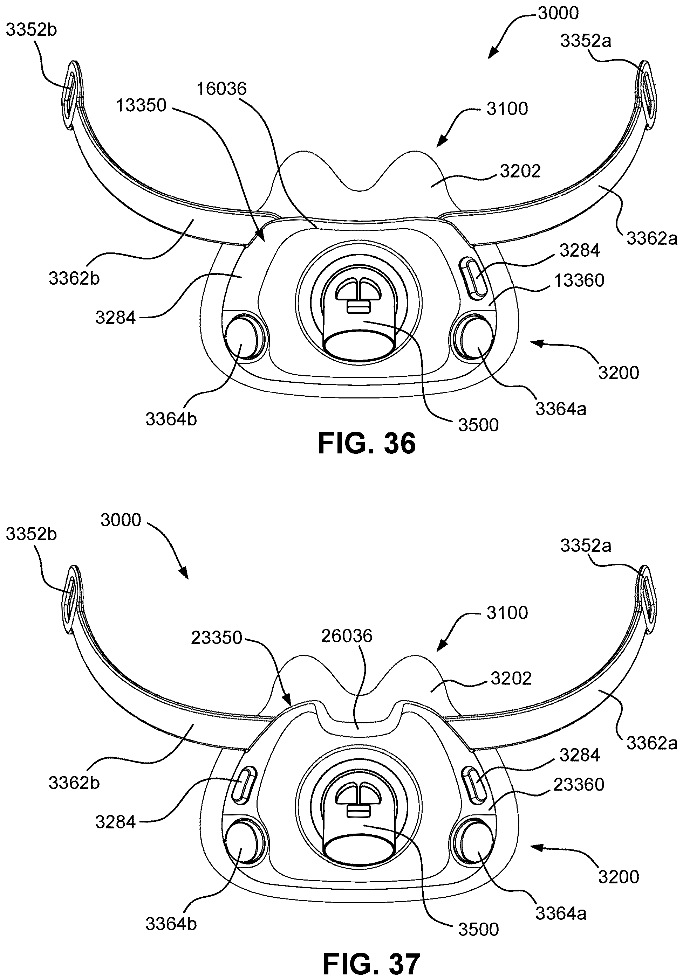

Fig. 36 is a front view of a frame in accordance with yet another form of the technology, illustrating a superior bar of the frame connected to the plenum chamber closer to the elbow.
Fig. 37 is a front view of a frame in accordance with yet another form of the technology, illustrating a superior bar of the frame spaced apart from the plenum chamber.

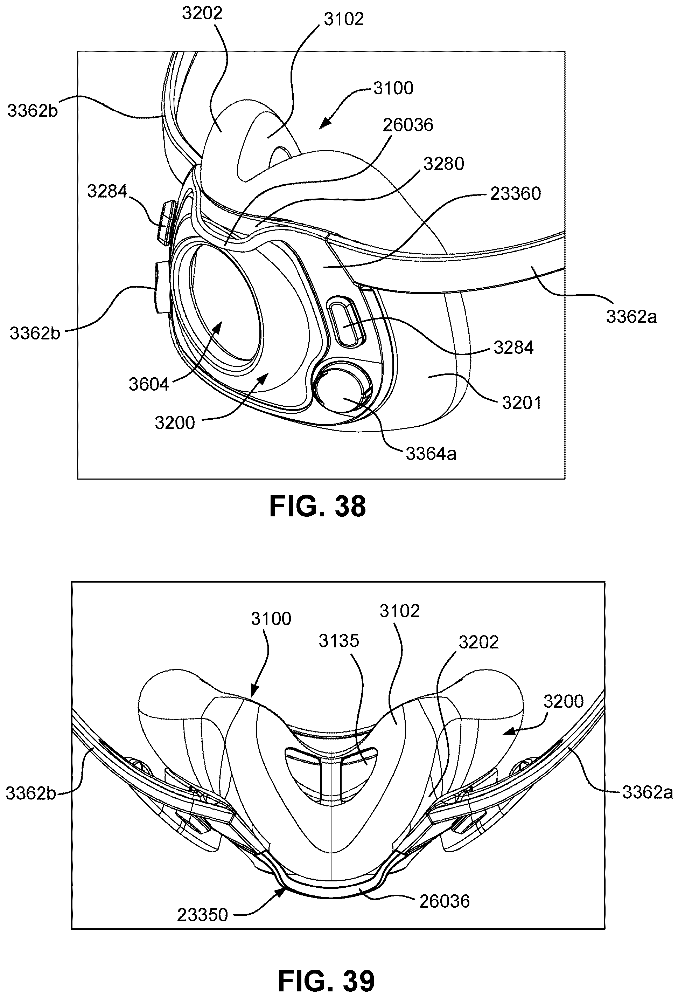

Fig. 38 is a perspective view of the frame of Fig. 37.
Fig. 39 is a top view of the frame of Fig. 37.

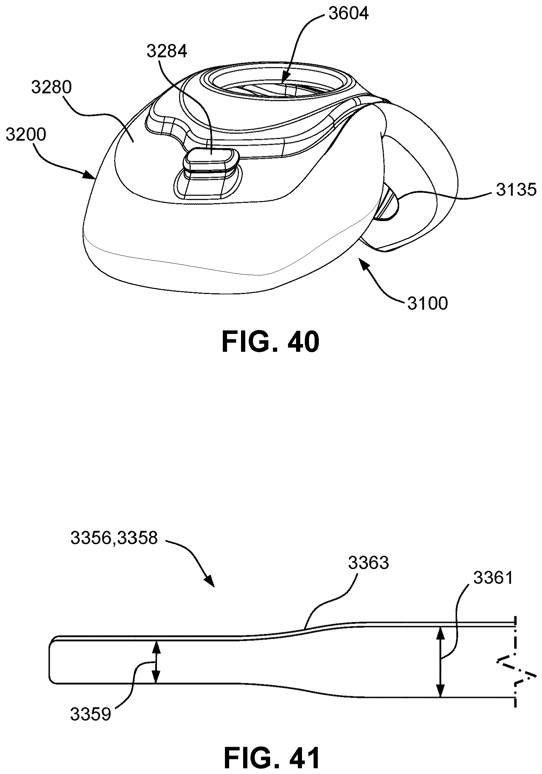

Fig. 40 is a side perspective view of a cushion in accordance with yet another from of the technology, illustrating the projection extending from the anterior surface of the plenum chamber.

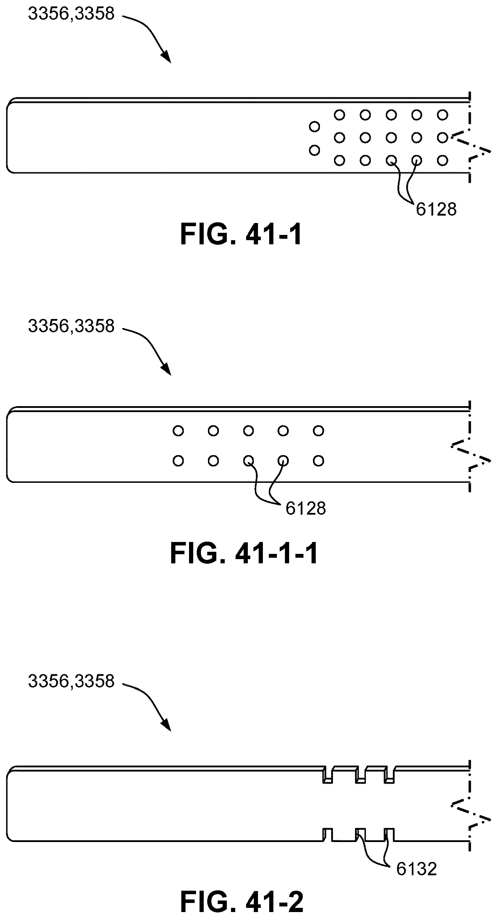

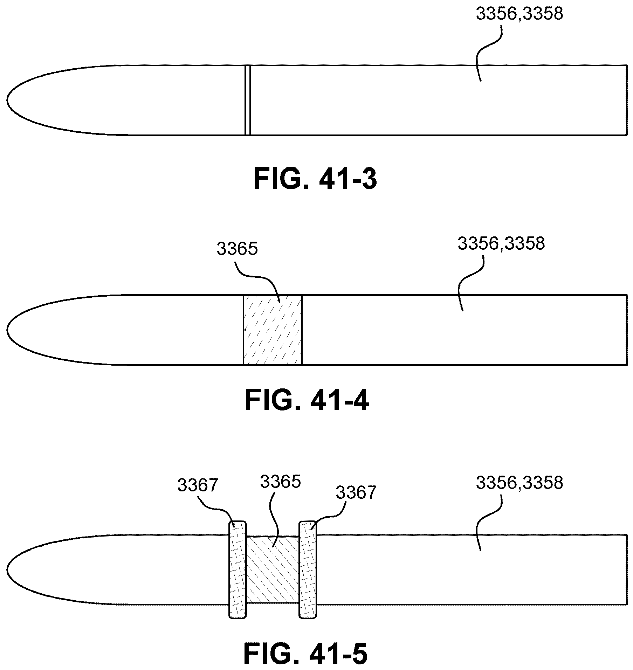

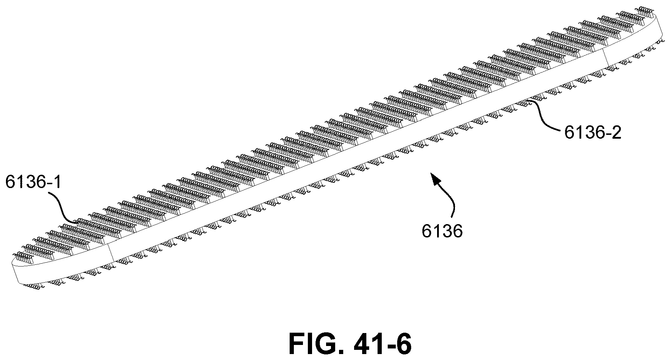

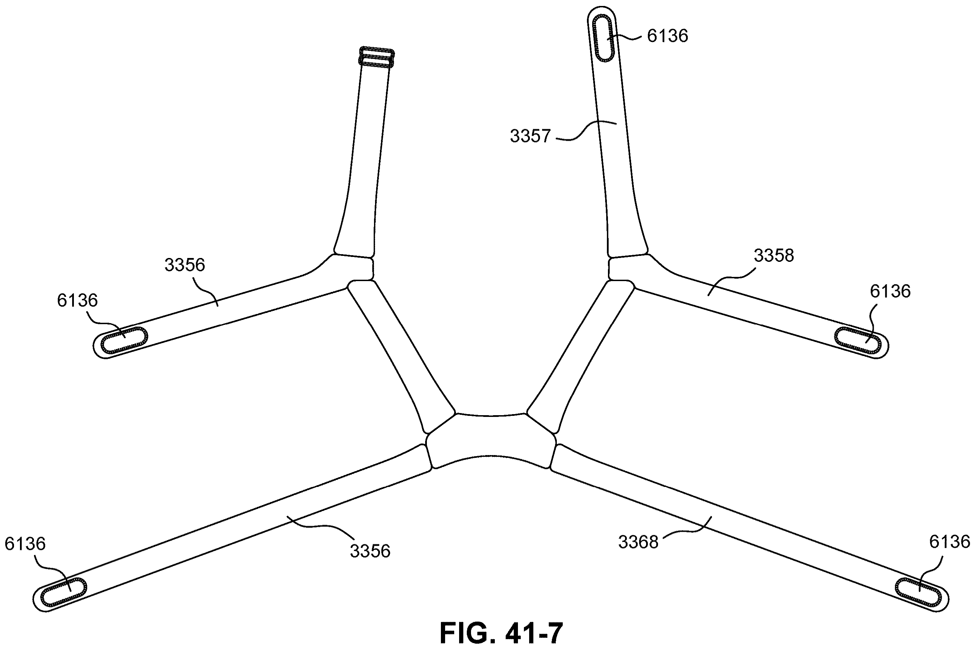

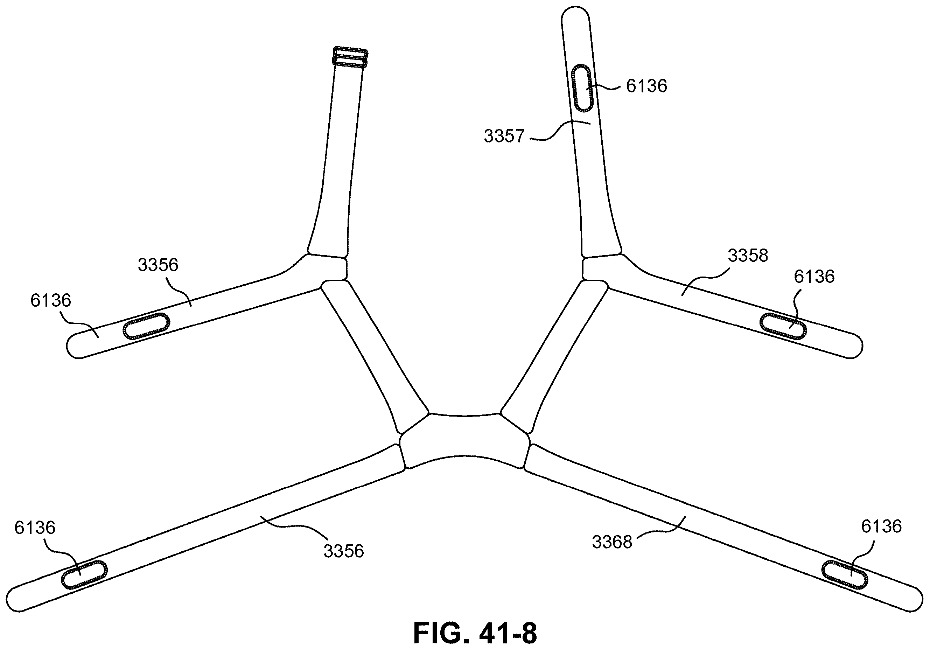

Fig. 41 is a detail view of a strap of a positioning and stabilizing structure usable with the patient interface of Fig. 23, the strap having a first width and a second width.
Fig. 41-1 is a detail view of an alternate example of a strap of a positioning and stabilizing structure usable with the patient interface of Fig. 23, the strap having raised portions.

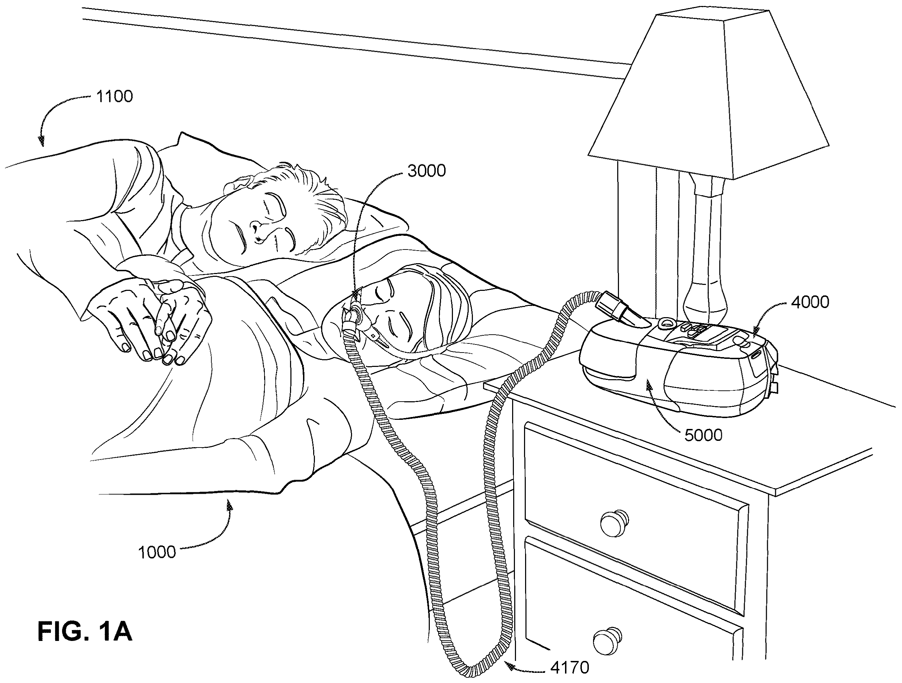

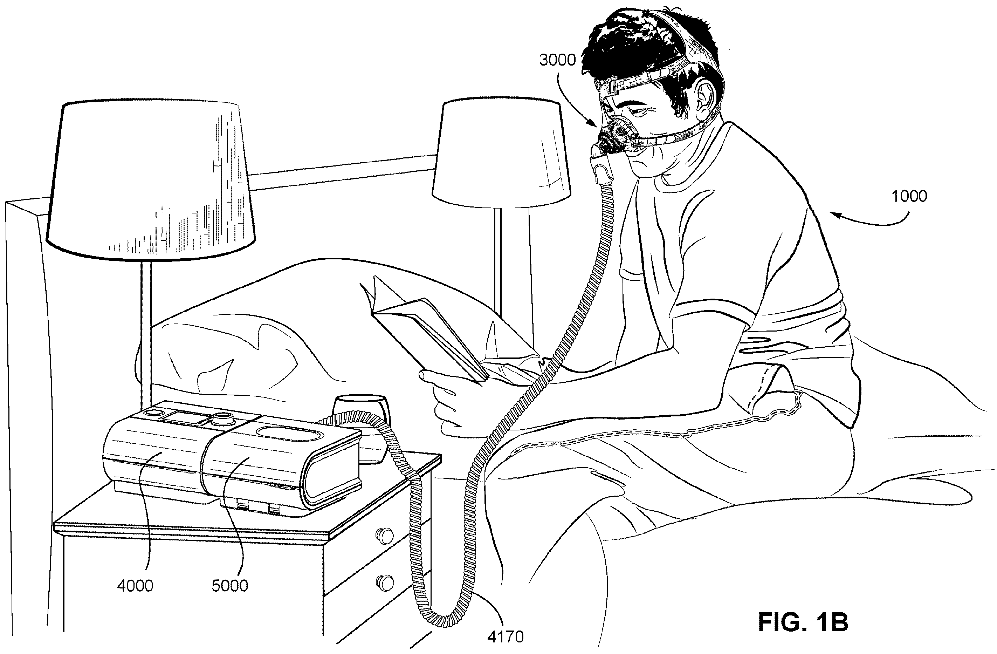

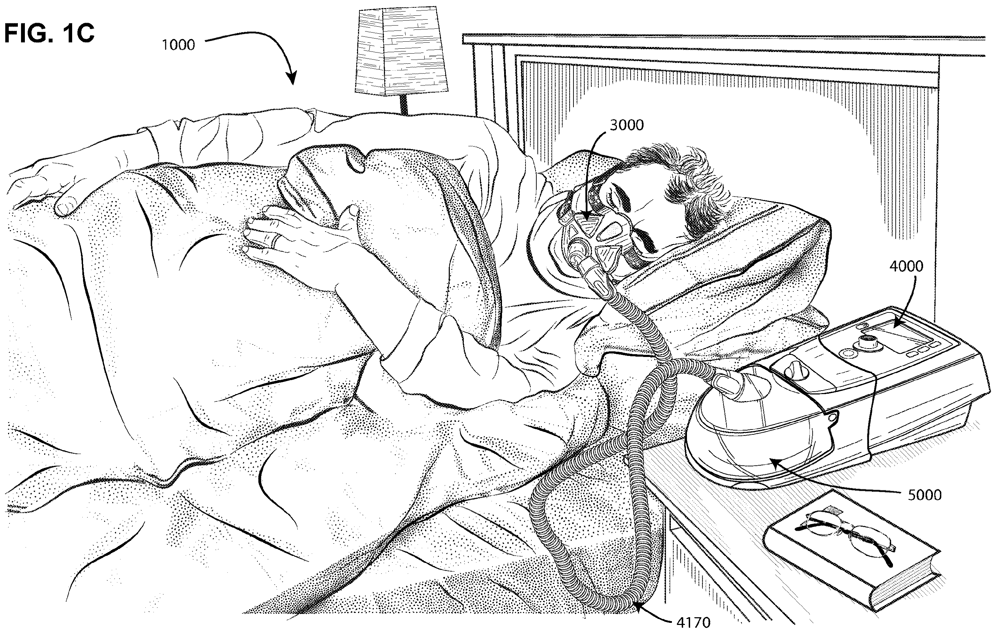

Fig. 41-1-1 is a detail view of an alternate example of a strap of a positioning and stabilising structure with the patient interface of Fig. 23, the strap having raised portions in an alternate configuration from the raised portions of Fig. 41-1.
Fig. 41-2 is a detail view of an alternate example of a strap of a positioning and stabilizing structure usable with the patient interface of Fig. 23, the strap having cut edges.
Fig. 41-3 is a detail view of an alternate example of a strap of a positioning and stabilizing structure usable with the patient interface of Fig. 23, the strap being positioned in a first position.
Fig. 41-4 is a detail view of the strap of Fig. 41-3 illustrating the strap in a second, extended position where an indicator becomes visible.
Fig. 41-5 is a detail view of an alternate example of a strap of a positioning and stabilizing structure usable with the patient interface of Fig. 23, the strap being positioned in a second, extended position where an indicator becomes visible.
Fig. 41-6 is a perspective view of a double-sided connecting member having hook or loop material.
Fig. 41-7 is a rear view of the double-sided connecting member of Fig. 41-6 connected to headgear in a first position.
Fig. 41-8 is a rear view of the double-sided connecting member of Fig. 41-6 connected to headgear in a second position.

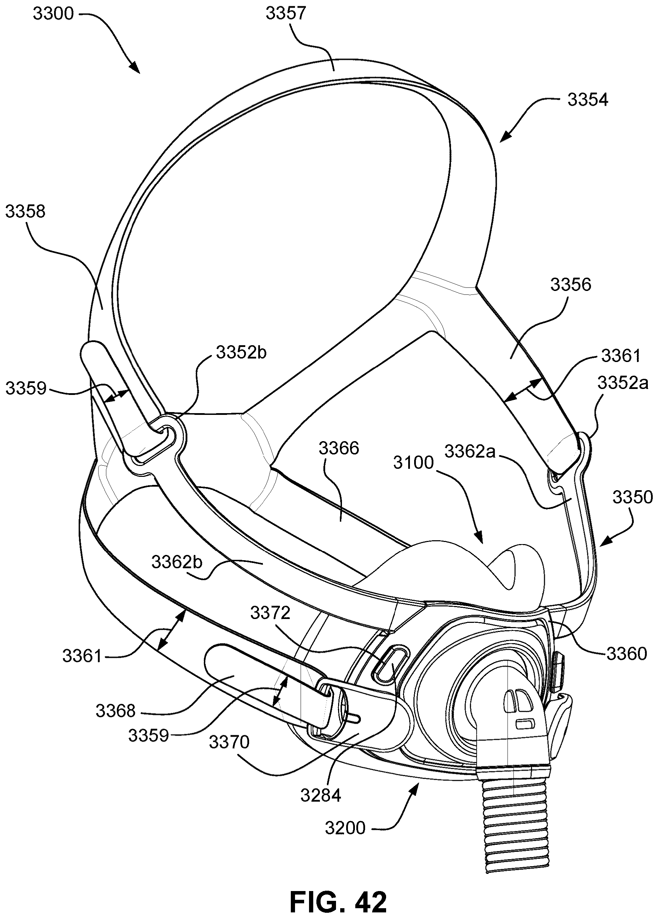

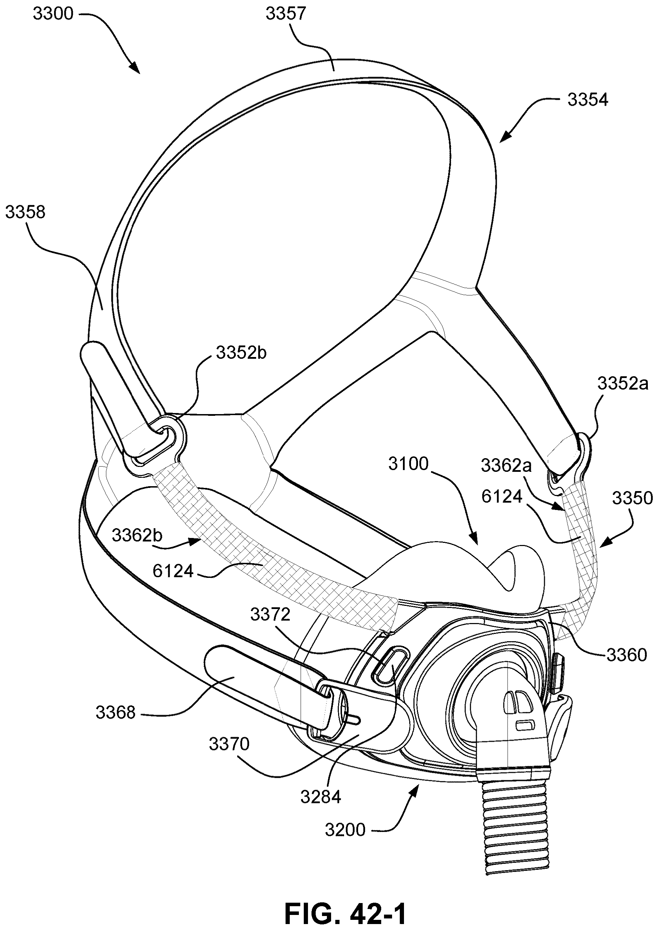

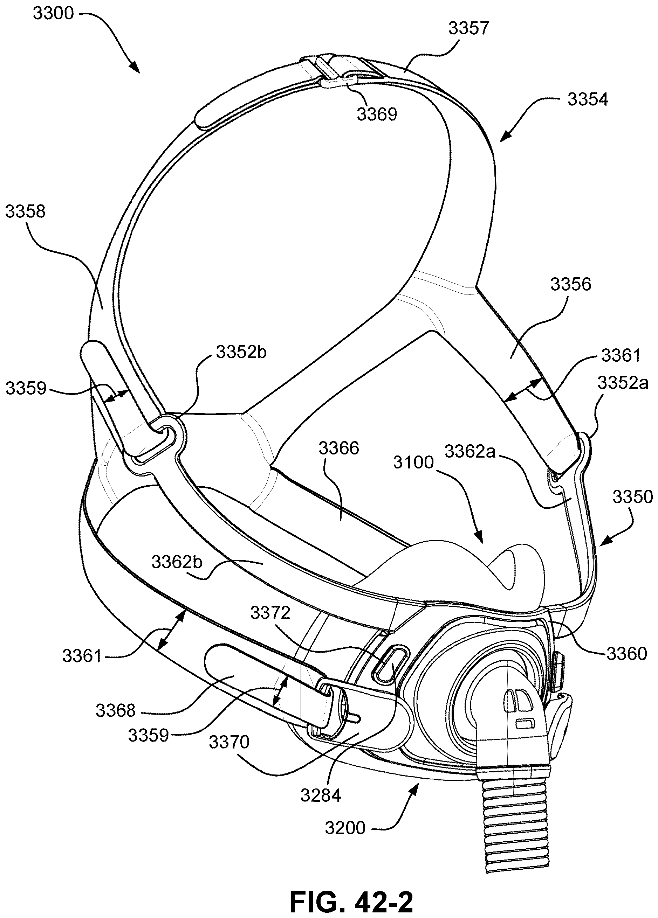

Fig. 42 is a front perspective view of a patient interface of Fig. 23 using the straps of Fig. 41.
Fig. 42-1 is a front perspective view illustrating an alternate version of Fig. 42 showing the patient interface with sleeves on the arms of the frame.
Fig. 42-2 is a front perspective view of a patient interface of Fig. 23 using an alternate version of the straps.

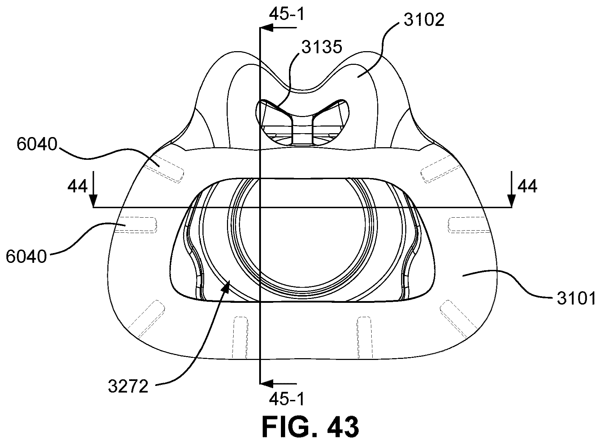

Fig. 43 is a rear view of an alternate form of a cushion, illustrating the stopper ribs in hidden lines.

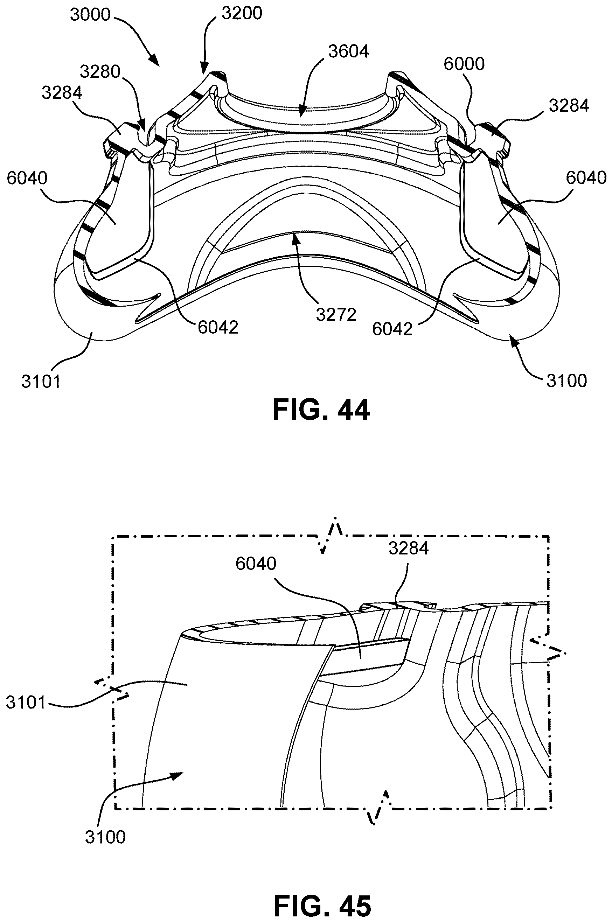

Fig. 44 is a cross-sectional view of the cushion of Fig. 43 viewed along line 44--44, illustrating a shape of the stopper ribs.

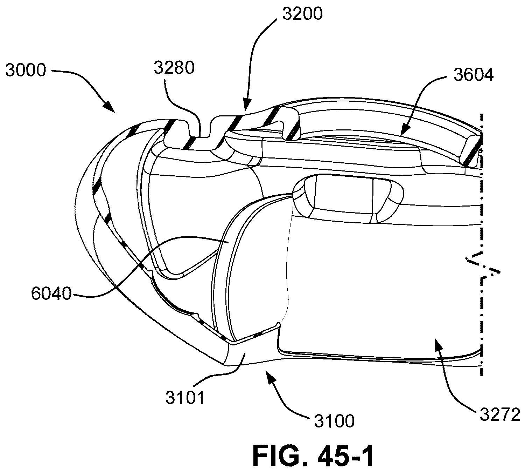

Fig. 45 is a side view of the cross-sectional view of Fig. 44.
Fig. 45-1 is a cross-sectional view of the cushion of Fig. 43 viewed along line 45-1--45-1, illustrating a shape of the stopper ribs.

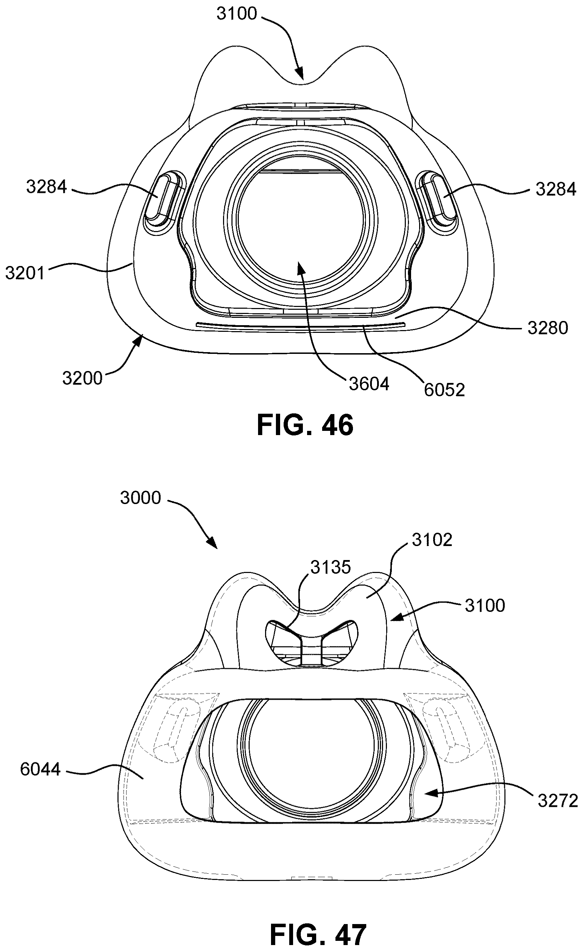

Fig. 46 is a front view of the plenum chamber of Fig. 26, illustrating the groove for receiving the frame.
Fig. 47 is a rear view of a cushion in accordance with yet another form of the present technology illustrating a stiffened region in hidden lines.
Fig. 47-1 is a cross-section of a cushion from the Fig. 47 illustrating the stiffened region.

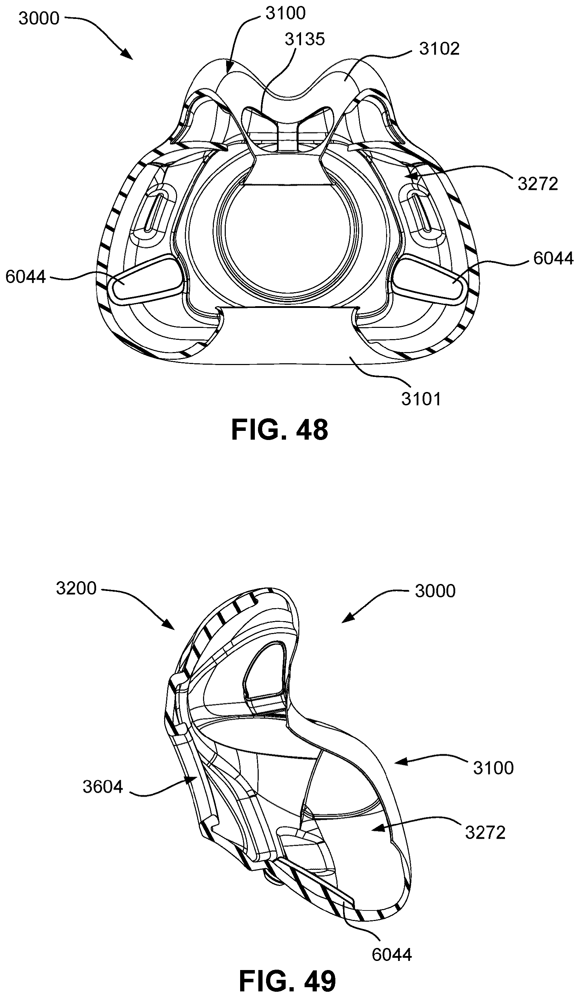

Fig. 48 is a cross-section of a cushion in accordance with yet another form of the present technology illustrating an alternate form of a stiffened region.
Fig. 49 is a cross-sectional view of the cushion of Fig. 48.

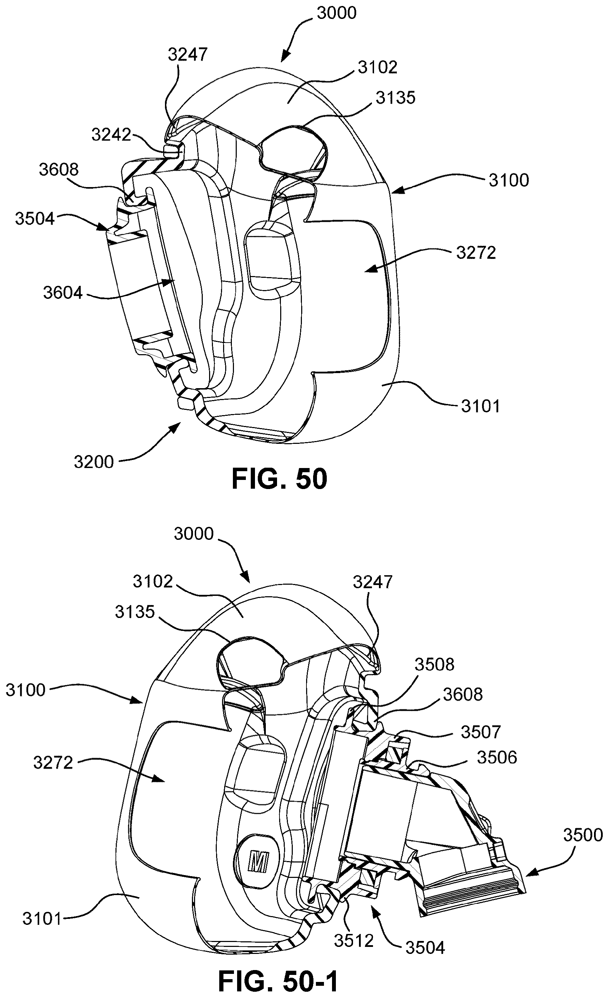

Fig. 50 is a cross-sectional view of a cushion in accordance with yet another form of the present technology illustrating spacing between the seal-forming structure and the anterior surface of the plenum chamber.
Fig. 50-1 is a cross-sectional view of a cushion in accordance with yet another form of the present technology illustrating a vent ring with an offset to create spacing between the seal-forming structure and the elbow.

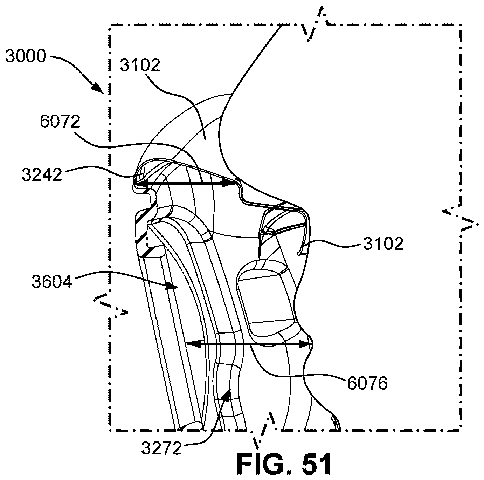

Fig. 51 is a cross-sectional view illustrating the cushion of Fig. 50 being worn by a patient.

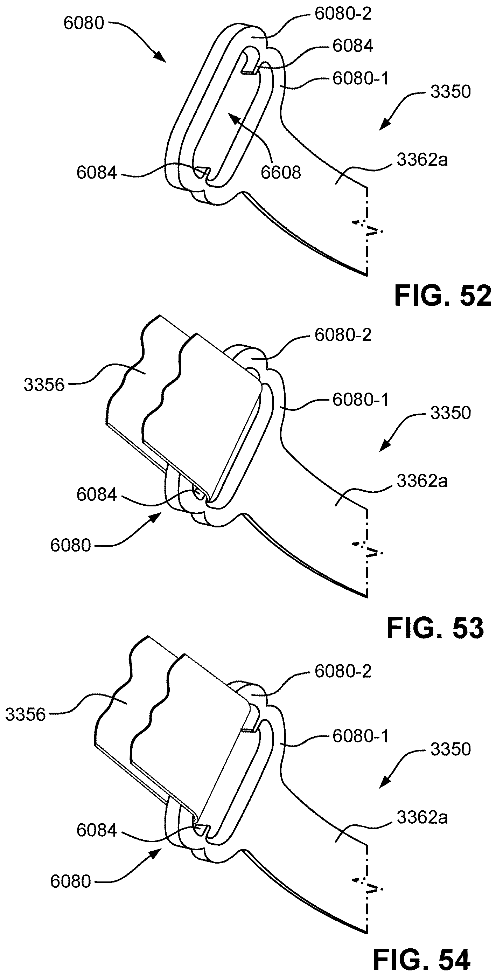

Fig. 52 is a perspective view of an arm of a frame according to another example, the frame including a first loop portion and a second loop portion.
Fig. 53 is a perspective view of the arm of Fig. 52, illustrating a headgear strap connected to the first loop portion under an optimal amount of tension.
Fig. 54 is a perspective view of the 52, illustrating a headgear strap connected to the second loop portion under an amount of tension that exceeds the optimal amount of tension.

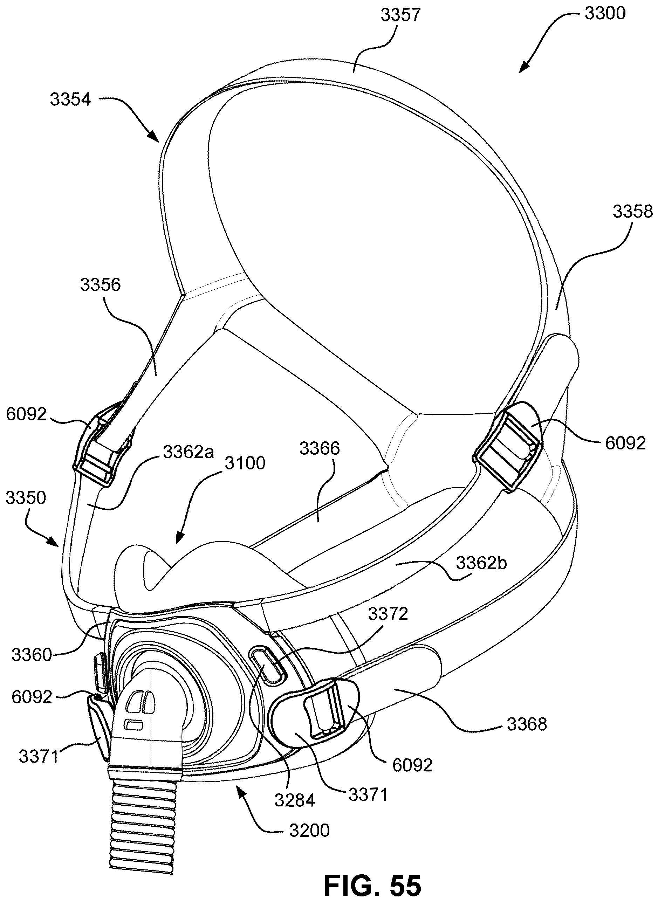

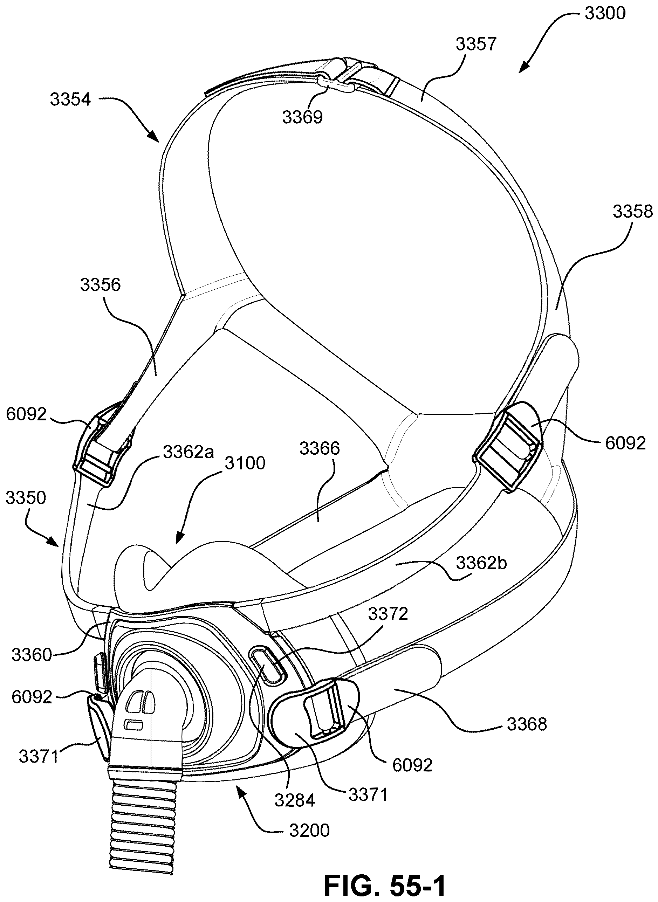

Fig. 55 is a perspective view of a patient interface according to another example, illustrating a frame with ladder locks connected to headgear straps.
Fig. 55-1 is a perspective view of a patient interface according to yet another example, illustrating a frame with ladder locks connected to headgear straps.

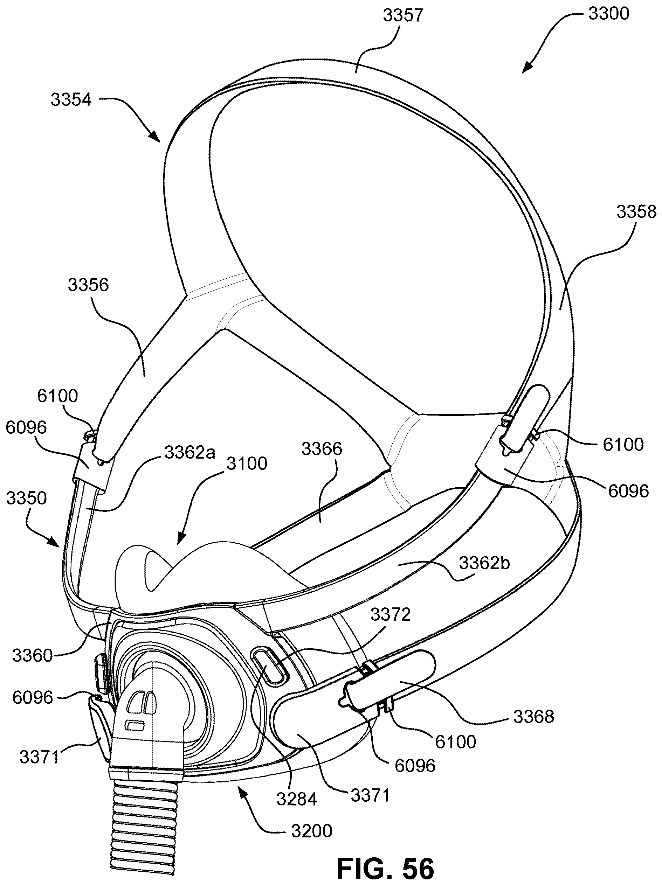

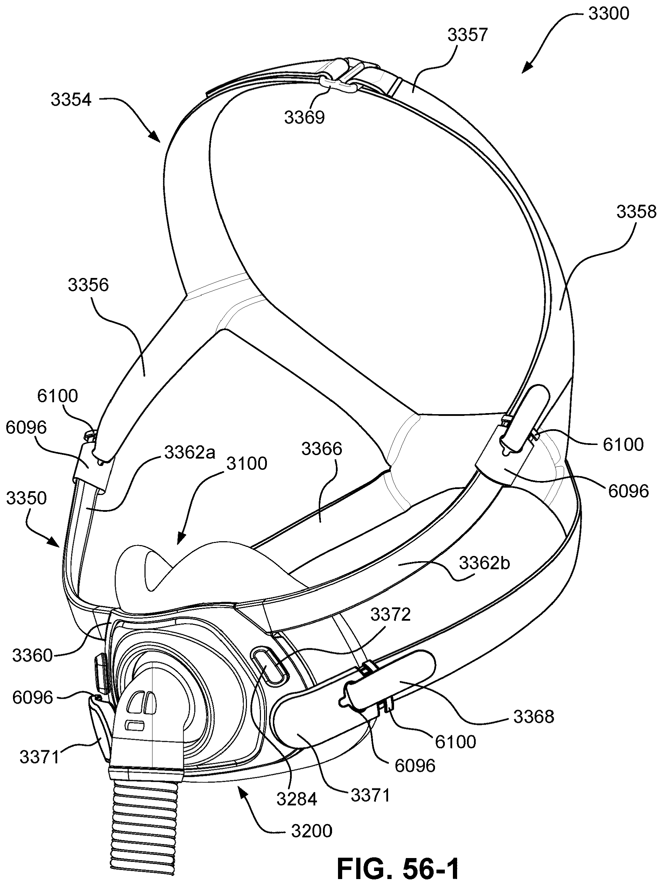

Fig. 56 is a perspective view of a patient interface according to another example, illustrating a frame with cord stoppers connected to headgear straps.
Fig. 56-1 is a perspective view of a patient interface according to yet another example, illustrating a frame with cord stoppers connected to headgear straps.

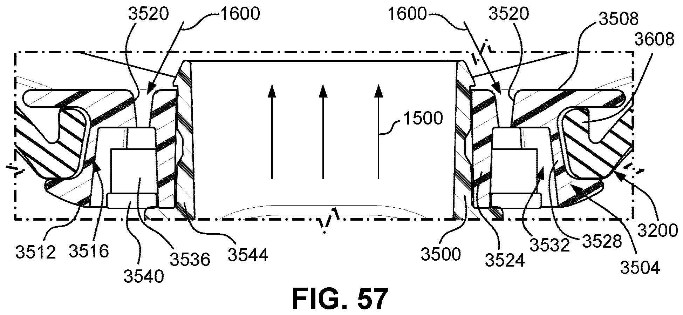

Fig. 57 is a cross-sectional view of a patient interface according to another example, illustrating a vent ring connected to a lip of the plenum chamber inlet port.

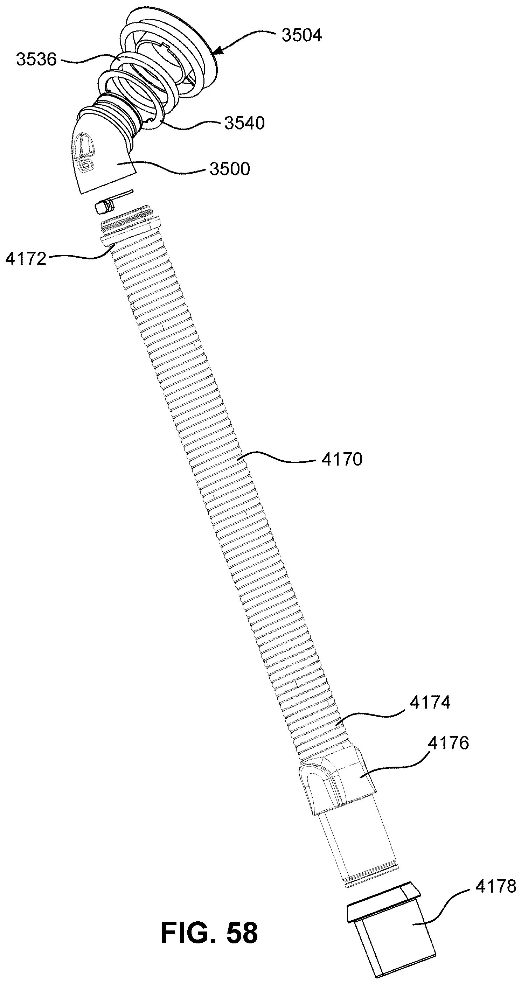

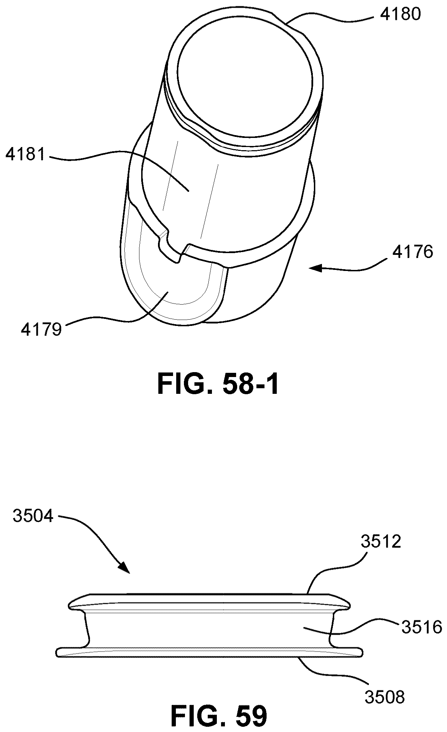

Fig. 58 is an exploded view of an air circuit for connecting to the patient interface of Fig. 57.
Fig. 58-1 is a perspective view of a quick-release connector used in the air circuit of Fig. 58.
Fig. 59 is a perspective view of the vent ring of Fig. 57.

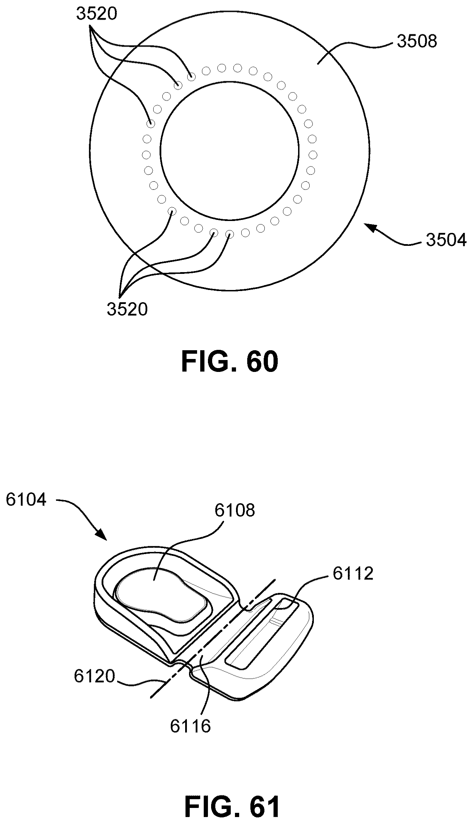

Fig. 60 is a bottom view of the vent ring of Fig. 59, illustrating a plurality of vent holes.
Fig. 61 is bottom view of an alternate version of a housing for a magnet that is removably connectable to a connection point of the frame in any previous example.

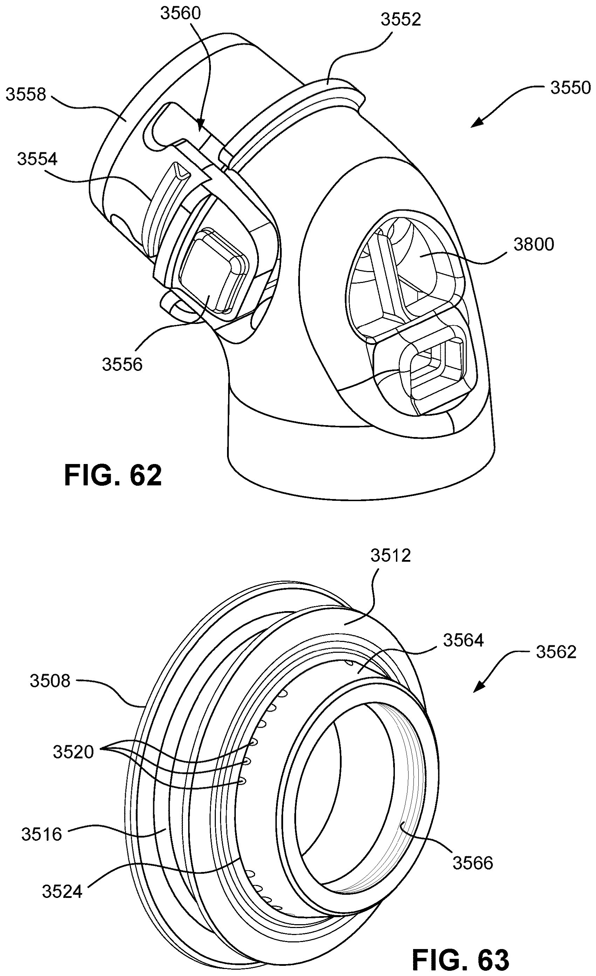

Fig. 62 is a perspective view of a decoupling structure according to another form, which includes a connector for engaging inside a vent ring.

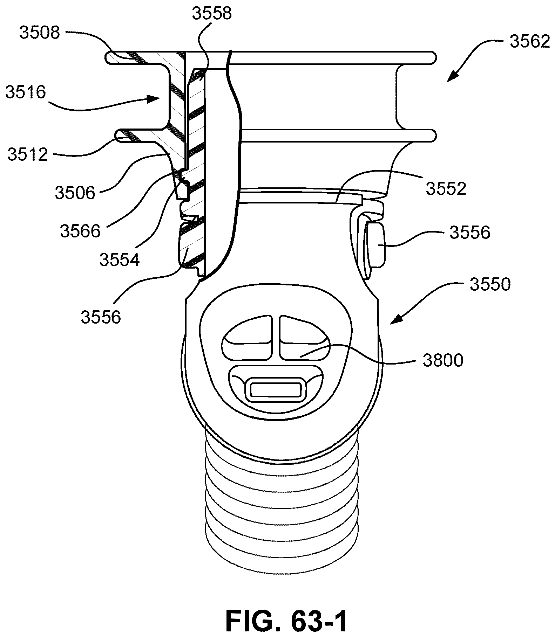

Fig. 63 is a perspective view of a vent ring according to another example for engaging with the decoupling structure of Fig. 62.
Fig. 63-1 is a cross-sectional view of the decoupling structure of Fig. 62 coupled to the vent ring of Fig. 63.

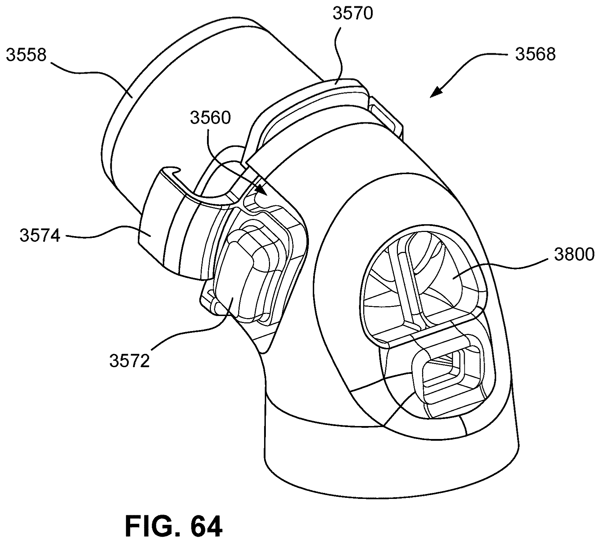

Fig. 64 is a perspective view of a decoupling structure according to another form, which includes a connector for engaging outside a vent ring.

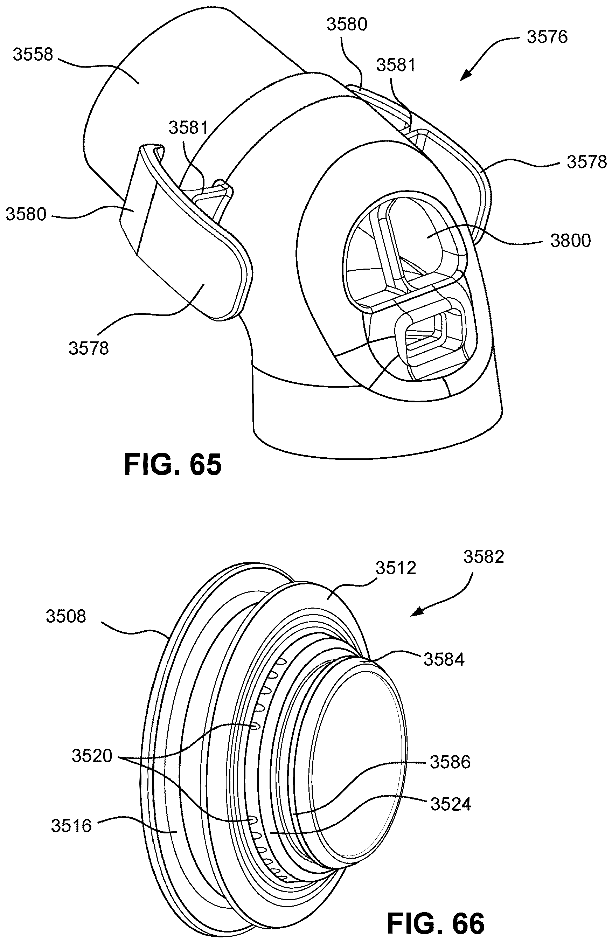

Fig. 65 is a perspective view of a decoupling structure according to yet another form, which includes a connector for engaging inside a vent ring.

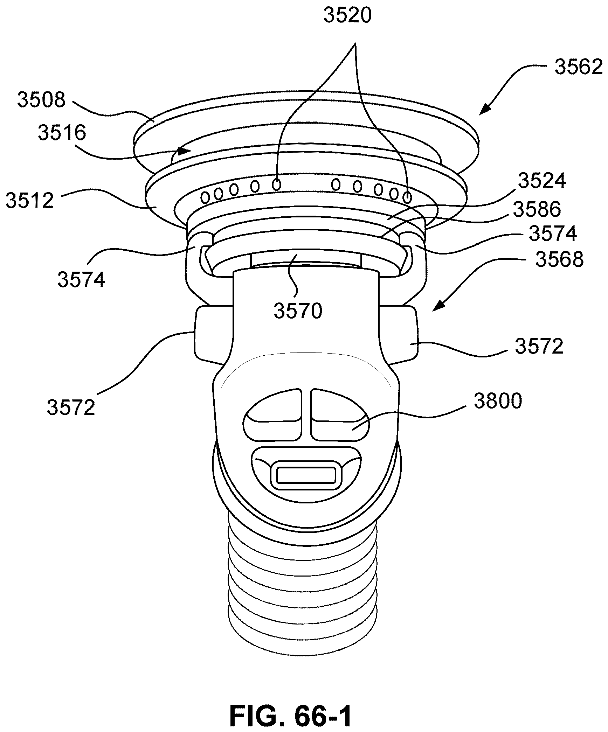

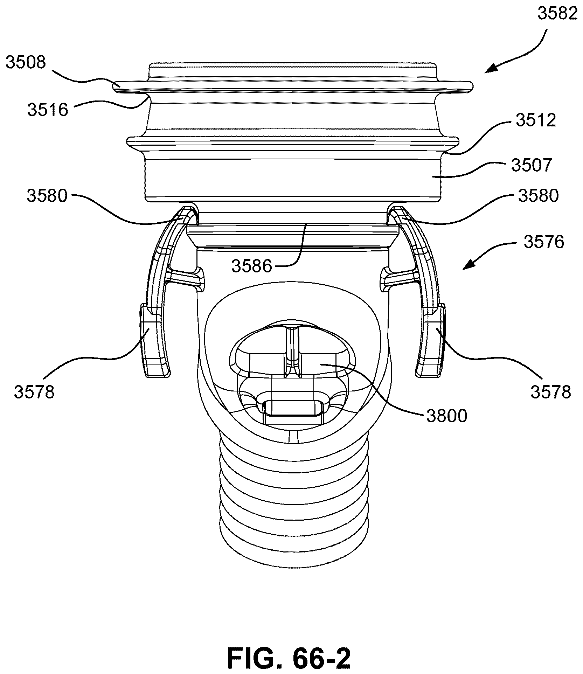

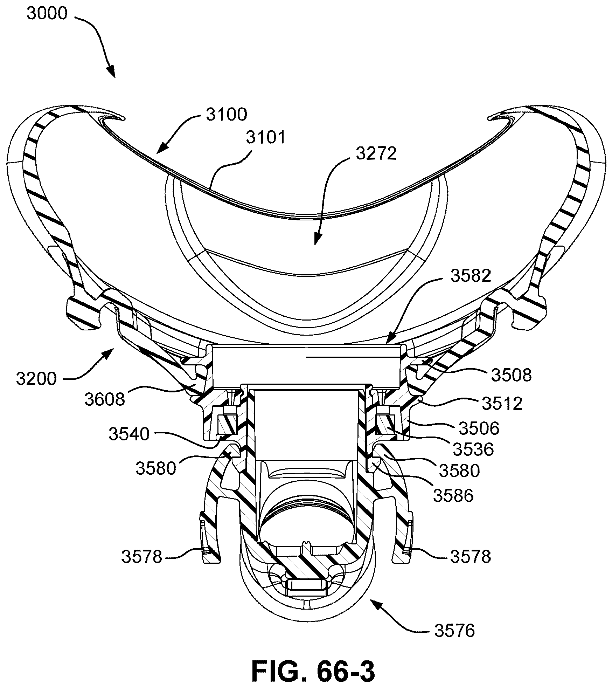

Fig. 66 is a perspective view of a vent ring according to another example for engaging with the decoupling structure of Fig. 64 or 65.
Fig. 66-1 is a perspective view of the decoupling structure of Fig. 64 coupled to the vent ring of Fig. 66.
Fig. 66-2 is a perspective view of the decoupling structure of Fig. 65 coupled to the vent ring of Fig. 66.
Fig. 66-3 is a cross-sectional view of the decoupling structure and vent ring of Fig. 66-2, coupled to a plenum chamber.

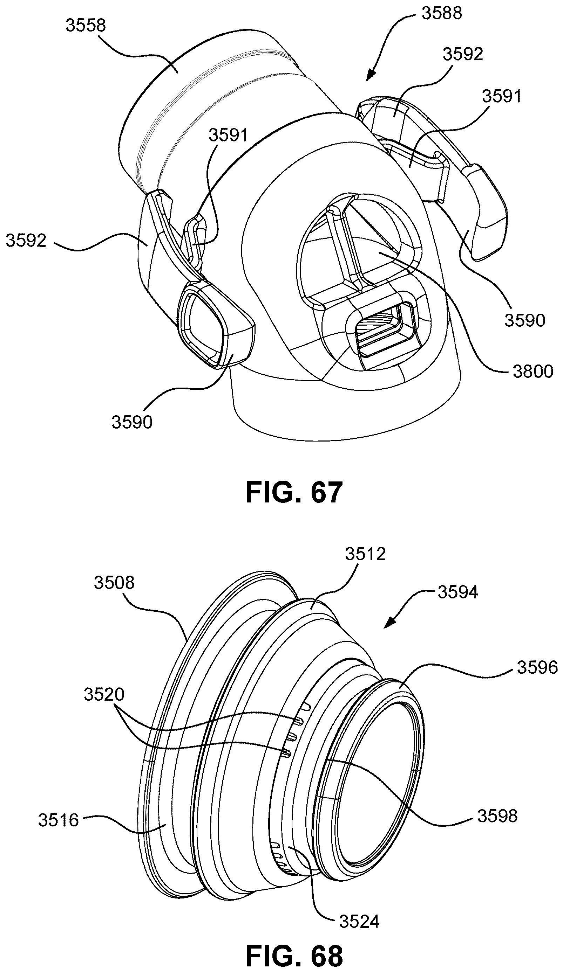

Fig. 67 is a perspective view of a decoupling structure according to yet another form, which includes a connector for engaging outside a vent ring.
Fig. 68 is a perspective view of a vent ring according to yet another example for engaging with the decoupling structure of Fig. 67.

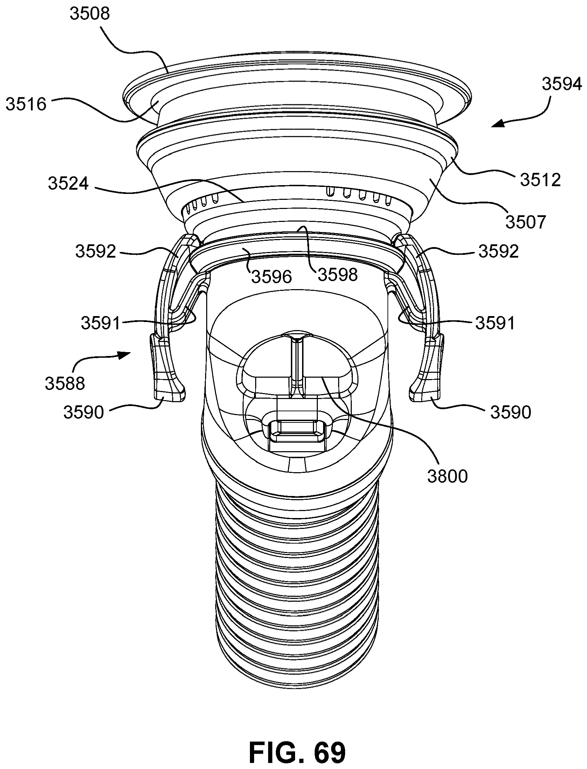

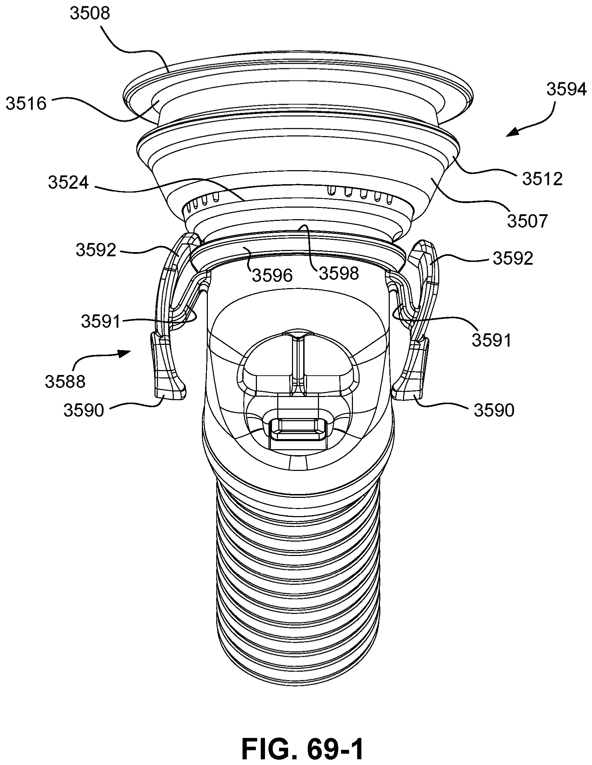

Fig. 69 is a perspective view of the decoupling structure of Fig. 67 coupled to the vent ring of Fig. 68, illustrating arms of the decoupling structure engaged to the vent ring.
Fig. 69-1 is a perspective view of the decoupling structure of Fig. 67 coupled to the vent ring of Fig. 68, illustrating arms of the decoupling structure disengaged from the vent ring.

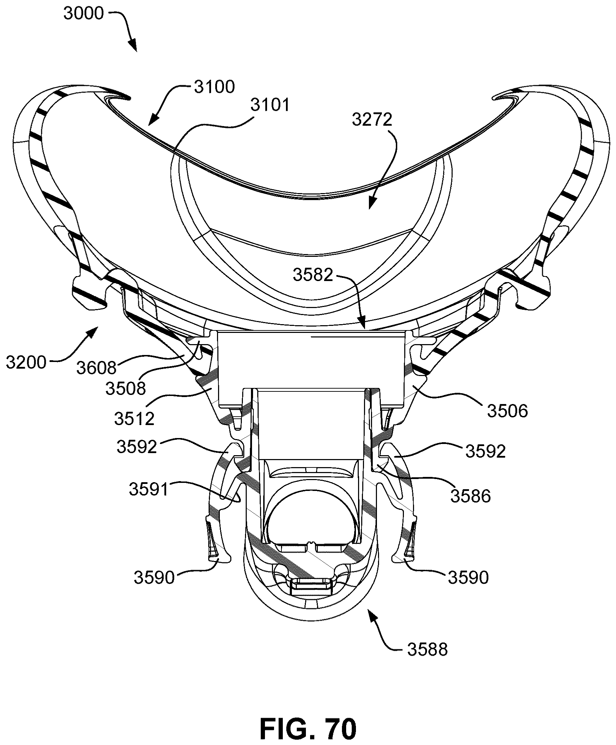

Fig. 70 is a cross-sectional view of the decoupling structure and vent ring of Fig. 69, coupled to a plenum chamber.

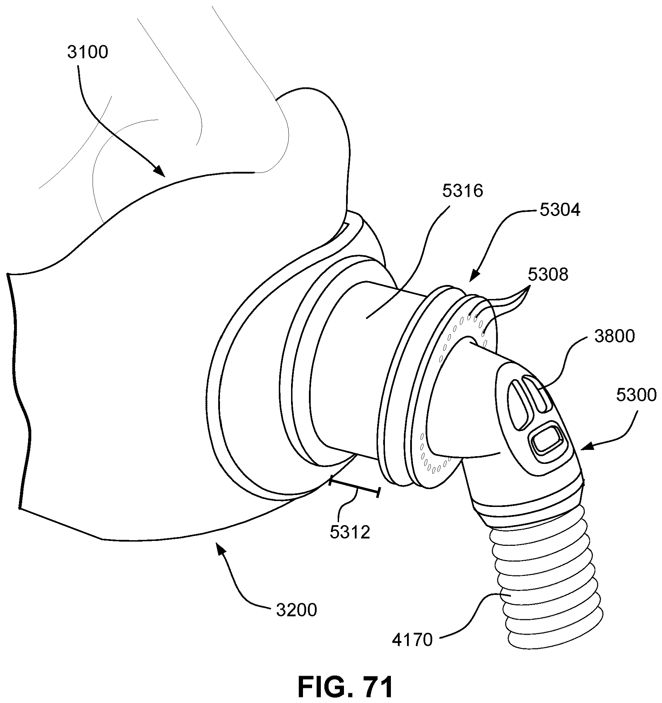

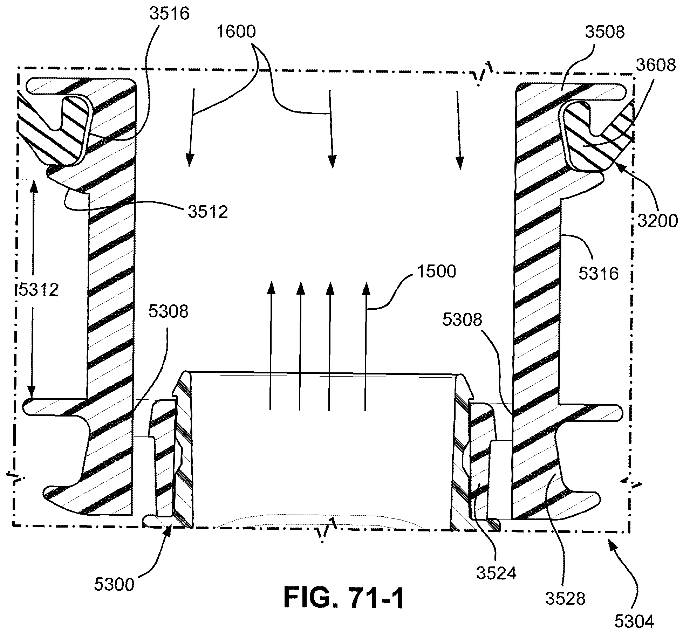

Fig. 71 is a perspective view of another form of an elbow connector connected to a plenum chamber and spaced apart from a surface of a plenum chamber when fully connected.
Fig. 71-1 is a cross-sectional view of the elbow connector and plenum chamber of Fig. 71.

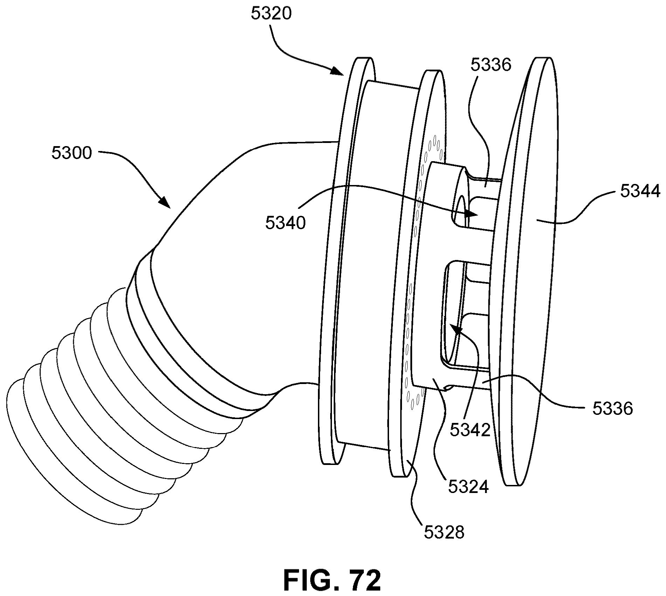

Fig. 72 is a side perspective view of yet another form of an elbow connector that includes a chevron

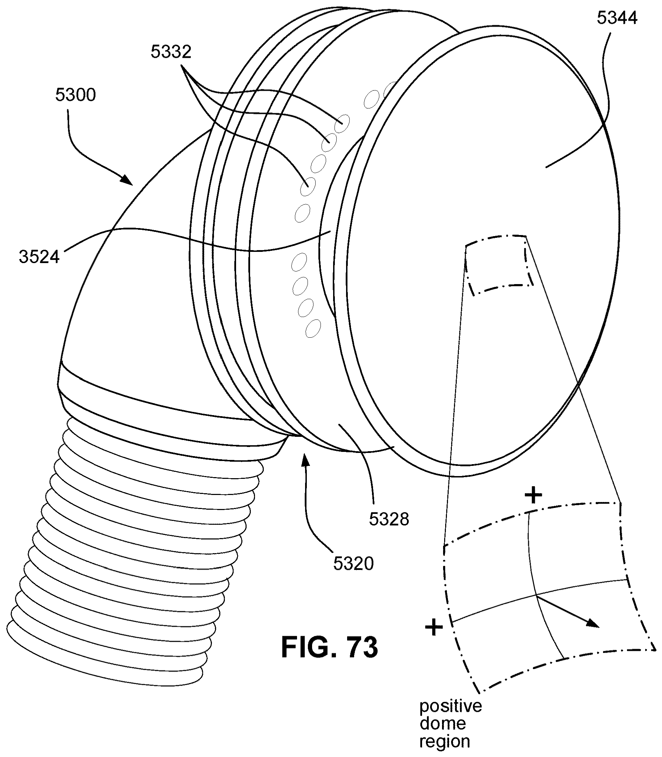

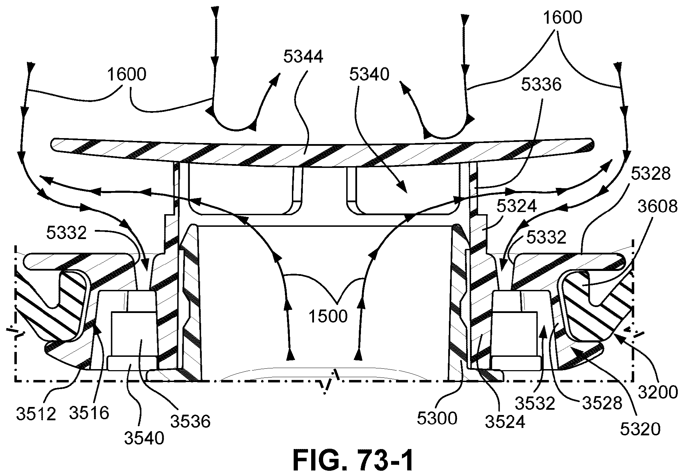

Fig. 73 is a front perspective view of the elbow connector of Fig. 72.
Fig. 73-1 is a cross-sectional view of the elbow connector of Fig. 73 connected to a plenum chamber.

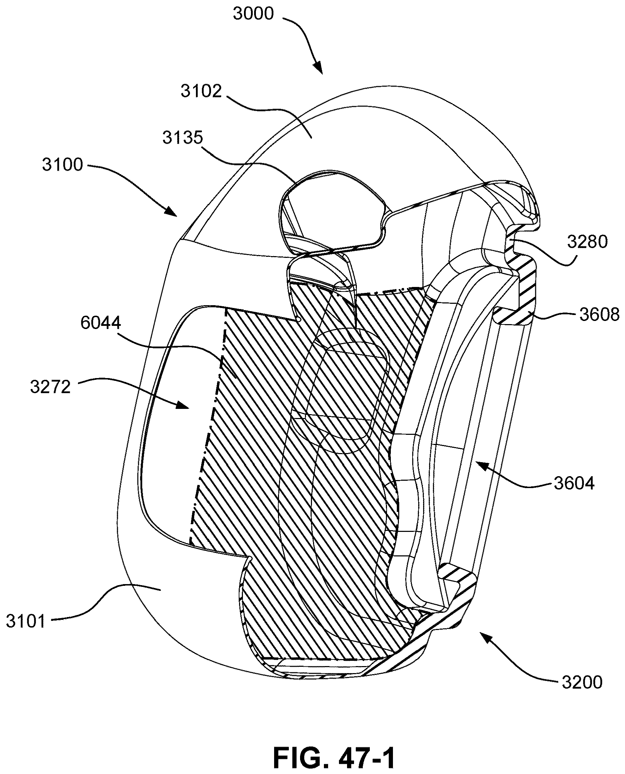

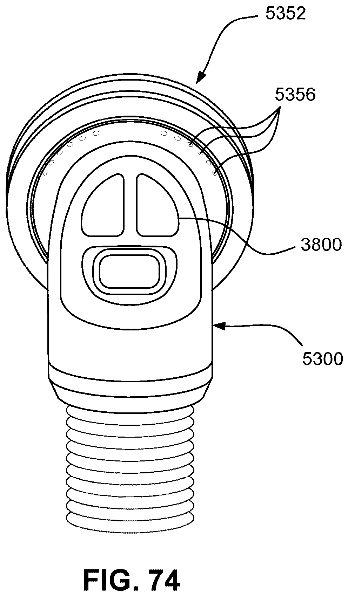

Fig. 74 is a perspective view of yet another form of an elbow connector having fewer vent holes.

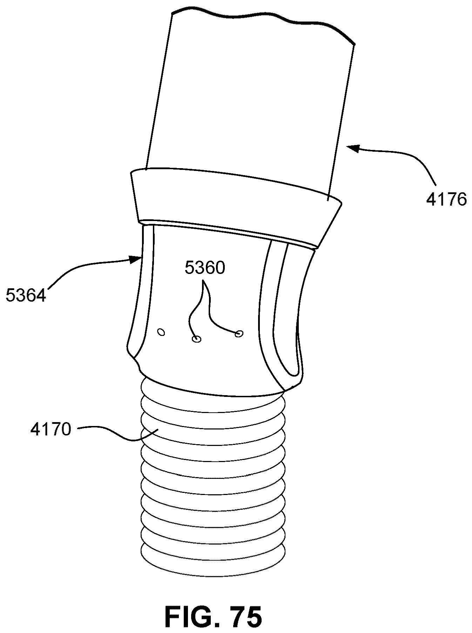

Fig. 75 is a perspective view of a swivel for use with the elbow connector of Fig. 74 illustrating vent holes.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

Where anatomical directional terms are used in describing aspects and examples of the present technology, such as "anterior", "posterior", "superior" and the like, the directions are to be read in the context of the present technology during use by the patient. For example, an anterior side of a patient interface refers to the side of the patient interface which is anterior with respect to the patient when the patient has donned the interface in the intended manner.

Where surfaces or portions are described as facing a direction, e.g. "superior facing", "anterior facing" and the like, unless the context clearly requires otherwise, the surfaces or portions are to be understood as at least partially facing in the particular direction. A portion may be "superior facing" if the portion generally faces a superior direction, even if it also partially faces another direction.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.

### 5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

In some examples of the present technology, the plenum chamber is at least partially formed by a shell 3250. In examples the shell 3250 or a portion of the shell 3250 may be somewhat flexible, as is discussed further below.

The patient interface in some examples of the technology is an oro-nasal patient interface, that is, the patient interface is configured to seal around both the patient's nasal airways and oral airway. In some examples the patient interface comprises separate seals around the each of the nasal airways and the oral airway.

In the examples shown in Figs 7-22 the seal forming structure at the nasal portion does not lie over a nose bridge region or nose ridge region of the patient's face and instead seals against inferior surfaces of the patient's nose.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH₂O with respect to ambient.

The patient interface 3000 in accordance with one form of the present technology is constructed and arranged to be able to provide a supply of air at a positive pressure of at least 10 cmH₂O with respect to ambient.

The patient interface 3000 in accordance with one form of the present technology is constructed and arranged to be able to provide a supply of air at a positive pressure of at least 20 cmH₂O with respect to ambient.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

As is described in greater detail below, in certain forms of the invention the seal forming structure 3100 comprises a first seal forming structure 3101 connected to an oral portion 3201 of the plenum chamber and constructed and arranged to form seal with a region of the patient's face surrounding an entrance to the patient's mouth, and a second seal-forming structure 3102 connected to a nasal portion 3202 of the plenum chamber 3200 constructed and arranged to form a seal with a region of the patient's face surrounding an entrance to the patient's nose. The phrase "connected to" is used herein to refer to portions or components which are formed as a single piece as well as to portions or components which are formed separately and subsequently joined together. In some cases components may be connected by an intermediate component.

In certain forms, the first seal forming structure 3101 seals independently against the patient's face than the second seal forming structure 3102.

In certain forms, the first seal forming structure 3101 and the second seal forming structure 3102 cooperate to form a single common seal against the patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.1.1 Sealing mechanisms

In one form, the seal-forming structure 3100 includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a spring-like element and functions to support the sealing flange from buckling in use. Limiting the occurrences of buckling may limit creases from forming in the seal-forming structure 3100, which may lead to leaks and loss of the therapeutic pressure.

In one form, the seal-forming structure 3100 may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure 3100 comprises a tension portion. In use, the tension portion is held in tension, e.g. by adjacent regions of the sealing flange.

In one form, the seal-forming structure 3100 comprises a region having a tacky or adhesive surface.

In certain forms of the present technology, a seal-forming structure 3100 may comprise one or more of a pressure-assisted sealing flange, a compression sealing portion, a gasket sealing portion, a tension portion, and a portion having a tacky or adhesive surface.

### 5.3.1.2 Nasal region

Referring next to Figs. 7 to 18, in certain forms of the present technology, the second seal forming structure 3102 comprises a central portion 3110 configured to seal to surfaces of the patient's nose in use. The central portion may seal to an inferior periphery of the patient's nose (e.g. surrounding the patient's nares) and to the patient's lip superior. In examples a portion of the seal forming structure may engage the patient's septum. The second seal forming structure 3102 may further comprise lateral portions 3111 on lateral sides of the central portion 3110. In examples, the seal forming structure 3102 may be configured to contact the patient's face below the bridge of the nose or below the pronasale.

As best seen in Figs. 10 and 16 - 19, posterior surfaces 3112 of the lateral portions 3111 slope forward in a superior/anterior direction from the boundary 3103 of the first and second seal forming structures 3101, 3102 such that in profile the posterior side of the nasal part of the mask slopes forward.

In embodiments provided with a ridge 3120 (as described further below), the posterior surfaces 3112 of the lateral portions 3111 may slope forward from the ridge 3120.

In some forms of the technology the posterior surfaces 3112 of the lateral portions 3111 form an angle with a mid-contact plane of the mask of between 20° and 90°. The mid-contact plane may be perpendicular to the sagittal plane, and may extend substantially along a length of the ridge 3120 and the chord 3210. This angle may be measured in the clockwise direction in Fig. 16, although the angle may also be measured in the counter-clockwise direction so that the measured angle is the complement of the above listed angles (i.e., between 90° and 160°).

As shown in Figs. 16-3 to 16-5, the second seal forming structure 3102 may be formed at different angles relative to the mid-contact plane so that the patients with different sized noses can each comfortably wear the patient interface 3000. As illustrated, two separate angles of the second seal forming structure 3102 may be modified on different sized patient interfaces 3000 in order to provide a better fit for a wide range of patients.

A first angle 6056 may be measured from the mid-contact plane to a first seal axis 6060 extending along a superior boundary 3247 of the second anterior wall portion 3242 and intersecting the nasal aperture 3135. In some forms, the first angle 6056 may be between approximately 50° and approximately 150°. In some forms, the first angle 6056 may be between approximately 75° and approximately 125°. In some forms, the first angle 6056 may be between approximately 100° and approximately 110°. For example, Fig. 16-3 may include a first angle 6056-1 of approximately 108°, Fig. 16-4 may include a first angle 6056-2 of approximately 105°, and Fig. 16-5 may include a first angle 6056-3 of approximately 109°. As illustrated, the first angle 6056 is measured in a counter-clockwise direction shown in Figs. 16-3 to 16-5. However, the first angle 6056 may be measure in the clockwise direction so that the measured angle is the complement of the above listed angles.

A second angle 6064 may be measured from the mid-contact plane to a second seal axis 6068 extending along the surface of the second seal forming structure 3102 configured to contact the patient and intersecting the nasal aperture 3135. In some forms, the second angle 6064 may be between approximately 50° and approximately 160°. In some forms, the second angle 6064 may be between approximately 100° and approximately 145°. In some forms, the second angle 6064 may be between approximately 110° and approximately 130°. For example, Fig. 16-3 may include a second angle 6064-1 of approximately 127.5°, Fig. 16-4 may include a second angle 6064-2 of approximately 116.5°, and Fig. 16-5 may include a second angle 6064-3 of approximately 122°. As illustrated, the second angle 6064 is measured in a counter-clockwise direction shown in Figs. 16-3 to 16-5. However, the second angle 6064 may be measure in the clockwise direction so that the measured angle is the complement of the above listed angles.

A difference between the first angle 6056 and the second angle 6064 may form an inclination of the second seal forming structure 3102. As described above, in some forms, this difference may be between approximately 1° and approximately 110°. In some forms, the difference may be between approximately 5° and approximately 50°. In some forms, the difference may be between approximately 10° and approximately 20°. For example, Fig. 16-3 may include a difference of approximately 19.5°, Fig. 16-4 may include a difference of approximately 11.5°, and Fig. 16-5 may include a difference of approximately 13°. In some forms, the difference may be similar to the angle measured between the posterior surface 3112 and the mid-contact plane described above, although these are separate angles.

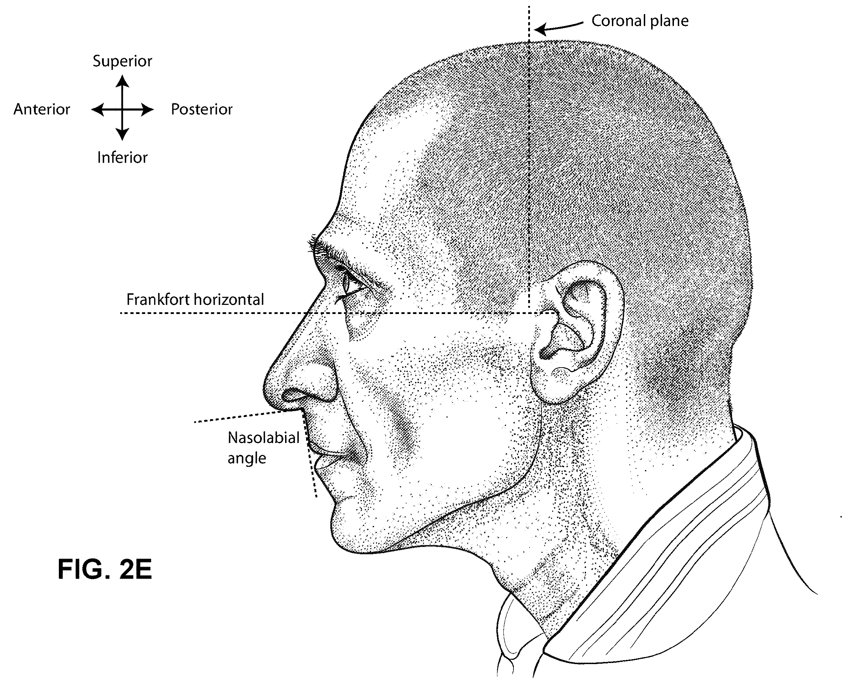

The different angles shown in Figs. 16-3 to 16-5 may correspond to patient's with different nasolabial angles (see e.g., Fig. 2E). The patient interfaces 3000 shown in each of the three figures may represent a different sized interface (e.g., small, medium, or large), each which may comfortably fit patients within a range of nasolabial angles.

As shown in Figs. 50 and 51, the first angle 6056 and the second angle 6064 of Figs. 16-3 to 16-5 may provide relief for a patient with a long nose tip (e.g., a large distance between the pronasale and the alar crest point). For example, the second anterior wall portion 3242 may be sufficiently spaced from the second seal forming structure 3102, so that contact between the patient's nares and the second seal forming structure 3102 maintains a sufficient distance with the second anterior wall 3242 to allow for airflow. This distance 6072 may extend between the second anterior wall portion 3242 and the nasal aperture 3135. The distance 6072 may be the shortest distance between the second anterior wall portion 3242 may be sufficiently spaced from the second seal forming structure 3102, and the distance may not extend along the first axis 6060. Although in other examples, the first distance 6072 may extend substantially along the first seal axis 6060. This may enable the second seal forming structure 3102 to stretch in the anterior direction (e.g., when the patient has a long nose tip) without contacting the second anterior wall portion 3242.

As shown in Fig. 19, in some embodiments the lateral portions 3111 are configured such that no part of the patient interface 3000 contacts the patient's alar crest point 1020 when in use.

Configuring the lateral portions 3111 to slope in this way results in a smaller portion of the nasal part of the interface 3000 extending over the sides of the ala than some similar interfaces of the prior art. In some forms of the technology this results in the portion of the ala which is in contact with the seal-forming structure 3100 being reduced relative to interfaces with lateral portions which slope backward, toward the patient's face, thereby reducing the proportion of the ala which can be deformed and occluded by the seal-forming structure 3100, for example when the patient sleeps on their side with the interface in contact with a pillow.

### 5.3.1.3 Boundary of oral and nasal regions

With particular reference to Figs. 7, 8 and 16-18, in one form of the technology the boundary between the first sealing forming structure 3101 and the second seal forming structure 3102 forms or comprises a corner or ridge 3120. In use, the corner or ridge 3120 may engage the patient's face above the lip superior and immediately below the nose.

In embodiments the corner or ridge 3120 forms a sharper angle than the equivalent portion or area of some oro-nasal masks of the prior art, for example those described in PCT application No. PCT/AU2019/050278.

The sharper angle reduces the likelihood of creases forming in the first and/or second seal forming structures 3101, 3102 on or adjacent the corner or ridge 3120 when the mask is donned and therapy is applied. Some oro-nasal patient interfaces which do not use such a structure may require a very thin, rounded formation in this area which may be less resistant to creasing. By contrast, the corner or ridge 3120 may be stiffer, and may hold its shape better, than such interfaces and may therefore seal better against the concavities and creases present around the patient's nose. This effect may be enhanced in embodiments which are provided with support portions, for example support portions 3260 as described herein, which resist or oppose compression of this region.

In some forms of the technology the radius of the corner or ridge 3120 may be less than 2mm, for example around 1.75mm. In one form of the technology the radius may vary from approximately 1.75mm in the centre of the ridge to approximately 0.75mm at the lateral portions.

The angle formed by the first and second sealing structures may be between 20 degrees and 90 degrees, for example 36 degrees.

In some forms of the technology, the corner or ridge 3120 may extend across substantially an entire boundary 3103 between the first seal forming structure 3101 and the second seal forming structure 3102. In embodiments the corner or ridge 3120 may engage the patient's face at least approximate the entrances to the nares, for example where the ala meets the face above the lip superior, as indicated by areas 1010 in Fig 20.

### 5.3.1.4 Oral region

As is described above, in one form the non-invasive patient interface 3000 comprises a first seal-forming structure 3101 that forms a seal in use around the patient's mouth. The first seal forming structure 3101 may form a seal on a chin-region of the patient's face.

In one form, the seal-forming structure includes a saddle-shaped region constructed to form a seal in use on a chin-region of the patient's face.

The seal forming structure 3100 comprises a lip inferior portion 3130 which forms a seal against the chin region of the patient and/or the lip inferior and/or supramenton of the patient. The lip inferior portion 3130 may be connected to (e.g. contiguous with) a lip superior portion 3131 via an oral hole peripheral portion 3132, as shown in Fig. 16.

The seal forming structure 3100 comprises a relatively low wall thickness (compared to other portions of the interface), for example less than 0.7mm, at the oral hole peripheral portion 3132, the lip inferior portion 3130 of the seal forming structure which lies against the chin region, and at least the centre of the lip inferior portion 3130. The low wall thickness in these locations assists in achieving an effective, comfortable seal. The seal forming structure in these regions is able to readily conform to any complex geometry.

In some forms of the technology the oral hole 3133 is substantially trapezoidal rather than oval or elliptical, in order to more accurately correspond to a shape of the patient's nose. This shape of oral hole may allow the interface 3000 to be particularly compact, and not be substantially wider than a width of the patient's nares.

As shown in Fig. 51, the patient's mouth may be spaced apart from the inlet port 3604 (and therefore from the elbow 3500 and/or the vent ring 3504 like in Fig. 50). This distance 6076 may be measured in a substantially parallel direction as the distance 6072. This distance 6076 provides sufficient clearance for a patient's lips, which extend into the cavity 3272 and more anterior to the second seal forming structure 3102. The patient may slightly compress the second seal forming structure 3102 in the anterior direction, and the distance 6076 may be sufficiently large to prevent contact with the elbow 3500 and/or vent ring 3504. However, the distance 6076 may not be overly large so that the patient interface 3000 maintains a compact profile.

Also or in addition as illustrated in Fig. 50-1, a vent ring 3504 (described in more detail below) may include an offset in order to create additional spacing between an elbow 3500 and the first seal forming structure 3101 (and therefore the patient's lips). The vent ring 3504 may include an offset 3506 (described below) that may space the elbow 3500 apart from the cavity 3272 of the plenum chamber 3200. For example, as shown in Fig. 57, the elbow 3500 contacts an anterior surface 3512 of the vent ring 3504 and extends beyond the posterior surface 3508 of the plenum chamber 3200. This allows for a more compact design (e.g., because it minimizes extension in the anterior direction away from the patient's face), but the elbow 3500 extends into the distance 6076. In other words, the distance 6076 (as measured in Fig. 51) is no longer the greatest distance of spacing between the patient's lips and an anterior portion of the patient interface 3000. Applying an offset 3506 may increase the distance that the patient interface 3000 extends in front of the patient's face, but also spaces the elbow 3500 apart from the patient's lips so that the distance 6076 remains the largest distance of separation in the anterior direction between the patient's lips and the patient interface 3000.

### 5.3.1.5 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.2 Plenum chamber

In some forms, the plenum chamber 3200 (or at least a portion of the plenum chamber 3200) and the seal-forming structure 3100 are formed from a single homogeneous piece of material (e.g., molded silicone). A combination of the seal-forming structure 3100 and the plenum chamber 3200 may be considered a cushion.

### 5.3.2.1 Angle of nasal portion is adjustable

With particular reference to Figs. 9, 10 and 16 to 18-2, in one form of the technology a first anterior wall portion 3240 of the nasal portion 3202 of the plenum chamber 3200 is more flexible than an immediately adjacent region of the oral portion 3201. The first anterior wall portion 3240 may be provided adjacent a boundary 3241 of the nasal and oral portions of the plenum chamber 3200. In embodiments the first anterior wall portion 3240 may be symmetrical about the mid-sagittal plane and may extend across at least 50% of the width of the nasal portion 3202 of the plenum chamber, for instance at least 80%. In some embodiments the first anterior wall portion 3240 may extend across substantially the entire width of the nasal portion 3202 of the plenum chamber.

In some forms of the technology a second anterior wall portion 3242 is less flexible than the immediately adjacent portions of the anterior wall. In some embodiments the second anterior wall portion 3242 is immediately adjacent the first anterior wall portion 3240 on an opposite side to the boundary 3241 of the nasal and oral portions of the plenum chamber. In embodiments the second anterior wall portion 3242 may be symmetrical about the mid-sagittal plane and may extend across at least 50% of the width of the nasal portion 3202 of the plenum chamber, for instance at least 80%. In some embodiments the second anterior wall portion 3242 may extend across substantially the entire width of the nasal portion 3202 of the plenum chamber.

The flexible first anterior wall portion 3240 may allow the patient contacting portions 3110 of the second seal forming structure 3102 to pivot or hinge about a region on the posterior side of the interface 3000. This may assist in allowing the interface to accommodate patients with a variety of angles between the bottom of the nose and the top lip (i.e. nasolabial angles).

In embodiments featuring a corner or ridge 3120 between the first and second seal forming structures 3101, 3102, such as have been described above, the patient contacting portions 3110 may pivot or hinge about an area at or adjacent the corner or ridge 3120. In embodiments provided with one or more support portions 3260 (described further below), the hinging or pivoting region may be immediately superior to the support portions 3260.

As shown in Fig. 9, the first anterior wall portion 3240 may have a superior boundary 3243 and an inferior boundary 3244. One or both of the superior and inferior boundaries 3243, 3244 may be curved, for example such that a central portion of the boundary is inferior to the lateral portions, as shown. The first anterior wall portion 3240 may be substantially the same height across its width (i.e., the superior and inferior boundaries may be substantially parallel) or the height may vary across the width, for example such that the height of a central portion of the first anterior wall portion 3240 is greater than the height of the lateral portions, as shown in the embodiment in Fig. 9. Varying the curvature of one or both of the boundaries 3243, 3244 and/or the height of the first anterior wall portion 3240 may change the stiffness of the first anterior wall portion 3240, that is, its resistance to collapsing or folding in response to forces on the patient-contacting portions 3110 of the second seal forming structure 3102.

Similarly, the second anterior wall portion 3242 may have a superior boundary 3247 and an inferior boundary 3248. In some forms of the technology the inferior boundary 3248 of the second anterior wall portion 3242 is the same as the superior boundary 3243 of the first anterior wall portion 3240. Both the superior and inferior boundaries 3247, 3248 of the second anterior wall portion 3242 may be curved, for example such that a central portion of the boundary is inferior to the lateral portions. The second anterior wall portion 3242 may be substantially the same height across its width (i.e., the superior and inferior boundaries may be substantially parallel) or the height may vary across the width, for example such that the height of a central portion of the second anterior wall portion 3242 is less than the height of the lateral portions.

In some forms of the technology other ways of configuring the first anterior wall portion 3240 to have a required stiffness may be used, in addition to or alternatively to curved boundaries. For example, the thickness of the first anterior wall portion 3240 may be selected to provide a required stiffness. In examples the first anterior wall portion 3240 may be thinner than the immediately adjacent portions of the plenum chamber wall. Additionally and/or alternatively, the first anterior wall portion 3240 may extend in a superior direction around a lateral edge of the second anterior wall portion 3242, as shown in Fig. 21, thereby providing a reduced stiffness/resistance to compression or collapse compared to embodiments in which the first anterior wall portion 3240 is not shaped this way.

The second anterior wall portion 3242 (e.g., the band 3270) may assist in preventing collapse of the nasal portion 3202, and may provide support for the patient-contacting portions 3110 of the second seal forming structure 3102, which are typically relatively thin. Insufficiently supported patient contacting portions may suffer from blowout of the sealing engagement with the patient's face. In one form the second anterior wall portion 3242 is thicker than the immediately adjacent portions of the plenum chamber wall. In certain forms the second anterior wall portion 3242 is provided as a thickened band of material 3270, as shown in Figs 16-19. The first and second anterior wall portions 3240, 3242 may be made from the same material, for example as part of an integrally moulded shell 3250.

In some forms, the first and second anterior wall portions 3240, 3242 may include different thicknesses. For example, the thickness of the second anterior wall portion 3242 may be greater than the thickness of the first anterior wall portion 3240, which may provide the increased stiffness in the second anterior wall portion 3242 (e.g., as compared to the first anterior wall portion 3240). Specifically, the second anterior wall portion 3242 may be a band 3270 that may extend into the cavity 3272 of the plenum chamber 3200. For example, the band 3270 may extend past the first anterior wall portion 3240, and extend toward a patient wearing the patient interface 3000. An exterior surface of the nasal portion 3202 may be substantially smooth, while an interior surface of the nasal portion (e.g., within the cavity 3272) may be stepped (or otherwise include a discontinuity).

As shown in Figs. 16-1 and 16-2, the first anterior wall portion 3240 may act as a hinge and allow the nasal portion 3202 to bend. The first anterior wall portion 3240 may be the thinnest region of the nasal portion 3202, and therefore may be the most susceptible to a bending moment. The increased thickness of the band 3270 directs the bending moment away from the second anterior wall portion 3242, and toward the thinner first anterior wall portion 3240. A larger height of the band 3270 (i.e., a larger distance between the superior and inferior boundaries 3247, 3248) may also make the nasal portion 3202 stiffer and less capable of bending about the first anterior wall portion 3240. The first and second anterior wall portions 3240, 3242 may move in the anterior direction (e.g., away from the patient) as bending occurs.

As shown in Figs. 18-1 and 18-2, the superior boundary 3243 of the first anterior wall portion 3240 is not the same as the inferior boundary 3248 of the second anterior wall portion 3242. Instead, the superior boundary 3243 is a least partially more superior than the inferior boundary 3248, and may be at least partially aligned with the superior boundary 3247 of the second anterior wall portion 3242. This allows the first anterior wall portion 3240 to be disposed at least partially alongside of the band 3270 (e.g., and surround the band 3270 on two or more sides). In other words, the first anterior wall portion 3240 may be disposed on at least one of the ends of the band 3270. This may provide the first anterior wall portion 3240 with greater flexibility so that the nasal portion 3202 is able to bend further (e.g., in the anterior direction).

As shown in Fig. 21-1, the nasal portion 3202 may also be formed without a hinge. In other words, a band may not be formed on the second anterior wall portion 3242, so that the first and second anterior wall portions 3240, 3242 have substantially uniform thicknesses. The nasal portion 3202 may still be able to bend even without band because it may be constructed from silicone, which permits some compliance in the nasal portion 3202 to accommodate different nasolabial angles.

### 5.3.2.2 Flexible shell

In some forms of the technology the shell 3250 may be made from a rigid material such as polycarbonate. However, in other forms of the technology the shell 3250, or portions of the shell 3250, may be somewhat flexible. For example, in examples the shell 3250 may be formed from a material which has a Young's modulus of 0.4 GPa or lower, for example foam. In some forms of the technology the shell 3250 may be made from a material having Young's modulus of 0.1GPa or lower, for example rubber. In other forms of the technology the shell 3250 may be made from a material having a Young's modulus of 0.7MPa or less, for example between 0.7MPa and 0.3MPa. An example of such a material is silicone.

In examples, the shell 3250 and one or both of the first and second seal forming structures 3101, 3102 may be formed from the same material (e.g., silicone, textile, etc.).

In some forms of the technology (see e.g., Figs. 23 to 28-2), the shell 3250 may be constructed substantially entirely from a flexible material, which may provide the shell 3250 with the greatest freedom of movement (i.e., substantially no rigid and/or thickened portions that limit bending). The shell 3250 may be sufficiently flexible that one or more components are added to provide a required stiffness in one or more areas or regions of the shell 3250 (e.g., a region that contacts the area 1010). For example, one or more of a vent module; a connection port; a headgear connector; a headgear connector connected to a rigidising arm and a rigidising member may be connected to the shell 3250 in such a way as to increase the stiffness of the plenum chamber 3200 in the area adjacent the component, for example as described further below. In some forms of the technology such components may be releasably connectable to the flexible shell 3250. Additionally or alternatively one more components may be permanently connected to the shell 3250, for example by bonding and/or overmolding. The rigidising member may also serve to increase the stiffness and/or support the shape of the seal forming structure 3100.

In some forms of the technology the shell 3250 may be generally flexible but may comprise stiffening portions having greater thickness than immediately adjacent portions of the shell 3250. Such stiffening portions may be configured as ribs or bands, for example extending laterally across the shell and/or extending in a superior-inferior direction, although many other configurations are possible. In some forms the shell may comprise a substantially rigid portion, for example manufactured from polycarbonate, as well as a somewhat flexible portion.

In some forms of the technology it may be preferable for a central portion 3251 of the anterior side of the oral portion 3201 of the plenum chamber to have a greater stiffness than the remainder of the plenum chamber 3200. In some forms of the technology the area of increased stiffness may be immediately inferior to the nasal portion 3202, as shown in Fig. 21 and described further below, and/or immediately superior to the oral portion 3201. In one form of the technology, a portion of, or the entirety of, the first anterior wall portion 3240 may be an area of increased stiffness, rather than an area of increased flexibility. Providing increased stiffness in one or more of these areas may provide shape stability and may limit the extent to which the shell 3250 deforms as a result of headgear forces. Excessive deformation may result in the second seal forming structure 3102 occluding the nares. Avoiding such deformation may be particularly advantageous to patients with relatively wide noses, and may be less important, or in some cases undesirable, for patients with narrow noses. In addition, the areas of increased stiffness described may assist in reducing torsional deformation of the interface which may otherwise result in one side of the second seal forming structure 3102 losing contact with the patient's nose, thereby creating a leak path.

As shown in Figs. 21 and 21 -1, in one form of the technology the shell 3250 may be provided with a rigid portion 3263, or at least a portion which is more rigid than the remainder of the shell, to which one or more connection ports 3600 are provided, e.g. moulded. In one form of the technology a rigid portion 3263 may be made from polycarbonate. This may provide more rigidity than a shell made exclusively of silicone. In one form the technology holes forming a vent 3400 are moulded into the rigid portion 3263. In some forms of the technology, connectors 3310 for a positioning and stabilising structure are mounted on arms 3320 which provide some rigidity to the shell.

In one form of the technology the rigid portion 3263 extends laterally across the anterior of the plenum chamber near a superior boundary of the first anterior wall portion 3240, for example immediately below the second anterior wall portion 3242. The rigid portion 3263 may extend continuously between the connection ports 3600, and may provide an airflow path for the flow of pressurized air entering the plenum chamber 3200 through the connection ports 3600.

In some forms of the technology the connection ports 3600 may have a substantially elliptical shape in cross-section. The connection ports 3600 may be orientated such that a centreline of each port is substantially parallel to an exterior surface of the plenum chamber adjacent the port.

In some forms of the technology the rigid portion 3263 may protrude in an anterior direction relative to an adjacent face of the first anterior wall portion 3240, and may be shaped to increase resistance to bending.

In some forms of the technology (see e.g., Fig. 21), the connectors 3310 and arms 3320 are provided inferior of the connection ports 3600, toward the lateral edges of the plenum chamber 3200. The connectors 3310 may be provided at lateral ends of the arms 3320. The connectors 3310 may provide additional rigidity to the plenum chamber 3200 and/or the seal forming structure 3100.

In some forms of the technology (see e.g., Fig. 21-1), the connectors 3310 do not include arms 3320, and are instead connected directly to the plenum chamber 3200. This may make the plenum chamber 3200 more flexible than the plenum chamber 3200 of Fig. 21.

Fig. 22 shows a plenum chamber 3200 with a vent mounting aperture 3410 into which a suitable vent portion or module may be inserted. The vent portion may be made from a relatively stiff material to increase the stiffness of the plenum chamber. In some forms of the technology the vent mounting aperture 3410 may be substantially elliptical in shape, with the minor axis of the ellipse being substantially parallel to a sagittal plane.

In the embodiment shown in Figure 22 the vent mounting aperture is provided toward a superior border of the oral portion 3201 of the plenum chamber 3200.

The embodiment shown in Fig. 22 is provided with connectors 3310 for a positioning and stabilising structure. The connectors 3310 may be mounted in relatively thicker regions of the shell 3250. In the embodiment shown the connectors 3310 are inferior of the vent mounting aperture 3410 and toward the lateral sides of the plenum chamber 3200. In some forms of the technology the connectors 3310 are substantially circular magnetic headgear connectors.

While inlet or connection ports are not shown in the drawings of the plenum chamber shown in Figs. 7-19, those skilled in the art will appreciate that in practice one or more inlet ports will be provided, for example inlet ports 3600 as shown in Figs. 21-22. The inlet ports 3600 allow connection of the interface to an air circuit 4170, as described further herein. In some forms of the technology one or more components of the air circuit 4170 may also act as components of a positioning and stabilising structure.

As shown in Fig. 24, some forms of the patient interface 3000 may include a single inlet port 3604. The inlet port 3604 may be substantially circular in shape and may be centered on the oral portion 3201 of the plenum chamber 3200.

As shown in Fig. 57, the inlet port 3604 may include a lip 3608 that extends around at least a portion of the perimeter of the inlet port 3604 (see e.g., Fig. 49). For example, the lip 3608 may extend around the entire circumference of the circular inlet port 3604. The lip 3608 may extend into the cavity 3272 (see e.g., Fig. 18-1). The lip 3608 may be inclined to the periphery of the circumference of the inlet port 3604.

In some forms, the lip 3608 is approximately perpendicular to the periphery of the circumference of the inlet port such that the diameter of the inlet port 3604 is approximately equivalent to a diameter of an opening formed by the lip 3608 within the cavity 3272. In other words, the lip 3608 may extend substantially parallel to a port axis 3612 that extends through the inlet port 3604.

In other forms, the lip 3608 is inclined between approximately 1° and approximately 60° with respect to the port axis 3612. In some forms, the lip 3608 is inclined between approximately 2° and approximately 50° with respect to the port axis 3612. In some forms, the lip 3608 is inclined between approximately 5° and approximately 30° with respect to the port axis 3612. In some forms, the lip 3608 is inclined between approximately 10° and approximately 15° with respect to the port axis 3612.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material, e.g. translucent silicone. The use of a translucent material can reduce the obtrusiveness of the patient interface, and help improve compliance with therapy.

In certain forms of the present technology, dedicated stiffening members or rigidising members (e.g., with no other function) may be included on the plenum chamber 3200. These members may be formed from a material more rigid than the plenum chamber 3200 (e.g., than the silicone). The dedicated stiffening members may be overmolded to the plenum chamber 3200 to provide more stiffness than the rigid portion 3263 of the shell 3250 or the arms 3320.

### 5.3.3 Support portions

As best seen in Figs. 12 and 14 - 18, in one form of the technology, support portions 3260 are provided on opposite sides of the interface 3000 between the second seal forming structure 3102 and an anterior wall of the plenum chamber 3200. As shown in Fig. 12, in an example each support portion 3260 extends to a lateral edge of the interface.

The support portions 3260 do not act as undercushions and are instead configured to resist or hinder compression in the anterior-posterior direction. The support portions 3260 thereby support and/or stiffen portions of the second seal forming structure 3102 which engages the patient's lip superior. In particular, the support portions 3260 may support and/or stiffen regions of the second seal forming structure 3102 that may contact an area 1010 of the patient's face proximate the entrances to the nares where the ala meets the area above the lip superior, as shown in Fig. 20. In other words, the area 1010 may be directly inferior to each of the lower corners of the patient's nose.

The support portions 3260 assist in ensuring that creases do not form in the seal forming structure 3100. Creases in a seal forming structure may form as a result of a very flexible seal forming structure, with a large radius of curvature, conforming to a patient's face. The seal forming structure may fold over itself, or crease, as a result of being too flexible, and lead to leaks in the seal forming structure. Creasing may be particularly of concern where the seal forming structure seals against the area 1010 of the patient's face. The support portions 3260 may be particularly advantageous when the seal forming structure is configured to create a corner and/or ridge 3120 as described herein. The corner and/or ridge 3120 may be a sharper curve (e.g., a curve with a lower radius of curvature) as compared to seal forming structures without the support portions 3260. The support and/or stiffness added by the support portions 3260 decreases the ability of the second seal forming structure 3102 from conforming to the patient's face. In order to retain comfort for the patient, the corner and/or ridge 3120 is selected and/or sized to substantially match the geometry (e.g., contours) of the patient's face. For example, the seal forming structure 3100 for a particular patient may be selected from a variety of sizes in order to substantially conform to the nasal alar region (i.e., proximate to the area 1010). The sharper curvature permits the second seal forming structure 3102 to seal against the various crevices around the patient's nose with reduced likelihood that creases will form.

As seen in Figs. 14-16 in particular, in one form of the technology the support portions 3260 are connected to the anterior side of the oral portion 3201 of the plenum chamber adjacent the boundary 3241 of the oral portion 3201 and the nasal portion 3202. In some embodiments the support portion 3260 may be curved when viewed in cross-section parallel to a sagittal plane (as shown in Figs. 16-18) and/or when viewed in cross-section parallel to a frontal plane (as shown in Figs. 14 and 15). The curvature may be positive or negative. In the illustrated example, the curvature may be negative (e.g., with respect to the patient's nose). In some examples, a lateral side wall portion 3245 of the plenum chamber 3200 may curve inwardly adjacent the boundary 3241 with the nasal portion 3202, and the support portion 3260 may be substantially contiguous with an adjacent lateral side wall portion 3245. As shown in Fig. 18, when viewed in cross-section parallel to a sagittal plane, at least a portion of the support portion 3260 may reduce in thickness between a first end 3261 adjacent the anterior wall of the plenum chamber 3200 and a second end 3262 adjacent the seal forming structure 3100. For example, the support portion 3260 may be thicker proximate to the first end 3261, which may assist in providing increased support and/or stiffness to the second seal forming structure 3102. In some examples, the support portion 3260 may vary between a thickness of 0.1 mm (e.g., proximate to the second end 3262), and a thickness of 3.5 mm (e.g., proximate to the first end 3261). In some examples, the support portion 3260 may vary between a thickness of 0.3 mm (e.g., proximate to the second end 3262), and a thickness of 3 mm (e.g., proximate to the first end 3261). In some examples, the support portion 3260 may vary between a thickness of 1.3 mm (e.g., proximate to the second end 3262), and a thickness of 2.5 mm (e.g., proximate to the first end 3261).

Support portions 3260 with different geometries may be used for different patients. For example, patient's that require more support and/or stiffness in the second seal forming structure 3102 may use a seal forming structure 3100 with a thicker (e.g., proximate to the first end 3261 and/or at any location along the length) and/or more curved (e.g., lower radius of curvature) support portion 3260. For example, patients that want a more flexible second seal forming structure 3102 may use a seal forming structure 3100 with a thinner (e.g., proximate to the first end 3261 and/or at any location along the length) and/or less curved (e.g., greater radius of curvature) support portion 3260.

As seen in particular in Figs. 14 and 15, in one form of the technology the support portion 3260 is connected to the oral portion 3201 of the plenum chamber adjacent a boundary of a lateral side wall portion 3245 of the oral portion 3201 and a lateral side wall portion 3246 of the nasal portion 3202.

In some forms of the technology, the support portions 3260 are shaped to provide a substantially clear flow path from the oral portion 3201 of the plenum chamber to the nasal aperture(s) 3135 during inspiration. In some forms of the technology no part of either support portion 3260 is directly inferior to the nasal aperture(s) 3135.

### 5.3.4 Overtightening Support

As shown in Figs. 43-45, the patient interface 3000 may include stopper ribs 6040 within the cavity 3272 of the plenum chamber 3200. The stopper ribs 6040 may be disposed within the oral portion 3201 of the plenum chamber 3200 and provide support around the patient's mouth. The stopper ribs 6040 may be formed integrally with the plenum chamber 3200 (e.g., during the same molding process) so that the stopper ribs 6040 are formed in one piece with the remainder of the plenum chamber 3200.

As shown in Fig. 43, the patient interface 3000 may include multiple stopper ribs 6040 that extend at least partially around the perimeter of the oral portion 3201 of the plenum chamber 3200 and the first seal forming structure 3101. As illustrated in Fig. 43, the stopper ribs 6040 may extend only partially around the patient interface 3000 (e.g., in a C-shape or U-shape), and no stopper ribs 6040 may be disposed proximate (e.g., directly anterior to) the lip superior portion 3131. In other forms, stopper ribs 6040 may extend around the entire perimeter of the oral portion 3201 and the first seal forming structure 3101, or may extend around a different portion of the oral portion 3201 and the first seal forming structure 3101 that what is shown in Fig. 43.

With continued reference to Fig. 43, the stopper ribs 6040 are spaced apart from one another, so that no stopper rib 6040 is in contact with any of the other stopper ribs 6040. In some forms, the stopper ribs 6040 may be equally spaced so that an equivalent distance exists between each pair of adjacent stopper ribs 6040. In other forms, the stopper ribs 6040 may be unequally spaced, so that at least some pairs of stopper ribs 6040 are closer together than other stopper ribs. For example, the stopper ribs 6040 may be closer together in locations that contact regions of the patient's face more adapted to receive force (e.g., proximate the nasolabial sulcus).

As shown in Figs. 44 to 45-1, the stopper ribs 6040 may be elongated members that extend from an inner surface of the plenum chamber 3200 toward the seal forming structure 3100. The stopper ribs 6040 may be connected to the oral portion 3201 of the plenum chamber 3200 and at least partially spaced from the first seal forming structure 3101 in a resting or non-use position. For example, an edge 6042 (e.g., a posterior edge) of each stopper rib 6040 may be inclined (e.g., not perpendicular to the anterior-posterior direction. This may provide added spaced between the stopper ribs 6040 and the first seal forming structure 3101 in the unused position.

In use, the patient's face contacts the seal-forming structure 3100 and pushes the first seal forming structure 3101 in the anterior direction. Because each patient may individually tighten the headgear straps 3354 (described in more detail below), patient's may overtighten the headgear straps 3354 and cause the seal-forming structure 3100 to press too firmly against the patient's face. This could result in patient discomfort and/or may degrade the quality of the seal if the overtightened seal-forming structure 3100 folds or wrinkles and allows pressurized air to escape the plenum chamber 3200. To limit the occurrence of overtightening, the stopper ribs 6040 are spaced apart from the first seal forming structure 3101 to allow only a predetermined amount of movement in the anterior direction. For example, the first seal forming structure 3101 contacts each stopper rib 6040 at the edge 6042 along its width (e.g., narrow width, measured substantially parallel to the first seal-forming structure 3101). Because the stopper ribs 6040 are longer than they are wide, the stopper ribs 6040 act as stiffening members and limit the total distance that the first seal forming structure 3101 can move. The stopper ribs 6040 may also be thicker in any direction than the first seal forming structure 3101. This may allow the stopper ribs 6040 to be sized so that a patient may tighten the headgear straps 3354 to an intended point to form a desired seal, but not beyond in order to minimize discomfort and/or seal degradation. The inclination of the edge 6042 may allow the patient's face enough room to fit comfortably within the plenum chamber 3200, while also limiting excess room (e.g., which can lead to overtightening), and provide support for the patient's face. The stopper ribs 6040 may also include a curvature along the surface of the width (see e.g., Fig. 45-1).

Additionally, each stopper rib 6040 may extend more than half way (e.g., two-thirds, three-quarters, etc.) along the length of the first seal forming structure 3101. This may help to ensure that movement is limited across substantially all of the first seal forming structure 3101.

As shown in Figs. 47-49, the patient interface 3000 may include a stiffened region 6044 within the cavity 3272 of the plenum chamber 3200. The stiffened region 6044 may be disposed within the oral portion 3201 of the plenum chamber 3200 and provide support around the patient's mouth. The stiffened region 6044 may be formed integrally with the plenum chamber 3200 (e.g., during the same molding process) so that the stiffened regions 6044 are formed in one piece with the remainder of the plenum chamber 3200.

The stiffened regions 6044 may be formed on an anterior surface 6046 of the plenum chamber 3200 within the cavity 3272, and proximate the lateral sides of the oral portion 3201. The stiffened regions 6044 may be positioned directly across from the patient while wearing the patient interface 3000. The stiffened regions 6044 may be substantially symmetrical on either side of the oral portion 3201 and may extend around a side region 6048 of the plenum chamber 3200.

As shown in Fig. 47, the stiffened regions 6044 may be substantially the same height as the inlet port 3604. In other words, the stiffened regions 6044 may extend substantially along the length of the oral portion 3201 of the plenum chamber 3200. In some forms, the thickness may vary along the stiffened regions 6044. For example, the stiffened regions 6044 may be thicker proximate to the inlet port 3604, and may decrease in thickness distal to the inlet port 3604 (although this may also be reversed). In other examples, the stiffened regions 6044 may be thicker proximate an inferior portion of the oral portion 3201, and thinner proximate a superior portion of the oral portion 3201 (e.g., proximate to the nasal portion 3202), although these may be reversed.

As shown in Fig. 47-1, some forms of the stiffened regions 6044 may be between approximately 0.01 mm and approximately 10 mm thicker than the surrounding unstiffened regions. In some forms, the stiffened regions 6044 may be between approximately 0.1 mm and approximately 5 mm thicker than the surrounding unstiffened regions. In some forms, the stiffened regions 6044 may be between approximately 1 mm and approximately 2 mm thicker than the surrounding unstiffened regions. In some forms, the stiffened regions 6044 may be approximately 1.8 mm thicker than the surrounding unstiffened regions. In some forms, this may be a constant thickness across the stiffened region 6044, while in other examples this may be a maximum across a variable thickness of the stiffened region 6044.

As shown in Figs. 48 and 49, an alternate version of the stiffened region 6044 may be smaller than the stiffened region described in Fig. 47. In the alternate version, the stiffened region may be disposed proximate to an inferior portion of the oral portion 3201 of the plenum chamber 3200. The stiffened regions 6044 may also extend in an inclined direction. For example, each stiffened regions 6044 may be inclined along the superior-inferior direction so that the stiffened regions 6044 is more superior proximate to the inlet port 3604 than distal to the inlet port 3604.

Some forms of the stiffened regions 6044 of Figs. 48 and 49 may extend only along the anterior surface 6046 of the cavity 3272 and may not extend along the side regions 6048 of the plenum chamber 3200. The stiffened regions 6044 may be disposed directly in front of the patient's face during use. As illustrated in Fig. 49, the stiffened region 6044 may extend up to the side regions 6048 (e.g., be adjacent to or contact the side regions 6048), but may not extend around the side regions 6048.

As shown in Fig. 48, the thickness of the stiffened regions 6044 may be vary across its surface. For example, the stiffened regions 6044 may be thicker proximate to a center, and thinner proximate to the sides. Although this may be reversed in other examples. In some forms, the stiffened regions 6044 may be stepped so that there is no gradual transition between the thicker and thinner portions. In other examples, the transition may be gradual.

While using the patient interfaces 3000 of Figs. 47-49, the patient's face contacts the seal-forming structure 3100 and pushes the first seal forming structure 3101 in the anterior direction. Because each patient may individually tighten the headgear straps 3354 (described in more detail below), patient's may overtighten the headgear straps 3354 and cause the seal-forming structure 3100 to press too firmly against the patient's face. This could result in patient discomfort and/or may degrade the quality of the seal if the overtightened seal-forming structure 3100 folds or wrinkles and allows pressurized air to escape the plenum chamber 3200. To limit the occurrence of overtightening, the stiffened regions 6044 are spaced apart from the first seal forming structure 3101 to allow only a predetermined amount of movement in the anterior direction. For example, the stiffened regions 6044 and limit compression of the first seal forming structure 3101 in the anterior direction. The stiffening regions 6044 may act similarly to the stopper ribs 6040 and limit the total distance that the first seal forming structure 3101 can move. However, the stiffening regions 6044 may not provide a stop, and may instead limit compression because the thicker region requires more force to compress. The stiffened regions 6044 may be sized so that a patient may tighten the headgear straps 3354 to an intended point to form a desired seal, but not beyond in order to minimize discomfort and/or seal degradation.

### 5.3.5 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

Figs. 21 and 22 show embodiments which are provided with connectors 3310, e.g. magnetic connectors, for connection to a positioning and stabilising structure 3300.

In some forms, the straps of the positioning and stabilizing structure 3300 may be constructed from an extensible (e.g., elastic) material. The positioning and stabilizing structure 3300 may stretch in order to accommodate patients with different head sizes.

In some forms, the straps of the positioning and stabilizing structure 3300 may be constructed from an at least partially inextensible material. This may limit the ability of the straps to stretch. Different sized straps may be used with different sized patients in order to accommodate different sized heads. The straps of the positioning and stabilizing structure 3300 may also be length adjustable.

As illustrated in Figs. 23-2, 24-1, 42-2, 55-1, and 56-1, certain forms of the positioning and stabilizing structure 3300 may include a superior strap 3357 that overlays the patient's parietal bone and/or frontal bone in use. The superior strap 3357 may be length adjustable in order to accommodate different sized heads (e.g., using a buckle 3369). Each patient may tighten or loosen the superior strap 3357 in order to fit their individual head. Tightening or loosening the strap adjusts the tension in the superior strap 3357 in order to provide comfort for the patient wearing the positioning and stabilizing structure 3300.

### 5.3.5.1 Frame

As shown in Figs. 23 to 28-2, a frame 3350 is coupled to the plenum chamber 3200 and assists in maintaining the therapeutically effective position of the seal-forming structure 3100. The plenum chambers 3200 illustrated in Figs. 23 to 28-2 specifically illustrate an elbow 3500 connected in front of the patient's face, although the frame may be used with other styles of plenum chambers 3200 (e.g., plenum chambers 3200 of Figs. 21-22 used with conduit headgear).

In some forms, the frame 3350 is constructed from a rigid or semi-rigid material, and provides support to the seal-forming structure 3100 and/or the plenum chamber 3200. For example, the frame 3350 may assist in maintaining the shape of the seal-forming structure 3100 and/or the plenum chamber 3200 in order to reduce leaks of pressurized air as a result of folding and/or creasing as the seal-forming structure 3100 engages the patient's face.

In some forms, the frame 3350 provides at least one connection point 3352, which may assist in indirectly connecting the headgear straps 3354 to the plenum chamber 3200 and/or seal-forming structure 3100. The connection point 3352 may be a loop (e.g., with a fully formed perimeter) that receives a portion of the headgear straps 3354. For example, a length of a left superior headgear strap 3356 may be threaded through a first loop 3352a, and pulled away from the plenum chamber 3200 in order to apply tension through the left superior headgear strap 3356. The left superior headgear strap 3356 may be folded against itself and retained in the selected length (e.g., using Velcro, magnets, adhesives, etc.) in order to maintain the applied tension. Similar steps may be performed regarding adjusting the tension in the right superior headgear strap 3358 in a second loop 3352b. In other forms, the connection point 3352 may include an open perimeter (e.g., a U-shape or a C-shape).

In some forms, each loop 3352a, 3352b may be oriented so that a force vector applied by the respective superior headgear strap 3356, 3358 is substantially perpendicular to a loop inner surface 3351, against which the superior headgear straps 3356, 3358 contact. As shown in Fig. 23, the right superior headgear strap 3358 engages the loop 3352b in substantially the center of the loop inner surface 3351. When the right superior headgear strap 3358 is tightened, the force vector is applied in a substantially straight direction, and not oblique relative to the loop inner surface 3351. This may improve the sealing of the seal-forming structure 3100, as the forces are directed along the arms 3362, and not oblique relative to the arms 3362, which may require the superior headgear straps 3356, 3358 to be further tightened to receive the same sealing effects (e.g., at the detriment to patient comfort), and/or may prevent the seal-forming structure 3100 from properly engaging the patient's face (e.g., leading to leaks).

As shown in Figs. 41 and 42, the superior headgear straps 3356, 3358 may include different widths along its length. For example, a first portion of each strap 3356, 3358 may be formed with a first width 3359, and a second portion of each strap 3356, 3358 may be formed with a second width 3361 that is greater than the first width. In some forms, the majority of the superior headgear straps 3356, 3358 may be formed with the second width 3361.

Although not illustrated, the inferior headgear straps 3366, 3368 may also include different widths (e.g., and include a tapered shape) similar to the description of the superior headgear straps 3356, 3358. Thus, any description related to the superior headgear straps 3356, 3358 may be applicable to the inferior headgear straps 3366, 3368.

In the illustrated example, each superior headgear strap 3356, 3358 may taper between the first width 3359 and the second width 3361. The superior headgear straps 3356, 3358 may include substantially linear sides 3363 between the first width 3359 and the second width 3361. In other examples, the superior headgear straps 3356, 3358 may be curved between the first width 3359 and the second width 3361. In still other examples, the superior headgear straps 3356, 3358 may include no transition between the first width 3359 and the second width 3361, and may instead be stepped between the first and second widths 3359, 3361.

As illustrated in Figs. 41-1 and 41-2, additional feedback features may be used in order to alert the patient that the superior headgear straps 3356, 3358 are overtightened. The illustrated examples show these features incorporated independently into the superior headgear straps 3356, 3358 (e.g., in a strap with a substantially uniform width). However, the feedback features of Figs. 41-1 and 41-2 may be incorporated into the tapered structure in Fig. 41 (or a stepped strap).

As illustrated in Fig. 41-1, the superior straps 3356, 3358 may include dots 6128 (e.g., silicone glue dots). The dots 6128 may provide increased resistance as the strap passes through the respective loop 3352a, 3352b of the frame 3350. In other words, the dots 6128 may rub against the respective loop 3352a, 3352b and provide the patient with a tactile response, which may alert the patient that the superior straps 3356, 3358 are too tight.

In some forms, the dots 6128 may be raised from the surface of the straps 3356, 3358 in order to contact the loops 3352a, 3352b and provide the tactile response to the patient.

In the illustrated form, the dots 6128 are used with a strap that is substantially uniform in width. The width may be about the width of the respective loops 3352a, 3352b (e.g., at least a portion of the strap width may be slightly larger than the width of the loops 3352a, 3352b). If the patient attempts to pull either superior strap 3356, 3358 through the respective loop 3352a, 3352b, the dots 6128 will provide further resistance to movement through the loops 3352a, 3352b. For example, the patient may feel the vibrations of the dots 6128 contacting the loops 3352a, 3352b.

In an alternate form, the dots 6128 are used in addition to the tapered shape of the superior straps 3356, 3358. The dots 6128, therefore, may act as an additional signal to the patient that the superior strap 3356, 3358 is overtightened. In other words, the dots 6128 may be positioned on the portion of each superior strap 3356, 3358 having the second width 3361. If the patient attempts to pull the second width 3361 of either superior strap 3356, 3358 through the respective loop 3352a, 3352b, the dots 6128 will provide further resistance to movement through the loops 3352a, 3352b.

In some forms, the dots 6128 may be evenly distributed on the superior straps 3356, 3358. In other forms, the dots 6128 may be unevenly distributed. For example, the concentration of dots 6128 may increase in a direction away from the free end of the respective strap 3356, 3358. This may provide the patient with an initial warning that overtightening is possible and may transition into a greater number of dots 6128 in order to alert the patient that overtightening has occurred.

In other examples (see e.g., Fig. 41-1-1), the dots 6128 may be used to assist in guiding the patient to the correct position. For example, the dots 6128 may be arranged in a linear pattern, evenly spaced apart. As the patient threads the strap 3356, 3358 through the respective loop 3352a, 3352b, the dots 6128 may provide feedback at each interval in order to communicate to the how far the strap has been threaded (e.g., similar to holes on a belt). In other words, the patient may adjust the strap to a particular position based on the number of discrete feedback signals that they receive.

In certain forms, this may assist in providing a more tailored adjustment for the patient's head. Instead of simply alerting to overtightening, the dots 6128 may alert the patient to the particular strap length beneficial to their head.

In certain forms, the shape, size, and/or density of the dots 6128 may change along the length of the strap 3356, 3358. For example, the dots 6128 may be evenly (or substantially evenly) spaced prior to overstretch in order to help the patient chose the appropriate length, as described above, The configuration of the dots 6128 may change (e.g., become more dense) at a position of the strap that would result in the patient overtightening the strap 3356, 3358. The change in the configuration of the dots 6128 may create a different tactile response, which may alert the patient to the overtightening.

In certain forms, the dots 6128 may extend through substantially the entire length of the respective strap 3356, 3358. This may provide tactile cues to patients with a large range of head sizes. In this example, the dots 6128 may be substantially evenly spaced (although there may be changes in configuration as described above). Instead of alerting to a particular overtightening positon, the dots 6128 may allow the patient to consistently select a desired position.

Although the description above relates to "dots", any suitable shape may be used. For example, the silicone glue (or other similar material) may include a triangular shape, a rectangular shape, or any other similar shape.

As illustrated in Fig. 41-2, the superior straps 3356, 3358 may include cut edges 6132 (e.g., squared cuts, triangular cuts, circular cuts, etc.). The cut edges 6132 may provide the patient with a tactile response, which may alert the patient that the superior straps 3356, 3358 are too tight.

In the illustrated form, the cut edges 6132 are used with a strap that is substantially uniform in width. The width may be about the width of the respective loops 3352a, 3352b (e.g., at least a portion of the strap width may be slightly larger than the width of the loops 3352a, 3352b). If the patient attempts to pull either superior strap 3356, 3358 through the respective loop 3352a, 3352b, the changes between the full width and the discontinuity of the cut edges 6132 will provide further indication to the patient to stop tightening the superior straps 3356, 3358 through the loops 3352a, 3352b.

In other forms, the cut edges 6132 are used in addition to the tapered shape of the superior straps 3356, 3358. The cut edges 6132, therefore, may act as an additional signal to the patient that the superior strap 3356, 3358 is overtightened. In other words, the cut edges 6132 may be positioned on the portion of each superior strap 3356, 3358 having the second width 3361. If the patient attempts to pull the second width 3361 of either superior strap 3356, 3358 through the respective loop 3352a, 3352b, the changes between the full second width 3361 and the discontinuity of the cut edges 6132 will provide further indication to the patient to stop tightening the superior straps 3356, 3358 through the loops 3352a, 3352b.

In some forms, the cut edges 6132 may be evenly distributed along a portion of the superior straps 3356, 3358. In other words, the distance between each adjacent cut on the respective cut edge 6132 may be equal. In other forms, the cut edges 6132 may be unevenly distributed. For example, the distance between each of the cuts and/or the size of the cuts on the cut edges 6132 may decrease in a direction away from the free end of each superior strap 3356, 3358.

In other examples, the cut edges 6132 may be used to assist in guiding the patient to the correct position. For example, the cut edges 6132 may be arranged in a linear pattern, evenly spaced apart. As the patient threads the strap 3356, 3358 through the respective loop 3352a, 3352b, the cut edges 6132 may provide feedback at each interval in order to communicate to the how far the strap has been threaded (e.g., similar to holes on a belt). In other words, the patient may adjust the strap to a particular position based on the number of discrete feedback signals that they receive.

In certain forms, this may assist in providing a more tailored adjustment for the patient's head. Instead of simply alerting to overtightening, the cut edges 6132 may alert the patient to the particular strap length beneficial to their head.

In certain forms, the shape, size, and/or density of the cut edges 6132 may change along the length of the strap 3356, 3358. For example, the cut edges 6132 may be evenly (or substantially evenly) spaced prior to overstretch in order to help the patient chose the appropriate length, as described above, The configuration of the cut edges 6132 may change (e.g., become smaller and/or closer together) at a position of the strap that would result in the patient overtightening the strap 3356, 3358. The change in the configuration of the cut edges 6132 may create a different tactile response, which may alert the patient to the overtightening.

In certain forms, the cut edges 6132 may extend through substantially the entire length of the respective strap 3356, 3358. This may provide tactile cues to patients with a large range of head sizes. In this example, the cut edges 6132 may be substantially evenly spaced (although there may be changes in configuration as described above). Instead of alerting to a particular overtightening positon, the cut edges 6132 may allow the patient to consistently select a desired position.

In use, each superior headgear strap 3356, 3358 is threaded through the respective loop 3352a, 3352b as described above. The first width 3359 may be selectively inserted through the opening of the respective loop 3352a, 3352b because the first width is less than the width of the loops 3352a, 3352b. At least some of the transition may also have a width less than the width of the loops 3352a, 3352b, so that the transition may also be received through the openings. However, the second width 3361 may be greater than the width of the loops 3352a, 3352b, and may be unable to slide through either opening. The larger second width 3361 may provide a stop, and limit the total length that may be threaded through the respective loop 3352a, 3352b. This may reduce patient overtightening because the second width 3361. In some forms, the length of the superior headgear straps 3356, 3358 with the first width 3359 (or the length of the first width 3359 plus the transition 3363) may be designed for a patient with a larger head. Thus, the superior headgear straps 3356, 3358 may be adjusted for ideal tightness at or before the second width reaches the respective loop 3352a, 3352b. The superior headgear straps 3356, 3358 may be under more tension if they are adjusted to the second width 3361 in patients with smaller heads. However, the second width 3361 may still prevent the patient from overly tightening the superior headgear straps 3356, 3358, and limit the seal-forming structure 3100 from deforming and creating leaks. Alternatively, different sized positioning and stabilizing structure 3300 may be constructed for patients with different sized heads (i.e., so that the second width 3361 acts as a stop to limit overtightening for patients with a variety of head sizes).

In other examples, strap extenders may be connected to the superior headgear straps 3356, 3358 to provide a greater length of the first width 3359. This may allow a single positioning and stabilizing structure 3300 designed for a smaller patient's head to be used with a larger patient's head.

In some forms, the strap extenders may have double-sided connectors. The illustrated example shows the same type of connector on either side, although the connector could be different on each side. For example, the strap extender may include hook and loop material. In some forms, loop material is formed on both sides while in other forms hook material is formed on both sides. In still other forms, hook material is formed on one side and loop material is formed on the other.

In some forms, the strap extenders may be used to provide extra length so that a patient does not overstretch the straps (e.g., either the superior straps 3356, 3358 and/or the inferior straps 3366, 3368).

In certain forms, the strap extenders may include similar features to the straps shown in Figs. 41 to 41-5. For example, the strap extenders may include a tapered shape as in Fig. 41, or may include a stepped shape. This may limit the distance that the strap extenders can pass through the loops 3352a, 3352b in order to prevent overtightening. The straps extenders may also include the dots 6128 (see e.g., Fig. 41-1) and/or the cut edges (see e.g., Fig. 41-2) as described above in order to provide an indication (e.g., a tactile response) of overtightening. The strap extenders may also include the hidden portion 3365 similar to Figs. 41-3 to 41-5, which may provide visual and/or tactile indications of overtightening. The hidden portion 3365 may alternatively be referred to as an alerting portion.

Any of the features described in Figs. 41 to 41-5 and included in a strap extender may alert the patient to adjust the strap extender relative to the headgear 3354. In some forms, this may involve adjusting the connected position of the strap extender relative to the respective strap on the headgear (e.g., the superior straps 3356, 3358 and/or the inferior straps 3366, 3368). For example, the patient may connect the strap extender closer to the portion with the second width 3361 in order to shorten the effective length of the strap or may connect the strap extender closer to the portion with the first width in order to increase the effective length of the strap.

In certain forms, straps of the headgear 3354 (e.g., the superior straps 3356, 3358 and/or the inferior straps 3366, 3368) may include an extended length of a connector configured to interface with a connector on the strap extender. For example, the superior straps 3356, 3358 may include hook or loop material along at least a portion of the first width 3359 and a/or a portion of the second width 3361.

The patient may adjust the position of the strap extender along the length of the respective strap in order to select the proper usable length for the patient (e.g., so that there is a sufficient sealing force without overextending the strap).

In certain forms, there may be different sized strap extenders (e.g., small, medium, large) so that a patient may make more tailored adjustments to their specific head and reduce the change of overstretching the strap extender.

Also, or in addition, the superior headgear straps 3356, 3358 may be constructed from an elastic material. The elastic material may change in visual appearance (e.g., may change in color) as a result of stretching under tension. The expansion of the headgear straps 3356, 3358 could also provide a tactile response to the patient that the straps 3356, 3358 are overstretched. This change in visual appearance may alert the patient (or a third party like a bed partner) that the superior headgear straps 3356, 3358 are over tensioned. This may be beneficial when the patient has fully tightened the superior headgear straps 3356, 3358 prior to the second width reaching the respective loop 3352a, 3352b.

For example, Figs. 41-3 to 41-5 illustrate examples of a strap (e.g., superior headgear straps 3356, 3358) which may include features that alert the patient that the straps have been overly stretched, which can alert the patient that the straps should be loosened and/or that a different sized (e.g., larger) headgear strap 3354 is needed.

In Fig. 41-3, the example strap is illustrated in a first position. This may be a position prior to use by the patient where the strap is un-stretched. Fig. 41-3 may also illustrate a strap tensioned to a correct length. For example, the strap may be used by the patient and adjusted to an appropriate length so that it is not overly stretched.

In Fig. 41-4, the example strap is illustrated in a second position, which is stretched and/or extended from the initial position. As described above, the strap may include a portion 3365 that is ordinarily hidden but becomes exposed in the second position. For example, the hidden portion 3365 may be elastic and stretches to an extended length when a tensile force applied to the strap exceeds a threshold value. The hidden portion 3365 may be a different color and/or texture in order to alert the patient that the tension in the strap has exceeded a predetermined limit. This change in appearance and/or feel may alert the patient that the strap is over stretched.

In some forms, the hidden portion 3365 may be luminescent and may glow when exposed. In a dark room, the glow from the hidden portion 3365 may be observable so that the patient is alerted to the overstretch condition of the headgear straps 3354.

In some forms, the hidden portion 3365 may include a noise output device (e.g., a speaker) that outputs a sound when the hidden portion 3365 is exposed in order to alert the patient.

In some forms, the hidden portion 3365 may include an electrical component (e.g., a light emitting element) that outputs light when the hidden portion 3365 is exposed. The output of light may alert the patient that the headgear strap 3354 is overstretched. In certain forms, the headgear strap 3354 may include a battery (not shown) and/or an electrical wire for providing power to the electrical component.

In some forms, the patient may be unable to see that the strap has exceeded the threshold tension and that the hidden portion 3365 is exposed (e.g., when donned by the patient, the straps may no longer be visible to the patient). As illustrated in Fig. 41-5, the strap may include a tactile response element 3367, which may provide a tactile response to the patient in order to provide an indication that the tension in the strap has exceeded the particular threshold.

In certain forms, the tactile response element 3367 are a pair of magnets. The magnets 3367 are connected to one another when the strap is in the first position. When the tension in the strap exceeds the magnetic force, the magnets 3367 separate and the hidden portion 3365 is exposed. Thus, the strap may be under tension and may stretch (e.g., if elastic) prior to the magnets 3367 separating.

When the magnets 3367 separate, the patient may receive a disenable response. This could be in the form of vibrations along the strap from the magnets 3367 disconnecting and/or in an auditory signal. Either or both of these may alert the patient that the strap is too tight. Removing the excess tension in the strap allows the magnets 3367 to reconnect to one another.

In other forms, the tactile response element 3367 may be an alternate connector (e.g., a mechanical connector). For example, the tactile response element 3367 may be constructed from plastic and/or silicone. Instead of a magnetic force, the tactile response element 3367 may be connected with a mechanical connection (e.g., a snap-fit), or a similar connection (e.g., hook and loop connectors). These alternate tactile response elements 3367 can have a retention force that allows for some extension below a separation threshold before the tactile response elements 3367. Like the magnets 3367, the separation of the tactile response element 3367 may create vibrations that the patient can detect and determine that the strap is overstretched.

In some forms, the tactile response element 3367 may be wider than the strap. This may provide additional visual cues for the patient to determine that the strap tension has exceeded the threshold.

Although not illustrated, the overstretch feature shown and described in Figs. 41-3 to 41-5 may also be incorporated into other straps on the headgear strap 3354. For example, the inferior headgear straps 3366, 3368 may be tightened on the magnet in a similar way such that overtightening could occur. Additionally or alternatively, the adjustable superior strap 3357 (see e.g., Figs. 23-2, 24-1, 42-2, 55-1, and 56-1) may include a hidden portion 3365 to alert the patient to overtightening. The adjustable superior strap 3357 may also include a tapered strap in order to limit overstretching. The tapered strap may be used with a strap extender in order to allow a single headgear strap 3354 to be used with multiple sized heads.

As shown in Figs. 41-6 to 41-8, the headgear straps 3354 may utilize a double-sided connecting member 6136 in order to allow the patient to adjust the usable length. As illustrated in Fig. 41-6, the double-sided connecting member 6136 may include hook material (or loop material) on both sides (e.g., first side 6136-1 and second side 6136-2 may be functionally the same). The patient may connect the hook material to loop material (or hook material) of the headgear straps 3354 at a desired location. For example, the entire length of the headgear straps 3354 may include the hook material so that there are an infinite number of locations to position the double-sided connecting members 6136. The patient may adjust this position as desired. For example, the patient may repeatedly connect and disconnect the double-sided connecting member 6136 from the headgear straps 3354. Once connected, the patient may use the headgear straps 3354 like other headgear straps 3354 and fold the free end of the strap back on itself in order to connect the exposed side of the double-sided connecting member 6136 to the headgear strap 3354 (e.g., in use, both sides are connected to the respective headgear strap 3354).

Because the double-sided connecting members 6136 may include the same connecting material on either side, the patient may connect either side to the headgear straps 3354.

In some forms, the double-sided connecting member 6136 may be used with headgear straps 3354 that otherwise lack affixed connectors.

As illustrated in Fig. 41-7, the double-sided connecting members 6136 may be positioned at a first location (e.g., proximate to the free ends of the various straps). This may create a longer usable length of the headgear straps 3354. For example, a patient with a larger head may attach the double-sided connecting members 6136 in the first position in order to achieve an appropriate fit.

In the first position, a person with a smaller head may overtighten the headgear straps 3354 in order to achieve an appropriate fit. Thus, the patient may move the double-sided connecting members 6136 to the second position (i.e., away from the free ends of the various straps as illustrated in Fig. 41-8). This may create a smaller usable length so that a patient with a smaller head can achieve a snug fit with the same headgear straps 3354 without overtightening.

In certain forms, the patient may cut the free ends of the straps beyond where the double-sided connecting members 6136 are located. In other forms, the excess free ends may remain so that a different patient (e.g., with a different sized head) may readjust the double-sided connecting members 6136 to fit their head.

In certain forms, the double-sided connecting member may have hook material on one side and loop material on the other so that the two sides are not functionally the same.

As shown in Fig. 27-2, certain forms of the loops 3352a, 3352b may include an eyelet cut 3353, which may be formed on the patient side of the respective loop 3352a, 3352b (e.g., the side proximate to the patient's skin while in use). The eyelet cut 3353 may form a region of reduced thickness along the perimeter of the respective loop 3352a, 3352b. The eyelet cut 3353 may extend around a portion of the perimeter of the respective loop 3352a, 3352b (e.g., less than 360°). In the illustrated example, the respective headgear strap 3354 may contact the eyelet cut 3353 when received through the loop 3352a, 3352b. The reduced thickness of the eyelet cut 3353 may result in less material being used to manufacture the loops 3352a, 3352b, which may lead to lower manufacturing times and/or lower manufacturing costs. The eyelet cut 3353 may also (or instead of) lead to reduced skin marks, and improved patient comfort.

In one form, at least one of the loops 3352a, 3352b may not be completely formed around an outer perimeter. In other words, the loops 3352a, 3352b may be C-shaped and/or U-shaped. The left and/or right superior headgear straps 3356, 3358 may be individually folded against themselves, and then inserted through into the respective loop 3352a, 3352b. This may allow the patient to maintain the same length adjustment in the respective superior headgear strap 3356, 3358, when the seal-forming structure 3100 is being removed from the therapeutically effective position.

As shown in Fig. 35-2, certain forms of the frame 3350 include a loop 3352 with a raised profile. For example, the illustrated loop 3352b includes a raised portion 3390 proximate to the arm 3362b. The sleeve 6124 may curve as a result of connecting to the arm 3362b. The raised portion 3390 may mirror the curvature of the sleeve in order to provide a more constant interface between the loop 3352b and the sleeve 6124.

The raised portion 3390 may include a rounded (e.g., semi-circular shape). In some forms, the raised portion 3390 may protrude about 0.01 mm to about 1 cm. In some forms, the raised portion 3390 may protrude about 0.05 mm to about 5 mm. In some forms, the raised portion 3390 may protrude about 0.1 mm to about 3 mm. In some forms, the raised portion 3390 may protrude about 0.5 mm to about 1.5 mm. In some forms, the raised portion 3390 may protrude about 1.2 mm.

As shown in Figs. 52-54, certain forms of the frame 3350 include alternate examples of loops 6080. In the illustrated example, the loops 6080 may include a dual-eyelet shape. For example, each loop 6080 may include the shape of two adjacent loops 3352a, 3352b with a dividing wall 6084 formed between. The dividing wall 6084 may divide the loop 6080 into a first loop section 6080-1 and a second loop section 6080-2. The first loop section 6080-1 may be adjacent to the respective arm 3362, and the second loop section 6080-2 may form a free end of the frame 3350. The dividing wall 6084 may not completely extend across the width of the loop 6080 so that each loop 6080 includes a single opening (e.g., as opposed to two openings if the dividing wall 6084 completely extended across). In other words, the first and second loop sections 6080-1, 6080-2 are not completely separated and a strap may move between the sections. The dividing wall 6084 may be formed in two segments with a central space 6088 formed in between.

As shown in Fig. 53, the superior headgear straps 3356, 3358 may be connected to the respective loop 6080 in a similar manner as described above with respect to the loops 3352a, 3352b. For example, the superior headgear straps 3356, 3358 may be threaded through the respective loop 6080, and apply a substantially perpendicular force in relation to the dividing wall 6084 in order to hold the seal-forming structure 3100 in the proper sealing position.

With continued reference to Fig. 53, the dividing wall 6084 may assist the patient in limiting overtightening of the superior headgear straps 3356, 3358. The width of each superior headgear strap 3356, 3358 may be greater than the space between the dividing wall 6084. Each superior headgear strap 3356, 3358 may rest on the respective dividing wall 6084 and provide the posteriorly-directed forced when the straps are tightened. While the superior headgear straps 3356, 3358 remain slack or within a range of desired tension, the superior headgear straps 3356, 3358 contact the dividing wall 6084 and remain within the respective first loop sections 6080-1.

As shown in Fig. 54, the superior headgear straps 3356, 3358 may move into the second loop section 6080-2 if the user applies excess tension (e.g., tightens the respective superior headgear straps 3356, 3358 upon the upper limit of the range of desired tensions). As a result of the excess tension, the superior headgear straps 3356, 3358 may bend or fold. In other words, the excess force pulls the respective superior headgear straps 3356, 3358 through the central space 6088 and into the second loop section 6080-2. The stiffness in the superior headgear straps 3356, 3358 may resist this bending motion (e.g., being pulled through the central space 6088) while the applied force remains within or below the range of desired tensions. Once the tension exceeds this range, the applied tension exceeds the stiffness of the superior headgear straps 3356, 3358 and causes the center of each superior headgear strap 3356, 3358 to bend (i.e., because the centers are unsupported by the dividing wall 6084). Once the superior headgear straps 3356, 3358 move through the central space 6088, the superior headgear straps 3356, 3358 contact the second loop section 6080-2 and apply the perpendicular force. The movement of the superior headgear straps 3356, 3358 may alert the patient or a third party (e.g., via visual and/or tactile stimulation) that the superior headgear straps 3356, 3358 are tightened to tight. The patient may loosen the superior headgear straps 3356, 3358 and reposition the superior headgear straps 3356, 3358 against the respective dividing wall 6084.

The superior headgear straps 3356, 3358 may also decrease in tension as a result of moving from the first loop section 6080-1 to the second loop section 6080-2 because the superior headgear straps 3356, 3358 do not need to be stretched as much while in the second loop section 6080-2. This may momentarily reduce the tension in the respective superior headgear strap 3356, 3358. However, there may be nothing to prevent further increases in tensioning.

As shown in Fig. 55, certain forms of the frame 3350 may include ladder locks 6092 in place of the loops (either loops 3352a, 3352b or loops 6080). The superior headgear straps 3356, 3358 may be threaded through the respective ladder lock 6092 in order to tighten the seal-forming structure 3100 against the patient's face. In some forms, the ladder locks 6092 may be molded to the arms 3362 of the frame 3350. In other examples, the ladder locks 6092 may be separate elements (e.g., if connection two straps together, like on a superior region of the patient's head). For example, the housing 3371 may include a ladder lock 6092.

As shown in Fig. 56, certain forms of the frame 3350 may include a stopper 6096 that may assist in adjusting the length of the superior headgear straps 3356, 3358. The stopper 6096 may be connected to either arm 3362 and used in place of the loops (e.g., the loops 3352a, 3352b or the loops 6080). Each superior headgear strap 3356, 3358 may be threaded through the respective stopper 6096. The stopper 6096 may include a spring-loaded button 6100 that secures the respective superior headgear strap 3356, 3358 in position. The patient may actuate the button 6100 and move the respective superior headgear strap 3356, 3358 (e.g., either tighten or loosen) in order to establish an appropriate fit. For example, the housing 3371 may include a stopper 6096.

As shown in Figs. 24-28, some forms of the frame 3350 includes a central portion 3360 that is coupled to the plenum chamber 3200. The central portion 3360 may have an annular shape, and may have a profile that corresponds to the shape of the plenum chamber 3200 (e.g., approximating a positive domed curvature).

In one form, a single size of the central portion 3360 may be used with a variety of sizes of plenum chambers 3200 and/or seal-forming structures 3100. For example, the seal-forming structure 3100 may come in multiple sizes (e.g., small, medium, large, etc.) and/or shapes (e.g., narrow, wide, etc.) in order to better seal against patients with a variety of facial shapes. An engagement region of the central portion 3360 may remain substantially the same regardless of the size of the plenum chamber 3200 and/or seal-forming structure 3100. Thus, the central portion 3360 may be coupled to a variety of shaped and/or sized cushions, and provide substantially the same support.

In one form, the central portion 3360 may be removable coupled to the plenum chamber 3200. A patient may use the same frame 3350 with multiple plenum chambers 3200. This may be useful when the patient is first beginning the therapy, and is trying different sized plenum chambers 3200, in order to find an appropriate fit. Removing the frame 3350 may also be helpful when cleaning the patient interface 3000, as the different elements of the patient interface 3000 may be cleaned separately, to help ensure a more thorough clean.

In other forms, different frames, with different sized and/or shaped central portions, may be used with different sized plenum chambers 3200 and/or with different sized patients in order to provide a comfortable fit for a wide variety of patients. As shown in Figs. 36-39, the central portions 13360, 23360 of the frame 13350, 23350 may include shapes to allow for comfortable sealing when used by a patient with a larger nose length (e.g., distance measured in the anterior-posterior direction between the patient's alar crest point and pronasale).

As shown in Fig. 36, the frame 13350 may include a central portion 13360 that is smaller than the central portion 3360 of Fig. 25. For example, a superior bar 16036 on the frame 13350 may be disposed inferior to the similar superior bar 6036 when the respective frames 3350, 13350 are connected to the plenum chamber 3200. This may allow a superior portion of the plenum chamber 3200 (e.g., the nasal portion 3202) and/or seal-forming structure 3100 (e.g., the second seal forming structure 3102) to move or flex when worn by the patient. This may be helpful for patients with larger noses. While a higher superior bar 6036 (e.g., like in Fig. 25) may offer more rigidized support, the plenum chamber 3200 and/or seal-forming structure 3100 may not be able to move a sufficient amount to receive a larger nose. Lowering the height of the superior bar 16036 (e.g., in Fig. 36), provides for the flexion and better fit in a patient with a larger nose.

As shown in Figs. 37-39, the frame 23350 may include a central portion 23360 with a superior bar 26036 that is out of plane from the remainder of the central portion 23360. For example, the superior bar 26036 may extend in the anterior direction from the remainder of the central portion 23360 (e.g., when connected to the plenum chamber 3200). Thus, the superior bar 26036 may be spaced apart from the nasal portion 3202 of the plenum chamber 3200. The central portion 23360 may include a substantially similar footprint as the central portion 3360 so that it may be used with the same plenum chamber 3200. However, the spacing between the plenum chamber 3200 and the superior bar 26036 may allow the plenum chamber 3200 and/or the seal-forming structure 3100 to deform further and receive a larger nose comfortably.

In some forms, the superior bar 26036 may still provide support to the patient in use. For example, the patient may deform the nasal portion 3202 of the plenum chamber 3200 as a result of wearing the patient interface 3000 so that the nasal portion 3202 contacts the superior bar 26036. The superior bar 26036 may be far enough away in the neutral (i.e., unworn) position in order to allow the nasal portion 3202 to comfortably expand when worn by the patient, but contact the superior bar 26036, which provides support and limits over-bending of the nasal portion 3202. This may simulate the frame 3350, but in a size conducive for patients with larger noses.

In some forms, the frame 3350 includes arms 3362 that extend away from the central portion 3360. The loops 3352a, 3352b are formed at ends of the arms 3362. In use, the arms 3362 may extend at least partially in the posterior direction, which may position the loops 3352a, 3352b more posterior than the plenum chamber 3200 and/or the seal-forming structure 3100. The arms 3362 may also extend in the lateral direction (e.g., left or right respectively) so as to generally extend along contours of the patient's face.

In some forms, the arms 3362 engage a portion of the patient's face while the patient interface 3000 is worn by the patient. For example, the arms 3362 may contact the patient's cheeks. The arms 3362 may be shaped in order to correspond to the curvatures of a patient's face (e.g., extend in posterior and lateral directions).

In some forms, the arms 3362 may not substantially stretch as a result of the tension applied by the respective headgear strap 3356, 3358 via the respective loop 3352a, 3352b (e.g., the arms 3362 may be rigidizers and/or may be inextensible). Tension may be transferred along the arm 3362 to the plenum chamber 3200 and/or seal-forming structure 3100 in order to maintain the therapeutically effective pressure and limit leaks from occurring.

In one form, the arms 3362 are constructed from a material that is more flexible than the material used to construct the central portion 3360. The two materials may be molded together so that the frame 3350 is constructed in an integral, one-piece construction. The arms 3362 may have some rigidity in order to assist in maintaining their shape. However, the arms 3362 may be bendable so that a patient may adjust the shape in order to correspond to their facial structure. Allowing a patient to adjust the shape of the arms 3362 may increase the comfort experienced by the patient, which may increase patient compliance with the therapy. In this way, the arms 3362 may bend or flex relative to the central portion 3360 (e.g., because of a cantilevered configuration), but may be unable to stretch further in the posterior direction (e.g., because of its inextensibility). Additionally, the relatively flexible material used to construct each of the arms 3362 may assist in reducing facial markings, and increase patient comfort.

In one form, the arms 3362 and the central portion 3360 are constructed from the same material. This material provides enough flexibility in order to permit shape adjustment, and also provides enough rigidity in order to maintain the adjusted shape. The central portion 3360 may be more rigid than the arms 3362 as a result of being coupled to the plenum chamber 3200. In addition or instead of, the central portion 3360 may be thicker than the arms 3362, which may also cause an increased rigidity of the central portion 3360. Each arm 3362 is formed as a cantilevered shape, so that the ends proximate to the respective loop 3352a, 3352b are unsupported. Additionally, the thickness of the frame 3350 may decrease along the length of each arm 3362 in the direction of the respective loop 3352a, 3352b (see e.g., Fig. 27-1). This may provide each arm 3362 with the flexibility necessary to be bent and/or shaped in order to substantially correspond to a shape of the patient's face (e.g., cheek). Decreasing the width of each arm 3362 may also reduce cheek contact between the respective arm 3362 and the patient's cheek, which may improve patient comfort. Decreasing the width along the length of each arm 3362 may also allow for greater flexibility proximate each respective loop 3352a, 3352b.

In one form, a sleeve 6124 may be used with the arms 3362 to provide additional comfort to the patient. As shown in Fig. 42-1, a sleeve 6124 may extend along substantially along the length of both arms 3362. For example, each sleeve 6124 may extend from the fixed end of each arm 3362 proximate to the central portion 3360 of the frame 3350. Each sleeve 6124 may extend toward the free end of each arm 3362 proximate to the loops 3352 (or 6080) of the frame 3350.

In the illustrated example, the sleeves 6124 may not cover the central portion 3360 of the frame 3350 or the loops 3352 (or 6080). In this way, the sleeves 6124 may not obscure straps from being connected to the loops 3352, and/or may not obscure the central portion 3360 from being connected to the plenum chamber 3200. In other examples, a sleeve could be used with central portion 3360 (e.g., so that the patient interface 3000 had the appearance of bedclothes).

In some forms, the sleeves 6124 may be constructed from a comfortable material, which may be flexible and/or soft to the touch. As described above, the arms 3362 may be bent and/or shaped in order to conform to the shape of the patient's face (e.g., the patient's cheeks). As a result, the at least a portion of the arms 3362 are in close contact with the patient's skin. By including the sleeves 6124, the patient's skin may be contact with material that is comfortable to wear for long periods of time. The material may be a textile and/or a foam, or any similar material.

In certain forms, the sleeves 6124 may be constructed from an elastomeric material. This may allow the sleeves to stretch over the loops 3352 so that the sleeves 6124 can be removed from the arms 3362 (e.g., to be cleaned). In other examples, the sleeves 6124 may wrap around the arms 3362 with a hook and loop, magnetic, or mechanical connection. The connection may be disconnected in order to remove the sleeves 6124 from the arms 3362.

In one form, each loop 3352 may include a raised portion 3390 with a rounded shape. The raised portion 3390 may include a smooth surface in the direction from the loop 3352 and toward the arm 3362, which may allow the sleeve 6124 to slide past the loop 3352 without snagging. An end 3392 of the raised portion 3390 proximate to the arm 3362 may be formed as a projection or overhang. This may limit the sleeve from sliding in the reverse direction (i.e., disconnecting from the loop frame 3350 by sliding over the loop 3352).

In other forms (not shown), the arms 3362 may include a comfortable material only on inner surfaces (e.g., surfaces include the scallop 3373 (see e.g., Fig. 27)) of the arms 3362. Instead of a sleeve, this may be a piece of material that is connected to the surface that contacts the patient's skin during use. This material may be connected to the arms 3362 with an adhesive and can be removed and/or replaced with a new piece of material (e.g., a clean piece of material).

In some forms, the fixed end of each arm 3362 may have a thickness of between approximately 2 mm and approximately 7 mm. In some forms, the fixed end of each arm 3362 may have a thickness of between approximately 2.5 mm and approximately 6 mm. In some forms, the fixed end of each arm 3362 may have a thickness of between approximately 3 mm and approximately 5 mm. In some forms, the fixed end of each arm 3362 may have a thickness of approximately 4 mm.

In some forms, the free end of each arm 3362 may have a thickness of between approximately 0 mm and approximately 4 mm. In some forms, the fixed end of each arm 3362 may have a thickness of between approximately 0.5 mm and approximately 3 mm. In some forms, the fixed end of each arm 3362 may have a thickness of between approximately 1 mm and approximately 2.5 mm. In some forms, the fixed end of each arm 3362 may have a thickness of approximately 2 mm.

As shown in Fig. 27-3, some forms of the cross-section of each arm 3362 may be substantially rectangular. Corners (e.g., one corner, two corners, four corners, etc.) of the substantially rectangular shape may be rounded. This may assist in improving patient comfort by reducing sharp surfaces that contact the patient's skin. Each arm 3362 may also have a height that is greater than its thickness. The height may be designed to comfortably distribute pressure across the patient's skin (e.g., by increasing the height), without obstructing the patient's peripheral vision, or otherwise causing the patient discomfort.

In some forms, the height of each arm 3362 may be between approximately 5 mm and approximately 15 mm. In some forms, the height of each arm 3362 may be between approximately 6.5 mm and approximately 13.5 mm. In some forms, the height of each arm 3362 may be between approximately 8 mm and approximately 12 mm. In some forms, the height of each arm 3362 may be between approximately 9.5 mm and approximately 10.5 mm. In some forms, the height of each arm 3362 may be approximately 10 mm.

As illustrated in Fig. 35, some forms of the arms 3362 of the frame 3350 may include a rib 3394, which may extend from an inferior surface of each arm 3362. Each arm 3362 may include at least one rib 3394. For example, the illustrated frame 3350 includes one rib 3394 on each arm 3362, with the ribs 3394 disposed proximate to the central portion 3360 of the frame 3350. In other examples, there may be more than one rib 3394 per arm 3362 and/or the rib(s) 3394 may be disposed at different locations along the length of the arm 3362 (e.g., proximate to the respective loop 3352 and/or on a different surface of the arm 3362).

In some forms, each rib 3394 may include a rounded profile (e.g., having a semi-circular shape). The rounded profile may reduce or limit discomfort caused by a sharp or angular shape.

As described above, a sleeve 6124 may be positioned around each arm 3362 in order to provide additional comfort to the patient. The dimensions of the arm 3362 (e.g., the thickness, height, and cross-section described above) provide a surface for the sleeve 6124 to extend around. However, in some examples, the sleeve 6124 may be larger than the arm 3362 (e.g., as a result of passing over the loop 3352) and may sag on the respective arm 3362. For example, a height of the arm 3362 may be greater proximate to the loop 3352. This may look and/or feel unpleasant to a patient wearing the patient interface 3000. The rib(s) 3394 on each arm 3362 may provide additional height (and/or thickness depending on the positioning of the ribs 3394) to the respective arm 3362 in order to stretch out the sleeve 6124 and minimize slack (e.g., in order to match the height of the arm 3362 proximate to the loop 3352).

The smooth profile of the ribs 3394 may limit the sleeve 6124 from snagging and/or ripping on the ribs 3394. It may also allow the sleeve 6124 to be connected or removed from the arm 3362 without damaging the sleeve 6124.

As shown in Figs. 26-4 and 26-5, certain forms of the frame 3350 may permit pivotable movement between each arm 3362 and the central portion 3360. The pivotable movement may be different than the bending movement described above in that the a pivot point 6012 may connect each arm 3362 to the central portion 3360, and allow the entire arm 3362 to move substantially the same angular distance. Each arm 3362 may also be able to bend, in addition to pivoting, as described above.

In one form, the pivot points 6012 are disposed in approximately the same position on either side of the frame 3350. In other words, each arm 3362 may be connected to the central portion 3360 at substantially the same height, and each arm 3362 may extend approximately the same length past the pivot point 6012. This may allow the patient to make mirrored adjustments to the arms 3362. The patient may then flex or bend each arm individually in order to make adjustments specific to one side.

In one form, each pivot point 6012 is a living hinge. In other words, the frame 3350 may be constructed from a substantially uniform material (or a transition between each arm 3362 and the central portion 3360 may be constructed from a substantially uniform material). The thickness at each hinge 6012 may be significantly less than the thicknesses of the arm 3362 and the central portion 3360 immediately adjacent to the hinge 6012. For example, the hinge 6012 may be formed as a groove on a surface of the frame 3350 that faces away from the patient while in use. Uniformly and integrally constructing the hinge 6012 out of substantially the same material as the arms 3362 and the central portion 3360 may assist in reducing manufacturing costs (e.g., as compared to including a hinge constructed of a different material).

Each arm 3362 may be pivotable about the respective hinge 6012 between a first position and a second position. The loops 3352a, 3352b may be spaced closer together in the second position than in the first position.

In one form, the first position may be a relaxed position, and the second position may be a biased position. The arms 3362 may move to the second position when an external force is applied, and may return to the first position when the external force is removed.

In one form, the arms 3362 may also be positioned in either the first positon or the second position without continued application of an external force. The arms 3362 may be moved into the second position so that the arms 3362 overlap one another (e.g., like eyeglasses). This may assist in providing a small footprint for packaging and/or storage.

In other forms (not shown), each arm 3362 may be separate from the central portion 3360, and may be coupled to the central portion 3360 with a rotational hinge (e.g., a pin joint). In still other forms, each arm 3362 may be coupled to the central portion 3360 by overmolding a flexible in between, which may allow for some pivotable movement.

In some forms, the frame 3350 further includes at least one secondary connection point 3364 that is spaced apart from the loops 3352a, 3352b. The secondary connection point(s) 3364 provides an additional connection location, which may further assist in indirectly connecting the headgear straps 3354 to the plenum chamber 3200 and/or seal-forming structure 3100.

In certain forms, the frame 3350 includes two secondary connection points 3364 (e.g., a left secondary connection point 3364a, and a right secondary connection point 3364b). The secondary connection points 3364a, 3364b may be inferior to the loops 3352a, 3352b while the patient interface 3000 is worn by the patient. The headgear straps 3354 may further include a left inferior headgear straps 3366 and a right inferior headgear strap 3368, each configured to couple to the respective secondary connection point 3364a, 3364b. The headgear 3354 as a whole may then be able to provide a force to the superior and inferior regions of the seal-forming structure 3100 and/or the plenum chamber 3200.

In certain forms, the secondary connection points 3364a, 3364b are formed directly on the central portion 3360. The secondary connection points 3364a, 3364b may be more anterior than the loops 3352a, 3352b while the patient interface 3000 is worn by the patient.

In certain forms, the secondary connection points 3364a, 3364b may be constructed from a single component, which may assist in reducing tooling and/or manufacturing costs.

In certain forms, the left and/or right inferior headgear straps 3366, 3368 are removably coupled to the respective secondary connection point 3364a, 3364b. The secondary connection points 3364a, 3364b may be magnetic, and a left and/or right inferior headgear straps 3366, 3368 may be threaded through a housing 3371 that includes a magnet 3370 with an opposite polarity as the secondary connection points 3364a, 3364b. The housing 3371 may be flexible or semi-rigid to assist with coupling and better retaining the magnet in a connected position. The length of the left and/or right inferior headgear straps 3366, 3368 may be adjusted by folding the respective strap 3366, 3368 on itself around a crossbar 3386 (e.g., as done with the left and/or right superior headgear straps 3356, 3358). Additionally, the inferior headgear straps 3366, 3368 may include a first width 3359 and a second width 3361. The illustrated strap in Fig. 41 may be similarly utilized as part of the inferior headgear straps 3366, 3368. Thus, the inferior headgear straps 3366, 3368 may include the stop with the second width 3361 in order to prevent or limit overtightening. In some forms (not illustrated), the housing 3371 may include a pair of loops and the crossbar 3386 may not extend completely between the two loops. Thus, the housing 3371 may include the shape described in Figs. 52-54, and may similarly alert the patient when the inferior headgear straps 3366, 3368 are overtightened. This may be in combination with the inferior headgear straps 3366, 3368 having the first and second widths 3359, 3361, or may be used instead.

Alternatively, or in addition, the left and/or right inferior headgear straps 3366, 3368 may include the dots 6128 and/or the cut edges 6132 as illustrated on the superior headgear straps 3356, 3358 (see e.g., Figs. 41-1 and 41-2). The description of the dots 6128 and/or the cut edges 6132 on the superior headgear straps may also apply to the inferior headgear straps 3366, 3368 in order to alert the patient that the straps are too tight.

In other examples, the ladder locks 6092 and/or the stoppers 6096 may be connected to the frame 3350 in order to provide adjustment for the inferior headgear straps 3366, 3368.

Each magnet 3370 may be removed from the respective secondary connection point 3364a, 3364b, without changing the length adjustment of the left and/or right inferior headgear straps 3366, 3368. A patient may be able to don and/or doff the patient interface 3000 while only removing the magnets 3370 from the respective secondary connection points 3364a, 3364b (e.g., without having to remove the left and/or right superior headgear straps 3356, 3358 from the respective loops 3352a, 3352b).

As shown in Figs. 28-1 and 28-2, each secondary connection point 3364a, 3364b may be constructed in order to improve retention with the respective magnet 3370. An outer casing 3376 of each secondary connection point 3364a, 3364b may include a substantially planar (e.g., flat) surface 3378, and a lip 3380 extending from the planar surface 3378.

In the illustrated example, the outer casing 3376 may have a substantially elliptical shape, and the lip 3380 may extend proximate to a vertex of the planar surface 3378. The lip 3380 may also extend around only a portion of the planar surface 3378 (e.g., less than 360°). In some examples, the lip 3380 may extend less than 180° around the planar surface 3378 (e.g., the lip 3380 may not extend to either co-vertex of the substantially elliptical planar surface 3378).

As shown in Fig. 28-2, each secondary connection point 3364a, 3364b may be coupled to the frame 3350 so that the lip 3380 is disposed proximate to a center of the frame 3350. In other words, each secondary connection point 3364a, 3364b may be oriented so that the lip 3380 is disposed proximate to an elbow 3500 when the frame 3350 is coupled to the plenum chamber 3200.

The magnet 3370 includes a substantially planar surface that may engage the substantially planar surface 3378 of the outer casing 3376. While engaging the planar surface 3378, the magnet 3370 may be magnetically coupled to the magnetic element 3382 of the secondary connection point 3364a, 3364b. An overhang 3384 may be spaced apart from the magnet 3370. As illustrated, the overhang 3384 may be positioned on one side of the lip 3380 (e.g., an inner side) and the magnet 3370 may be positioned on the other side of the lip 3380 (e.g., an outer side). In use, the patient may adjust the length of a respective inferior headgear strap 3366, 3368. Tightening one of the inferior headgear straps 3366, 3368 may apply a force directed away from the center of the frame 3350 (e.g., in a laterally outward direction). This force may be greater than a magnetic force between the magnet 3370 and the magnetic element 3382, and may cause the magnet 3370 to move relative to the planar surface 3378.

The overhang 3384 may prevent the magnet 3370 from becoming disengaged from the magnetic material 3382 as a result of tightening the respective inferior headgear strap 3366, 3368. As the magnet 3370 begins to move, the overhang 3384 may contact the lip 3380, limiting further movement away from the center of the frame 3350. Engagement between the lip 3380 and the overhang 3384 therefore assist in reducing accidental disconnection of the magnet 3370 from the respective secondary connection point 3364a, 3364b when tightening the respective inferior headgear strap 3366, 3368, but does not limit the ability of the patient to move the magnet 3370 substantially perpendicular to the frame 3350 (e.g., in order to doff the positioning and stabilizing structure 3300).

As shown in Figs. 29-35, other constructions may be used in order to improve retention of the connections points 3364a, 3364b with the respective magnet 3370. The forms described in Figs. 29-35 may be used alone or in any combination with each other. Any of the forms described in Figs. 29-35 may also be used with the lip 3380 illustrated in Figs. 28-1 and 28-2.

As shown in Figs. 29 and 30, the oral portion 3201 of the plenum chamber 3200 may include at least one clip support 6016. As shown in Fig. 29, the clip support 6016 may be disposed radially outside of the groove 3280 proximate to an edge of the oral portion 3201. In some forms, a second clip support 6016 may be disposed on the other side of the plenum chamber 3200 in a mirrored location as the clip support 6016 illustrated in Fig. 29. The clip support 6016 may be spaced apart from the frame 3350 (and therefore the connection points 3364a, 3364b) when the frame 3350 is connected to the plenum chamber 3200.

As shown in Fig. 29, some forms of the clip support(s) 6016 may be spaced apart from the groove 3280 so that the distance between the respective connection point 3364a, 3364b and the clip support 6016 is approximately the length of the housing or connection member 3371. In other words, a length between the overhang 3384 (see e.g., Fig. 28-2) and the crossbar 3386 (see e.g., Fig. 28) is approximately the length between the respective connection point 3364a, 3364b and the clip support 6016.

With continued reference to Fig. 29, some forms of the clip support 6016 may project from the surface of the oral portion 3201. The clip support 6016 may be integrally formed with the oral portion 3201 in a one piece construction (e.g., using an injection molding process). Thus, the clip support 6016 may be formed from the same material as the oral portion 3201. For example, the clip support 6016 may be constructed from a silicone material and may be flexible, bendable, and/or compressible. The increased thickness of the clip support 6016 (e.g., as compared to the rest of the oral portion 3201) may limit the flexibility, bendability, and/or compressibility of the clip support 6016. The increased thickness may also enable the clip support 6016 to act as a further rigidizing member (e.g., in addition to the frame 3350) in order to further increase the stiffness of the oral portion 3201.

In some forms, the clip supports 6016 may also be curved or extend along an arcuate path. As shown in Fig. 29, the clip support 6016 may include a constant curvature (although varying curvatures may be utilized). The center of curvature may be the location of the respective connection point 3364a, 3364b.

As shown in Fig. 46, some forms of the clip supports 6016 may connect to the groove ribs 6052, and may be formed as a single molded piece. The shape may change along the length to separately form the clip supports 6016 and the groove ribs 6052. In other forms, the clip supports 6016 may be spaced apart from the groove ribs 6052.

As shown in Fig. 30, the frame 3350 may be connected to the oral portion 3201, and the magnet 3370 may be connected to the respective connection point 3364a, 3364b. The end of the connection member 3371 proximate to the crossbar 3386 may extend up to and/or slightly beyond the respective clip support 6016. In some forms where the connection member 3371 is rotatable relative to the frame 3350, the rotation of the connection member 3371 may substantially follow the curvature of the clip support 6016, so that the connection member 3371 extends the same distance relative to the clip support 6016 throughout its angular motion.

In some forms, a height of the clip support 6016 from the oral portion 3201 may be less than the distance between the crossbar 3386 and the oral portion 3201. Thus, the crossbar 3386 and the clip support 6016 may be slightly spaced apart in a neutral position. This spacing may provide clearance for the respective inferior strap 3366, 3368 to extend around the crossbar 3386. Once the respective inferior strap 3366, 3368 is connected to the respective connection member 3371, the connection member 3371 may be substantially close or touching the clip support 6016. This may limit or prevent the connection member 3371 from rocking and/or pivoting toward the surface of the oral portion 3201. The rocking or pivoting may occur if the lip 3380 and the overhang 3384 (see e.g., Fig. 28-2) are not fully engaged when the inferior straps 3366, 3368 are tightened, and/or if the patient presses against the connection member 3371 proximate to the crossbar 3386 (e.g., while the patient is sleeping). With the inclusion of the clip support 6016, the crossbar 3386 contacts the clip support 6016 prior to substantial rocking or pivoting movement. Thus, the magnet 3370 may remain connected to the respective connection point 3364a, 3364b if the patient inadvertently contacts the connection member 3371 in their sleep, therefore helping to ensure the seal-forming structure 3100 remains in its proper position. During use, the clip support 6016 may be at least partially covered so that the patient interface 3000 may maintain its compact appearance.

As shown in Fig. 31, an alternate form may include a clip support 6020 on the frame 3350 instead of on the plenum chamber 3200. The clip support 6020 may be integrally formed with the frame 3350. The clip support 6020 and the frame 3350 may be constructed from the same material, or different materials may be used to construct each (e.g., a separate material may be introduced into a mold in order to form the clip support 6020). The clip support 6020 may be shaped substantially similarly to the clip support 6016. For example, the clip support 6020 may extend beyond the respective connection point 3364a, 3364b by approximately the length of the connection member 3371. The clip support 6020 may also extend in a curved or arcuate path, which may include a constant curvature around the respective connection point 3364a, 3364b.

In some forms, the clip support 6020 may be at least partially flush with the central portion 3360 of the frame 3350. For example, an interface between a proximate end of the clip support 6020 and the central portion 3360 may be substantially flush so that the central portion 3360 and the clip support 6020 each have the same thickness. In other forms, the clip support 6020 may have a thickness that is less than the central portion 3360. In this case, the clip support 6020 may be flush with only a portion of the central portion 3360 (e.g., the clip support 6020 may be flush with either the upper or lower edge of the central portion 3360).

In some forms, a distal end 6021of the clip support 6020 opposite the proximate end may be curved or bent, and extend away from the surface of the oral portion 3201. The curve or bend at the distal end 6021 may form the similarly shaped projection in the clip support 6016 of Figs. 29 and 30. In other words, the distal portion 6021 may extend the same distance from the surface of the oral portion 3201. The engaged connection member 3371 may therefore be limited or prevented from rocking or pivoting in substantially the same manner as with the clip support 6016.

As shown in Figs. 32 and 33, the frame 3350 may include at least one rib 6024 proximate to each of the connection points 3364a, 3364b. The ribs 6024 may limit the rotational movement of the connection member 3371, which may maintain a desired orientation of the inferior straps 3366, 3368 (e.g., in order to maintain the proper force vectors).

As shown in Fig. 32, the central portion 3360 of the frame 3350 may include a pair of ribs 6024 proximate to each connection point 3364a, 3364b. In the illustrated example, one rib 6024 may be disposed superior to the respective connection point 3364a, 3364b, and one rib 6024 may be disposed inferior to the respective connection point 3364a, 3364b. In some forms, the ribs 6024 may be evenly spaced about the respective connection point 3364a, 3364b.

In the illustrated example, the ribs 6024 may be substantially rectangular in shape, although any similar shape (e.g., elliptical, circular, triangular, etc.) may be used. The ribs 6024 may project from the surface of the central portion 3360. In some forms, the ribs 6024 may extend to a height substantially equal to the height of the connection member 3371, although the ribs 6024 may extend to a position below an upper surface of the connection member 3371.

When connected, each connection member 3371 fits between the respective pair of ribs 6024. Each rib 6024 may be in contact with the respective connection member 3371, or may be substantially close to the respective connection member 3371. This limits or prevents the connection members 3371 from rotating about a perpendicular axis through each respective connection point 3364a, 3364b. This may assist in maintaining the desired orientation of the force vector of each inferior strap 3366, 3368, and also help to minimize accidental disengagement of the magnet 3370 from the respective connection point 3364a, 3364b.

As shown in Fig. 33, another form of the frame 3350 may include a rib 6024 disposed adjacent to the respective connection point 3364a, 3364b. This form may include only a single rib 6024 associated with each connection point 3364a, 3364b, although the pair of ribs 6024 from Fig. 32 may also be included. The single rib 6024 may be disposed such that it is approximately aligned with a center of the respective connection point 3364a, 3364b (e.g., neither superior nor inferior to the center of the connection point 3364a, 3364b).

The connection member 3371 may include a cut-out 6028 between the overhang 3384 and the crossbar 3386. The cut-out 6028 may be shaped to substantially correspond to the shape of the rib 6024. When the connection member 3371 engages the respective connection point 3364a, 3364b, the cut-out 6028 receives the rib 6024. The rib 6024 may contact the cut-out 6028, or be substantially close to the cut-out 6028 in order to prevent or limit rotational movement of the respective connection member 3371. Thus, the single rib 6024 may achieve substantially the same benefit as the multiple ribs 6024.

As shown in Figs. 34 and 35, the connection points 3364a, 3364b may not be formed directly on the central portion 3360 of the frame 3350. Instead, the frame 3350 may include arms 6032 that extends radially outward from either side of the central portion 3360. Each arm 6032 may be connected to the frame 3350 at approximately the same position as the connection points 3364a, 3364b are connected in the previous examples (e.g., Fig. 28). Each connection point 3364a, 3364b may instead be connected to a distal end of the respective arm 6032.

Positioning the connection point 3364a, 3364b at an end of the arm 6032 may move the connection point 3364a, 3364b in the lateral and/or rearward direction. In some forms, the connection point 3364a, 3364b on either arm 6032 may be spaced apart from the central portion 3360 (e.g., measured along the surface of the arm 6032) between approximately 5 mm to approximately 50 mm. In some forms, the connection point 3364a, 3364b on either arm 6032 may be spaced apart from the central portion 3360 between approximately 10 mm to approximately 40 mm. In some forms, the connection point 3364a, 3364b on either arm 6032 may be spaced apart from the central portion 3360 between approximately 20 mm to approximately 30 mm. In some forms, the connection point 3364a, 3364b on either arm 6032 may be spaced apart from the central portion 3360 by approximately 29 mm. In some forms, the connection point 3364a, 3364b on either arm 6032 may be spaced apart from the central portion 3360 by approximately 21 mm.

In some forms, the arms 6032 may be integrally formed with the central portion 3360 with a one-piece construction. In this example, the central portion 3360 and the arms 6032 may be constructed from substantially the same materials. In other examples, the arms 6032 may be constructed from a different material, but integrally formed with the central portion 3360 (e.g., multiple materials are introduced during an injection molding process). In other examples, the arms 6032 may be formed using a separate process than the central portion 3360, and may be connected to the frame 3350 using an attachment means (e.g., an adhesive, a magnet, a mechanical fastener). In this example, the arms 6032 may be permanently connected, or may be removable.

As shown in Figs. 35 and 35-1, the arms 6032 may be substantially flush with an upper surface of the central portion 3360. This may assist in promoting a compact aesthetic because the arms 6032 do not project further than the frame 3350 in the anterior direction. This may also assist in promoting patient compliance as the compact design may not substantially interfere with the patient's line of sight.

The arms 6032 in Figs. 34 to 35-1 are arranged in a cantilevered configuration so that the distal end including the connection point 3364a, 3364b are unsupported. In some examples, the arms 6032 may be flexible or semi-rigid and may be capable of bending or flexing. For example, the arms 6032 may flex in the posterior direction as a result of the patient tightening the connected inferior straps 3366, 3368. While the arms 6032 may be capable of flexing, the total distance that the arms 6032 are capable of flexing may be minimal. This way, the arms 6032 may maintain the force vectors of the inferior straps 3366, 3368. If the arm 6032 flexes, the arm 6032 returns to its original position when the force is removed (e.g., when the magnet 3370 is removed from the respective connection point 3364a, 3364b).

As shown in Fig. 35-1, some forms of the arms 6032 may include a groove 6033, which may be positioned on an anterior and/or posterior surface. The groove 6033 may act as a hinge to assist in bending the respective arm 6032. The frame 3350 in Fig. 35-1 may be a separate frame, or the groove 6033 may be incorporated into the frames of Figs. 34 and/or 35 (or any other frame described herein).

In use, the patient may connect the connection members 3371 to the respective connection point 3364a, 3364b in a similar manner than when the connection points 3364a, 3364b are directly connected to the central portion 3360. Thus, there may be no impact on the patient's ability to connect the magnets 3370 and the connection points 3364a, 3364b. Additionally, creating a wider connection point 3364a, 3364b (i.e., spaced further apart from the central portion 3360 of the frame 3350) may limit the occurrence of the magnet 3370 unintentionally disengaging from the respective connection point 3364a, 3364b. For example, the at least partially flexible nature of the arms 6032 may allow for bending in the posterior direction, which may limit rocking of the connection member 3371 on the respective connection point 3364a, 3364b. Thus, the magnets 3370 are less likely to become accidentally disengaged, and the seal between the patient's face and the seal-forming structure 3100 may be better maintained.

In some forms, the arms 6032 may bend or flex relative to the central portion 3360. For example, the arms 6032 may bend inwardly toward the plenum chamber 3200 (and toward the patient's face when the patient interface 3000 is worn). The bending or flexing may be the result of the force of the positioning and stabilizing structure 3300 (e.g., tightening the straps 3366, 3368 may cause bending motion in the arms 6032). As a result of the bending, the arms 6032 may be disposed in close proximity to or in contact with the plenum chamber 3200 and/or the patient's face. This may help to reduce the occurrence of rocking or accidental disengagement of the magnets 3370.

In some forms, the connection points 3364a, 3364b on the arms 6032 may not include the lip 3380. Instead, the connection points 3364a, 3364b may be substantially planar. The displacement of the connection points 3364a, 3364b away from the central portion 3360 combined with the bendability of the arms 6032 may reduce the likelihood of the of the magnet accidentally disengaging during use. Specifically, the rearward positioning of the connection points 3364a, 3364b may reduce torque on the housing 3371 and magnets 3370, which may cause the decoupling.

As shown in Fig. 61, some forms of the patient interface 3000 may include a bendable housing 6104 that may removably connect the straps to the frame 3350. The bendable housing 6104 may be substantially similar to the housing 3371. For example, the bendable housing 6104 may include a magnet 6108 (or similar type of connector) to removably engage with a connection point 3364a, 3364b. Additionally, the bendable housing 6104 includes a crossbar 6112 to receive a strap (e.g., any one of straps 3356, 3358, 3366, 3368). A bendable section 6116 may be disposed between the magnet 6108 and the crossbar 6112. The bendable section 6116 may be a thin section of the bendable housing 6104, and may be able to resiliently flex without breaking. For example, the bendable section 6116 may bend about bending axis 6120 so that the crossbar 6112 pivots relative to the magnet 6108. Bending about the bending axis 6120 may occur as a result of tension applied in the respective strap around the crossbar 6112 when the magnet 6108 is connected to the respective connection point 3364a, 3364b. Movement about the bending axis 6120 may be preferable than movement of the magnet 6108 so that the housing 6104 does not accidentally disengage from the respective connection point 3364a, 3364b. In some forms, the bendable housing 6104 may be used in place of the arms 6032 (e.g., the bendable housing 6104 is used when the connections points 3364a, 3364b are on the central portion 3360). In some forms, the bendable housing 6104 may be used with the arms 6032.

The length of the bendable section 6116 may determine the total amount of bending allowed in the bendable housing 6104. Longer bendable sections 6116 may permit more bending than shorter bendable sections 6116. In some forms, the bendable section 6116 is between approximately 0.1 mm and approximately 20 mm. In some forms, the bendable section 6116 is between approximately 1 mm and approximately 10 mm. In some forms, the bendable section 6116 is approximately 5 mm.

As shown in Fig. 24, the engagement region of the plenum chamber 3200 may include a groove 3280. In the illustrated example, the groove 3280 may be included on the oral portion 3201 of the plenum chamber 3200, and may be radially outside of the central portion 3251. The central portion 3360 of the frame 3350 may be positioned within the groove 3280. The shape of the central portion 3360 may substantially correspond to the shape of the groove 3280, which may assist the patient in properly orienting the frame 3350 with respect to the plenum chamber 3200 (e.g., in examples where the frame 3350 is removably coupled to the plenum chamber 3200).

As shown in Figs. 37-39, a superior bar 6036 of the central portion 3360 may extend in the anterior direction from the remainder of the central portion 3360 and may be spaced apart from the groove 3280. In other words, the superior bar 6036 may not contact the groove 3280, or any other portion of the plenum chamber 3200 prior to use.

In some forms, the groove 3280 may have a completely formed perimeter with a substantially annular shape. The perimeter may have substantially the same length in any sized cushion.

In some forms, the groove 3280 is recessed relative to a remainder of the outer surface of the plenum chamber 3200. The recessed groove 3280 may not extend substantially into the plenum chamber 3200 and obstruct the patient's face. The groove 3280 may have substantially the same depth throughout its perimeter.

In some forms, a width of the groove 3280 may be less than a width of central portion 3360 of the frame 3350. The compliant nature of the cushion may allow the wider central portion 3360 to be received within the groove 3280. This may allow the central portion 3360 to couple to the groove 3280 via a press fit, friction fit, and/or snap-fit. The engagement between the groove 3280 and the central portion 3360 may assist in providing rigidity to the plenum chamber 3200 and/or the seal-forming structure 3100, because the rigidity of the frame 3350 (e.g., as compared to the plenum chamber 3200) may limit some flexibility of the plenum chamber 3200 alone.

In some forms, the cushion may be molded to the frame 3350 so that the groove 3280 may be created during a molding process. The material of the plenum chamber 3200 (e.g., silicone) may be molded at least partially around the central portion 3360 of the frame 3350, and may limit the central portion 3360 from being removed from the groove 3280.

As shown in Fig. 25, the frame 3350 may be constructed from multiple pieces (e.g., each constructed from a different material). For example, the central portion may be constructed from a first material (e.g., hard plastic). The arms 3362 may be coupled to the central portion 3360 (e.g., via glue, molding, etc.), and may be constructed from a second material (e.g., flexible plastic, foam, etc.) that is more flexible than the first material. A third material may be a magnetic material, and may be coupled to the central portion 3360 (e.g., via an adhesive). Once coupled together, the arms 3362 may be able to similarly move as in the integral, one-piece constructions described above.

As shown in Figs. 26 and 27, the frame 3350 may be constructed from a single piece of material. For example, the frame 3350 may be constructed from a TPE material, like Hytrel. As described above, the material used in constructing the frame 3350 may provide both rigidity and flexibility to the frame 3350. The magnets 3370 may be overmolded (or otherwise coupled to) the central portion 3360.

In some forms, the central portion 3360 includes slots 3372, which may be formed on either lateral side of the central portion 3360. The slots 3372 may have a generally elongated shape (e.g., rectangular, elliptical, etc.), and may be completely formed within the boundary of the central portion 3360.

As shown in Fig. 27, some forms of the central portion 3360 may include a tapered slot 3372. Specifically, an opening to the slot 3372 may be wider proximate a posterior surface of the central portion 3360 (e.g., the surface in contact with the plenum chamber 3200). The opening to the slot 3372 may decrease uniformly toward the anterior surface of the central portion 3360.

Fig. 27 also illustrates that some forms of the arm 3362a (or arm 3362b) may include an indent or scallop 3373. The scallop 3373 may be formed on an inner surface of the arm 3362a, and may be positioned proximate to the patient's skin when the positioning and stabilizing structure 3300 is worn by the patient. The scallop 3373 reduces the thickness along a portion of the arm 3362a, and may decrease the likelihood of developing sink marks in the arm 3362a during a manufacturing (e.g., injection molding) process. The scallop 3373 may also result in less material being used to manufacture the arm 3362a, which may lead to lower manufacturing times and/or lower manufacturing costs.

Returning to Figs. 26 to 26-2a, the plenum chamber 3200 may include projections 3284 on the oral portion 3201. The projections may be elongated, and may have a similar shape to the slots 3372 (e.g., tapered). The projections 3284 may be disposed within the groove 3280 so that they cooperate with the frame 3350 during assembly.

As shown in Fig. 40, the projections 3284 may extend substantially oblique to the anterior surface of the plenum chamber 3200. The anterior surface of the plenum chamber 3200 may be partially curved or angled, and the projections 3284 may extend in a non-perpendicular direction relative to the surface so that the projections 3284 are aligned along the insertion direction of the frame 3350. In other words, the projections 3284 may extend along the anterior-posterior direction when the patient interface 3000 is worn.

As shown in Figs. 26-2 and 26-2a, some forms of the projections 3284 may include an overhang 6000 that extends at least partially over the groove 3280. The overhang 6000 may be asymmetrically formed around the perimeter of the projection 3284. In other words, the overhang 6000 may extend further over the groove 3280 on one side than on the other side. As shown in Fig. 26-2a, the opposite side 6002 may extend outwardly and form a slight overhang. For example, the opposite side 6002 may extend between approximately 1° and approximately 20° (e.g., 10°) further than the example shown in Fig. 26-2, which may add between approximately 0.1 mm to approximately 5 mm (e.g., approximately 1 mm) of length to the opposing side 6002. This may provide additional retaining forces for keeping the frame 3350 in the groove 3280.

As shown in Fig. 40, the top surface of the projection 3284 may be slightly inclined so that the overhang 6000 may extend substantially parallel to a surface of the groove 3280. This may allow a greater portion of the projections 3284 to extend over the groove 3280. As described in more detail below, this may assist in better retaining the frame 3350 within the groove 3280 and limiting accidental disengagements.

In some forms, the groove 3280 may also be asymmetrical. The groove 3280 may include an undercut 6004 on one side of the projection 3284, and an inclined surface 6008. The overhang 6000 may extend further over the undercut 6004 than over the inclined surface 6008. The length of the overhang 6000 extending over the undercut 6004 may limit the ability of the frame 3350 to be removed in a perpendicular direction from the plenum chamber 3200. The angle of the inclined surface 6008 may be directed toward the overhang 6000, and limit the ability of the frame 3350 to be removed in an oblique direction.

When the frame 3350 is being assembled to the plenum chamber 3200 (e.g., via a press fit, friction fit, snap-fit, etc.), the patient may align the projections 3284 with the slots 3372, so that the projections 3284 are received within the slots 3372 in use. The wider openings of the slots 3372 proximate to the posterior surface may assist the patient in aligning each projection 3284 within the respective slot 3372. The projections 3284 may be slightly wider than the anterior opening of each slot 3372, but are able to be received within the slot 3372 because of the flexible properties of the plenum chamber 3200 (e.g., being constructed from a resilient material like silicone). The projections 3284 may slightly deform as they enter the respective slot 3372 (e.g., as a result of the slot 3372 narrowing).

Once the projections 3284 are through the slot 3372, the projections 3284 may substantially return to their original shape. For example, the overhang 6000 may deform (e.g., elastically deform) as the slot 3372 receives the projection 3284, and may return to its initial position once the central portion 3360 is received within the groove 3280. The patient may experience this deformation as a tactile response, in order to more easily observe proper connection between the frame 3350 and the plenum chamber 3200. The overhangs 6000 of the projections 3284 may be wider than the anterior opening of the respective slot 3372 in a relaxed or initial position.

Additionally, the width of the undercut 6004 may be substantially the same as the central portion 3360 so that the frame 3350 is snuggly received within the groove 3280. The flexible material of the plenum chamber 3200 may allow the undercut 6004 to be slightly smaller than the central portion 3360, so that the undercut 6004 may flex and receive the frame 3350 via the press-fit, friction fit, snap-fit, etc.

Thus, the frame 3350 may not be easily removed from the plenum chamber 3200. The patient may have to apply a force to the frame 3350 in order to remove it from the groove 3280. This user applied force may allow the frame 3350 to be moved in either the perpendicular or oblique direction, and overcome the retention provided by the overhang 6000. For example, the patient may lift the frame 3350 from the region of the central portion 3360 that contacts the inclined surface 6008 in order to provide a force for disengaging the frame 3350 from the plenum chamber 3200.

This force may exceed a force produced by normal movements of the plenum chamber 3200 and/or seal forming structure 3100. In other words, the seal-forming structure 3100 and/or plenum chamber 3200 may move relative to the frame 3350 while the projections 3284 are received within the respective slot 3372. This may allow the seal-forming structure 3100 and/or the plenum chamber 3200 to flex and conform to a patient's face without coming loose from the frame 3350, and inadvertently causing the slots 3372 to disengage from the projections 3284.

In other forms, the plenum chamber 3200 may be molded to the frame 3350, and the projections 3284 may result from the molding process in order to permanently retain the position of the frame 3350 relative to the plenum chamber 3200.

As shown in Fig. 26-3, some forms of the frame 3350 may be oriented in order to reduce cheek contact while the connected frame 3350 and plenum chamber 3200 are worn by the patient. For example, the arms 3362a, 3362b may be oriented (e.g., curved) in order to substantially compliment at least a portion of the patient's face (e.g., their cheek). In some examples however, the entire length of each arm 3362 may not contact the patient's skin as a patient may feel it is uncomfortable to have a foreign object in contact with their face. Instead, a portion of each arm 3362 may be spaced apart from the patient's face so that a smaller surface area of the arm 3362 contacts the patient's face. While maintaining substantially the same curvature in each arm 3362, moving a contact point between each arm 3362 and the plenum chamber 3200 (e.g., via a manufacturing process) may change the total length of each arm 3362 that contacts the patient's face. For example, if each arm 3362 contacts the plenum chamber 3200 closer to the elbow 3500, a maximum gap between each arm 3362 and the patient's face is greater. This means that a greater portion of each arm 3362 will be spaced apart from the patient's face (e.g., as opposed to if each arm 3362 contacted the plenum chamber 3200 distal to the elbow 3500). Less contact between the each arm 3362 and the patient's face may improve patient comfort.

Additionally, each arm 3362 contacting the plenum chamber 3200 proximate to the elbow, and therefore distal to the posterior surface 3112, may produce less support at the lateral portions 3111 of the plenum chamber 3200 (e.g., because the rigid or semi-rigid arm engages less of the lateral portion 3111). The lateral portions 3111 may therefore have a greater degree of flexibility, and may be able to better conform to the shape of a patient's nose and create an effective seal.

As shown in Figs. 26-4 and 26-5, some forms of the frame 3350 may include the hinges 6012 between each arm 3362 and the central portion 3360. The hinges 6012 may permit movement (e.g., pivotable movement) of the arms 3362 without affecting the connection between the frame 3350 and the plenum chamber 3200. In other words, pivoting either arm 3362 about the respective hinge 6012 may not cause the projection 3284 to disengage from the slot 3372.

In some forms, the patient may move the arms 3362 into the second position in order to provide a better fit. For example, the distance between the loops 3352a, 3352b in the first position may be larger than the size of an average patient's head. Accordingly, the patient may move the arms 3362 toward the second position in order to provide an appropriate fit (e.g., contact between the arms 3362 and the patient's head). The arms 3362 may be retained in the second position by adjustment of the superior headgear straps 3356, 3358 (i.e., the superior headgear straps 3356, 3358 may provide the external force).

Similarly to the arms 3362, the plenum chamber 3200 and/or the seal-forming structure 3100 may be larger than the size of the average patient's face. For example, the seal-forming structure 3100 may not snuggly engage the patient's mouth and/or nose. The plenum chamber 3200 and/or the seal-forming structure 3100 may also move toward a more compact position in order to better engage an individual patient's face.

As shown in Fig. 26-5, movement of the arms 3362 into the second position may directly cause the seal-forming structure 3100 and/or the plenum chamber 3200 to move to a more compact position. The arms 3362 may contact the plenum chamber 3200 along the second anterior wall portion 3242. The arms 3362 may also contact the second anterior wall portion 3242 proximate to a center of the plenum chamber 3200, and distal to the lateral portions 3111.

Pivoting movement of each arm 3362 about the hinge 6012 may provide an inwardly directed force to the plenum chamber 3200. This may reduce a distance between the lateral portions 3111 of the second seal-forming structure 3102, and snuggly position the second seal-forming structure 3102 against the patient's nose. Compressing the seal-forming structure 3100 and/or the plenum chamber 3200 may provide a better seal against the patient's face (e.g., by limiting the occurrence of leaks). Additionally, applying the inwardly directed force proximate to the center of the plenum chamber 3200 (e.g., as described with respect to Fig. 26-3) may limit creasing from forming in the seal-forming structure 3100 by limiting the total compression of the plenum chamber 3200.

As shown in Fig. 46, the plenum chamber 3200 may include a groove rib 6052, which may be disposed anterior to the groove 3280 when the patient interface 3000 is worn by the patient. The groove rib 6052 may be integrally formed with the plenum chamber 3200, and may be formed together during the molding process. The groove rib 6052 may project in the anterior direction from the surface of the plenum chamber 3200, and may form a lower boundary of the groove 3280. The groove rib 6052 may be thicker than the surrounding portions of the plenum chamber 3200 and may act as a stiffening member. The groove rib 6052 may limit bending or flexing of the lower part of the oral portion 3201. This may help maintain the shape of the groove 3280, which may help keep the frame 3350 within the groove 3280.

As shown in Fig. 28, the frame 3350 may be shaped similarly to the frame in Figs. 26 and 27. In other words, the frame 3350 may be constructed from a single material (e.g., with semi-rigid properties). The frame 3350 may be thicker proximate the central portion 3360, and thinner toward each respective loop 3352a, 3352b.

In some forms, the central portion 3360 of the frame 3350 may be substantially solid throughout, and may not include slots 3372, and the plenum chamber 3200 may not include projections 3284 within the groove 3280. Instead, the plenum chamber 3200 may include protrusions 3288 disposed radially inside of the groove 3280. The protrusions 3288 may be raised from the rest of the anterior surface of the plenum chamber 3200. The protrusions 3288 may also extend at least partially in a radially outward direction. In other words, the protrusions 3288 may extend at least partially over the groove 3280 (e.g., the protrusions 3288 are spaced apart from the groove 3280).

While assembling a removable frame 3350 to the plenum chamber 3200, a patient may be required to position the frame 3350 so that it extends into the groove 3280, and underneath of the protrusions 3288. Once the central portion 3260 is positioned, the protrusions 3288 may assist in retaining the central portion 3360 in place. To decouple the frame 3350 from the plenum chamber 3200, the patient may push on at least one of the protrusions 3288 (e.g., in a lateral direction toward the other protrusion 3288) so that the protrusion no longer extends over the groove 3280. In other forms, the plenum chamber 3200 may be molded to the frame 3350, the protrusions 3288 may prevent the central portion 3360 from being removed.

In some forms, the frame 3350 of any of the examples described above may be substantially flush with the outer surface of the plenum chamber 3200 when positioned within the groove 3280. The depth of the groove 3280 substantially corresponds to a thickness of the central portion 3360. Similarly, the shape of the central portion may substantially approximate the shape of the cushion as described above. The resulting assembly may have a substantially uniform surface. This assists in maintaining a low profile look of the patient interface 3000, because the frame 3350 is not projecting in front of the cushion where it may obstruct the patient's line of sight.

### 5.3.6 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO₂ by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

While no vent structures are shown in Figs. 7-18, embodiments of the technology shown in Figs. 7-18 may be provided with a suitable vent structure, for example in the plenum chamber (one example of which is shown in Fig. 21).

### 5.3.7 Decoupling structure(s)

In one form the patient interface 3000 includes at least one decoupling structure 3500, for example, a swivel or a ball and socket. In some examples, the decoupling structure may be an elbow 3500 that is connected (e.g., removably connected, permanently connected, etc.) to the plenum chamber 3200 (e.g., a plenum chamber inlet port).

As shown in Figs. 23-28, the central portion 3360 of the frame 3350 has an annular shape, so that a central region of the oral portion 3201 of the plenum chamber 3200 is not covered by the frame 3350. In some examples, the oral portion 3201 includes an opening for receiving the elbow 3500. The opening may be significantly wider than the opening for receiving the elbow 3500, so that the frame 3350 is completely spaced apart from the opening. In other words, there is a length between the inner edge of the central portion 3360 and the opening for receiving the elbow 3500. The arms 3362a, 3362b and the secondary connection portions 3342a, 3342b are each spaced apart from the opening and the elbow 3500, so that the headgear straps 3358 do not interfere with movement (e.g., rotation) of the elbow.

While not explicitly shown in the drawings of the plenum chamber shown in Figs. 7-19, those skilled in the art will appreciate that in practice an elbow 3500 may be provided (e.g., as illustrated in Fig. 23), and allow connection of the interface to an air circuit 4170.

As shown in Fig. 57, the elbow 3500 may be connected to the plenum chamber 3200 in order to provide airflow through the inlet port 3604. In the illustrated example, the elbow 3500 is indirectly connected to the plenum chamber 3200, although the elbow 3500 may be directly connected to the plenum chamber 3200 in other examples.

As shown in Figs. 57 and 58, the elbow 3500 is connected to a vent ring 3504. The vent ring 3504 may directly connect to both the plenum chamber 3200 and to the elbow 3500 in order to establish an indirect connection and an airflow path between the air circuit 4170 (via the elbow) and the patient (via the plenum chamber 3200).

As shown in Fig. 59, the vent ring 3504 includes a posterior surface 3508, an anterior surface 3512, and a ring body 3516 extending there between. In the illustrated example, the vent ring 3504 includes a substantially cylindrical shape where the posterior surface 3508 and the anterior surface 3512 are circular (although other shapes, like ovals, triangles, rectangles, etc., may be used). In some forms, the posterior surface 3508 and/or the anterior surface 3512 may extend beyond (i.e., have a larger diameter than) the ring body 3516. The ring body 3516 may thus be formed as a recessed groove.

In some forms, the posterior surface 3508 has a larger diameter than the anterior surface 3512 (although these may be reversed). In some forms, the diameter of the posterior surface 3508 may be between approximately 1 mm and approximately 100 mm. In some forms, the diameter of the posterior surface 3508 may be between approximately 10 mm and approximately 75 mm. In some forms, the diameter of the posterior surface 3508 may be between approximately 20 mm and approximately 50 mm. In some forms, the diameter of the posterior surface 3508 may be between approximately 30 mm and approximately 40 mm. In some forms, the diameter of the posterior surface 3508 may be approximately 39 mm.

In some forms, the ring body 3516 may extend in an inclined manner between the posterior surface 3508 and the anterior surface 3512. For example, the diameter of ring body 3516 may be greater proximate to the anterior surface 3512 than proximate the posterior surface 3508 (or vice versa). In some forms, the angle between the posterior surface 3508 and the ring body 3516 may be approximately the same as the inclination of the lip 3608. This may allow the vent ring 3504 to engage the lip 3608 in a sealing configuration.

Returning to Fig. 57, the vent ring 3504 may be connected to the plenum chamber 3200 by engaging the lip 3608. In the illustrated example, the length of the lip 3608 may be approximately the same as the length of the ring body 3516. The substantially similar angles in both the ring body 3516 and the lip 3608 allows the lip 3608 to rest adjacent and parallel to the surface of the ring body 3516. Because the lip 3608 is substantially the same length as the ring body 3516, the lip 3608 may contact the posterior and anterior surfaces 3508, 3512 while the vent ring 3504 is engaged with the plenum chamber 3200. The length of the lip 3608 may limit or prevent the vent ring 3504 from moving (e.g., along the port axis 3612) during use of the patient interface 3000. This contact may create a sealing engagement so that airflow cannot escape between the vent ring 3504 and the inlet port 3604.

With continued reference to Fig. 57, the vent ring 3504 may be connected to the plenum chamber 3200 such that the posterior surface 3508 is positioned within the cavity 3272 (e.g., within the pressurized environment in use), and the anterior surface remains outside of the cavity 3272 (e.g., exposed to the ambient in use). The flexible material of the plenum chamber 3200 may allow the vent ring 3504 to be partially inserted through the inlet port 3604 and into the operational position. Once properly positioned, the posterior surface 3508 may extend beyond the lip 3608. In other words, the diameter of the posterior surface 3508 is greater than a diameter of the lip within the cavity 3272 of the plenum chamber 3200. This may help to secure or lock the vent ring 3504 in place and limit or prevent accidental disengagement between the vent ring 3504 and the inlet port 3604.

In some examples, the diameter of the posterior surface 3508 may extend proximate to or adjacent to the inner surface of the plenum chamber 3200 within the cavity 3272. For example, the diameter of the posterior surface 3508 may substantially match the profile of the plenum chamber 3200. The close proximity between the posterior surface 3508 and the wall of the plenum chamber 3200, or may create two simultaneous areas of contact (i.e., the lip 3608 and the wall of the plenum chamber 3200). The multiple areas of contact may increase the total retention force of the vent ring 3504, thus making accidental removal more difficult.

In some forms, the combination of the vent ring 3504 and the lip 3608 may create a retention force between approximately 1 N and approximately 50 N. In some forms, the combination of the vent ring 3504 and the lip 3608 may create a retention force between approximately 20 N and approximately 45 N. In some forms, the combination of the vent ring 3504 and the lip 3608 may create a retention force between approximately 25 N and approximately 40 N. In some forms, the combination of the vent ring 3504 and the lip 3608 may create a retention force between approximately 30 N and approximately 35 N. In some forms, the combination of the vent ring 3504 and the lip 3608 may create a retention force of approximately 33 N. In some forms, the combination of the vent ring 3504 and the lip 3608 may create a retention force greater than approximately 20 N. The greater retention forces may limit accidental disengagement of vent ring 3504 during use (and maintain the flow of pressurized breathable gas to the patient).

In some forms, the vent ring 3504 may include openings or vent holes 3520, which may allow air (e.g., exhaled carbon dioxide) to exit the cavity 3272 of the plenum chamber 3200. In some forms, there may be at least two vent holes 3520. In other forms, there may be four or five vent holes 3520. As shown in Fig. 60, certain forms of vent holes 3520 may extend substantially around the entire perimeter of the vent ring 3504. In certain forms, the vent holes 3520 may be equally spaced around the perimeter of the vent ring 3504.

As shown in Figs. 57 and 58, the ring body 3516 may include an inner wall 3524 and an outer wall 3528. The outer wall 3528 may be inclined while the inner wall 3524 may be substantially perpendicular to the posterior and anterior 3508, 3512. The inner and outer walls 3524, 3528 may be spaced apart from another in order to form a groove 3532. The vent holes 3520 may extend through the posterior surface 3508 and into the groove 3532 in order to vent to the ambient.

In some forms, a ring seal 3536 may be positioned within the groove 3532. The ring seal 3536 may extend around an outer surface of the inner wall 3524 of the ring body 3516. In certain forms, the ring seal 3536 may not completely fill the groove 3532, and may leave space for air to escape the plenum chamber 3200 and exit to the ambient through the vent holes 3520.

In certain forms, a washer 3540 may be positioned in the groove 3532 and may contact the ring seal 3536. The washer 3540 may include an outer diameter greater than or equal to the outer diameter of the ring seal 3536 in order to obscure the ring seal 3536 from view (e.g., from a bed partner). The washer 3540 may appear visually similar (e.g., color, texture, etc.) in order to provide an appearance that the anterior surface 3512 is substantially continuous between the inner and outer walls 3524, 3528.

As illustrated in Fig. 57, the elbow 3500 may be inserted into the vent ring 3504. For example, the elbow 3500 may connect to the vent ring 3504 with a snap-fit, friction fit, and/or press fit when the elbow 3500 is inserted within the inner diameter of the inner wall 3524. The elbow 3500 may also include a flange 3544 with an outer diameter greater than the outer diameter of the inner wall 3524 but less than the inner diameter of the outer wall 3528. In other words, the flange 3544 may extend partially, but not completely, across the width of the groove 3532. Thus, the flange 3544 may contact the washer 3540 (or the ring seal 3536), but not completely block the flow path through the vent holes 3520. The contact between the flange 3544 and the washer 3540 may assist in holding the ring seal 3536 and the washer 3540 within the groove 3532.

As described above, in some forms, the elbow 3500 may extend beyond the posterior surface 3508 when fully connected to the vent ring 3504 (e.g., the elbow 3500 contacts the posterior surface 3508 in order to form the snap-fit). In this position, the elbow may (slightly) extend into the distance 6076 (Fig. 51) so that an anterior portion of the patient interface 3000 is closer to the patient's lips than the distance 6076. As shown in Fig. 57, the distance that the elbow 3500 extends beyond the posterior surface 3508 may be small and not substantially noticeable to the patient (e.g., the patient's lips may not be able to stretch and contact the elbow 3500).

In some forms, the force required to remove the elbow 3500 from the vent ring 3504 may be less than the force required to remove the vent ring 3504 from the inlet port 3604. Said another way, the elbow 3500 may be easier to remove than the vent ring 3504. However, the force require to disconnect the elbow 3500 may be large enough in order to limit accidental disengagement.

As shown in Fig. 50-1, an alternate form of the vent ring 3504 may include an offset 3506 that extends beyond the anterior surface 3512 of the vent ring 3504. In some forms, the offset 3506 may be about 0.1 mm to about 50 mm. In some forms, the offset 3506 may be about 1 mm to about 25 mm. In some forms, the offset 3506 may be about 2 mm to about 10 mm. In some forms, the offset 3506 may be about 5 mm.

The offset 3506 may cause the elbow 3500 to extend further from the posterior surface 3508 when connected to the vent ring 3504. In the illustrated example, an extension 3507 may further extend from the anterior surface 3512 and further space the elbow from the posterior surface 3508. With or without the extension 3507, the offset 3506 may limit the elbow from extending closer to the patient's lips than the posterior surface 3508. This may keep the distance 6076 as the largest distance anterior to the patient's lips, and limit feeling of uncomfortableness while wearing the patient interface 3000.

With continued reference to Fig. 57, the elbow 3500 may deliver a flow of pressurized gas 1500 into the plenum chamber 3200 through the inlet port 3604 (see e.g., Fig. 49). After passing through the elbow 3500, the pressurized gas 1500 may fill and pressurize the plenum chamber 3200. The patient may inhale the pressurized gas 1500 and exhale into the plenum chamber 3200. Exhaled gas 1600 (e.g., a mixture of CO₂ and water vapor) may leave the plenum chamber through the vent holes 3520 in order to exhaust to ambient and limit rebreathing (e.g., re-inhaling the exhaled CO₂). In order to escape, the exhaled gas 1600 must travel in the opposite direction as the pressurized gas 1500, which at least partially impede the flow of the exhaled gas 1600.

### 5.3.7.1 Internal Connection

As shown in Figs. 62 to 63-1, an alternate form of an elbow 3550 may be connected to the plenum chamber 3200 in order to provide airflow through the inlet port 3604. In the illustrated example, the elbow 3550 is indirectly connected to the plenum chamber 3200, although the elbow 3550 may be directly connected to the plenum chamber 3200 in other examples.

In some forms, the elbow 3550 may include an outer lip 3552 and an inner lip 3554. In the illustrated example, the outer lip 3552 extends substantially around the perimeter of the elbow 3550. The inner lip 3554 may be spaced apart from the outer lip 3552 and may extend around only a portion of the perimeter of the elbow 3550.

In some forms, the elbow 3550 may include at least one button 3556 that is selectively engagable by the patient. The illustrated example includes a pair of buttons 3556 spaced approximately 180° that can be simultaneously engaged by the patient (e.g., using their thumb and index finger).

In some forms, the buttons 3556 may be formed as a cantilever structure. For example, the buttons 3556 may be fixed proximate to a first end 3558 of the elbow (e.g., the end proximate to the plenum chamber 3200). The elbow 3550 may also include a channel 3560 that extends substantially around the perimeter of each respective button 3556. Each channel 3560 may create a free end of each button 3556 opposite of the fixed end.

In certain forms, the outer lip 3552 may intersect each button 3556 along the perimeter of the elbow 3550. The channels 3560 may create discontinuities so that the outer lip 3552 does not extend continuously around the perimeter of the elbow 3550.

In certain forms, the inner lip 3554 may be disposed only on the button 3556. For example, the inner lip 3554 may include two segments, each spaced approximately 180° apart. Additionally, the length of each segment of the inner lip 3554 may be approximately the width of each button 3556 (e.g., the distance across the button 3556 between the channel 3560).

In some forms, the cantilever structure of each button 3556 may allow each button to flex or bend when the patient applies a force. For example, each channel 3560 may allow the free end of the button 3556 to move relative to the remainder of the elbow 3550. The free end of each button 3556 may be able to move toward a center of the elbow 3550 (i.e., toward the opposite button 3556) as a result of the patient's applied force. The buttons 3556 (or the elbow 3550 in general) may be constructed from a resilient material so that the buttons 3556 return to their original position after application of the force.

As shown in Fig. 63, a vent ring 3562 may be substantially similar to the vent ring 3504 described above. Only some similarities and difference may be described below.

As illustrated in Fig. 63, the vent ring 3562 may include a central column 3564 that projects from the inner wall 3524. In other words, the central column 3564 may have a smaller perimeter than the inner wall 3524, and may include an opening to still allow fluid flow through the vent ring 3562. In some forms, the central column 3564 may project beyond the anterior surface 3512. In other words, an end of the central column 3564 may project away from the plenum chamber 3200 and may the anterior-most portion of the vent ring 3562.

In some forms, the central column 3564 may include a groove 3566, which may extend at least partially around the inner perimeter of the central column 3564. In the illustrated example, the groove 3566 may extend entirely around the central column 3564. In other examples, the groove 3566 may extend around only a portion of the central column 3564, or a projection may replace the groove 3566. The projection may similarly extend around at least a portion of the inner perimeter (e.g., the entire inner perimeter) of the central column 3564.

As shown in Fig. 63-1, the patient may insert the elbow 3550 into the vent ring 3562 in order to engage the two pieces (e.g., via a snap-fit). The first end 3558 of the elbow 3550 may have a smaller diameter than the central column 3564 and may be insertable into the central column 3564. In the illustrated example, the inner lips 3554 may be inclined in order to make insertion easier. Once inserted, the inner lip 3554 may be received within the groove 3566 and the outer lip 3552 may remain outside the central column 3564 (e.g., and act as a stop to limit additional insertion). Once received within the groove 3566, the inner lip 3554 may allow the elbow 3550 to rotated 360°. However, the elbow 3550 may not be disconnected from the vent ring 3562 because the inner lip 3554 locks the elbow 3550 in place. To remove the elbow 3550, the patient may engage the buttons 3556, a portion of which are not within the central column 3564. Actuating the buttons 3556 (e.g., simultaneously) may move the inner lip 3554 out of the groove 3566 so that the elbow 3550 may translate relative to the vent ring 3562 and be disconnected.

With continued reference to Fig. 63-1, the elbow 3550 may not extend past the posterior surface 3508 of the vent ring 3562 (e.g., further toward the cavity 3272 than the vent ring 3562). The groove 3566 may be positioned in the anterior direction (e.g., away from the patient's face) in order to space the elbow 3550 apart from the cavity 3272 in use. This may be further assisted by the offset 3506. By spacing the elbow 3550 apart from the cavity 3272, the elbow 3550 may not encroach on the distance 6076 (shown in Fig. 51).

### 5.3.7.2 External connection

As shown in Figs. 64 to 66-3, further alternate forms of an elbow may be connected to the plenum chamber 3200 in order to provide airflow through the inlet port 3604. The elbows may be indirectly connected to the plenum chamber 3200, although the elbows may be directly connected to the plenum chamber 3200 in other examples.

As shown in Fig. 64, one form of an elbow 3568 may include a lip 3570 that extends from the body of the elbow 3568. In the illustrated form, the lip 3570 may extend only partially around the perimeter of the elbow 3568, although in other examples, the lip 3570 may extend entirely around the elbow 3568.

With continuing reference to Fig. 64, the elbow 3568 may include a button 3572. The button 3572 may be similarly shaped to the button 3556 (e.g., a cantilever structure with a fixed end and a free end). Thus, the button 3572 may be movable relative to the rest of the elbow 3568.

In the illustrated example, the button 3572 may form a portion of the outer surface of the elbow 3568. For example, the shape of the button may substantially match the surrounding shape of the elbow 3568. This means that the button 3572 may be curved in order to substantially match the shape of the elbow 3568. When the button 3572 is in a relaxed position, the curvature of the button 3572 may be substantially aligned with the curvature of the elbow 3568. However, when the patient applies the external force to the button 3572, the button 3572 moves inwardly and the curvature of the button 3572 is moved out of alignment with the curvature of the elbow 3568.

Unlike the button 3556, the button 3572 may not include a lip. Instead, the button 3572 may include a finger 3574. The finger 3574 may be hook shaped and may be connected proximate to the fixed end of the button 3572. In the illustrated example, the finger 3574 may extend past the fixed end of the button 3572 and away from the free end. For example, the button 3572 may be cantilevered relative to a first end 3558, and the finger 3574 may be cantilevered relative to the button 3572. The first end 3558 may therefore be a fulcrum point disposed between the button 3572 and the finger 3574.

In some forms, each button 3572 may include a finger 3574 spaced part on opposite sides of the elbow 3568 (e.g., about 180° apart). The fingers 3574 may be wider than the remainder of the button 3572, and may be spaced apart by a first distance. In other words, the fingers 3574 may not form a portion of the perimeter of the surface of the elbow 3568. When actuating the buttons 3572, the patient pushes the free ends of the buttons 3572 toward one another. When this occurs, the fingers 3574 move further apart from one another (i.e., spaced apart by a second distance).

As shown in Fig. 65, another example of an elbow 3576 may be similar to the elbow 3568 described above. Only some similarities and differences between the elbows 3568, 3576 may be described below.

With continued reference to Fig. 65, the elbow 3576 may include at least one button 3578 and at least one finger 3580 (e.g., a pair of each). Both the button 3578 and the finger 3580 may project from the surface of the elbow 3576 and may not be included as forming part of a perimeter of the elbow 3576. The button 3578 may operate similarly to the button 3572. For example, the patient may engage the buttons 3578 to move them closer together. In doing so, the fingers 3580 may move from a first distance apart to a second distance apart that is greater than the first distance.

In some forms, an arm 3581 may extend from the surface of the elbow 3576 (e.g., perpendicularly) in order to connect the button 3578 and the finger 3580 to the surface of the elbow 3576. The arm 3581 is disposed between the finger 3580 and the button 3578 (e.g., halfway) and forms a pivot point or fulcrum. Engaging the button 3578 in order to move the finger 3580 causes pivoting movement about the arm 3581.

As shown in Fig. 66, a vent ring 3582 may be substantially similar to the vent ring 3562 described above. Only some similarities and difference may be described below.

As illustrated in Fig. 66, the vent ring 3582 may include a central column 3584 that projects from the inner wall 3524. In other words, the central column 3584 may have a smaller perimeter than the inner wall 3524, and may include an opening to still allow fluid flow through the vent ring 3582. In some forms, the central column 3584 may project beyond the anterior surface 3512. In other words, an end of the central column 3584 may project away from the plenum chamber 3200 and may the anterior-most portion of the vent ring 3582.

In some forms, the central column 3584 may include a lip 3586, which may extend at least partially around the outer perimeter of the central column 3584. In the illustrated example, the lip 3586 may extend entirely around the central column 3584. In other examples, the lip 3586 may extend around only a portion of the central column 3584, or a groove may replace the lip 3586. The groove may similarly extend around at least a portion of the outer perimeter (e.g., the entire outer perimeter) of the central column 3584.

As shown in Figs. 66-1 to 66-3, the patient may insert either elbow 3568, 3576 into the vent ring 3582 in order to engage the two pieces (e.g., via a snap-fit). The first end 3558 of the elbow 3568, 3576 may have a smaller diameter than the central column 3584 and may be insertable into the central column 3584. Once inserted, fingers 3574, 3580 may remain outside the central column 3584. Additionally, the lip 3570 may act as a stop to limit additional insertion in the elbow 3568. When inserting either elbow 3568, 3576, the shape of either finger 3574, 3580 may allow either to pass over the lip 3586. However, the patient may also actuate the buttons 3572, 3578 in order to allow the fingers to pass over the lip 3586. The fingers 3574, 3580 may grab onto the lip 3586, and may limit the respective elbow 3568, 3576 from being disconnected. The fingers 3574, 3580 may also allow the respective elbow 3568, 3576 to rotate 360°. To remove the elbow 3568, 3576, the patient may engage the buttons 3572, 3578. Actuating the buttons 3572, 3578 (e.g., simultaneously) may space the fingers 3574, 3580 apart from the lip 3586 (e.g., wider than an outer perimeter of the lip 3586) so that the elbow 3568, 3576 may be disconnected.

In some forms, either example of the elbow 3568, 3576 may be unable to translate while connected to the vent ring 3582. For example, a washer 3540 may be spaced apart from the lip 3586 so that the respective fingers 3574, 3580 can fit there between. The space may be just large enough for the respective finger 3574, 3580 such that the fingers 3574, 3580 cannot move while in between the lip 3586 and the washer 3540. Limiting transition of the elbow 3568, 3576 may limit disturbances in flow or irritations to the patient that could be caused by the translational movement of the elbow 3568, 3576 during use.

In certain forms, the washer 3540 may be formed as an integral piece of the central column 3584 and may not be a separate piece that can be removed. In this example, the washer 3540 (or support surface) would still limit the translation of the elbow 3568, 3576 while connected to the vent ring 3582.

As shown in Fig. 66-3, the elbow 3576 may not extend further toward the cavity 3272 than the mount 5324. The lip 3586 may extend in the anterior direction in order to space the elbow 3576 apart from the cavity 3272. This may be further assisted by the offset 3506. By spacing the elbow 3576 apart from the cavity 3272, the elbow may not encroach on the distance 6076. Although this is shown and described with respect to the elbow 3576, the description also applies to similar elbows (e.g., the elbow 3568).

As shown in Fig. 67, another example of an elbow 3588 may be similar to the elbows 3568 and 3576 described above. Only some similarities and differences between the elbows 3568, 3576, and 3588 may be described below.

With continued reference to Fig. 67, the elbow 3588 may include at least one button 3590 and at least one finger 3592 (e.g., a pair of each). Both the button 3590 and the finger 3592 may project from the surface of the elbow 3588 and may not be included as forming part of a perimeter of the elbow 3588. The button 3590 may operate similarly to the button 3572 or the button 3580. For example, the patient may engage the buttons 3590 to move them closer together. In doing so, the fingers 3592 may move from a first distance apart to a second distance apart that is greater than the first distance.

In some forms, an arm 3591 may extend from the surface of the elbow 3588 (e.g., perpendicularly) in order to connect the button 3590 and the finger 3592 to the surface of the elbow 3588. The arm 3591 is disposed between the finger 3592 and the button 3590 (e.g., halfway) and forms a pivot point or fulcrum. Engaging the button 3590 in order to move the finger 3592 causes pivoting movement about the arm 3591.

In certain forms, the arm 3591 may include a curved shape, which may be an S shape. The curved shape of the arm 3591 may reduce stress points caused when engaging the button 3590.

As shown in Fig. 68, a vent ring 3594 may be substantially similar to the vent rings 3562 and 3582 described above. Only some similarities and difference may be described below.

As illustrated in Fig. 68, the vent ring 3594 may include a central column 3596 that projects from the inner wall 3524. In other words, the central column 3596 may have a smaller perimeter than the inner wall 3524, and may include an opening to still allow fluid flow through the vent ring 3594. In some forms, the central column 3596 may project beyond the anterior surface 3512. In other words, an end of the central column 3596 may project away from the plenum chamber 3200 and may be the anterior-most portion of the vent ring 3594.

In some forms, the central column 3596 may include a lip 3598, which may extend at least partially around the outer perimeter of the central column 3596. In the illustrated example, the lip 3598 may extend entirely around the central column 3596. In other examples, the lip 3598 may extend around only a portion of the central column 3596, or a groove may replace the lip 3598. The groove may similarly extend around at least a portion of the outer perimeter (e.g., the entire outer perimeter) of the central column 3596.

As shown in Figs. 69 to 70, the patient may insert the elbow 3588 into the vent ring 3594 in order to engage the two pieces (e.g., via a snap-fit). The first end 3558 of the elbow 3588 may have a smaller diameter than the central column 3596 and may be insertable into the central column 3596. Once inserted, fingers 3592 may remain outside the central column 3596. Additionally, the lip 3598 may act as a stop to limit additional insertion in the elbow 3588. When inserting the elbow 3588, the shape of the finger 3592 may allow either to pass over the lip 3596. However, the patient may also actuate the buttons 3590 in order to allow the fingers 3592 to pass over the lip 3598. The fingers 3592 may grab onto the lip 3596, and may limit the elbow 3588 from being disconnected. The fingers 3592 may also allow the elbow 3588 to rotate 360°. To remove the elbow 3588, the patient may engage the buttons 3590. Actuating the buttons 3590 (e.g., simultaneously) may space the fingers 3592 apart from the lip 3598 (e.g., wider than an outer perimeter of the lip 3598) so that the elbow 3588 may be disconnected (see e.g., Fig. 69-1 illustrating a single button 3590 actuated to move a finger 3592).

In some forms, the elbow 3588 may be unable to translate while connected to the vent ring 3594. For example, a washer 3540 may be spaced apart from the lip 3598 so that the fingers 3592 can fit there between. The space may be just large enough for the finger 3592 such that the fingers 3592 cannot move while in between the lip 3598 and the washer 3540. Limiting transition of the elbow 3588 may limit disturbances in flow or irritations to the patient that could be caused by the translational movement of the elbow 3588 during use.

In certain forms, the washer 3540 may be formed as an integral piece of the central column 3596 and may not be a separate piece that can be removed. In this example, the washer 3540 (or support surface) would still limit the translation of the elbow 3588 while connected to the vent ring 3594.

In some forms, the elbow 3588 may be connected to the vent ring 3582 of Fig. 66. Additionally, the elbows 3568, 3576 may be used with the vent ring 3594. The descriptions above may similarly apply to these combinations.

As shown in Fig. 70, the elbow 3588 may not extend further toward the cavity 3272 than the mount 5324. The lip 3598 may extend in the anterior direction in order to space the elbow 3588 apart from the cavity 3272. This may be further assisted by the offset 3506. By spacing the elbow 3588 apart from the cavity 3272, the elbow may not encroach on the distance 6076.

### 5.3.8 Connection port

Connection port 3600 allows for connection (e.g., a removable connection via a snap-fit, a permanent connection, etc.) to the air circuit 4170. The patient interface 3000 may include two connection ports 3600, one on either side of the plenum chamber 3200. Conduits may connect to the connection ports 3600 in order to convey pressurized breathable gas to the patient. In some forms, the conduits may be conduit headgear, and may contact the patient's head. The conduits may extend toward the crown of the patient's head, where the decoupling structure 3500 is located.

As described above, the inlet port 3604 of the plenum chamber 3200 may include a lip 3608 that extends into the cavity 3272 and assists in securing the vent ring 3504 and/or the elbow 3500 to the plenum chamber 3200. In the illustrated example, the vent ring 3504 may engage the lip 3608 with a snap-fit, or any other similar engagement technique.

### 5.3.9 Forehead support

In one form, the patient interface 3000 includes a forehead support 3700.

### 5.3.10 Anti-asphyxia valve

In one form, the patient interface 3000 includes an anti-asphyxia valve. For example, the elbow 3550 may include the anti-asphyxia valve 3800 that may be selectively closed as a result of the flow of pressurized air. When the flow of pressurized air stops, the patient may be able to breathe through the anti-asphyxia valve 3800.

### 5.3.11 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH₂O, or at least 10cmH₂O, or at least 20 cmH₂O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors 4272 and flow rate sensors.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air Filter

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000 or 3800.

### 5.4.1.2 Muffler

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 5.4.1.3 Pressure Generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH2O to about 20 cmH2O, or in other forms up to about 30 cmH2O when delivering respiratory pressure therapy. The blower may be as described in any one of the following patents or patent applications the contents of which are incorporated herein by reference in their entirety: U.S. Patent No. 7,866,944; U.S. Patent No. 8,638,014; U.S. Patent No. 8,636,479; and PCT Patent Application Publication No. WO 2013/020167.

The pressure generator 4140 may be under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device algorithms

As mentioned above, in some forms of the present technology, the central controller may be configured to implement one or more algorithms expressed as computer programs stored in a non-transitory computer readable storage medium, such as memory. The algorithms are generally grouped into groups referred to as modules.

In other forms of the present technology, some portion or all of the algorithms may be implemented by a controller of an external device such as the local external device or the remote external device. In such forms, data representing the input signals and/or intermediate algorithm outputs necessary for the portion of the algorithms to be executed at the external device may be communicated to the external device via the local external communication network or the remote external communication network. In such forms, the portion of the algorithms to be executed at the external device may be expressed as computer programs stored in a non-transitory computer readable storage medium accessible to the controller of the external device. Such programs configure the controller of the external device to execute the portion of the algorithms.

In such forms, the therapy parameters generated by the external device via the therapy engine module (if such forms part of the portion of the algorithms executed by the external device) may be communicated to the central controller to be passed to the therapy control module.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated herewithin in its entirety by reference.

As shown in Fig. 58, the air circuit 4170 is formed as a flexible conduit that is connected to the elbow 3500 at a first end 4172. A second end 4174 opposite the first end 4172 may include a quick-release connector 4176. The quick-release connector 4176 may assist the patient in disconnecting the air circuit 4170 from the RPT device 4000.

In some forms, the quick-release connector 4176 may be a swivel cuff. For example, the quick-release connector 4176 of the air circuit 4170 may attach to a complementary connector 4178 (e.g., either of the RPT device 4000 or of an intermediate conduit). The quick-release connector 4176 and the complementary connector 4178 may each be rotatable relative to one another. This may allow for increased movement between the RPT device 4000 and the patient interface 3000, and may allow the patient to move while asleep without disturbing the connection between the air circuit 4170 and the RPT device 4000.

In certain forms, connection between the quick-release connector 4176 and the complementary connector 4178 may be formed with a rotational snap. For example, the complementary connector 4178 may receive the quick-release connector 4176 (or vice versa), and a locking mechanism (not shown) may rotate in order to lock the two pieces together. The locking mechanism may rotate (e.g., in the clockwise direction) with a quarter turn (or a half turn, a full turn, etc.) in order to form the locking connection, and may be released with a quarter turn in the opposite rotational direction (e.g., the counter clockwise direction). Rotation of the locking mechanism to facilitate the connection between the quick-release connector 4176 and the complementary connector 4178 may not affect the relative rotation between the two connectors 4176, 4178. Rotation in the disengagement direction (e.g., the counter clockwise direction) may push the complementary connector 4178 away from the quick-release connector 4176 in order to facilitate rapid disengagement.

In certain forms, the connection between the quick-release connector 4176 and the complementary connector 4178 may be formed with catches (not shown). In other examples, this may be reversed. For example, the quick-release connector 4176 may include movable latches that are receivable within the complementary connector 4178. The movable latches may be biased toward a locked position in order to secure the two connectors 4176, 4178 together. Once secured, the movable latches may be able to move (e.g., slide) around the perimeter of the complementary connector 4178 so that the connectors 4176, 4178 are still rotatable relative to one another. To disconnect the air circuit 4170 from the RPT device 4000, the patient may engage a button 4179 on the quick-release connector 4176, which may move the latches into an unlocked position and allow the air circuit 4170 to freely translate and disengage from the complementary connector 4178. In some forms, the quick-release connector 4176 may be detachable from the complementary connector 4178 with one hand. For example, the patient may be able to disconnect the quick-release connector 4176 and the complementary connector 4178 using only his thumb and index finger. This may allow a patient to more easily disconnect the quick-release connector 4176 and the complementary connector 4178.

As shown in Fig. 58-1, certain forms of the quick-release connector 4176 may include flat surfaces 4181. In other words, the quick-release connector 4176 may not be entirely circular. This may assist in better distributing leak from the system so that less leak occurs through the vent holes 3520, and more humidity is retained within the plenum chamber 3200.

### 5.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000.

### 5.6 HUMIDIFIER

### 5.6.1 Humidifier overview

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 5.6.2 Humidifier Components

### 5.6.2.1 Water Reservoir

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 5.6.2.2 Conductive Portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 5.6.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 5.6.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 5.6.3 Passive humidification

As shown in Figs. 71 to 75, coupling can be connected to the plenum chamber 3200 in order to provide a humid environment within the plenum chamber during use. These coupling may be used in conjunction with a humidifier 5000, and may help to increase the efficiency of the humidifier by retaining a greater percentage of the supplied humidity within the plenum chamber 3200 during use. These couplings may also be used without the humidifier 5000. Instead, the couplings may assist in retaining the moisture that the patient naturally exhales within the plenum chamber 3200.

In some forms, the passive humidification may occur solely through the shape and/or geometry of a plenum chamber, vent ring, conduit, and/or elbow. In other words, the shape and/or size of the structures alone may influence the airflow so that humidity within a cavity of the plenum chamber may be greater than the surrounding environment. A heat and moisture exchanger (HME) may not be needed to achieve the passive humidification.

In certain forms, an HME and/or a humidifier 5000 may be used with the passive humidification described below in order to allow for more humid environments within the cavity.

### 5.6.3.1 Increased spacing

As shown in Figs. 71 and 71-1, a coupling or elbow 5300 may be connected to a plenum chamber 3200. The elbow 5300 may connect to a vent ring 5304 in a similar manner to any of the elbows described above (e.g., an internal connection or an external connection). The vent ring 5304 may be similar to the previously described vent rings and may include vent holes 5308. In the illustrated example, the vent holes 5308 may be spaced apart from the connection port 3600 by a distance 5312. The vent ring 5304 may include an extension 5316 that extends the distance 5312. For example, the extension 5316 may extend from a posterior surface (e.g., posterior surface 3508 in Fig. 57). The extension 5316 may connect directly to the connection port 3600 (e.g., in a similar manner as the vent ring 3504 shown in Fig. 57).

In other forms, the extension 5316 may be connected to the plenum chamber 3200 as part of the connection port 3600 (e.g., the extension 5316 may be permanently connected to the plenum chamber 3200). The vent ring 5304 may be removably connected to the extension 5316 (e.g., in the same manner that the vent ring 3504 is connected to the connection port 3600 described above).

In some forms, the distance 5312 may be between approximately 1 mm and approximately 50 mm. In some forms, the distance 5312 may be between approximately 5 mm and approximately 30 mm. In some forms, the distance 5312 may be between approximately 15 mm and approximately 25 mm. In some forms, the distance 5312 may be approximately 20 mm. The distance may be optimized in order to balance an increase in humidity (i.e., maximize) with an increase of carbon dioxide (i.e., minimize).

In use, the flow of pressurized breathable gas is delivered to the patient through the elbow 5300 and the extension 5316. This air may be actively humidified (e.g., using a humidifier 5000) or may have the humidity of the ambient air. The patient inhales this breathable air and exhales a mixture of gas (e.g., carbon dioxide) and water vapor. As described above, this air is allowed to escape through the vent holes 5308 so that the plenum chamber 3200 can be replenished with the pressurized breathable gas and so that the patient is not inhaling exhaled gases. However, the escaping gas carries the water vapor into the ambient environment. Particularly when the patient is not using a humidifier, the loss of water vapor during a period of use (e.g., one night) can lead the patient to wake up with dry mouth and feel uncomfortable (e.g., which may discourage future use). By spacing the vent holes 5308 further from the connection port 3600 (and therefore further from the patient's nose or mouth) the exhaled air has further to travel in order to vent to ambient. This impedance may make it more difficult for the air and the water vapor to escape, which may retain more moisture (e.g., from the exhaled water vapor) in the plenum chamber 3200. During the course of a use, the patient's mouth may therefore be less likely to dry out. By spacing the vent holes 5308 apart by a distance 5312, a sufficient amount of exhaled gas may still be able to escape the plenum chamber 3200 (e.g., so that the patient is not breathing recycled air) but enough water vapor may remain to help passively humidify the flow of pressurized breathable gas.

In certain forms, the vent holes 5308 are not blocked or otherwise obstructed by a physical barrier that redirects or prevents fluid flow through the vent holes 5308. Instead, the impedance may be generated by the distance 5312, which causes the exhaled air to travel further while flowing against the oppositely directed pressurized flow of air.

In certain forms, and HME may be placed within the extension 5316 in order to further increase the humidity that may be retained within the plenum chamber 3200. The HME may further increase the impedance for the exhaled air to exit the patient interface and is designed to trap at least some exhaled water vapor so that the flow of pressurized air picks up and allows the patient to re-inhale the water. The material of the HME may be designed so that CO₂ accumulation within the plenum chamber 3200 does not substantially increase.

As shown in Fig. 71-1, the vent ring 5304 may be connected to the plenum chamber 3200 in a similar manner as the vent ring 3504 (see e.g., Fig. 57). For example, the lip 3608 may engage the ring body 3516 (e.g., in a substantially flush orientation between the anterior and posterior surface 3512). In use, the flow of pressurized gas 1500 travels through the elbow 5300 and toward the plenum chamber 3200 to pressurize the plenum chamber 3200 and allow the patient to inhale the pressurized air. When the patient exhales, the exhaled air 1600 not only has further to travel to the vent holes 5308 in order to exhaust to ambient (e.g., as compared to Fig. 57), but the distance 5312 of the extension 5316 also means that the exhaled air 1600 is traveling against the pressurized air 1500 for a greater distance (e.g., creating the impedance). This allows less of the exhaled air 1600 to exhaust, while allowing the pressurized air 1500 to pick up the exhaled water vapor from the exhaled air 1600 so that it can be re-inhaled.

### 5.6.3.2 Barrier

As shown in Figs. 72 to 73-1, an elbow 5300 may be connected to another example of a vent ring 5320, which may have a similar structure to the vent ring 5304. The vent ring 5320 may include a mount 5324 connect to the posterior surface 5328 of the vent ring 5320.

In some forms, the mount 5324 may be connected to the posterior surface 5328 radially inside of the vent openings 5332 and may not substantially occlude the holes of the vent openings 5332.

As shown in Fig. 72, some forms of the mount 5324 may include arms 5336 that extend away from the posterior surface 5328. In the illustrated example, the arms 5336 are spaced apart from one another (e.g., equally spaced) around the perimeter of the mount 5324. A space 5340 may be formed between the arms 5336 (e.g., within the mount 5324) through which airflow may pass.

In some forms, the perimeter of the opening for the inner wall 3524 may be larger than the mount 5324. For example, there may be space radially outside the mount 5324. This may allow fluid flow between perimeter of the inner wall 3524 and the mount 5324.

In some forms, the posterior surface 5328 may abut the mount 5324. For example, there may be substantially no space between the posterior surface 5328 and the mount 5324 for fluid flow. Instead, the space 5340 may include an opening 5342 that may communicate with the opening of the inner wall 3524. For example, there may be an air path through the vent ring 5304 by way of the space 5340.

In some forms, the posterior surface 5328 may be ring shaped and an inner perimeter of the posterior surface 5328 may be spaced apart from the mount 5324. For example, there may be space radially outside the mount 5324 and inside of an inner perimeter of the posterior surface 5328. This may allow fluid flow between perimeter of the posterior surface 5328 and the mount 5324. In some forms, the mount 5324 may not include the opening 5342.

As shown in Fig. 73, some forms of the mount 5324 may include a disk 5344 connected to the arms 5336. The disk 5344 may include a domed shape (e.g., a positive domed shape facing the patient in use). The disk 5344 may form one side of the space 5340 (e.g., a posterior wall of the space 5340). The outer perimeter of the disk 5344 may also be approximately the same as the outer diameter of the posterior surface 5328. The disk 5344 may cover the vent openings 5332 from the perspective of the patient. However, the disk 5344 may not obstruct the vent openings 5332 because the arms 5336 space the disk 5344 apart from the vent openings 5332.

In some forms, different shaped disks 5344 may be used in order to retain different amounts of moisture within the plenum chamber 3200. For example, the disk 5344 with a smaller radius of curvature may extend closer to a patient's nose and/or mouth than a disk 5344 with a larger radius of curvature. In other words, if both the smaller curvature and larger curvature disks 5344 include the same end-to-end distance (e.g., equal to or exceeding the diameter between the vent openings 5332), an edge of the smaller curvature disk 5344 will extend closer to the patient's face. This may cause more exhaled air to be redirected (i.e., increase the impedance) so that more water vapor is retained within the plenum chamber 3200.

In certain forms, a smaller radius of curvature may assist the flow of pressurized breathable gas in flowing around the disk 5344 because the smaller radius of curvature directs more airflow around the disk 5344 and not back toward the elbow 5300.

In certain forms, the larger radius of curvature may be more comfortable for a patient because the disk 5344 is spaced further from the patient's face. For example, the patient may desire a maximized second distance 6076 (see e.g., Fig. 51) between his mouth and the plenum chamber 3200. Using a larger radius of curvature for the disk 5344 may be able to achieve a larger distance 6076 than is achievable with the smaller radius of curvature disk 5344.

In other forms, the disk 5344 may have substantially no curvature and may be substantially planar with respect to the patient. For example, the disk 5344 may be substantially parallel to the posterior surface 5328.

In some forms, the disk 5344 may be constructed from an air impermeable material and may act as a barrier to increase the impedance for exhaled to reach the vent openings 5332. The disk 5344 may block or obstruct direct (e.g., straight line) flow paths between the patient's airways (e.g., nose or mouth) and the vent openings 5332.

In some forms, the disk 5344 may be constructed from a semi-permeable material that may allow some airflow to pass through. For example, the disk 5344 may include an HME (or may be constructed from HME material) so that some exhaled air may pass through the disk 5344 but water vapor may be trapped. Alternatively, the disk 5344 may be constructed from another semi-permeable material that does not retain moisture but still increases the impedance of the airflow exiting the plenum chamber 3200. Because the disk 5344 may be semi-permeable, some exhale air may still be directed back toward the patient (and not exhaust through the vent openings 5332).

As described above, patients lose moisture as they exhale and may experience an uncomfortable dry mouth as a result of losing too much moisture (especially if the patient interface 3000 is used without a humidifier 5000). Like the vent ring 5304, the vent ring 5320 works to retain moisture that the patient exhales within the plenum chamber in order to reduce the occurrence of dry mouth.

As shown in Fig. 73-1, the mount 5324 may face the patient during use so that the disk 5344 has a positive domed shape relative to the patient's mouth or nose. Because the disk 5344 has substantially the same width as the posterior surface 5328, the disk 5344 may obstruct exhaled airflow 1600 from venting through the vent openings 5332 and may direct air back into the plenum chamber 3200. However, the negative domed shape of the disk 5344 relative to the flow direction of pressurized air 1500 may allow the airflow to pass around the disk 5344 to the patient without substantial obstruction. For example, the flow of pressurized gas 1500 may either flow between the inner perimeter of the posterior surface 5328 and the mount 5324, or through the opening 5342 of the mount 5324 and through the space 5340. As described above, the radius of curvature of the disk 5344 may influence an impedance of the pressurized air 1500 entering the plenum chamber 3200 (e.g., smaller radius of curvature creates less impedance).

Exhaled air may still be able to pass around the disk 5344 in order to exhaust out through the vent openings 5332. However, the disk 5344 may make this harder (i.e., increase an impedance) and may cause more exhausted air to remain in the plenum chamber 3200 than if the disk 5344 was not present. As described above, the radius of curvature of the disk 5344 may influence an impedance of the exhausted air 1600 leaving the plenum chamber 3200 (e.g., smaller radius of curvature creates more impedance). The disk 5344 acts similar to the distance 5312 described above in that the disk 5344 prevents some water vapor from leaving the plenum chamber 3200 by directing exhaled air back into the plenum chamber 3200. Exhaled air 1600 that is not redirected toward the patient's mouth has a further distance to travel to reach the vent openings 5332 (e.g., as compared to Fig. 57). This increased distance (e.g., similar to distance 5316 of Fig. 71-1) combined with the oppositely directed flow of pressurized gas 1500 may allow of more water vapor to be retained and re-inhaled by the patient. The increased impedance created by the disk 5344 with respect to the exhaled air 1600 may be without the assistance of an HME (although one may be used), and may simply be the result of the shape and/or size of the disk 5344. This may assist in reducing mouth dryness in a patient.

### 5.6.3.3 Multiple venting locations

As shown in Figs. 74 to 75, an elbow 5300 may be used with a vent ring 5352 that is substantially similar to the vent ring 3504, and only some similarities and difference may be described. As shown in Fig. 74 for example, the vent ring 5352 may include fewer vent openings 5356 than the vent ring 3504. In the illustrated example, the vent openings 5356 may be collected at a particular region of the vent ring 5352. In other examples, the vent openings 5356 may be evenly spaced around a perimeter of the vent ring 5352.

As shown in Fig. 75, additional vent openings 5360 may be disposed on a complementary connector 5364, which may be removably connected to the quick-release connector 4176. The vent openings 5360 may be collected at a particular region of the connector 5364. In other examples, the vent openings 5360 may be evenly spaced around a perimeter of the connector 5364.

In use, vent openings 5356 may operate like the previously described vent openings and allow exhaled air to escape the plenum chamber 3200. In this example, creating fewer vent openings 5356 reduces the flow rate of exhaust gas venting from the plenum chamber 3200. Because less gas can vent through the vent openings 5356 as a whole, more exhaust gas, and therefore more water vapor, remains in the plenum chamber 3200, which can help limit the patient's mouth from drying out.

Because less venting occurs in the plenum chamber 3200, venting may need to occur elsewhere so that the system does not become over pressurized. The vent openings 5360 of the connector 5364 may allow pressurized gas to vent to ambient prior to reaching the patient. While the pressure reaching the patient may be slightly less, more air remains in the plenum chamber 3200 so the net pressure is substantially the same (e.g., as with the vent ring 3504).

### 5.6.3.4 Combination

In some forms, any combination of the examples described above may be used in combination with one another. For example, a vent ring could include a fewer number of vent openings could also include either a disk or be spaced apart a distance from the connection port. Including any two (or all three) of these features together may increase the amount of water vapor that is retained within the plenum chamber 3200 that the patient may inhale on their next breath in order to help limit their mouth from drying out.

### 5.7 BREATHING WAVEFORMS

Fig. 6 shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 5.8 RESPIRATORY THERAPY MODES

Various respiratory therapy modes may be implemented by the disclosed respiratory therapy system including CPAP therapy and bi-level therapy.

### 5.9 GLOSSARY

For the purposes of the present technology disclosure, in certain forms of the present technology, one or more of the following definitions may apply. In other forms of the present technology, alternative definitions may apply.

### 5.9.1 General

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol *Q*. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy (RPT):* The application of a supply of air to an entrance to the airways at a treatment pressure that is typically positive with respect to atmosphere.

*Ventilator:* A mechanical device that provides pressure support to a patient to perform some or all of the work of breathing.

### 5.9.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.9.1.2 Mechanical properties

*Resilience:* Ability of a material to absorb energy when deformed elastically and to release the energy upon unloading.

*Resilient:* Will release substantially all of the energy when unloaded. Includes e.g. certain silicones, and thermoplastic elastomers.

*Hardness*: The ability of a material *per se* to resist deformation (e.g. described by a Young's Modulus, or an indentation *hardness* scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

*Stiffness (or rigidity) of a structure or component:* The ability of the structure or component to resist deformation in response to an applied load. The load may be a force or a moment, e.g. compression, tension, bending or torsion. The structure or component may offer different resistances in different directions. The inverse of stiffness is *flexibility.*

*Floppy structure or component:* A structure or component that will change shape, e.g. bend, when caused to support its own weight, within a relatively short period of time such as 1 second.

*Rigid structure or component:* A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.9.2 Respiratory cycle

*Apnea:* According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

*Breathing rate:* The rate of spontaneous respiration of a patient, usually measured in breaths per minute.

*Duty cycle:* The ratio of inhalation time, *Ti* to total breath time, *Ttot.*

*Effort (breathing):* The work done by a spontaneously breathing person attempting to breathe.

*Expiratory portion of a breathing cycle:* The period from the start of expiratory flow to the start of inspiratory flow.

*Flow limitation:* Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
*(i) Flattened:* Having a rise followed by a relatively flat portion, followed by a fall.
(ii) *M-shaped:* Having two local peaks, one at the leading edge, and one at the trailing edge, and a relatively flat portion between the two peaks.
(iii) *Chair-shaped:* Having a single local peak, the peak being at the leading edge, followed by a relatively flat portion.
(iv) *Reverse-chair shaped:* Having a relatively flat portion followed by single local peak, the peak being at the trailing edge.

*Hypopnea:* According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

*Hyperpnea*: An increase in flow to a level higher than normal.

*Inspiratory portion of a breathing cycle:* The period from the start of inspiratory flow to the start of expiratory flow will be taken to be the inspiratory portion of a breathing cycle.

*Patency (airway):* The degree of the airway being open, or the extent to which the airway is open. A *patent* airway is open. Airway patency may be quantified, for example with a value of one (1) being patent, and a value of zero (0), being closed (obstructed).

*Positive End-Expiratory Pressure (PEEP):* The pressure above atmosphere in the lungs that exists at the end of expiration.

*Peak flow rate (Qpeak):* The maximum value of flow rate during the inspiratory portion of the respiratory flow waveform.

*Respiratory flow rate,* patient *airflow rate, respiratory airflow rate (Qr):* These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

*Tidal volume (Vt):* The volume of air inhaled or exhaled during normal breathing, when extra effort is not applied. In principle the inspiratory volume *Vi* (the volume of air inhaled) is equal to the expiratory volume Ve (the volume of air exhaled), and therefore a single tidal volume *Vt* may be defined as equal to either quantity. In practice the tidal volume *Vt* is estimated as some combination, e.g. the mean, of the inspiratory volume *Vi* and the expiratory volume *Ve.*

*(inhalation) Time (Ti):* The duration of the inspiratory portion of the respiratory flow rate waveform.

*(exhalation) Time (Te):* The duration of the expiratory portion of the respiratory flow rate waveform.

*(total) Time (Ttot):* The total duration between the start of one inspiratory portion of a respiratory flow rate waveform and the start of the following inspiratory portion of the respiratory flow rate waveform.

*Typical recent ventilation:* The value of ventilation around which recent values of ventilation *Vent* over some predetermined timescale tend to cluster, that is, a measure of the central tendency of the recent values of ventilation.

*Upper airway obstruction (UAO):* includes both partial and total upper airway obstruction. This may be associated with a state of flow limitation, in which the flow rate increases only slightly or may even decrease as the pressure difference across the upper airway increases (Starling resistor behaviour).

*Ventilation (Vent):* A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.9.3 Ventilation

*Adaptive Servo-Ventilator (ASV):* A servo-ventilator that has a changeable, rather than fixed target ventilation. The changeable target ventilation may be learned from some characteristic of the patient, for example, a respiratory characteristic of the patient.

*Backup rate:* A parameter of a ventilator that establishes the minimum breathing rate (typically in number of breaths per minute) that the ventilator will deliver to the patient, if not triggered by spontaneous respiratory effort.

*Cycled:* The termination of a ventilator's inspiratory phase. When a ventilator delivers a breath to a spontaneously breathing patient, at the end of the inspiratory portion of the breathing cycle, the ventilator is said to be cycled to stop delivering the breath.

*Expiratory positive airway pressure (EPAP):* a base pressure, to which a pressure varying within the breath is added to produce the desired interface pressure which the ventilator will attempt to achieve at a given time.

*End expiratory pressure (EEP):* Desired interface pressure which the ventilator will attempt to achieve at the end of the expiratory portion of the breath. If the pressure waveform template Π(Φ) is zero-valued at the end of expiration, i.e. Π(Φ) = 0 when Φ = 1, the EEP is equal to the EPAP.

*Inspiratory positive airway pressure (IPAP):* Maximum desired interface pressure which the ventilator will attempt to achieve during the inspiratory portion of the breath.

*Pressure support:* A number that is indicative of the increase in pressure during ventilator inspiration over that during ventilator expiration, and generally means the difference in pressure between the maximum value during inspiration and the base pressure (e.g., *PS = IPAP - EPAP*). In some contexts pressure support means the difference which the ventilator aims to achieve, rather than what it actually achieves.

*Servo-ventilator:* A ventilator that measures patient ventilation, has a target ventilation, and which adjusts the level of pressure support to bring the patient ventilation towards the target ventilation.

*Spontaneous*/*Timed (S*/*T):* A mode of a ventilator or other device that attempts to detect the initiation of a breath of a spontaneously breathing patient. If however, the device is unable to detect a breath within a predetermined period of time, the device will automatically initiate delivery of the breath.

*Swing:* Equivalent term to pressure support.

*Triggered:* When a ventilator delivers a breath of *air* to a spontaneously breathing patient, it is said to be triggered to do so at the initiation of the respiratory portion of the breathing cycle by the patient's efforts.

### 5.9.4 Anatomy

### 5.9.4.1 Anatomy of the face

*Ala:* the external outer wall or "wing" of each nostril (plural: alar)
*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the pronasale to the upper lip.

*Columella angle:* The angle between the line drawn through the midpoint of the nostril aperture and a line drawn perpendicular to the Frankfort horizontal while intersecting subnasale.

*Frankfort horizontal plane:* A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Lip, lower (labrale inferius):* A point on the face between the mouth and supramenton, lying in the median sagittal plane.

*Lip, upper (labrale superius):* A point on the face between the mouth and nose, lying in the median sagittal plane.

*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils):* Approximately ellipsoidal apertures forming the entrance to the nasal cavity. The singular form of nares is naris (nostril). The nares are separated by the nasal septum.

*Naso-labial sulcus or Naso-labial fold:* The skin fold or groove that runs from each side of the nose to the corners of the mouth, separating the cheeks from the upper lip.

*Naso-labial angle:* The angle between the columella and the upper lip, while intersecting subnasale.

*Otobasion inferior:* The lowest point of attachment of the auricle to the skin of the face.

*Otobasion superior:* The highest point of attachment of the auricle to the skin of the face.

*Pronasale:* the most protruded point or tip of the nose, which can be identified in lateral view of the rest of the portion of the head.

*Philtrum:* the midline groove that runs from lower border of the nasal septum to the top of the lip in the upper lip region.

*Pogonion:* Located on the soft tissue, the most anterior midpoint of the chin.

*Ridge (nasal):* The nasal ridge is the midline prominence of the nose, extending from the Sellion to the Pronasale.

*Sagittal plane:* A vertical plane that passes from anterior (front) to posterior (rear). The midsagittal plane is a sagittal plane that divides the body into right and left halves.

*Sellion:* Located on the soft tissue, the most concave point overlying the area of the frontonasal suture.

*Septal cartilage (nasal):* The nasal septal cartilage forms part of the septum and divides the front part of the nasal cavity.

*Subalare:* The point at the lower margin of the alar base, where the alar base joins with the skin of the superior (upper) lip.

*Subnasal point:* Located on the soft tissue, the point at which the columella merges with the upper lip in the midsagittal plane.

*Supramenton:* The point of greatest concavity in the midline of the lower lip between labrale inferius and soft tissue pogonion

### 5.9.4.2 Anatomy of the skull

*Frontal bone:* The frontal bone includes a large vertical portion, the *squama frontalis,* corresponding to the region known as the forehead.

*Mandible:* The mandible forms the lower jaw. The mental protuberance is the bony protuberance of the jaw that forms the chin.

*Maxilla:* The maxilla forms the upper jaw and is located above the mandible and below the orbits. The *frontal process of the maxilla* projects upwards by the side of the nose, and forms part of its lateral boundary.

*Nasal bones:* The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone:* The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit:* The bony cavity in the skull to contain the eyeball.

*Parietal bones:* The parietal bones are the bones that, when joined together, form the roof and sides of the cranium.

*Temporal bones:* The temporal bones are situated on the bases and sides of the skull, and support that part of the face known as the temple.

*Zygomatic bones:* The face includes two zygomatic bones, located in the upper and lateral parts of the face and forming the prominence of the cheek.

### 5.9.4.3 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx:* The larynx, or voice box houses the vocal folds and connects the inferior part of the pharynx (hypopharynx) with the trachea.

*Lungs:* The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity:* The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.9.5 Patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

*Frame:* Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane:* Membrane will be taken to mean a typically thin element that has, preferably, substantially no resistance to bending, but has resistance to being stretched.

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*Shell:* A shell will be taken to mean a curved, relatively thin structure having some bending, tensile and compressive stiffness. For example, a curved structural wall of a mask may be a shell. In some forms a shell or portions of a shell, may not be rigid. In some forms, a shell may be faceted. In some forms a shell may be airtight. In some forms a shell may not be airtight.

*Stiffener:* A stiffener will be taken to mean a structural component designed to increase the bending resistance of another component in at least one direction.

*Strut:* A strut will be taken to be a structural component designed to increase the compression resistance of another component in at least one direction.

*Swivel* (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie* (noun): A structure designed to resist tension.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.9.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

To facilitate describing the shape of the three-dimensional structures and the surfaces, we first consider a cross-section through a surface of the structure at a point, *p.* See Fig. 3B to Fig. 3F, which illustrate examples of cross-sections at point *p* on a surface, and the resulting plane curves. Figs. 3B to 3F also illustrate an outward normal vector at *p.* The outward normal vector at *p* points away from the surface. In some examples we describe the surface from the point of view of an imaginary small person standing upright on the surface.

### 5.9.6.1 Curvature in one dimension

The curvature of a plane curve at *p* may be described as having a sign (e.g. positive, negative) and a magnitude (e.g. 1/radius of a circle that just touches the curve at *p).*

*Positive curvature:* If the curve at *p* turns towards the outward normal, the curvature at that point will be taken to be positive (if the imaginary small person leaves the point *p* they must walk uphill). See Fig. 3B (relatively large positive curvature compared to Fig. 3C) and Fig. 3C (relatively small positive curvature compared to Fig. 3B). Such curves are often referred to as concave.

*Zero curvature:* If the curve at *p* is a straight line, the curvature will be taken to be zero (if the imaginary small person leaves the point *p*, they can walk on a level, neither up nor down). See Fig. 3D.

*Negative curvature:* If the curve at *p* turns away from the outward normal, the curvature in that direction at that point will be taken to be negative (if the imaginary small person leaves the point *p* they must walk downhill). See Fig. 3E (relatively small negative curvature compared to Fig. 3F) and Fig. 3F (relatively large negative curvature compared to Fig. 3E). Such curves are often referred to as convex.

### 5.9.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

*Principal curvatures and directions:* The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at *p* are the curvatures in the principal directions.

*Region of a surface:* A connected set of points on a surface. The set of points in a region may have similar characteristics, e.g. curvatures or signs.

*Saddle region:* A region where at each point, the principal curvatures have opposite signs, that is, one is positive, and the other is negative (depending on the direction to which the imaginary person turns, they may walk uphill or downhill).

*Dome region:* A region where at each point the principal curvatures have the same sign, e.g. both positive (a "concave dome") or both negative (a "convex dome").

*Cylindrical region:* A region where one principal curvature is zero (or, for example, zero within manufacturing tolerances) and the other principal curvature is non-zero.

*Planar region:* A region of a surface where both of the principal curvatures are zero (or, for example, zero within manufacturing tolerances).

*Edge of a surface:* A boundary or limit of a surface or region.

*Path:* In certain forms of the present technology, 'path' will be taken to mean a path in the mathematical - topological sense, e.g. a continuous space curve from *f(0)* to *f*(1) on a surface. In certain forms of the present technology, a 'path' may be described as a route or course, including e.g. a set of points on a surface. (The path for the imaginary person is where they walk on the surface, and is analogous to a garden path).

*Path length:* In certain forms of the present technology, 'path length' will be taken to mean the distance along the surface from *f*(0) to f(1), that is, the distance along the path on the surface. There may be more than one path between two points on a surface and such paths may have different path lengths. (The path length for the imaginary person would be the distance they have to walk on the surface along the path).

*Straight-line distance:* The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.9.6.3 Space curves

*Space curves:* Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

*Tangent unit vector (or unit tangent vector):* For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

*Unit normal vector:* As the imaginary person moves along the curve, this tangent vector itself changes. The unit vector pointing in the same direction that the tangent vector is changing is called the unit principal normal vector. It is perpendicular to the tangent vector.

*Binormal unit vector:* The binormal unit vector is perpendicular to both the tangent vector and the principal normal vector. Its direction may be determined by a right-hand rule (see e.g. Fig. 3P), or alternatively by a left-hand rule (Fig. 3O).

*Osculating plane:* The plane containing the unit tangent vector and the unit principal normal vector. See Figures 3O and 3P.

*Torsion of a space curve:* The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 5.9.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.10 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are incorporated herein by reference in their entirety to disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the spirit and scope of the technology.

### 5.11 REFERENCE SIGNS LIST

| | |
|---|---|
| patient | 1000 |
| area proximate ala | 1010 |
| alar crest | 1020 |
| bed partner | 1100 |
| pressurized gas | 1500 |
| exhaled air | 1600 |
| seal forming structure | 3100 |
| first seal forming structure | 3101 |
| second seal forming structure | 3102 |
| boundary | 3103 |
| central portion | 3110 |
| lateral portion | 3111 |
| posterior surfaces of lateral portion | 3112 |
| ridge | 3120 |
| lip inferior portion | 3130 |
| lip superior portion | 3131 |
| peripheral portion | 3132 |
| oral hole | 3133 |
| nasal aperture(s) | 3135 |
| plenum chamber | 3200 |
| oral portion of plenum chamber | 3201 |
| nasal portion of plenum chamber | 3202 |
| chord | 3210 |
| superior point | 3220 |
| inferior point | 3230 |
| first anterior wall portion | 3240 |
| boundary of oral and nasal portion | 3241 |
| second anterior wall portion | 3242 |
| first anterior wall portion superior boundary | 3243 |
| first anterior wall portion inferior boundary | 3244 |
| lateral side wall portion | 3245 |
| lateral side wall portion | 3246 |
| second anterior wall portion superior boundary | 3247 |
| second anterior wall portion inferior boundary | 3248 |
| shell | 3250 |
| central portion | 3251 |
| support portions | 3260 |
| first end of support portion | 3261 |
| second end of support portion | 3262 |
| rigid portion | 3263 |
| band | 3270 |
| cavity | 3272 |
| groove | 3280 |
| projection | 3284 |
| protrusion | 3288 |
| positioning and stabilising structure | 3300 |
| connectors | 3310 |
| arms | 3320 |
| frame | 3350 |
| loop inner surface | 3351 |
| connection point | 3352 |
| first loop | 3352a |
| second loop | 3352b |
| eyelet cut | 3353 |
| headgear straps | 3354 |
| left superior headgear strap | 3356 |
| superior strap | 3357 |
| right superior headgear strap | 3358 |
| first width | 3359 |
| central portion | 3360 |
| second width | 3361 |
| arm | 3362 |
| linear sides | 3363 |
| secondary connection point | 3364 |
| left secondary connection point | 3364a |
| right secondary connection point | 3364b |
| hidden portion | 3365 |
| left inferior strap | 3366 |
| tactile response element | 3367 |
| right inferior strap | 3368 |
| buckle | 3369 |
| magnet | 3370 |
| slot | 3372 |
| scallop | 3373 |
| outer casing | 3376 |
| planar surface | 3378 |
| lip | 3380 |
| magnetic member | 3382 |
| overhang | 3384 |
| crossbar | 3386 |
| raised portion | 3390 |
| end | 3392 |
| rib | 3394 |
| vent | 3400 |
| vent mounting aperture | 3410 |
| elbow | 3500 |
| vent ring | 3504 |
| offset | 3506 |
| extension | 3507 |
| posterior surface | 3508 |
| anterior surface | 3512 |
| ring body | 3516 |
| vent hole | 3520 |
| inner wall | 3524 |
| outer wall | 3528 |
| groove | 3532 |
| ring seal | 3536 |
| washer | 3540 |
| elbow | 3550 |
| outer lip | 3552 |
| inner lip | 3554 |
| button | 3556 |
| first end | 3558 |
| channel | 3560 |
| vent ring | 3562 |
| central column | 3564 |
| groove | 3566 |
| elbow | 3568 |
| lip | 3570 |
| button | 3572 |
| finger | 3574 |
| elbow | 3576 |
| button | 3578 |
| finger | 3580 |
| arm | 3581 |
| vent ring | 3582 |
| central column | 3584 |
| lip | 3586 |
| connection port | 3600 |
| forehead support | 3700 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| air filter | 4110 |
| inlet air filter | 4112 |
| muffler | 4120 |
| outlet muffler | 4124 |
| pressure generator | 4140 |
| blower | 4142 |
| motor | 4144 |
| anti-spillback valve | 4160 |
| air circuit | 4170 |
| first end | 4172 |
| second end | 4174 |
| quick-release connector | 4176 |
| complementary connector | 4178 |
| button | 4179 |
| supplementary gas | 4180 |
| flat side | 4181 |
| electrical components | 4200 |
| PCBA | 4202 |
| electrical power supply | 4210 |
| input device | 4220 |
| transducers | 4270 |
| humidifier | 5000 |
| humidifier inlet | 5002 |
| humidifier outlet | 5004 |
| humidifier base | 5006 |
| humidifier reservoir | 5110 |
| conductive portion | 5120 |
| reservoir dock | 5130 |
| locking lever | 5135 |
| water level indicator | 5150 |
| heating element | 5240 |
| elbow | 5300 |
| vent ring | 5304 |
| vent hole | 5308 |
| distance | 5312 |
| extension | 5316 |
| vent ring | 5320 |
| mount | 5324 |
| posterior surface | 5328 |
| vent hole | 5332 |
| arm | 5336 |
| space | 5340 |
| disk | 5344 |
| vent ring | 5352 |
| vent opening | 5356 |
| vent opening | 5360 |
| complementary connector | 5364 |
| overhang | 6000 |
| opposite side | 6002 |
| undercut | 6004 |
| inclined surface | 6008 |
| pivot point | 6012 |
| clip support | 6016 |
| clip support | 6020 |
| rib | 6024 |
| cut-out | 6028 |
| arm | 6032 |
| superior bar | 6036 |
| stopper rib | 6040 |
| edge | 6042 |
| stiffened region | 6044 |
| anterior surface | 6046 |
| groove rib | 6052 |
| first angle | 6056 |
| first seal axis | 6060 |
| second angle | 6064 |
| second seal axis | 6068 |
| first distance | 6072 |
| second distance | 6076 |
| loop | 6080 |
| first loop section | 6080-1 |
| second loop section | 6080-2 |
| dividing wall | 6084 |
| central space | 6088 |
| ladder lock | 6092 |
| stopper | 6096 |
| button | 6100 |
| bendable housing | 6104 |
| magnet | 6108 |
| crossbar | 6112 |
| bendable section | 6116 |
| bending axis | 6120 |
| sleeve | 6124 |
| dots | 6128 |
| cut edge | 6132 |
| double-sided connecting member | 6136 |
| frame | 13350 |
| central portion | 13360 |
| superior bar | 16036 |
| frame | 23350 |
| central portion | 23360 |
| superior bar | 26036 |

Further preferred embodiments are described by the following items:
1. A patient interface comprising:
   a plenum chamber having a cavity pressurisable to a therapeutic pressure of at least 6 cmH₂O above ambient air pressure;
   a first seal-forming structure constructed and arranged to form a seal with a region of the patient's face surrounding an entrance to the patient's mouth, the first seal-forming structure constructed and arranged to maintain said therapeutic pressure in the cavity of the plenum chamber throughout the patient's respiratory cycle in use;
   a second seal-forming structure constructed and arranged to form a seal with a region of the patient's face surrounding an entrance to the patient's nares, the second seal-forming structure constructed and arranged to maintain said therapeutic pressure in the cavity of the plenum chamber throughout the patient's respiratory cycle in use; and
   a vent structure to allow a continuous flow of gases exhaled by the patient from an interior of the plenum chamber to vent to ambient, said vent structure being sized and shaped to maintain the therapeutic pressure in the plenum chamber in use;
   the patient interface further comprising:
      at least one stopper rib disposed in the cavity of the plenum chamber spaced apart from the first seal-forming structure in a rest position, the first seal-forming structure configured to contact the at least one stopper rib in an operational position, the at least one stopper rib configured to oppose compression of the first seal-forming structure in an anterior direction; and
      wherein the second seal-forming structure is not configured to contact the at least one stopper rib.
2. The patient interface of item 1, further comprising a lip superior portion disposed inferior to the second seal-forming structure and configured to contact the patient's lip superior, the lip superior portion not configured to contact the at least one stopper rib.
3. The patient interface of any one of items 1 or 2, wherein the at least one stopper rib includes a plurality of stopper ribs spaced apart from one another within the cavity.
4. The patient interface of item 3, wherein the plurality of stopper ribs are arranged in a c-shape.
5. The patient interface of any one of items 3 to 4, wherein the plurality of stopper ribs are unequally spaced apart from one another within the cavity.
6. The patient interface of any one of items 3 or 5, when dependent from claim 3, wherein the plurality of stopper ribs extend around a majority of a perimeter of the first seal-forming structure.
7. The patient interface of any one of items 1 to 6, wherein the at least one stopper rib is an elongated member.
8. The patient interface of any one of items 1 to 7, wherein the at least one stopper rib includes an inclined edge.
9. The patient interface of item 8, wherein the inclined edge is a posterior edge, and the inclined edge configured to contact the first seal-forming structure in use in order to limit anterior movement of the first seal-forming structure in the operational position.
10. The patient interface of any one of items 8 to 9, wherein the inclined edge is disposed further from the first seal-forming structure toward a center of the plenum chamber in the rest position.
11. The patient interface of any one of items 1 to 10, wherein the at least one stopper rib includes a length measured substantially perpendicularly to the first seal-forming structure and a width measured substantially perpendicularly to the length, and wherein the length is greater than the width.
12. The patient interface of item 11, wherein first seal-forming structure is configured to contact the at least one stopper rib along the width, in the operational position.
13. The patient interface of any one of items 11 to 12, wherein the width is substantially uniform along the length of the at least one stopper rib.
14. The patient interface of any one of items 11 to 12, wherein the width varies along the length of the at least one stopper rib.
15. The patient interface of any one of items 1 to 14, wherein the at least one stopper rib is integrally formed with the plenum chamber.
16. A patient interface comprising:
   a plenum chamber being formed entirely from a flexible material, the plenum chamber including:
      a cavity that is pressurisable to a therapeutic pressure of at least 6 cmH₂O above ambient air pressure,
      an inlet port configured to provide fluid communication between the cavity and ambient, and
      a groove formed on a surface of the plenum chamber outside the cavity and exposed to ambient pressure, the groove formed radially outside of the inlet port, and the groove forming an enclosed perimeter;
   a seal-forming structure constructed and arranged to form a seal with a region of the patient's face surrounding an entrance to the patient's airways, the seal-forming structure constructed and arranged to maintain said therapeutic pressure in the cavity of the plenum chamber throughout the patient's respiratory cycle in use;
   a positioning and stabilizing structure configured to maintain the seal-forming structure in a therapeutically effective position, the positioning and stabilizing structure comprising:
      a frame coupled to the plenum chamber, the frame including,
         a central portion including an inner perimeter and an outer perimeter removably positionable within the groove, the inner perimeter configured to be spaced apart from the inlet port while positioned within the groove,
         a pair of upper arms that extend away from the central portion, and
         a pair of lower arms that extend away from the central portion; and
      headgear straps coupled to the pair of upper arms and the pair of lower arms of the frame, and configured to provide a tensile force to the seal-forming structure into the patient's face via the frame.
17. The patient interface of item 16, wherein the pair of upper arms are longer than the pair of lower arms.
18. The patient interface of any one of items 16 to 17, wherein the pair of upper arms each include a first type of connector and the pair of lower arms each include a second type of connector that is different from the first type of connector.
19. The patient interface of item 18, wherein the first type of connector is selected from a group consisting of a loop, a ladder lock, and a cord stopper.
20. The patient interface of any one of items 18 to 19, wherein the first type of connector is the loop, the loop including a first loop portion and a second loop portion adjacent to the first loop portion.
21. The patient interface of item 20, wherein the loop includes a dividing wall at least partially dividing the first loop portion from the second loop portion.
22. The patient interface of item 21, wherein a strap of the headgear straps is configured to be selectively inserted into the first loop portion and contact the dividing wall, in use while under an optimal tension, and wherein the strap is configured to move into the second loop portion while under tension greater than the optimal tension.
23. The patient interface of any one of items 18 to 22, wherein the second type of connector is a magnetic member.
24. The patient interface of item 23, wherein the magnetic member includes a substantially elliptical shape.
25. The patient interface of any one of claims 23 to 24, wherein a housing is removably and magnetically connected to the magnetic member.
26. The patient interface of item 25, wherein the housing includes a strap connector for connecting to a strap of the headgear straps, the strap connector is selected from a group consisting of a loop, a ladder lock, and a cord stopper.
27. The patient interface of any one of items 25 to 26, wherein the housing is configured to engage a lip of an outer casing.
28. The patient interface of any one of items 18 to 27, wherein at least one strap of the headgear straps includes an elastic portion stretchable between a first position and a second position, and wherein the at least one strap includes an alerting portion configured to be visible in the second position and substantially hidden in the first position.
29. The patient interface of item 28, wherein the at least one strap further includes a tactile response element includes a first portion and a second portion disposed on either side of the alerting portion, and wherein the first portion and the second portion of the tactile response element are connecting in the first position and are configured to separate in the second position in order to alert the patient.
30. The patient interface of item 29, wherein the tactile response element is a magnet.
31. The patient interface of any one of items 18 to 28, wherein at least one strap of the headgear straps includes a first width and a second width that is greater than the first width.
32. The patient interface of item 31, wherein the first width is selectively receivable through the first type of connector, and wherein the second width is substantially blocked from passing through the first type of connector.
33. The patient interface of any one of items 18 to 28, wherein at least one strap of the headgear straps includes a plurality of raised portions configured to provide a tactile response when passing through the first type of connector.
34. The patient interface of item 33, wherein the plurality of raised portions are glue dots.
35. The patient interface of any one of items 18 to 28, wherein at least one strap of the headgear straps includes a cut edge configured to provide a tactile response when passing through the first type of connector.
36. The patient interface of item 35, wherein the cut edge includes a triangular shape, a rectangular shape, and/or a round shape.
37. The patient interface of any one of items 16 to 32, wherein the pair of upper arms and the pair of lower arms are integrally formed with the central portion.
38. The patient interface of any one of items 16 to 37, wherein the plenum chamber includes at least one projection that extends from the groove, and wherein the central portion of the frame includes at least one slot configured to selectably receive the at least one projection.
39. The patient interface of any one of items 16 to 38, wherein the pair of lower arms are substantially flush with the central portion.
40. The patient interface of any one of items 16 to 39, wherein the seal-forming structure further includes:
   a first seal-forming structure constructed and arranged to form a seal with a region of the patient's face surrounding an entrance to the patient's mouth;
   a second seal-forming structure constructed and arranged to form a seal with a region of the patient's face surrounding an entrance to the patient's nares;
   wherein the groove is disposed on the plenum chamber inferior to the second seal-forming structure and opposite to the first seal-forming structure.
41. The patient interface of any one of items 16 to 40, wherein a textile sleeve is connected to each upper arm of the pair of upper arms.
42. A system for providing pressurized air to a patient, the system comprising:
   the patient interface of any one of items 16 to 41;
   a respiratory therapy system (RPT) device configured to provide a flow of air at the therapeutic pressure; and
   a conduit connecting the RPT device to the patient interface, the conduit configured to convey the flow of air to the patient interface;
   wherein the conduit is connected to the inlet port of the plenum chamber.
43. A system for providing pressurized air to a patient, the system comprising:
   a plenum chamber including a cavity that is pressurisable to a therapeutic pressure of at least 6 cmH2O above ambient air pressure, said plenum chamber including a plenum chamber inlet port sized and structured to receive a flow of air at the therapeutic pressure for breathing by a patient, wherein the plenum chamber includes a lip extending at least partially around the plenum chamber inlet port and into the cavity;
   a seal-forming structure constructed and arranged to form a seal with a region of the patient's face surrounding an entrance to the patient's airways such that the flow of air at said therapeutic pressure is delivered to the airways, the seal-forming structure constructed and arranged to maintain said therapeutic pressure in the plenum chamber throughout the patient's respiratory cycle in use;
   a positioning and stabilizing structure configured to maintain the seal-forming structure in a therapeutically effective position; and
   an air circuit removably connected to the plenum chamber and configured to provide the flow of air at the therapeutic pressure from a flow generator to the cavity, the air circuit including a vent ring configured to be inserted at least partially through the plenum chamber inlet port, the vent ring comprising:
      a posterior surface having a first diameter,
      an anterior surface having a second diameter less than the first diameter, and
      a ring body extending between the anterior surface and the posterior surface, ring body includes an inner wall, an outer wall, and a groove disposed between the inner wall and the outer wall;
   wherein:
      the posterior surface is configured to be positioned within the cavity and the anterior surface is configured to remain outside the cavity when connected to the plenum chamber;
      the posterior surface includes at least one vent opening configured to provide communication between the cavity and an ambient environment through the groove;
      the lip is configured to contact the vent ring along the ring body between the posterior surface and the anterior surface; and
      the first diameter is greater than a diameter of the lip.
44. The system of item 43, wherein the ring body is inclined between the posterior surface and the anterior surface.
45. The system of any one of items 43 to 44, wherein the posterior surface is configured to contact the lip and an anterior wall of the plenum chamber within the cavity, in use.
46. The system of item 43, further comprising a ring seal positioned within the groove.
47. The system of item 46, wherein an outer diameter of the ring seal is less than an outer diameter of the groove.
48. The system of any one of items 43 to 47, wherein the ring body includes a central opening and the system further comprises an elbow connector, the central opening configured to receive the elbow connector.
49. The system of item 48, wherein the elbow connector is rotatably connected to the ring body and wherein the ring body is rotatably connected to the plenum chamber.
50. The system of any one of items 48 to 49, wherein the elbow connector includes at least one button configured to be selectively actuated in order to disengage the elbow connector from the plenum chamber inlet port.
51. The system of item 50, wherein the at least one button is formed as a cantilever structure.
52. The system of item 51, wherein an arm connects the at least one button to a body of the elbow connector, the arm having an S shape.
53. The system of any one of items 50 to 52, wherein the at least one button includes a button lip configured to selectively engage the ring body and move relative to a remainder of the elbow connector when the at least one button is actuated.
54. The system of item 53, wherein the button lip is configured to selectively engage an inner perimeter of the inner wall of the ring body.
55. The system of any one of items 50 to 51, wherein the at least one button includes a finger configured to selectively engage the ring body and move relative to a remainder of the elbow connector when the at least one button is actuated.
56. The system of item 55, wherein the finger is configured to selectively engage the ring body radially outside the inner wall.
57. The system of any one of items 43 to 56, wherein the at least one vent opening is a plurality of vent openings spaced around a perimeter of the groove.
58. The system of any one of items 43 to 57, wherein the plenum chamber inlet port includes an extension and the ring body is connected to the extension and spaced apart from a surface of the plenum chamber.
59. The system of any one of items 43 to 58, further comprising a quick-release connector.
60. The system of item 59, wherein the quick-release connector is a rotatable connector or a latch connector.
61. The system of any one of items 59 to 60, wherein the quick-release connector is configured to connect to a complementary connector.
62. The system of any one of items 59 to 61, wherein the quick-release connector includes at least one flat surface.
63. The system of item 62, wherein the at least one flat surface is a pair of flat surface spaced approximately 180° apart.
64. The system of any one of items 54 to 55, when dependant on claim 57, wherein the at least one flat surface is configured to redistribute leak of the flow of air from the system.
65. The system of any one of items 59 to 64, wherein the quick-release connector includes vent openings configured to exhaust a portion of the flow of air to ambient.
66. The system of any one of items 43 to 65, wherein the seal-forming structure is spaced apart from the vent ring, and wherein a patient's face is not configured to contact the vent ring.
67. The system of any one of items 43 to 66, wherein the ring body includes an extension.
68. The system of any one of items 43 to 67, wherein the ring body includes disk configured to be positioned within the cavity and limit exhaled air from venting to ambient.
69. The system of item 68, wherein the disk extends radially beyond a portion of the posterior surface including the at least one vent opening.

## Claims

1. A patient interface comprising:
a plenum chamber being formed entirely from a flexible material, the plenum chamber including:
a cavity that is pressurisable to a therapeutic pressure of at least 6 cmH₂O above ambient air pressure,
an inlet port configured to provide fluid communication between the cavity and ambient, and
a groove formed on a surface of the plenum chamber outside the cavity and exposed to ambient pressure, the groove formed radially outside of the inlet port, and the groove forming an enclosed perimeter;
a seal-forming structure constructed and arranged to form a seal with a region of the patient's face surrounding an entrance to the patient's airways, the seal-forming structure constructed and arranged to maintain said therapeutic pressure in the cavity of the plenum chamber throughout the patient's respiratory cycle in use;
a positioning and stabilizing structure configured to maintain the seal-forming structure in a therapeutically effective position, the positioning and stabilizing structure comprising:
a frame coupled to the plenum chamber, the frame including,
a central portion including an inner perimeter and an outer perimeter removably positionable within the groove, the inner perimeter configured to be spaced apart from the inlet port while positioned within the groove,
a pair of upper arms that extend away from the central portion in a posterior direction, the pair of upper arms being configured to, in use, extend over the patient's cheeks, and
a pair of lower arms that extend away from the central portion; and
headgear straps coupled to the pair of upper arms and the pair of lower arms of the frame and configured to provide a tensile force to the seal-forming structure into the patient's face via the frame.

2. The patient interface of claim 2, wherein the upper arms are longer than the lower arms.

3. The patient interface of any one of claims 1 and 2, wherein the pair of upper arms each include a first type of connector and the pair of lower arms each include a second type of connector that is different from the first type of connector.

4. The patient interface of claim 3, wherein the first type of connector is selected from a group consisting of a loop, a ladder lock, and a cord stopper.

5. The patient interface of any one of claims 3 and 4, wherein the second type of connector is a magnetic member.

6. The patient interface of claim 5, wherein a housing is removably and magnetically connected to the magnetic member.

7. The patient interface of claim 6, wherein the housing includes a strap connector for connecting to a strap of the headgear straps, the strap connector is selected from a group consisting of a loop, a ladder lock, and a cord stopper.

8. The patient interface of any one of claims 1 to 7, wherein at least one strap of the headgear straps includes a first width and a second width that is greater than the first width.

9. The patient interface of claim 8, wherein the first width is selectively receivable through the first type of connector, and wherein the second width is substantially blocked from passing through the first type of connector.

10. The patient interface of any one of claims 1 to 9, wherein the pair of upper arms and the pair of lower arms are integrally formed with the central portion.

11. The patient interface of any one of claims 1 to 10, wherein the plenum chamber includes at least one projection that extends from the groove, and wherein the central portion of the frame includes at least one slot configured to selectably receive the at least one projection.

12. The patient interface of any one of claims 1 to 11, wherein the pair of lower arms is substantially flush with the central portion.

13. The patient interface of any one of claims 1 to 12, wherein the seal-forming structure further includes:
a first seal-forming structure constructed and arranged to form a seal with a region of the patient's face surrounding an entrance to the patient's mouth;
a second seal-forming structure constructed and arranged to form a seal with a region of the patient's face surrounding an entrance to the patient's nares;
wherein the groove is disposed on the plenum chamber inferior to the second seal-forming structure and opposite to the first seal-forming structure.

14. The patient interface of any one of claims 1 to 13, wherein a textile sleeve is connected to each upper arm of the pair of upper arms.

15. A system for providing pressurized air to a patient, the system comprising:
the patient interface of any one of claims 1 to 14;
a respiratory therapy system (RPT) device configured to provide a flow of air at the therapeutic pressure; and
a conduit connecting the RPT device to the patient interface, the conduit configured to convey the flow of air to the patient interface;
wherein the conduit is connected to the inlet port of the plenum chamber.
